(19) Europäisches Patentamt
European Patent Office
Office européen des brevets

(11) EP 4 241 789 A2

(12) **EUROPEAN PATENT APPLICATION**
published in accordance with Art. 153(4) EPC

(43) Date of publication:
**13.09.2023 Bulletin 2023/37**

(21) Application number: **21731365.9**

(22) Date of filing: **25.03.2021**

(51) International Patent Classification (IPC):
***A61K 47/68*** *(2017.01)* ***A61K 47/65*** *(2017.01)*
***A61P 35/00*** *(2006.01)*

(52) Cooperative Patent Classification (CPC):
**A61K 47/6889; A61K 47/6803; A61K 47/6851**

(86) International application number:
**PCT/CN2021/083024**

(87) International publication number:
**WO 2021/115497 (17.06.2021 Gazette 2021/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.12.2020 CN 202011423832**

(71) Applicant: **Harbour Biomed (Shanghai) Co., Ltd**
**Shanghai 201203 (CN)**

(72) Inventors:
- **HE, Yun**
**Shanghai 201203 (CN)**
- **SHI, Lei**
**Shanghai 201203 (CN)**
- **CAO, Yang**
**Shanghai 201203 (CN)**
- **WANG, Ming**
**Shanghai 201203 (CN)**
- **ZHANG, Yun**
**Shanghai 201203 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

# (54) PROTEIN-DRUG CONJUGATE AND SITE-SPECIFIC CONJUGATING METHOD

(57) The present application relates to a protein-drug conjugate, containing an antigen binding protein portion and a drug conjugate portion. The antigen binding protein portion comprises one or more antigen binding fragments, and at least two linker peptides are directly or indirectly connected to the antigen binding fragments. The two linker peptides can each independently contain a first cysteine Cys1 and a second cysteine Cys2. The drug conjugate portion is covalently bonded on Cys1. The present application further relates to a preparation method for the protein-drug conjugate, and a use for the protein-drug conjugate in preventing or treating tumors or other diseases.

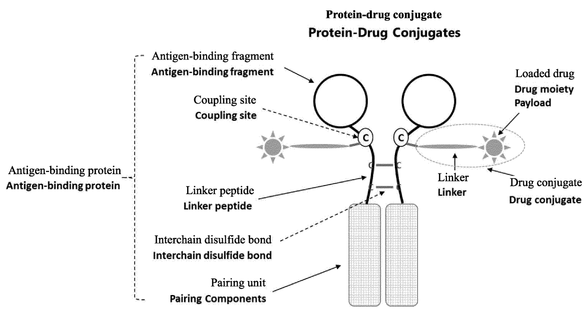


FIG. 41

EP 4 241 789 A2

## Description

The present application claims priority to Chinese Patent Application No. 202011423832.6 filed on December 8, 2020.

## TECHNICAL FIELD

The present invention generally relates to the field of biomedicine, and in particular to a protein-drug conjugate and a method for preparing the same.

## BACKGROUND

Antibody-drug conjugate (ADC) is produced by the coupling of an antibody targeting a tumor antigen with highly-cytotoxic small molecule chemical drug through a linker. By utilizing the characteristic of specific binding between the antibody and a target antigen, small molecule drugs are targeted and delivered to tumor cells, thereby exerting a killing effect on tumors. In recent years, with the continuous development of new linkers, small molecule toxins, antibodies, and target discovery technologies, the early problems of ADC have been continuously overcome, and ADC drugs have been launched one after another and demonstrate good clinical effects on hematologic tumors and solid tumors. On the other hand, in recent years, ADC technology has also been applied in non-tumor fields; for example, the AbbVie company conjugates steroids to anti-TNF$\alpha$ antibodies for the treatment of multiple TNF$\alpha$-mediated autoimmune diseases. Coupling methods and linker structures are crucial for the stability of ADC drugs. The cysteine coupling technology is a representative of early chemical coupling methods; it utilizes four natural interchain disulfide bonds of IgG1 antibodies as active sulfhydryls for coupling. Compared to the lysine amide coupling technology, this technology has a controllable drug/antibody ratio (DAR), but still produces a mixture with a DAR distributed from 0 to 8. Due to the uneven components of the coupling products produced by such technologies, their stability and pharmaceutical properties have significant shortcomings. In recent years, with the continuous development of cytotoxic drugs and site-specific conjugation methods, the third-generation ADC technology has improved the stability and pharmacokinetic performance of antibody-drug conjugates by obtaining stable DAR through site-specific conjugation between drugs and antibodies. The representative coupling technologies for the third-generation ADC mainly include ThioMab, ThioBridge, introduction of unnatural amino acids, transpeptidaton, N-saccharide chain coupling, etc. ThioMab technology was first developed by the Genentech company. Based on cysteine coupling technology, two or more cysteines are introduced as coupling sites at specific sites in antibodies, except for natural disulfide bonds, to produce site-specific and highly-homogeneous antibody-drug conjugate with a DAR of 2; however, due to the fact that the mutations introduced are not naturally occurring amino acid sequences, there is a potential risk of molecular stability and immunogenicity. ThioBridge technology is to reduce the disulfide bond of the monoclonal antibody itself. Specifically, by using dibromomaleinimide or dibromopyridine diphenyl ether and other reagents to react with a reduced interchain cysteine, the monoclonal antibody can be re-bridged, and a product with a main component with a DAR of 4 is obtained, but this technology still has the risk of disulfide bond mismatch. Unnatural amino acid coupling technology is to use a special tRNA synthetase encoding unnatural amino acids, which enables cells to synthesize various antibodies with *p*-acetylphenylalanine residues, and by utilizing the coupling reaction between the carbonyls of *p*-acetylphenylalanine residues and the linker with alkoxylamine groups, a product with a main component with a DAR of 2 is obtained; due to the need for the use of modified host cells to produce unnatural amino acids, the obvious drawbacks of this technology are the low yield and high cost of antibody production. The coupling technology using transpeptidaton is to introduce a sequence of LPETG at the C' terminus of the heavy or light chain of the antibody, a transpeptidase Sortase A specifically recognizes this sequence and hydrolyzes a peptide bond between threonine and glycine, and then, a conjugate comprising glycine is connected to threonine; however, due to additional peptide sequences are introduced in a coupling product generated by this technology, there is a potential risk of immunogenicity. N-saccharide chain coupling technology, such as GlycoConnect technology, is to use endoglycosidase Endo S2 to trim a saccharide chain of Asn297 (Eu numbering) located in an antibody CH2 region, then use the mutant galactosyl transferase GalT (Y289L) and N-azidoacetyl galactosamine (GalNAz) to introduce azido, and couple the azido with a small molecule drug by using click chemistry to produce stable and uniform antibody-drug conjugate with a DAR of 2; due to the need for the use of special mutant enzymes, the process development cost of this technology is relatively high.

At present, although various coupling technologies that can generate controllable DAR through site-specific conjugation have been developed, these technologies, including either the introduction of amino acid mutations, or the introduction of additional amino acid sequences, or the change of saccharide chain structures on Fc, or the introduction of unnatural amino acids, have complex process development and high cost. Moreover, there is no common coupling technology for preparing a protein-drug conjugate for heavy-chain antibodies, single-chain antibodies, bispecific antibodies, or fusion proteins in the prior art. Therefore, there is still an urgent need to develop new site-specific coupling technologies that do not require complex preparation processes to improve the stability of coupling products and reduce immunogenicity.

## SUMMARY

The present application provides a site-specific conjugation method for a protein-drug conjugate and a protein-drug conjugate prepared by the method. The present application has one or more of the following properties: 1) a coupling product with a uniform drug/antibody ratio (DAR) or a uniform conjugate/antibody ratio (CAR) can be produced through site-specific conjugation; 2) compared to other site-specific coupling technologies such as THIOMAB technology, natural hinge region amino acid sequences can be utilized without introducing amino acid mutations or additional peptide sequences and changing saccharide chain structures on Fc; 3) the length or site-specific amino acid types of the natural hinge region amino acid sequences can be optimized to improve the expression quantity and uniformity of a product; 4) there is no need for particularly complex coupling technologies or processes; 5) the site-specific conjugation method has fewer byproducts and strong developability; 6) antigen-binding fragments can be extended to various forms such as fully human VH domain, camelidae VHH domain, single-chain antibody scFv and soluble receptor fusion protein; 7) antigen-binding fragments can be extended into multivalent and multispecific forms; 8) good stability and half-life are achieved; 9) a better target cell internalization effect is achieved; and 10) better tissue penetration is achieved.

In one aspect, the present application provides a protein-drug conjugate, comprising an antigen-binding protein moiety and a drug conjugate moiety, the antigen-binding protein moiety comprising one or more antigen-binding fragments, and at least two linker peptides directly or indirectly connected with the antigen-binding fragment; the at least two linker peptides comprising a first linker peptide and a second linker peptide, wherein the first linker peptide comprises a first cysteine Cys1 and a second cysteine Cys2, and the drug conjugate moiety is coupled to the first linker peptide through the Cys1; and the second linker peptide comprises a first cysteine Cys1 and a second cysteine Cys2, and the drug conjugate moiety is coupled to the second linker peptide through the Cys1.

In certain embodiments, the Cys2 of the first linker peptide is connected to the Cys2 of the second linker peptide through a disulfide bond.

In certain embodiments, the one or more antigen-binding fragments do not comprise a functional group capable of affecting coupling of a drug conjugate with the first linker peptide and/or the second linker peptide at the Cys1, or a conjugate formed by the functional group and other drug conjugates, and the affection is manifested in significantly reducing a coupling probability of the drug conjugate at a Cys1 site.

In certain embodiments, the one or more antigen-binding fragments do not comprise a functional group capable of forming a disulfide bond with the Cys1 of the first linker peptide and/or the Cys1 of the second linker peptide, or a conjugate formed by the functional group and other drug conjugates.

In certain embodiments, the one or more antigen-binding fragments do not comprise a cysteine capable of forming a disulfide bond with the Cys1 of the first linker peptide and/or the Cys1 of the second linker peptide, or a conjugate formed by the cysteine and other drug conjugates.

In certain embodiments, amino acid sequences of the first linker peptide and the second linker peptide can be identical or different.

In certain embodiments, the first linker peptide and/or the second linker peptide comprise/comprises an amino acid sequence set forth in Cys1-(X)n-Cys2 from an N-terminus to a C-terminus, wherein n is an integer greater than or equal to 3, and X is any amino acid other than cysteine.

In certain embodiments, the first linker peptide and/or the second linker peptide are/is derived from an antibody hinge region sequence or a derivative sequence thereof. In particular, the derivative sequence is obtained by modifying the antibody hinge region sequence, and the modification does not involve changes in Cys1 and Cys2 in the antibody hinge region. For example, the modification is adjusting a type and number of amino acids between Cys1 and Cys2 in the antibody hinge region sequence, and/or adding flexible linker amino acids before Cys1. In certain embodiments, the linker peptide is derived from a human IgG hinge region. For example, the antibody hinge region is a human IgG1 hinge region.

In certain embodiments, the linker peptide is the human IgG1 hinge region, sequences of the first linker peptide and the second linker peptide are each independently set forth in SEQ ID NO: 73, the Cys1 in the linker peptides is Cys-220 according to EU numbering in the human IgG1 hinge region, and the Cys2 is Cys-226 according to EU numbering in the human IgG1 hinge region.

In certain embodiments, the first linker peptide and the second linker peptide can be each independently derived from a derivative sequence of the human IgG1 hinge region, and an amino acid sequence thereof is set forth in any one of SEQ ID NOs: 74-105 and 145-146; the Cys1 in the first linker peptide is Cys corresponding to Cys-220 in the human IgG1 hinge region, and the Cys2 in the first linker peptide is Cys corresponding to Cys-226 in the human IgG1 hinge region; the Cys1 in the second linker peptide is Cys corresponding to Cys-220 in the human IgG1 hinge region, and the Cys2 in the second linker peptide is Cys corresponding to Cys-226 in the human IgG1 hinge region.

In certain embodiments, the linker peptide is a mouse IgG2c hinge region, sequences of the first linker peptide and the second linker peptide are each independently set forth in SEQ ID NO: 147, the Cys1 in the linker peptides is a first cysteine in a sequence of the mouse IgG2c hinge region, and the Cys2 is a second cysteine in a sequence thereof.

In certain embodiments, the plurality of antigen-binding fragments comprised by the antigen-binding protein moiety of the protein-drug conjugate are connected to the same linker peptide or respectively connected to the different linker peptides.

In certain embodiments, the plurality of antigen-binding fragments can be partially or completely identical or different from each other.

In certain embodiments, the plurality of antigen-binding fragments comprise a first antigen-binding fragment and a second antigen-binding fragment. The first antigen-binding fragment and the second antigen-binding fragment can be identical or different. For example, the first antigen-binding fragment and the second antigen-binding fragment bind to the same target and have the same amino acid sequence; for example, the first antigen-binding fragment and the second antigen-binding fragment bind to the same target but have different amino acid sequences; for example, the first antigen-binding fragment and the second antigen-binding fragment bind to different targets.

In certain embodiments, the plurality of antigen-binding fragments comprise a first antigen-binding fragment, a second antigen-binding fragment, a third antigen-binding fragment and a fourth antigen-binding fragment. The first antigen-binding fragment, the second antigen-binding fragment, the third antigen-binding fragment and the fourth antigen-binding fragment can be partially or completely identical or different from each other. For example, the first antigen-binding fragment and the second antigen-binding fragment bind to the same target and have the same amino acid sequence; for example, the first antigen-binding fragment and the second antigen-binding fragment bind to the same target but have different amino acid sequences; for example, the first antigen-binding fragment and the second antigen-binding fragment bind to different targets. For example, the third antigen-binding fragment and the fourth antigen-binding fragment bind to the same target and have the same amino acid sequence; for example, the third antigen-binding fragment and the fourth antigen-binding fragment bind to the same target but have different amino acid sequences; for example, the third antigen-binding fragment and the fourth antigen-binding fragment bind to different targets. For example, the first antigen-binding fragment, the second antigen-binding fragment, the third antigen-binding fragment and the fourth antigen-binding fragment bind to different targets.

In certain embodiments, the one or more antigen-binding fragments are capable of each independently being derived protein structures such as a VHH domain, a VH, a VL, an Fab, an ScFv, a receptor protein soluble extracellular region, a ligandin, a lipocalin, a neuronal cell adhesion molecule NCAM, a fibronectin and/or a designed ankyrin repeat protein DARPin.

In certain embodiments, the one or more antigen-binding fragments are capable of specifically binding to a tumor antigen or a non-tumor antigen.

In certain embodiments, the tumor antigen comprises CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG (TMPRSS2 ETS fusion gene), NA17, PAX3, androgen receptor (AR), Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, Cadherin17, CD32b, EGFRvIII, GPNMB, GPR64, HER3, LRP6, LYPD8, NKG2D, SLC34A2, SLC39A6, SLITRK6, GUCY2C, 5T4 and/or TACSTD2, etc.

In certain embodiments, the first antigen-binding fragment and/or the second antigen-binding fragment each independently comprise HCDR1, HCDR2, and HCDR3, and the antigen-binding fragments comprise amino acid sequences selected from any one of (1) HCDR1: SEQ ID NO: 7, HCDR2: SEQ ID NO: 22, HCDR3: SEQ ID NO: 38; (2) HCDR1: SEQ ID NO: 10, HCDR2: SEQ ID NO: 25, HCDR3: SEQ ID NO: 41; (3) HCDR1: SEQ ID NO: 11, HCDR2: SEQ ID NO: 26, HCDR3: SEQ ID NO: 42; (4) HCDR1: SEQ ID NO: 13, HCDR2: SEQ ID NO: 28, HCDR3: SEQ ID NO: 44; (5) HCDR1: SEQ ID NO: 14, HCDR2: SEQ ID NO: 29, HCDR3: SEQ ID NO: 42; (6) HCDR1: SEQ ID NO: 9, HCDR2: SEQ ID NO: 24, HCDR3: SEQ ID NO: 40; and (7) HCDR1: SEQ ID NO: 8, HCDR2: SEQ ID NO: 23, HCDR3: SEQ ID NO: 39.

In certain embodiments, the antigen-binding fragment comprises a VH, and the VH comprises an amino acid sequence set forth in any one of SEQ ID NOs: 55-59, 61 and 62.

In certain embodiments, the first antigen-binding fragment and/or the second antigen-binding fragment each independently comprise HCDR1, HCDR2, HCDR3, LCDR1, LCDR2 and LCDR3, and the HCDR1, HCDR2, HCDR3, LCDR1, LCDR2 and LCDR3 respectively and sequentially comprise amino acid sequences set forth in SEQ ID NO: 12, SEQ ID NO: 27, SEQ ID NO: 43, SEQ ID NO: 49, SEQ ID NO: 51 and SEQ ID NO: 53.

In certain embodiments, the first antigen-binding fragment and/or the second antigen-binding fragment each independently comprise a VH and a VL, and the VL comprises an amino acid sequence set forth in SEQ ID NO: 63, and the VH comprises an amino acid sequence set forth in SEQ ID NO: 60.

In certain embodiments, the first antigen-binding fragment and/or the second antigen-binding fragment each independently comprise one pair of VH and VL and/or another type of VH, the pair of VH and VL respectively comprises amino acid sequences set forth in SEQ ID NO: 60 and SEQ ID NO: 63, and the another type of VH comprises an amino acid sequence set forth in SEQ ID NO: 62. For example, the pair of VH and VL can form scFv.

In certain embodiments, the first antigen-binding fragment and/or the second antigen-binding fragment each independently comprise a VH, and the VH comprises an amino acid sequence set forth in SEQ ID NO: 62; and the third antigen-binding fragment and/or the fourth antigen-binding fragment each independently comprise one pair of VH and VL, and the pair of VH and VL respectively comprises amino acid sequences set forth in SEQ ID NO: 130 and SEQ ID NO: 131. For example, the third antigen-binding fragment and/or the fourth antigen-binding fragment can comprise an Fab.

In certain embodiments, the antigen-binding protein moiety in the protein-drug conjugate can further comprise a pairing unit moiety enabling the first linker peptide and the second linker peptide to be paired, the pairing unit can comprise at least a first pairing subunit and a second pairing subunit, and the first pairing subunit can interact with the second pairing subunit through a covalent bond or a non-covalent bond.

In certain embodiments, the pairing unit is an Fc fragment or a mutant Fc fragment.

In certain embodiments, an N-terminus of the first pairing subunit is connected to a C-terminus of the first linker peptide, and an N-terminus of the second pairing subunit is connected to a C-terminus of the second linker peptide; or the N-terminus of the first pairing subunit is connected to the C-terminus of the second linker peptide, and the N-terminus of the second pairing subunit is connected to the C-terminus of the first linker peptide.

In certain embodiments, the protein-drug conjugate comprises an amino acid sequence set forth in any one of SEQ ID NOs: 64-71, 106-129 and 148-149.

In certain embodiments, the drug conjugate moiety of the protein-drug conjugate comprises two drug conjugates respectively connected to the first linker peptide and the second linker peptide, and the drug conjugate comprises a loaded drug and optionally a linker.

In certain embodiments, the drug conjugate in the protein-drug conjugate comprises at least one loaded drug. The loaded drug can comprise, but is not limited to, a small molecule compound, a toxin molecule, an antibiotic, an oligonucleotide, a proteolysis targeting chimera (PROTAC), an affinity ligand, a fluorescently or nuclide-labeled group, a polypeptide, an immunomodulator, etc.

In certain embodiments, the linker comprises a cleavable linker or a non-cleavable linker, wherein the cleavable linker comprises a protease cuttable linker.

In certain embodiments, the drug conjugate comprises mc-vc-PAB-MMAE (as shown in 0).

In certain embodiments, the drug conjugate comprises PROTAC, such as a BRD4 protein degrading agent (as shown in 0).

In certain embodiments, the protein-drug conjugate is an antibody-drug conjugate (ADC).

In another aspect, the present application provides a method for preparing the protein-drug conjugate of the present application, comprising covalently binding the drug conjugate with a reactive sulfhydryl of the Cys1 on the first linker peptide and/or the second linker peptide.

In another aspect, the present application provides a method for preparing a protein-drug conjugate. The protein-drug conjugate comprises an antigen-binding protein moiety and a drug conjugate moiety; the antigen-binding protein moiety comprises one or more antigen-binding fragments, and at least two linker peptides directly or indirectly connected with the antigen-binding fragment; the two linker peptides comprise a first linker peptide and a second linker peptide, wherein the first linker peptide comprises a first cysteine Cys1 and a second cysteine Cys2, and the second linker peptide comprises a first cysteine Cys 1 and a second cysteine Cys2; and the method comprises binding the drug conjugate moiety with the first linker peptide through the Cys1 of the first linker peptide, and/or binding the drug conjugate moiety with the second linker peptide through the Cys1 of the second linker peptide.

In certain embodiments, the one or more antigen-binding fragments do not comprise a functional group capable of affecting coupling of a drug conjugate with the first linker peptide and/or the second linker peptide at the Cys1, or a conjugate formed by the functional group and other drug conjugates, and the affection is manifested in significantly reducing a coupling probability of the drug conjugate at a Cys1 site.

In certain embodiments, the one or more antigen-binding fragments in the method do not comprise a functional group capable of forming a disulfide bond with the Cys1 of the first linker peptide and/or the Cys1 of the second linker peptide, or a conjugate formed by the functional group and other drug conjugates.

In certain embodiments, the one or more antigen-binding fragments do not comprise a cysteine capable of forming a disulfide bond with the Cys1 of the first linker peptide and/or the Cys1 of the second linker peptide, or a conjugate formed by the cysteine and other drug conjugates.

In certain embodiments, amino acid sequences of the first linker peptide and the second linker peptide in the method can be identical or different.

In certain embodiments, the first linker peptide and/or the second linker peptide in the method comprise/comprises an amino acid sequence set forth in Cysl-(X)n-Cys2 from an N-terminus to a C-terminus, wherein n is an integer greater than or equal to 3, and X is any amino acid other than cysteine.

In certain embodiments, the first linker peptide and/or the second linker peptide are/is derived from an antibody hinge region sequence or a derivative sequence thereof.

In certain embodiments, the derivative sequence in the method is obtained by modifying the antibody hinge region sequence, and the modification does not involve changes in Cys1 and Cys2 in the antibody hinge region. In certain embodiments, the modification is adjusting a type number of amino acids between Cys1 and Cys2 in the antibody hinge region sequence, and/or adding flexible linker amino acids before Cys1.

In certain embodiments, the linker peptide in the method is derived from a human IgG hinge region, preferably a human IgG1 hinge region.

In certain embodiments, the first linker peptide in the method is the human IgG1 hinge region, a sequence of the first linker peptide is set forth in SEQ ID NO: 73, the Cys1 in the first linker peptide is Cys-220 according to EU numbering in the human IgG1 hinge region, and the Cys2 is Cys-226 according to EU numbering in the human IgG1 hinge region.

In certain embodiments, the second linker peptide in the method is the human IgG1 hinge region, a sequence of the second linker peptide is set forth in SEQ ID NO: 73, the Cys1 in the second linker peptide is Cys-220 according to EU numbering in the human IgG1 hinge region, and the Cys2 is Cys-226 according to EU numbering in the human IgG1 hinge region.

In certain embodiments, the first linker peptide in the method can be derived from a derivative sequence of the human IgG1 hinge region, an amino acid sequence thereof is a sequence as set forth in any one of SEQ ID NOs: 74-105 and 145-146, the Cys1 in the first linker peptide is Cys corresponding to Cys-220 in the human IgG1 hinge region, and the Cys2 in the linker peptide is Cys corresponding to Cys-226 in the human IgG1 hinge region.

In certain embodiments, the second linker peptide in the method can be derived from a derivative sequence of the human IgG1 hinge region, an amino acid sequence thereof is a sequence as set forth in any one of SEQ ID NOs: 74-105 and 145-146, the Cys1 in the second linker peptide is Cys corresponding to Cys-220 in the human IgG1 hinge region, and the Cys2 in the linker peptide is Cys corresponding to Cys-226 in the human IgG1 hinge region.

In certain embodiments, the first linker peptide in the method is a mouse IgG2c hinge region, the amino acid sequence thereof is set forth in SEQ ID NO: 147, the Cys1 in the first linker peptide is a first cysteine in the sequence thereof, and the Cys2 is a second cysteine in the sequence thereof.

In certain embodiments, the second linker peptide in the method is a mouse IgG2c hinge region, the amino acid sequence thereof is set forth in SEQ ID NO: 147, the Cys1 in the second linker peptide is a first cysteine in the sequence thereof, and the Cys2 is a second cysteine in the sequence thereof.

In certain embodiments, the method comprises steps of reduction, coupling, and optionally purification.

In certain embodiments, the reduction comprises reducing a disulfide bond between the Cys1 of the first linker peptide and the Cys1 of the second linker peptide under a condition of a reducing agent.

In certain embodiments, the reduction comprises using DTT and/or TCEP as a reducing agent; in certain embodiments, the reducing agent is TCEP.

In certain embodiments, an amount of usage of the reducing agent is 1 to 50 molar multiples; in certain embodiments, the amount of usage of the reducing agent is 1 to 6 molar multiples; in certain embodiments, the amount of usage of the reducing agent is 1.5 to 3 molar multiples.

In certain embodiments, reaction time for the reduction is 1 to 17 hours; in certain embodiments, the reaction time for the reduction is 1 to 3 hours; in certain embodiments, the reaction time for the reduction is 1.5 to 2 hours.

In certain embodiments, a reaction temperature for the reduction is 0 °C to 40 °C; in certain embodiments, the reaction temperature for the reduction is 0 °C or room temperature (25 °C to 30 °C) or 37 °C.

In certain embodiments, a pH of a reaction buffer for the reduction is 4.0 to 9.0; in certain embodiments, the pH of the reaction buffer for the reduction is 5.0 to 8.0; in certain embodiments, the pH is 5.0 to 7.0; in certain embodiments, the pH is 5.0 to 6.0.

In certain embodiments, the coupling comprises covalently binding the drug conjugate with a reactive sulfhydryl of the Cys1 on the first linker peptide and/or the second linker peptide.

In certain embodiments, the drug conjugate comprises mc-vc-PAB-MMAE or PROTAC or a CpG oligonucleotide.

In certain embodiments, an amount of usage of the drug conjugate is 1 to 50 molar multiples; in certain embodiments, the amount of usage of the drug conjugate is 1 to 10 molar multiples; in certain embodiments, the amount of usage of the drug conjugate is 3 to 7 molar multiples.

In certain embodiments, the drug conjugate is mc-vc-PAB-MMAE, and an amount of usage of the mc-vc-PAB-MMAE is 1 to 50 molar multiples; in certain embodiments, the amount of usage of the mc-vc-PAB-MMAE is 3 to 18 molar multiples; in certain embodiments, the amount of usage of the mc-vc-PAB-MMAE is 3 to 7 molar multiples.

In certain embodiments, an amount of usage of the drug conjugate PROTAC is 10 to 15 molar multiples.

In certain embodiments, reaction time for the coupling is 0.5 to 10 hours; in certain embodiments, the reaction time for the coupling is 0.5 to 3 hours; in certain embodiments, the reaction time for the coupling is 0.5 to 1 hours.

In certain embodiments, a reaction temperature for the coupling is 0 °C to 40 °C; in certain embodiments, the reaction temperature for the coupling is room temperature (25 °C to 30 °C). In certain embodiments, pH of a reaction buffer for the coupling is 4.0 to 9.0; in certain embodiments, the pH of the reaction buffer for the coupling is 5.0 to 8.0; in certain embodiments, the pH is 5.0 to 7.0; in certain embodiments, the pH is 7.0 to 8.0; in certain embodiments, the pH is 5.0 to 6.0.

In certain embodiments, a purity of the protein-drug conjugate is greater than 80%. For example, the purity of the protein-drug conjugate is greater than 82%, greater than 85%, greater than 88% or greater than 90%.

In certain embodiments, the preparation method can provide a combination of reaction steps including "reduction" and "coupling" and reaction condition parameters; the combination of the reaction steps and the reaction condition parameters can further increase the content of a single component in the coupling product, reducing the need for an additional "purification" step; in certain embodiments, after the step of coupling, a highly-homogeneous coupling product with a purity greater than 90% can be obtained without an additional purification step; furthermore, in certain embodiments, the highly-homogeneous coupling product is a protein-drug conjugate with CAR = 2 site-specifically coupled to Cys1.

In another aspect, the present application provides a pharmaceutical composition comprising the protein-drug conjugate and a pharmaceutically acceptable carrier.

In another aspect, the present application provides a method for preventing and/or treating diseases, comprising administering the protein-drug conjugate and/or the pharmaceutical composition, wherein the protein-drug conjugate and/or the pharmaceutical composition are optionally in combination with other therapies or drugs.

In another aspect, the present application provides use of the protein-drug conjugate and/or the pharmaceutical composition in preparation of a drug, wherein the drug is used for treating tumors or other diseases.

In another aspect, the present application provides use of the protein-drug conjugate, in combination with other therapies or drugs, in preparation of a drug, wherein the drug is used for treating tumors or other diseases.

In certain embodiments, the other therapies or drugs are selected from: chemotherapy, radiotherapy, miRNA and oligonucleotides.

In certain embodiments, the tumor is selected from any one or more of the following groups: lymphoma, multiple myeloma, breast cancer, ovarian cancer, kidney cancer, endometrial cancer, melanoma, pancreatic cancer, lung cancer, stomach cancer, liver cancer, mesothelioma, esophageal cancer, head and neck cancer, bile duct cancer, gallbladder cancer, bladder cancer, thymus cancer and colorectal cancer.

In another aspect, the present application provides a method for diagnosing a specific disease, comprising using the protein-drug conjugate and/or the pharmaceutical composition.

In another aspect, the present application provides a method for detecting a specific target, comprising using the protein-drug conjugate and/or the pharmaceutical composition, wherein preferably the detection is for non-disease diagnostic purposes.

In another aspect, the present application provides a kit for detection, comprising the protein-drug conjugate and/or the pharmaceutical composition, and optionally instructions for use.

In another aspect, the present application provides an administration device, comprising the protein-drug conjugate and/or the pharmaceutical composition, and a device for administering the protein-drug conjugate and/or the pharmaceutical composition.

The inventor of the present application surprisingly discovered that: in the absence of a light chain, the Cys-220 (Eu-numbered) in the human IgG1 heavy chain hinge region has the opportunity to form a disulfide bond between heavy chains, however, the disulfide bond is not as stable as other disulfide bonds, and only a weaker reducing agent is needed to separate the disulfide bond of the Cys-220 from other disulfide bonds, so that the disulfide bond of the Cys-220 can be effectively cleaved while the other disulfide bonds remain intact. Furthermore, the Cys-220 becomes almost the only free cysteine residue that acts as a site-specific conjugation site for covalent binding with drug conjugates. The hinge region derivative sequence, formed by adjusting the type, the number of amino acids between Cys-220 and Cys-226 in the human IgG1 natural hinge region or by adding a flexible linker peptide before Cys-220, can also serve as a linker peptide to achieve site-specific conjugation corresponding to a Cys-220 site in the natural hinge region.

The above conditions may be combined arbitrarily to obtain preferred embodiments of the present application on the basis of the general knowledge in the art.

## BRIEF DESCRIPTION OF THE DRAWINGS

FIG. 1 shows a disulfide bond structure of a human IgG1 antibody.

FIG. 2 shows a structure of a heavy-chain antibody HCAb with a human IgG1 antibody hinge region sequence and a difference in a hinge region disulfide bond structure between human IgG1 and HCAb.

FIG. 3 shows, in an HCAb structure, two possible states of a cysteine Cys-220 located at position 220 (Eu numbering) in a hinge region: Cys-220 forms or does not form a disulfide bond between heavy chains.

FIG. 4 shows a mass spectrometry deconvolution processing map of molecular weight analysis of non-reduced samples of HCAb PR000020.

FIG. 5 shows analysis results of papain cleavage of HCAb PR000020: (A) hypothesis of whether Cys-220 forms a disulfide bond between heavy chains and speculation of corresponding papain cleavage product components; (B) results of non-reduced SDS-PAGE of a papain cleavage product.

FIG. 6 shows (A) a schematic diagram of ADCs comprising a drug conjugate mc-vc-PAB-MMAE and a structure of the mc-vc-PAB-MMAE (also known as VcMMAE), wherein the mc-vc-PAB-MMAE is composed of a linker mc-vc-PAB and a loaded drug MMAE, (wherein "antibody" and "ADCs" are only general indications and do not refer to specific structures of "antigen-binding protein" or "protein-drug conjugate" of the present invention); and (B) a characteristic fragment structure of the mc-vc-PAB-MMAE in mass spectrometry analysis.

FIG. 7 shows binding activity of HCAb PR000020 and a coupling product PR000020-ADC thereof to cells highly expressing CTLA4.

FIG. 8 shows HIC-HPLC analysis results of HCAb PR000020 and a coupling product PR000020-ADC thereof: (A) before HCAb coupling; (B) after coupling and before purification; (C) after coupling and one-step HIC purification.

FIG. 9 shows RP-HPLC analysis results of HCAb PR000020 and a coupling product PR000020-ADC thereof: (A) before HCAb coupling; (B) after coupling and before purification; (C) after coupling and one-step HIC purification.

FIG. 10 shows mass spectrometry deconvolution processing maps of molecular weight analysis of a coupling product PR000020-ADC of HCAb PR000020: (A) non-reduced samples; (B) reduced samples.

FIG. 11 shows analysis of compound coupling sites of PR000020-ADC by using LC-MS peptide map: (A) an amino acid sequence of PR000020-ADC, polypeptide fragments comprising Cys-IAM labeled with a gray background; (B) polypeptide fragments comprising Cys-IAM in PR000020-ADC samples; (C) polypeptide fragments comprising Cys-IAM in PR000020 samples; definitions of column names in tables in (B) and (C) can be found below (C).

FIG. 12 shows SEC-HPLC analysis results of samples after HCAb PR000759 protein expression and purification.

FIG. 13 shows HIC-HPLC analysis results of samples after one-step HIC purification of a coupling product PR000759-ADC of HCAb PR000759.

FIG. 14 shows analysis of compound coupling sites of PR000759-ADC by using LC-MS peptide map, and screened peptide fragments with coupling sites; definitions of column names in the table are the same as FIG. 11 (C).

FIG. 15 shows mass spectrometry deconvolution processing maps of molecular weight analysis of HCAb PR000759 and a coupling product PR000759-ADC thereof: (A) non-reduced samples of PR000759; (B) non-reduced samples of PR000759-ADC; (C) reduced samples of PR000759-ADC.

FIG. 16 shows SEC-HPLC analysis results of samples after HCAb PR001046 protein expression and purification.

FIG. 17 shows HIC-HPLC analysis results of samples after one-step HIC purification of a coupling product PR001046-ADC of HCAb PR001046.

FIG. 18 shows analysis of compound coupling sites of PR001046-ADC by using LC-MS peptide map, and screened peptide fragments with coupling sites; definitions of column names in the table are the same as FIG. 11 (C).

FIG. 19 shows mass spectrometry deconvolution processing maps of molecular weight analysis of HCAb PR001046 and a coupling product PR001046-ADC thereof: (A) non-reduced samples of PR001046; (B) non-reduced samples of PR001046-ADC; (C) reduced samples of PR001046-ADC.

FIG. 20 shows binding activity of HCAb PR001046 and a coupling product PR001046-ADC thereof to cells highly expressing BCMA.

FIG. 21 shows specific cytotoxicity of PR001046-ADC: (A) specific killing of $BCMA^+$ cells NCI-H929 and mixed cells; (B) an effect of different purities of ADC (D2 component) on cytotoxicity.

FIG. 22 shows SEC-HPLC analysis results of samples after HCAb PR004432 protein expression and purification.

FIG. 23 shows RP-HPLC analysis results of samples after one-step HIC purification of a coupling product PR004432-ADC of HCAb PR004432.

FIG. 24 shows analysis of compound coupling sites of PR004432-ADC by using LC-MS peptide map, including screened peptide fragments with coupling sites and corresponding coupling site coverage rates.

FIG. 25 shows mass spectrometry deconvolution processing maps of molecular weight analysis of a coupling product PR004432-ADC of HCAb PR004432: (A) non-reduced samples; (B) reduced samples.

FIG. 26 shows binding activity of HCAb PR004432 and a coupling product PR004432-ADC thereof to cells highly expressing 5T4.

FIG. 27 shows specific cytotoxicity of PR004432-ADC on cells highly expressing 5T4.

FIG. 28 shows SEC-HPLC analysis results of samples after HCAb PR004433 protein expression and purification.

FIG. 29 shows HIC-HPLC analysis results of samples after one-step HIC purification of a coupling product PR004433-ADC of HCAb PR004433.

FIG. 30 shows HIC-HPLC analysis results of HCAb PR004433 and a coupling product PR004433-ADC thereof: (A) before HCAb coupling; (B) after coupling and before purification; (C) after coupling and one-step HIC purification.

FIG. 31 shows mass spectrometry deconvolution processing maps of molecular weight analysis of a coupling product PR004433-ADC of HCAb PR004433: (A) non-reduced samples; (B) reduced samples.

FIG. 32 shows binding activity of HCAb PR004433 and a coupling product PR004433-ADC thereof to cells expressing BCMA: (A) binding to cells HEK293T-hBCMA highly expressing human BCMA; (B) binding to cells NCI-H929 highly expressing human BCMA; (C) non-binding to BCMA-negative cells SNU-16.

FIG. 33 shows specific cytotoxicity of PR004433-ADC: (A) specific cytotoxicity on HEK293T-hBCMA; (B) no cytotoxicity on HEK293T; (C) specific cytotoxicity on NCI-H929; (D) no cytotoxicity on SNU-16.

FIG. 34 shows analysis of compound coupling sites of PR004433-ADC by using LC-MS peptide map, including screened peptide fragments with coupling sites and corresponding coupling site coverage rates.

FIG. 35 shows a secondary map corresponding to screened peptide fragments with coupling sites when compound coupling sites of PR000020-ADC are analyzed by using LC-MS peptide map.

FIG. 36 shows a XIC diagram of a peptide fragment of enzymatic digestion PHGSDIWGQGTMVTVSSEPKSC#DK (# represents a coupling site) in an ADC sample (upper) and a monoclonal antibody sample (lower) when compound coupling sites of PR000020-ADC are analyzed by using LC-MS peptide map.

FIG. 37 shows analysis of a disulfide bond of Cys-220 in PR002129 by LC-MS: (A) showing two possible states of Cys-220 in a structure of PR002129; (B) a mass spectrometry deconvolution processing map of molecular weight analysis of non-reduced samples of PR002129.

FIG. 38 shows DSC thermodynamic analysis curve graphs of HCAb PR000184 and a C220S derived variant thereof: (A) PR000184; (B) PR000184 (C220S).

FIG. 39 shows DSC thermodynamic analysis curve graphs of HCAb PR000453 and a C220S derived variant thereof: (A) PR000453; (B) PR000453 (C220S).

FIG. 40 shows melting temperatures Tm of HCAb PR004432, a C220S derived variant thereof and a coupling product thereof measured by using a Uncle analysis platform: (A) PR004432; (B) PR004432 (C220S); (C) PR004432-ADC.

FIG. 41 illustrates the components and related terms of the "protein-drug conjugate" in the specific embodiments of the present invention.

FIG. 42 shows exemplary antigen-binding proteins: (A) an antigen-binding protein with two antigen-binding fragments and two linker peptides; (B) an antigen-binding protein with four antigen-binding fragments and two linker peptides.

FIG. 43 shows exemplary protein-drug conjugates produced by coupling using the antigen-binding proteins as shown in FIG. 42: (A) a protein-drug conjugate with two antigen-binding fragments and two linker peptides; (B) a protein-drug conjugate with four antigen-binding fragments and two linker peptides.

FIG. 44 shows hinge region sequences and disulfide bond structures of different species: (A) hinge region sequences of different IgG isotypes of humans, mice and alpacas, with cysteine Cys identified; (B) a human IgG1 hinge region sequence and a disulfide bond structure; (C) a human IgG4 hinge region sequence and a disulfide bond structure; (D) a mouse IgG1 hinge region sequence and a disulfide bond structure; (E) a mouse IgG2a hinge region sequence and a disulfide bond structure.

FIG. 45 shows Total Ion Chromatograms (TICs) in mass spectrums obtained by LC-MS analysis of the complete molecular weights of non-reduced samples of coupling products prepared by HCAb PR004433 under different reduction and coupling reaction conditions, corresponding to experiment numbers in Table 0-1 and Table 0-2 of 0 respectively: (A) Experiment #8; (B) Experiment #9; (C) Experiment #11; (D) Experiment #12.

FIG. 46 shows binding activity of HCAb PR004433 and a variant molecule thereof to NCI-H929 cells.

FIG. 47 shows a mass spectrometry deconvolution processing map of complete molecular weight analysis of non-reduced samples of a coupling product PR006468-ADC of HCAb PR006468.

FIG. 48 shows analysis of compound coupling sites of PR006468-ADC by using LC-MS peptide map, including screened peptide fragments with coupling sites and corresponding coupling site coverage rates.

FIG. 49 shows binding activity of BCMA binding proteins (HCAb PR004433, HCAb PR006468) and coupling products (PR004433-ADC, PR006468-ADC) thereof to NCI-H929 cells.

FIG. 50 shows cytotoxicity of PR006468-ADC and PR004433-ADC on NCI-H929 cells.

FIG. 51 shows HIC-HPLC analysis results of a coupling product PR002129-ADC of PR002129: (A) after coupling and before purification; (B) after coupling and one-step HIC purification.

FIG. 52 shows mass spectrometry deconvolution processing maps of molecular weight analysis of a coupling product PR002129-ADC of PR002129: (A) non-reduced molecular weights of samples before HIC purification; (B) reduced molecular weights of samples after HIC purification; (C) non-reduced molecular weights of samples after HIC purification.

FIG. 53 shows analysis of compound coupling sites of PR002129-ADC by using LC-MS peptide map, including screened peptide fragments with coupling sites and corresponding coupling site coverage rates.

FIG. 54 shows binding activity of PR002129 and a coupling product PR002129-ADC thereof to cells highly expressing ROR1.

FIG. 55 shows HIC-HPLC analysis results of samples before purification of a coupling product PR006345-ADC of SIRPa-Fc fusion protein PR006345.

FIG. 56 shows a mass spectrometry deconvolution processing map of non-reduced molecular weight analysis of samples before purification of a coupling product PR006345-ADC of SIRPa-Fc fusion protein PR006345.

FIG. 57 shows analysis of compound coupling sites of PR006345-ADC by using LC-MS peptide map, including screened peptide fragments with coupling sites and corresponding coupling site coverage rates.

FIG. 58 shows binding activity of PR006345 and a coupling product PR006345-ADC thereof to cells highly expressing CD47.

FIG. 59 shows a structure of a tetravalent bispecific antigen-binding protein PR005744.

FIG. 60 shows internalization of PR005744 on NCI-H929 cells.

FIG. 61 shows analysis of compound coupling sites of PR005744-ADC by using LC-MS peptide map, including screened peptide fragments with coupling sites and corresponding coupling site coverage rates.

FIG. 62 shows binding activity of PR005744 and a coupling product PR005744-ADC thereof to NCI-H929 cells.

FIG. 63 shows cytotoxicity of PR005744-ADC on NCI-H929 cells.

FIG. 64 shows a molecular structure of a BRD4 protein degrading agent (PROTAC).

FIG. 65 shows a mass spectrometry deconvolution processing map of non-reduced molecular weight analysis of samples before purification of a product PR004433-PROTAC by coupling of HCAb PR004433 and PROTAC.

FIG. 66 shows binding activity of HCAb PR004433 and a coupling product PR004433-PROTAC thereof to NCI-H929 cells.

FIG. 67 shows a structure of a bivalent bispecific antigen-binding protein 2129/4433.

FIG. 68 shows HIC-HPLC analysis results of samples before purification of a coupling product 2129/4433-ADC of 2129/4433.

FIG. 69 shows analysis of compound coupling sites of 2129/4433-ADC by using LC-MS peptide map, including screened peptide fragments with coupling sites and corresponding coupling site coverage rates.

FIG. 70 shows a secondary map corresponding to screened peptide fragments with coupling sites when compound coupling sites of PR001046-ADC are analyzed by using LC-MS peptide map.

FIG. 71 shows a secondary map corresponding to screened peptide fragments with coupling sites when compound coupling sites of PR006468-ADC are analyzed by using LC-MS peptide map.

FIG. 72 shows a secondary map corresponding to screened peptide fragments with coupling sites when compound coupling sites of 2129/4433-ADC are analyzed by using LC-MS peptide map.

## DETAILED DESCRIPTION

The embodiments of the present invention are described below with reference to specific examples, and other advantages and effects of the present invention will be readily apparent to those skilled in the art from the disclosure of the present specification.

In the present application, the terms "immunoglobulin" (Ig) and "antibody" are used interchangeably herein. The basic four-chain antibody unit is a heterotetramer protein, which is composed of two identical light chains (L) and two identical heavy chains (H). In the case of IgG, each L chain is connected to the H chain through a covalent disulfide bond, and two H chains are connected to each other through one or more disulfide bonds. The number of disulfide bonds depends on the isotype of the H chain. Each H and L chain also has regularly-spaced intrachain disulfide bonds. Each H chain has a variable domain (VH) at an amino terminus, followed by three (for each $\alpha$ and $\gamma$ chain) or four (for $\mu$ and $\varepsilon$ isotype) constant domain (CH). Each L chain has a variable domain (VL) at an amino terminus, followed by one constant domain (CL). Human IgG has four subtypes: IgG1, IgG2, IgG3 and IgG4. The L chain of human or mouse antibodies is further divided into $\kappa$ chain and $\lambda$ chain, and accordingly, its variable domain is divided into Y$\kappa$ and V$\lambda$, and its constant domain is divided into C$\kappa$ and C$\lambda$.

In the present application, the term "binding protein" or "antigen-binding protein" generally refers to a protein comprising an antigen-binding moiety, and optionally a scaffold or framework moiety that allows the antigen-binding moiety to adopt a conformation that facilitates the binding of the antigen-binding protein to the antigen. The "binding protein" or "antigen-binding protein" may typically comprise an antibody light chain variable region (VL) or an antibody heavy chain variable region (VH), or both. The VH and VL regions can be further divided into hypervariable regions termed complementary determining regions (CDRs), and relatively conserved framework regions (FRs). Each VH and VL can be composed of three CDRs and four FRs. The VH and VL comprise binding sites that interact with antigens. The three CDRs of VH are denoted as HCDR1, HCDR2 and HCDR3, respectively, and may also be denoted as VH CDR1, VH CDR2 and VH CDR3, respectively; and the three CDRs of VL are denoted as LCDR1, LCDR2 and LCDR3, respectively, and may also be denoted as VL CDR1, VL CDR2 and VL CDR3, respectively. Examples of the antigen-binding proteins include, but are not limited to, antibodies, antigen-binding fragments (Fab, Fab', $F(ab)_2$, Fv fragment, $F(ab')_2$, scFv, di-scFv and/or dAb), immunoconjugates, multispecific antibodies (e.g., bispecific antibodies), antibody fragments, antibody derivatives, antibody analogs, receptor protein soluble extracellular regions, ligandin or other forms of fusion proteins, as long as they exhibit the desired antigen-binding activity. In the present application, the amino acid sequences of the CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-948, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See the table below for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

Table-I. The schemes for numbering the CDRs of the antibody of the present application Laa-Lbb can refer to an amino acid sequence from position aa (the Chothia scheme) to position bb (the Chothia scheme) beginning at the N-terminus of the light chain of the antibody; and Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H32 can refer to the amino acid sequence from position 26 to position 32 according to the Chothia scheme beginning at the N-terminus of the heavy chain of the antibody. It should be known to those skilled in the art that there are positions where insertion sites are present in numbering CDRs with the Chothia scheme (see http://bioinf.org.uk/abs/).

| | **Kabat** | **Chothia** | **Combined** |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26-H32 | H26-H35 |
| HCDR2 | H50--H65 | H52--H56 | H50-H65 |
| HCDR3 | H95--H102 | H95--H102 | H95-H102 |

In the present application, the term "monoclonal antibody" generally refers to an antibody obtained from a population of substantially homogeneous cells, that is, the antibodies in the population are identical except for a small amount of natural mutations that may exist. Monoclonal antibodies are generally highly specific for a single antigenic site. Moreover, unlike conventional polyclonal antibody formulations (which generally have different antibodies directed against different determinants), each monoclonal antibody is directed against a single determinant on the antigen. In addition to their specificity, the advantage of monoclonal antibodies lies in that they can be synthesized through hybridoma culture and are not contaminated by other immunoglobulins. The modifier "monoclonal" represents the characteristics of the antibody obtained from a population of substantially homogeneous antibodies, but is not to be construed as requiring production of the antibody by any particular method. For example, monoclonal antibodies used according to the present invention can be prepared in hybridoma cells or can be prepared by the recombinant DNA method.

In the present application, the term "fully human antibody" generally refers to an antibody that is expressed by a genetically engineered antibody gene-deleted animal into which the entire gene that encodes an antibody in human is transferred. All parts of the antibody (including variable and constant regions of the antibody) are composed of amino acid sequences of human origin. The fully human antibody can greatly reduce the immune side effects caused in the human body by the heterologous antibody. Methods for obtaining fully human antibodies in the art can include phage display, transgenic mice, and the like.

In the present application, the term "specific binding to" generally refers to that an antigen-binding protein binds to an antigenic epitope, and that the binding requires some complementarity between the antigen-binding protein and the epitope. According to this definition, an antibody is said to "specifically bind to" an antigen when the antibody more easily binds to an epitope via its antigen-binding protein than binds to a random, unrelated epitope.

"Epitope" refers to a specific atomic group (e.g., saccharide side chains, phosphoryl, sulfonyl) or an amino acid on an antigen that binds to an antigen-binding protein (e.g., an antibody).

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In certain embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form the other polypeptide chain, in which case an Fab (cross VH/VL) structure is formed; in certain embodiments, CH1 of the Fab is not linked to the hinge region, but rather the C-terminus of CL is linked to the hinge region of the heavy chain, in which case a Fab (cross Fd/LC) structure is formed.

In the present application, the term "VH" generally refers to the heavy chain variable region VH domain of an antibody. In certain embodiments, "VH" may be a heavy chain variable region VH of a conventional antibody (H2L2 structure) from humans or other animals. In certain embodiments, "VH" may also be a heavy chain variable region VHH of a heavy-chain antibody (HCAb structure) from animals such as camelidae. In certain embodiments, "VH" may also be a heavy chain variable region VH of a fully-human heavy-chain antibody (HCAb structure) produced by a Harbour HCAb transgenic mouse.

In the present application, the term "antigen-binding fragment" generally refers to any protein functional region that can specifically bind to an antigen. In certain embodiments, "antigen-binding fragment" may be "Fab". In certain embodiments, "antigen-binding fragment" may also be "VH". In certain embodiments, "antigen-binding fragment" may also be a single chain antibody scFv. In certain embodiments, "antigen-binding fragment" may further be other antigen-binding forms (e.g., a receptor protein soluble extracellular region, a ligandin, a lipocalin, a neuronal cell adhesion molecule (NCAM), a fibronectin, a designed ankyrin repeat protein (DARPin) and other derived protein structures).

In the present application, the term "pairing unit" generally refers to a group of structures comprising at least two subunits that can be paired with each other. The paired subunits can bind with each other, and the binding requires complementarity between the subunits; covalent bond or non-covalent bond interaction can occur between the paired subunits; the non-covalent bond interaction can include van der Waals force, hydrogen bond, hydrophobic interaction, electrostatic interaction, dipole interaction, etc. "Pairing unit" may be a set of naturally occurring or modified polypeptide chains in the form of homologous or heterologous multimers; for example, "pairing unit" may be a dimer form of antibody Fc fragments or variants thereof; "pairing unit" may be $\alpha$ chain and $\beta$ chain of CD79; "pairing unit" may be $\alpha$ chain and $\beta$ chain of CD8; "pairing unit" may be $\alpha$ chain and $\beta$ chain of a T-cell receptor (TCR).

In the present application, the term "Fc fragment" generally refers to the fragment crystallizable (Fc) of the antibody, that is, comprising a polypeptide chain composed of heavy chain constant domains CH2 and CH3. In certain embodiments, an Fc fragment may be in the form of a natural dimer; in other embodiments, an Fc fragment may be in the form of a modified monomer.

In the present application, the term "CTLA4" generally refers to the cytotoxic T lymphocyte-associated antigen-4 (also known as CD152), a functional variant thereof and/or a functional fragment thereof. The sequence of CTLA4 is known in the art. For example, the sequence of an exemplary full-length human CTLA4 can be found under Uniprot accession No. P16410; and the sequence of an exemplary full-length cynomolgus monkey CTLA4 can be found under Uniprot accession No. G7PL88. Drugs directed against CTLA4 may be applied to treat melanoma, lung cancer, colon cancer, liver cancer, head and neck cancer, kidney cancer, breast cancer, pancreatic cancer, bladder cancer and other tumors.

In the present application, the term "BCMA" generally refers to tumor necrosis factor receptor superfamily member 17 (also known as CD269, B cell maturation protein), a functional variant thereof and/or a functional fragment thereof. The sequence of BCMA is known in the art. For example, the sequence of an exemplary full-length human BCMA can be found under Uniprot accession No. Q02223; and the sequence of an exemplary full-length cynomolgus monkey BCMA can be found under NCBI accession No. XP_005591343. Drugs directed against BCMA may be applied to treat multiple myeloma and other hematological malignant tumors.

In the present application, the term "MSLN" generally refers to a mesothelin molecule (also known as MPF), a functional variant thereof and/or a functional fragment thereof. The sequence of MSLN is known in the art. For example, the sequence of an exemplary full-length human MSLN can be found under Uniprot accession No. Q13421; and the sequence of an exemplary full-length cynomolgus monkey MSLN can be found under NCBI accession No. XP_005590873. Drugs directed against MSLN may be applied to treat mesothelioma, lung cancer, pancreatic cancer, breast cancer, ovarian cancer, endometrial cancer and other tumors. In the present application, the term "5T4" generally refers to a trophoblast glycoprotein (also known as TPBG), a functional variant thereof and/or a functional fragment thereof. The sequence of 5T4 is known in the art. For example, the sequence of an exemplary full-length human 5T4 can be found under Uniprot accession No. Q13641; and the sequence of an exemplary full-length cynomolgus monkey 5T4 can be found under Uniprot accession No. Q4R8Y9. Drugs directed against 5T4 may be applied to treat thymus cancer, lung cancer, esophageal cancer, stomach cancer, small intestine cancer, pancreatic cancer, liver cancer, gallbladder cancer, kidney cancer, bladder cancer, ovarian cancer, endometrial cancer, cervical cancer and other tumors.

In the present application, the term "ROR1" generally refers to an inactivated tyrosine protein kinase transmembrane receptor ROR1 (also known as NTRKR1), a functional variant thereof and/or a functional fragment thereof. The sequence of ROR1 is known in the art. For example, the sequence of an exemplary full-length human ROR1 can be found under Uniprot accession No. Q01973; and the sequence of an exemplary full-length cynomolgus monkey ROR1 can be found under NCBI accession No. XP_015290264. Drugs directed against ROR1 may be applied to treat leukemia, non-Hodgkin lymphoma, mantle cell lymphoma, breast cancer, lung cancer, ovarian cancer and other tumors.

In the present application, the term "CD47" generally refers to a leukocyte surface antigen CD47, a functional variant thereof and/or a functional fragment thereof. The sequence of CD47 is known in the art. For example, the sequence of an exemplaryfull-length human CD47 can be found under Uniprot accession No. Q08722. Drugs directed against CD47 may be applied to treat leukemia, non-Hodgkin lymphoma, multiple myeloma, melanoma, head and neck cancer and other tumors.

In the present application, the term "SIRPα" generally refers to a signal regulatory protein α (also known as BIT, MFR, MYD1, PTPNS1, SHPS1 or SIRP), a functional variant thereof and/or a functional fragment thereof. SIRPα is a receptor for CD47. The sequence of SIRPα is known in the art. For example, the sequence of an exemplaryfull-length human SIRPα can be found under Uniprot accession No. P78324.

In the present application, the term "protein-drug conjugate" generally refers to a class of drug molecules formed by binding a drug conjugate to an antigen-binding protein through a covalent bond. The drug conjugate comprises at least one loaded drug. A variety of protein-drug conjugates and a preparation method thereof are known in the art. In certain embodiments, the protein-drug conjugate may be an antibody-drug conjugate (ADC). For example, exemplary protein-drug conjugates can include, but are not limited to, trastuzumab, ocrelizumab, pertuzumab, rituximab and other antibodies. Loaded drug modules can be covalently attached to antibodies via linker units to form antibody-drug conjugates to achieve targeted therapeutic effects. In certain embodiments, "protein-drug conjugate" may comprise an antigen-binding fragment, a linker peptide and a drug conjugate. In certain embodiments, the drug conjugate of "protein-drug conjugate" may be mc-vc-PAB-MMAE (also known as VcMMAE).

In the present application, the term "drug conjugate" generally refers to a class of aggregations of atoms and groups with specific structures that can bind to other molecules by covalent bonds through specific functional groups; it comprises at least one loaded drug, and optionally comprises a linker. A variety of loaded drugs, a variety of linkers or linker members are known in the art. In certain embodiments, the drug conjugate may be mc-vc-PAB-MMAE (also known as VcMMAE).

In the present application, the term "loaded drug" generally refers to the forms of a class of small molecule compounds or toxins or other drug molecules with pharmaceutical activity, which can be, but are not limited to, small molecule compounds, toxin molecules, antibiotics, oligonucleotides, proteolysis targeting chimeras (PROTACs), affinity ligands, fluorescent groups, nuclide groups, polypeptides, immunomodulatory molecules, etc. A variety of loaded drugs are known in the art, such as monomethyl auristatin E ("MMAE"), mertansine ("DM1"), and toll-like receptor agonist molecules.

In the present application, the term "linker peptide" or "peptide fragment" generally refers to a part of a polypeptide chain in the "protein-drug conjugate" in the present application. The linker peptide comprises at least one cysteine as a site for covalent binding with the drug conjugate. In the present application, the linker peptide may comprise a first linker peptide and/or a second linker peptide, and amino acid sequences of the first linker peptide and the second linker peptide may be identical or different.

In the present application, the term "linker" or "linker unit" generally refers to a chemical functional module that can be used to connect one or more loaded drugs to the antigen-binding protein to form "protein-drug conjugate". The linker may comprise one or more linker members. A variety of linker members are known in the art, such as maleimidocaproyl ("MC"), valine-citrulline ("val-cit" or "vc") and *p*-aminobenzyloxycarbonyl ("PAB"). In the present application, the "linker" may include a polypeptide linker composed of amino acids, but the "linker" is different from the aforementioned "linker peptide". The aforementioned "linker peptide" is a part of the antigen-binding protein.

In the present application, the term "conjugate" generally refers to the aggregation of atoms and groups formed by covalent binding between the "drug conjugate" in the present application and a specific functional group. In certain embodiments, the conjugate may be formed by the reaction of the drug conjugate with sulfhydryl. For example, in certain embodiments, the conjugate may be an aggregation of stable atoms/groups formed by the addition reaction between the active maleimide reaction group of mc-vc-PAB-MMAE and the sulfhydryl.

In the present application, the term "DAR" (Drug-Antibody Ratio) generally refers to the ratio of a loaded drug to an antibody, that is, the average number of loaded drugs connected to the antibody. The drug loading of a current protein-drug conjugate is generally 0 to 8 loaded drug molecules (D0 to D8)/antibody. Generally, "D0", "D2", "D4", "D6" and "D8" are also used to represent protein-drug conjugate components in a coupling product without coupled loaded drug molecules (DAR = 0), coupled with 2 loaded drug molecules (DAR = 2), coupled with 4 loaded drug molecules (DAR = 4), coupled with 6 loaded drug molecules (DAR = 6), and coupled with 8 loaded drug molecules (DAR = 8), respectively. In certain embodiments, "D0" is also known as "naked antibody". The HIC-HPLC analysis method is a commonly used analysis method for determining the DAR and drug loading distribution of a protein-drug conjugate.

In the present application, the term "CAR" (Conjugate/Antigen binding protein Ratio) is used to represent the ratio of drug conjugates to an antigen-binding protein, that is, the average number of drug conjugates connected to the antigen-binding protein. Generally, one drug conjugate comprises at least one loaded drug; in some embodiments, the prior art enable one drug conjugate moiety to only comprise one loaded drug, in which case the values of "CAR" and "DAR" may be equivalent; in other embodiments, the prior art can enable one drug conjugate moiety to connect a plurality of loaded drugs through linkers (see Kumara et al., Bioorganic & Medicinal Chemistry Letters (2018), 28, 3617-3621), in which case the value of "DAR" may be several times that of "CAR"; for example, when one drug conjugate comprises two loaded drugs, the value of DAR may be twice that of CAR.

In specific embodiments of the present application, the drug conjugate in the present application is linked by using the cysteine Cys-220 (Eu numbering) in the natural sequence (SEQ ID NO: 73) of the human IgG1 antibody heavy chain hinge region as a coupling site, so that a protein-drug conjugate with a uniform CAR value can be generated. In particular, a protein-drug conjugate with uniform CAR = 2 can be generated using the method. In certain specific embodiments of the present application, when mc-vc-PAB-MMAE is used as a drug conjugate for coupling, since one mc-vc-PAB-MMAE only comprises one loaded drug MMAE, the value of CAR and the value of DAR may be equivalent, or the value of DAR may be used to refer to the number of drug conjugates connected to one antigen-binding protein; in particular, a protein-drug conjugate with a uniform DAR value can be generated using the method; for example, the DAR of the protein-drug conjugate is 2.

In the present application, the term "derived from" generally refers to derivative amino acid or nucleotide sequences obtained from parental amino acid or nucleotide sequences. It generally refers to the structural similarity between the parental sequence and the derivative sequence, without implying or containing limitations on the process or source of derivative sequences derived from parental sequences. Therefore, when "derived from" is used to discuss proteins or polynucleotides, their physical origins are not considered. In certain cases, "derived from" can refer to the derivative sequence being a part of the unmodified parental sequence. For example, in specific embodiments of the present application, Cys1 and Cys2 may be a part of the unmodified natural antibody hinge region sequence.

In one aspect, the present application provides the following embodiments:

1. A protein-drug conjugate, substantially comprising a first polypeptide chain and a second polypeptide chain, wherein the first polypeptide chain comprises a first cysteine (Cys1) and a second cysteine (Cys2), the second polypeptide chain comprises a first cysteine (Cys1) and a second cysteine (Cys2), and the Cys1 of the first polypeptide chain is coupled with a structure - L1-P1, and the Cys1 of the second polypeptide chain is coupled with a structure -L2-P2.
2. The protein-drug conjugate according to embodiment 1, wherein the structure -L1-P1 may not have L1.
3. The protein-drug conjugate according to any one of embodiments 1 to 2, wherein the structure -L2-P2 may not have L2.
4. The protein-drug conjugate according to any one of embodiments 1 to 3, wherein the L1 and/or the L2 comprise/comprises a linker.
5. The protein-drug conjugate according to embodiment 4, wherein the linker comprises a cleavable linker.
6. The protein-drug conjugate according to any one of embodiments 4 to 5, wherein the linker comprises a protease-sensitive linker.
7. The protein-drug conjugate according to any one of embodiments 4 to 6, wherein the linker comprises mc-vc-PAB.
8. The protein-drug conjugate according to any one of embodiments 1 to 7, wherein the P1 and/or the P2 comprise/comprises a loaded drug.
9. The protein-drug conjugate according to embodiment 8, wherein the loaded drug comprises a active drug ingredient and a labeled molecule.
10. The protein-drug conjugate according to any one of embodiments 8 to 9, wherein the loaded drug includes small molecule compounds, toxin molecules, oligonucleotides, proteolysis targeting chimeras (PROTAC), affinity ligands, labeled groups, antibiotics, polypeptides and/or immunomodulatory molecules.
11. The protein-drug conjugate according to any one of embodiments 1 to 10, wherein the P1 and the P2 may be identical or different.
12. The protein-drug conjugate according to any one of embodiments 1 to 11, wherein the L1 and the L2 may be identical or different.

13. The protein-drug conjugate according to any one of embodiments 1 to 12, wherein the L1 and the P1 are connected in a covalent binding manner.

14. The protein-drug conjugate according to any one of embodiments 1 to 13, wherein the L2 and the P2 are connected in a covalent binding manner.

15. The protein-drug conjugate according to any one of embodiments 1 to 14, wherein the first polypeptide chain comprises a first antigen-binding fragment.

16. The protein-drug conjugate according to any one of embodiments 1 to 15, wherein the second polypeptide chain comprises a second antigen-binding fragment.

17. The protein-drug conjugate according to embodiment 15 or 16, wherein the first antigen-binding fragment and/or the second antigen-binding fragment target/targets a tumor antigen or a non-tumor antigen.

18. The protein-drug conjugate according to any one of embodiments 15 to 17, wherein the antigen-binding fragments include a VH, a VL, an scFv, an Fab, a dAb, a receptor protein soluble extracellular region and/or derived protein structures, and the like.

19. The protein-drug conjugate according to any one of embodiments 1 to 18, further comprising a pairing unit.

20. The protein-drug conjugate according to embodiment 19, wherein the pairing unit is an Fc domain.

21. The protein-drug conjugate according to any one of embodiments 1 to 20, wherein the first polypeptide chain comprises a first linker peptide, and the second polypeptide chain comprises a second linker peptide.

22. The protein-drug conjugate according to any one of embodiments 15 to 21, wherein the antigen-binding fragment is connected to the pairing unit through the linker peptide.

23. The protein-drug conjugate according to any one of embodiments 15 to 22, wherein a C-terminus of the antigen-binding fragment is connected to an N-terminus of the first linker peptide and/or the second linker peptide.

24. The protein-drug conjugate according to any one of embodiments 15 to 23, wherein a C-terminus of the first linker peptide and/or the second linker peptide is connected to an N-terminus of the pairing unit.

25. The protein-drug conjugate according to any one of embodiments 1 to 24, wherein the first polypeptide chain or the second polypeptide chain each independently comprises an amino acid sequence set forth in any one of SEQ ID NOs: 64-77, 106-129 and 148-149.

26. The protein-drug conjugate according to any one of embodiments 1 to 25, wherein the first polypeptide chain sequentially comprises the first antigen-binding fragment, the first linker peptide (such as a hinge region of IgG) and the pairing unit from an N-terminus to a C-terminus.

27. The protein-drug conjugate according to any one of embodiments 1 to 26, wherein the second polypeptide chain sequentially comprises the second antigen-binding fragment, the second linker peptide (such as a hinge region of IgG), and the pairing unit from an N-terminus to a C-terminus.

28. The protein-drug conjugate according to any one of embodiments 1 to 27, wherein the first polypeptide chain sequentially comprises a VH of the first antigen-binding fragment, a VL of the first antigen-binding fragment, the first linker peptide (such as the hinge region of IgG), $CH_2$ and $CH_3$ from the N-terminus to the C-terminus.

29. The protein-drug conjugate according to embodiment 28, wherein the second polypeptide chain sequentially comprises a VH of the second antigen-binding fragment, a VL of the second antigen-binding fragment, the second linker peptide (such as the hinge region of IgG), $CH_2$ and $CH_3$ from the N-terminus to the C-terminus.

30. The protein-drug conjugate according to any one of embodiments 1 to 27, wherein the first polypeptide chain sequentially comprises a VL of the first antigen-binding fragment, a VH of the first antigen-binding fragment, the first linker peptide (such as the hinge region of IgG), $CH_2$ and $CH_3$ from the N-terminus to the C-terminus.

31. The protein-drug conjugate according to embodiment 30, wherein the second polypeptide chain sequentially comprises a VL of the second antigen-binding fragment, a VH of the second antigen-binding fragment, the second linker peptide (such as the hinge region of IgG), $CH_2$ and $CH_3$ from the N-terminus to the C-terminus.

32. The protein-drug conjugate according to any one of embodiments 1 to 27, wherein the first polypeptide chain sequentially comprises a VH of the first antigen-binding fragment, the first linker peptide (such as the hinge region of IgG), $CH_2$ and $CH_3$ from the N-terminus to the C-terminus.

33. The protein-drug conjugate according to embodiment 32, wherein the second polypeptide chain sequentially comprises a VH of the second antigen-binding fragment, the second linker peptide (such as the hinge region of IgG), $CH_2$ and $CH_3$ from the N-terminus to the C-terminus.

34. A protein-drug conjugate, the protein-drug conjugate according to any one of embodiments 1 to 33 and a third polypeptide chain and/or a fourth polypeptide chain.

35. The protein-drug conjugate according to any one of embodiments 1 to 34, further comprising a third antigen-binding fragment and/or a fourth antigen-binding fragment.

36. The protein-drug conjugate according to embodiment 34 or 35, wherein the third antigen-binding fragment and/or the fourth antigen-binding fragment comprise/comprises an antibody or an antigen-binding fragment thereof.

37. The protein-drug conjugate according to any one of embodiments 35 to 36, wherein the first polypeptide chain sequentially comprises the third antigen-binding fragment, the first antigen-binding fragment, the first linker peptide (such as a hinge region of IgG) and the pairing unit from the N-terminus to the C-terminus.

38. The protein-drug conjugate according to any one of embodiments 35 to 37, wherein the second polypeptide chain sequentially comprises the fourth antigen-binding fragment, the second antigen-binding fragment, the second linker peptide (such as the hinge region of IgG) and the pairing unit from the N-terminus to the C-terminus.

39. The protein-drug conjugate according to any one of embodiments 35 to 38, wherein the first polypeptide chain sequentially comprises a VL of the third antigen-binding fragment, a CL of the third antigen-binding fragment, a VH of the first antigen-binding fragment, the first linker peptide (such as the hinge region of IgG), $CH_2$ and $CH_3$ from the N-terminus to the C-terminus.

40. The protein-drug conjugate according to any one of embodiments 35 to 39, wherein the second polypeptide chain sequentially comprises a VL of the fourth antigen-binding fragment, a CL of the fourth antigen-binding fragment, a VH of the second antigen-binding fragment, the second linker peptide (such as the hinge region of IgG), $CH_2$ and $CH_3$ from the N-terminus to the C-terminus.

41. The protein-drug conjugate according to any one of embodiments 35 to 40, wherein the third polypeptide chain sequentially comprises a VH of the third antigen-binding fragment and $CH_1$ of the third antigen-binding fragment from an N-terminus to a C-terminus.

42. The protein-drug conjugate according to any one of embodiments 35 to 41, wherein the fourth polypeptide chain sequentially comprises a VH of the fourth antigen-binding fragment and $CH_1$ of the fourth antigen-binding fragment from an N-terminus to a C-terminus.

43. The protein-drug conjugate according to any one of embodiments 35 to 42, wherein the third antigen-binding fragment and/or the fourth antigen-binding fragment are/is Fab.

44. The protein-drug conjugate according to any one of embodiments 35 to 43, wherein amino acid sequences of the first antigen-binding fragment and the second antigen-binding fragment are identical, and/or amino acid sequences of the third antigen-binding fragment and the fourth antigen-binding fragment are identical.

45. The protein-drug conjugate according to any one of embodiments 35 to 44, wherein amino acid sequences of the first polypeptide chain and the second polypeptide chain are identical, and/or amino acid sequences of the third polypeptide chain and the fourth polypeptide chain are identical.

46. The protein-drug conjugate according to any one of embodiments 1 to 27, wherein the first polypeptide chain sequentially comprises a receptor protein soluble extracellular region of the first antigen-binding fragment, the first linker peptide (such as the hinge region of IgG), $CH_2$ and $CH_3$ from the N-terminus to the C-terminus.

47. The protein-drug conjugate according to embodiment 46, wherein the second polypeptide chain sequentially comprises a receptor protein soluble extracellular region of the second antigen-binding fragment, the second linker peptide (such as the hinge region of IgG), $CH_2$ and $CH_3$ from the N-terminus to the C-terminus.

## Antigen-binding protein

In one aspect, the present application provides an antigen-binding protein.

In the present application, the antigen-binding protein comprises at least two linker peptides, and one or more antigen-binding fragments.

In the present application, the two linker peptides are respectively a first linker peptide and a second linker peptide; and amino acid sequences of the first linker peptide and the second linker peptide may be identical or different.

In the antigen-binding protein of the present application, the first linker peptide can at least comprise a first cysteine Cys1 and a second cysteine Cys2, and the second linker peptide can at least comprise a first cysteine Cys1 and a second cysteine Cys2.

In the antigen-binding protein of the present application, a disulfide bond, known as "disulfide bond Cys1-Cys1", can be formed between the Cys1 of the first linker peptide and the Cys1 of the second linker peptide.

In the antigen-binding protein of the present application, a disulfide bond, known as "disulfide bond Cys2-Cys2", can be formed between the Cys2 of the first linker peptide and the Cys2 of the second linker peptide.

In the antigen-binding protein of the present application, the "disulfide bond Cys1-Cys1" is weaker than the "disulfide bond Cys2-Cys2". In particular, in specific embodiments, the disulfide bond Cys1-Cys1 is more easily cleaved by a reducing agent compared to the disulfide bond Cys2-Cys2.

In the present application, the Cys1 and the Cys2 of the first linker peptide in the antigen-binding protein can be separated by at least 3 (such as 4, 5, 6, 7, 8, 9, 10, 11, 12, 13, 14, 15 or more) amino acids other than cysteine.

In the present application, the Cys1 and the Cys2 of the second linker peptide in the antigen-binding protein can be separated by at least 3 (such as 4, 5, 6, 7, 8, 9, 10, 11, 12, 13, 14, 15 or more) amino acids other than cysteine.

For example, the first linker peptide can comprise an amino acid sequence set forth in Cys1-(X)n-Cys2 from the N-terminus to the C-terminus, wherein n can be an integer greater than or equal to 3 (for example, n may be 3, n may be 4, n may be 5, n may be 6, n may be 7, n may be 8, n may be 9, n may be 10, n may be 11, n may be 12, n may be 13, n may be 14, n may be 15, or n may be an integer greater than 15); and the X refers to any amino acid other than Cys.

For example, the second linker peptide can comprise an amino acid sequence set forth in Cys1-(X)n-Cys2 from the N-terminus to the C-terminus, wherein n can be an integer greater than or equal to 3 (for example, n may be 3, n may be 4, n may be 5, n may be 6, n may be 7, n may be 8, n may be 9, n may be 10, n may be 11, n may be 12, n may be 13, n may be 14, n may be 15, or n may be an integer greater than 15); and the X refers to any amino acid other than Cys.

In the present application, the linker peptide in the antigen-binding protein may be a naturally occurring amino acid sequence comprising at least two cysteines (Cys1 and Cys2), or a derivative sequence obtained by modifying "naturally occurring amino acid sequence comprising at least two cysteines". The "modifying" does not involve changes in the Cys1 and the Cys2 in the naturally occurring amino acid sequence; for example, the modification is adjusting the type or number of amino acids between the Cys1 and the Cys2 in the naturally occurring amino acid sequence, and/or adding flexible linker peptides (such as GSGS and GGGGS) before Cys1. For example, the first linker peptide or the second linker peptide can be derived from a human IgG hinge region (SEQ ID NO: 73), and the Cys1 is a Cys-220 according to EU numbering in the human IgG hinge region.

For example, the first linker peptide can be a human IgG1 hinge region, a sequence of the linker peptide is set forth in SEQ ID NO: 73, the Cys1 in the linker peptide is Cys-220 according to EU numbering in the natural IgG1 hinge region, and the Cys2 is Cys-226 according to EU numbering in the natural IgG1 hinge region. For another example, the first linker peptide can be a derivative sequence derived from the human IgG1 hinge region, an amino acid sequence thereof is a sequence as set forth in any one of SEQ ID NOs: 74-105 and 145-146, the Cys1 in the linker peptide is Cys corresponding to Cys-220 in the human IgG1 hinge region, and the Cys2 in the linker peptide is Cys corresponding to Cys-226 in the human IgG1 hinge region.

For example, the second linker peptide can be a human IgG1 hinge region, a sequence of the linker peptide is set forth in SEQ ID NO: 73, the Cys1 in the linker peptide is Cys-220 according to EU numbering in the natural IgG1 hinge region, and the Cys2 is Cys-226 according to EU numbering in the natural IgG1 hinge region. For another example, the second linker peptide can be a derivative sequence derived from the human IgG1 hinge region, an amino acid sequence thereof is a sequence as set forth in any one of SEQ ID NOs: 74-105 and 145-146, the Cys1 in the linker peptide is Cys corresponding to Cys-220 in the human IgG1 hinge region, and the Cys2 in the linker peptide is Cys corresponding to Cys-226 in the human IgG1 hinge region. For example, the first linker peptide or the second linker peptide can be a mouse IgG2c hinge region (SEQ ID NO: 147), the Cys1 is a first cysteine in the sequence thereof, and the Cys2 is a second cysteine in the sequence thereof.

In the present application, the "more antigen-binding fragments" in the "one or more antigen-binding fragments" may be partially or completely identical or all different; the "identical" refers to the amino acid sequences of two or more antigen-binding fragments being identical, the "different" refers to the amino acid sequences of two or more antigen-binding fragments being different. Two or more antigen-binding fragments can identify different antigens or epitopes, or two or more antigen-binding proteins can identify the identical antigen or identical epitope, but with different sequences and structures.

In the present application, the antigen-binding protein can comprise at least two the antigen-binding fragments. In the present application, the antigen-binding protein can comprise a first antigen-binding fragment and a second antigen-binding fragment (a structure as shown in 0(A)), amino acid sequences of the first antigen-binding fragment and the second antigen-binding fragment may be identical or different, and targets of the first antigen-binding fragment and the second antigen-binding fragment may be identical or different.

In the present application, the antigen-binding protein can further comprise a third antigen-binding fragment and/or a fourth antigen-binding fragment (a structure as shown in 0(B)); the first, second, third and fourth antigen-binding fragments can be partially or completely identical or different.

In the present application, the one or more antigen-binding fragments can be connected to the same linker peptide or to different linker peptides. The antigen-binding fragment can be located at the N-terminus or C-terminus of the linker peptide.

In the present application, the antigen-binding protein comprises a first linker peptide and a second linker peptide, comprising a first antigen-binding fragment and a second antigen-binding fragment. The antigen-binding protein can comprise the following structures:

(a) the C-terminus of the first antigen-binding fragment can be directly or indirectly connected to the N-terminus of the first linker peptide, and the C-terminus of the second antigen-binding fragment can be directly or indirectly connected to the N-terminus of the second linker peptide, with a structure as shown in 0(A);
(b) the N-terminus of the first antigen-binding fragment can be directly or indirectly connected to the C-terminus of the first linker peptide, and the C-terminus of the second antigen-binding fragment can be directly or indirectly connected to the N-terminus of the second linker peptide;
(c) the N-terminus of the first antigen-binding fragment can be directly or indirectly connected to the C-terminus of the first linker peptide, and the N-terminus of the second antigen-binding fragment can be directly or indirectly connected to the C-terminus of the second linker peptide; and

(d) the C-terminus of the first antigen-binding fragment can be directly or indirectly connected to the N-terminus of the first linker peptide, and the N-terminus of the second antigen-binding fragment can be directly or indirectly connected to the C-terminus of the second linker peptide. In the present application, the first antigen-binding fragment and/or the second antigen-binding fragment can refer to any protein functional region that can specifically bind to the antigen, which can be a VHH domain derived from camels or HCAb transgenic mice, or a single chain antibody scFv; or other antigen binding forms (such as receptor protein soluble extracellular regions, ligandins, lipocalins, neuronal cell adhesion molecules (NCAMs), fibronectins, designed ankyrin repeat proteins (DARPins) and other derived protein structures). However, the first antigen-binding fragment and/or the second antigen-binding fragment do/does not comprise a functional group capable of affecting the coupling of drug conjugates and linker peptides at Cys1.

In the present application, the first antigen-binding fragment and/or the second antigen-binding fragment do/does not comprise a functional group capable of affecting the coupling of drug conjugates and linker peptides at Cys1. For example, the first antigen-binding fragment and/or the second antigen-binding fragment do/does not comprise a functional group capable of forming a covalent bond, such as a disulfide bond, with side chain sulfhydryl of the Cys1 of the first linker peptide and/or the second linker peptide. For example, in certain specific embodiments, the first antigen-binding fragment and/or the second antigen-binding fragment do/does not comprise a light chain or light chain fragment comprising a constant region Cys-214 (Eu numbering).

In the present application, the antigen-binding protein can further comprise a third antigen-binding fragment and/or a fourth antigen-binding fragment. The third antigen-binding fragment and/or fourth antigen-binding fragment further comprised are/is further connected to the N-terminus or C-terminus of the antigen-binding protein in (a) or (b) or (c) or (d) described above: For example, the C-terminus of the third antigen-binding fragment can be directly or indirectly connected to the N-terminus of the first antigen-binding fragment, and the C-terminus of the first antigen-binding fragment can be connected to the N-terminus of the first linker peptide; and/or the C-terminus of the fourth antigen-binding fragment can be directly or indirectly connected to the N-terminus of the second antigen-binding fragment, and the C-terminus of the second antigen-binding fragment can be connected to the N-terminus of the second linker peptide, with a structure as shown in 0(B).

In the structure of the antigen-binding protein of the present application, the third antigen-binding fragment and/or the fourth antigen-binding fragment can comprise any protein functional region that can specifically bind to the antigen, which can be "Fab", or "VH", or a single chain antibody scFv; or other antigen binding forms (such as receptor protein soluble extracellular regions, ligandins, lipocalins, neuronal cell adhesion molecules (NCAMs), fibronectins, designed ankyrin repeat proteins (DARPins) and other derived protein structures). In the present application, the antigen-binding protein can further comprise a pairing unit enabling the first linker peptide and the second linker peptide to be paired, the pairing unit can comprise a first pairing subunit and a second pairing subunit, and the first pairing subunit can interact with the second pairing subunit through a covalent bond or a non-covalent bond.

In certain embodiments, "pairing unit" may be in a dimer form of an antibody Fc fragment or a variant thereof.

In certain embodiments, "pairing unit" may be in an asymmetric dimer form. For example, the first pairing subunit is an Fc fragment monomer with a "knob" mutation (T366W), and the second pairing subunit is an Fc fragment monomer with a "hole" mutation (T366S/L368A/Y407V).

In certain embodiments, "pairing unit" may be in an asymmetric dimer form. For example, the first pairing subunit is an $\alpha$ chain of CD8, and the second pairing subunit is a $\beta$ chain of CD8.

In certain embodiments, "pairing unit" may be in an asymmetric dimer form. For example, the first pairing subunit is an $\alpha$ chain of CD79, and the second pairing subunit is a $\beta$ chain of CD79. In certain embodiments, "pairing unit" may be in an asymmetric dimer form. For example, the first pairing subunit is an $\alpha$ chain of a T-cell receptor, and the second pairing subunit is a $\beta$ chain of a T-cell receptor.

In certain embodiments, an N-terminus of the first pairing subunit can be connected to a C-terminus of the first linker peptide, and an N-terminus of the second pairing subunit is connected to a C-terminus of the second linker peptide; or the N-terminus of the first pairing subunit can be connected to the C-terminus of the second linker peptide, and the N-terminus of the second pairing subunit is connected to the C-terminus of the first linker peptide.

In certain specific embodiments, the present application provides an antigen-binding protein with a structure as shown in 0(A), having the following structural features:

the antigen-binding protein can comprise a first linker peptide and a second linker peptide, a first antigen-binding fragment and a second antigen-binding fragment, as well as a pairing unit;

amino acid sequences of the first linker peptide and the second linker peptide may be identical or different;

targets of the first antigen-binding fragment and the second antigen-binding fragment may be identical or different;

amino acid sequences of the first antigen-binding fragment and the second antigen-binding fragment may be identical or different;

the pairing unit may be a homologous or heterologous dimer of an Fc fragment;

the C-terminus of the first antigen-binding fragment can be directly or indirectly connected to the N-terminus of the first linker peptide, and the C-terminus of the second antigen-binding fragment can be directly or indirectly connected to the N-terminus of the second linker peptide; the C-terminuses of the first linker peptide and the second linker peptide can be respectively connected to the N-terminus of the Fc fragment;

the first linker peptide can comprise at least two cysteines Cys1 and Cys2, and the Cys1 and the Cys2 are separated by at least 3 amino acids other than Cys;

the second linker peptide can comprise at least two cysteines Cys1 and Cys2, and the Cys1 and the Cys2 are separated by at least 3 amino acids other than Cys;

a disulfide bond can be formed between the Cys1 of the first linker peptide and the Cys1 of the second linker peptide;

a disulfide bond can be formed between the Cys1 of the first linker peptide and the Cys2 of the second linker peptide;

the first linker peptide and/or the second linker peptide can be derived from a human IgG1 hinge region sequence (SEQ ID NO: 73) or a derivative sequence thereof (SEQ ID NOs: 74-105 and 145-146), or derived from a mouse IgG2c hinge region sequence (SEQ ID NO: 147);

the first antigen-binding fragment and/or the second antigen-binding fragment can refer to any protein functional region that can specifically bind to the antigen, which can be a VHH domain derived from camels or HCAb transgenic mice, or a single chain antibody scFv; or other antigen binding forms (such as receptor protein soluble extracellular regions, ligandins, lipocalins, neuronal cell adhesion molecules (NCAMs), fibronectins, designed ankyrin repeat proteins (DARPins) and other derived protein structures);

the first antigen-binding fragment and the second antigen-binding fragment do not comprise a functional group capable of affecting the coupling of the drug conjugate and the linker peptide at Cys1; for example, they do not comprise a functional group capable of forming a covalent bond with side chain sulfhydryl of the Cys1 in the first linker peptide and the second linker peptide; for example, they do not comprise a functional group capable of forming a disulfide bond with side chain sulfhydryl of the Cys1 in the first linker peptide and the second linker peptide; for example, they do not comprise a light chain or a light chain fragment comprising a constant region Cys-214 (Eu numbering);

in certain embodiments, the one or more antigen-binding fragments do not comprise a functional group capable of affecting the coupling of the drug conjugate with the first linker peptide and/or the second linker peptide at the Cys1, or a conjugate formed by the functional group and other drug conjugates, the affection is manifested in significantly reducing a coupling probability of the drug conjugate at a Cys1 site, and the significant reduction can include one or more of the following:

1) significantly reducing the probability of coupling the Cys1 with the drug conjugate;
2) when the functional group or the conjugate is present, the probability of coupling the Cys1 of the linker peptide with the drug conjugate may be reduced to 50%, 40%, 30%, 20%, 10%, or lower;
3) when the functional group or the conjugate is present, the purity of the prepared protein-drug conjugate is less than 50%, for example, less than 40%, 30%, 20%, 10%, or lower, and
4) when the functional group or the conjugate is present, the functional group or the conjugate interacts with the Cys1, so that the Cys1 cannot be coupled with the drug conjugate, or the Cys1 cannot be coupled with the drug conjugate in the method in the present application;

in certain embodiments, the one or more antigen-binding fragments may not comprise a functional group capable of forming a disulfide bond with the Cys1 of the first linker peptide and/or the Cys1 of the second linker peptide, or a conjugate formed by the functional group and other drug conjugates; and

in certain embodiments, the one or more antigen-binding fragments may not comprise a cysteine capable of forming a disulfide bond with the Cys1 of the first linker peptide and/or the Cys1 of the second linker peptide, or a conjugate formed by the cysteine and other drug conjugates.

In certain specific embodiments, the present application further provides an antigen-binding protein with a structure as shown in 0(B), having the following structural features:

having the structural features of the antigen-binding protein with the structure as shown in 0(A);

further comprising a third antigen-binding fragment and a fourth antigen-binding fragment on the basis of the antigen-binding protein with the structure as shown in 0(A);

the C-terminus of the third antigen-binding fragment can be directly or indirectly connected to the N-terminus of the first antigen-binding fragment, and the C-terminus of the first antigen-binding fragment can be connected to the N-terminus of the first linker peptide;

the C-terminus of the fourth antigen-binding fragment can be directly or indirectly connected to the N-terminus of the second antigen-binding fragment, and the C-terminus of the second antigen-binding fragment can be connected to the N-terminus of the second linker peptide;

the first antigen-binding fragment, the second antigen-binding fragment, the third antigen-binding fragment and/or the fourth antigen-binding fragment may be partially or completely identical or different; for example, the first and second antigen-binding fragments are identical, the third and fourth antigen-binding fragments are identical, the first and third antigen-binding fragments are different, and the second and fourth antigen-binding fragments are different;

the third antigen-binding fragment and/or the fourth antigen-binding fragment can comprise any protein functional region that can specifically bind to the antigen, which can be "Fab", or "VH", or a single chain antibody scFv; or other antigen binding forms (such as receptor protein soluble extracellular regions, ligandins, lipocalins, neuronal cell adhesion molecules (NCAMs), fibronectins, designed ankyrin repeat proteins (DARPins) and other derived protein structures); the third antigen-binding fragment and/or the fourth antigen-binding fragment also do/does not comprise a functional group capable of affecting the coupling of the drug conjugate and the linker peptide at Cys1 or a conjugate formed by the functional group and other drug conjugates; for example, they do not comprise a functional group capable of forming a disulfide bond with side chain sulfhydryl of the Cys1 in the first linker peptide and the second linker peptide;

the third antigen-binding fragment and/or the fourth antigen-binding fragment do/does not comprise a functional group capable of forming a disulfide bond with the Cys1 of the first linker peptide and/or the Cys1 of the second linker peptide, or a conjugate formed by the functional group and other drug conjugates; and

the third antigen-binding fragment and/or the fourth antigen-binding fragment do/does not comprise a cysteine capable of forming a disulfide bond with the Cys1 of the first linker peptide and/or the Cys1 of the second linker peptide, or a conjugate formed by the cysteine and other drug conjugates.

The antigen-binding protein of the present application can target a specific antigen.

The antigen-binding protein can comprise one or more antigen-binding fragments, which can bind to a specific antigens on a target cell. For example, the specific antigen on the target cell can be a tumor antigen. For another example, the tumor antigen can be CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG (TMPRSS2 ETS fusion gene), NA17, PAX3, androgen receptor (AR), Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, Cadherin17, CD32b, EGFRvIII, GPNMB, GPR64, HER3, LRP6, LYPD8, NKG2D, SLC34A2, SLC39A6, SLITRK6, GUCY2C, 5T4 and/or TACSTD2, etc.

For example, the tumor antigen may be MSLN; for example, the tumor antigen may be BCMA; for example, the tumor antigen may be 5T4; for example, the tumor antigen may be ROR1.

In certain embodiments, the isolated antigen-binding protein can comprise an antibody or an antigen-binding fragment thereof. For example, the antigen-binding fragment can comprise an antibody heavy chain variable region VH; for another example, the antigen-binding fragment can comprise a single chain antibody variable region scFv; for another example, the antigen-binding fragment can comprise a receptor protein soluble extracellular region fusion protein. For example, the antigen-binding protein can comprise an antibody targeting CTLA4 or an antigen-binding fragment thereof, an antibody targeting MSLN or an antigen-binding fragment thereof, an antibody targeting BCMA or an antigen-binding fragment thereof, an antibody targeting 5T4 or an antigen-binding fragment thereof, an antibody targeting ROR1 or an antigen-binding fragment thereof and/or a soluble fusion protein derived from SIRP$\alpha$ extracellular region targeting CD47.

In the present application, the CTLA4 antigen-binding fragment can comprise a heavy chain variable region; the heavy chain variable region VH comprises HCDR1, HCDR2 and HCDR3, which are amino acid sequences set forth in SEQ ID NOs: 7, 22 and 38, respectively. The amino acid sequences of the listed CDRs are shown according to the Chothia scheme. For example, the heavy chain variable region VH can comprise an amino acid sequence set forth in SEQ ID NO: 55. For another example, the CTLA4 antigen-binding protein can comprise a polypeptide chain, as an amino acid sequence set forth in SEQ ID NO: 64.

In the present application, the CTLA4 antigen-binding fragment can comprise a heavy chain variable region; the heavy chain variable region VH can comprise HCDR1, HCDR2 and HCDR3, which are amino acid sequences set forth in SEQ ID NOs: 8, 23 and 39, respectively. The amino acid sequences of the listed CDRs are shown according to the Chothia scheme. For example, the heavy chain variable region VH can comprise an amino acid sequence set forth in SEQ ID NO: 56. For another example, the CTLA4 antigen-binding protein can comprise a polypeptide chain, as an amino acid sequence set forth in SEQ ID NO: 65.

In the present application, the MSLN antigen-binding fragment can comprise a heavy chain variable region; the heavy chain variable region VH can comprise HCDR1, HCDR2 and HCDR3, which are amino acid sequences set forth in SEQ ID NOs: 10, 25 and 41, respectively. The amino acid sequences of the listed CDRs are shown according to the Chothia scheme. For example, the heavy chain variable region VH can comprise an amino acid sequence set forth in SEQ ID NO: 58. For another example, the MSLN antigen-binding protein can comprise a polypeptide chain, as an amino acid sequence set forth in SEQ ID NO: 67.

In the present application, the 5T4 antigen-binding fragment can comprise a heavy chain variable region; the heavy chain variable region VH can comprise HCDR1, HCDR2 and HCDR3, which are amino acid sequences set forth in SEQ ID NOs: 13, 28 and 44, respectively. The amino acid sequences of the listed CDRs are shown according to the Chothia scheme. For example, the heavy chain variable region VH can comprise an amino acid sequence set forth in SEQ ID NO: 61. For another example, the 5T4 antigen-binding protein can comprise a polypeptide chain, as an amino acid sequence set forth in SEQ ID NO: 69.

In the present application, the BCMA antigen-binding fragment can comprise a heavy chain variable region; the heavy chain variable region VH can comprise HCDR1, HCDR2 and HCDR3, which are amino acid sequences set forth in SEQ ID NOs: 9, 24 and 40, respectively. The amino acid sequences of the listed CDRs are shown according to the Chothia scheme. For example, the heavy chain variable region VH can comprise an amino acid sequence set forth in SEQ ID NO: 57. For another example, the BCMA antigen-binding protein can comprise a polypeptide chain, as an amino acid sequence set forth in SEQ ID NO: 66.

In the present application, the BCMA antigen-binding fragment can comprise a heavy chain variable region; the heavy chain variable region VH can comprise HCDR1, HCDR2 and HCDR3, which are amino acid sequences set forth in SEQ ID NOs: 11, 26 and 42, respectively. The amino acid sequences of the listed CDRs are shown according to the Chothia scheme. For example, the heavy chain variable region VH can comprise an amino acid sequence set forth in SEQ ID NO: 59. For another example, the BCMA antigen-binding protein can comprise a polypeptide chain, as an amino acid sequence set forth in SEQ ID NO: 68.

In the present application, the BCMA antigen-binding fragment can comprise a heavy chain variable region; the heavy chain variable region VH can comprise HCDR1, HCDR2 and HCDR3, which are amino acid sequences set forth in SEQ ID NOs: 14, 29 and 42, respectively. The amino acid sequences of the listed CDRs are shown according to the Chothia scheme. For example, the heavy chain variable region VH can comprise an amino acid sequence set forth in SEQ ID NO: 62. For another example, the BCMA antigen-binding protein can comprise a polypeptide chain, as an amino acid sequence set forth in SEQ ID NO: 70. For another example, the BCMA antigen-binding protein can comprise a polypeptide chain, as an amino acid sequence set forth in any one of SEQ ID NOs: 106-129 and 148.

In the present application, the ROR1 antigen-binding fragment can comprise a light chain variable region and a heavy chain variable region; the light chain variable region VL can comprise LCDR1, LCDR2 and LCDR3, which are amino acid sequences set forth in SEQ ID NOs: 49, 51 and 53, respectively; the heavy chain variable region VH can comprise HCDR1, HCDR2 and HCDR3, which are amino acid sequences set forth in SEQ ID NOs: 12, 27 and 43, respectively. The amino acid sequences of the listed CDRs are shown according to the Chothia scheme. For example, the light chain variable region VL can comprise an amino acid sequence set forth in SEQ ID NO: 63, and the heavy chain variable region VH can comprise an amino acid sequence set forth in SEQ ID NO: 60. For another example, the ROR1 antigen-binding protein can comprise a polypeptide chain, as an amino acid sequence set forth in SEQ ID NO: 71.

In the present application, the CD47 antigen-binding protein can comprise a polypeptide chain, as an amino acid sequence set forth in SEQ ID NO: 149.

In the present application, the antigen-binding protein can further comprise heavy chain constant regions CH2 and CH3 as well as a hinge region, a sequence of the heavy chain constant regions (comprising a hinge region) comprises an amino acid sequence set forth in SEQ ID NO: 72, and a sequence of the hinge region is preferably a human IgG1 antibody heavy chain hinge region sequence (SEQ ID NO: 73).

In the present application, the antigen-binding protein can further have a symmetrical structure as shown in 0, comprising four antigen-binding fragments and four polypeptide chains; the antigen-binding protein comprises an antigen-binding fragment A and an antigen-binding fragment B; the antigen-binding fragment A and the antigen-binding fragment B target different antigens or antigenic epitopes; the antigen-binding fragment A is an Fab structure, and the antigen-binding fragment B is a VH structure; in the antigen-binding protein, the number of the antigen-binding fragment A is two, and the number of the antigen-binding fragment B is two; the antigen-binding protein sequentially comprises the antigen-binding fragment A, the antigen-binding fragment B and Fc from the N-terminus to the C-terminus, wherein the antigen-binding fragment A is directly connected to the antigen-binding fragment B, and the antigen-binding fragment B is connected to the Fc through a linker peptide (or a hinge region). The antigen-binding protein has four polypeptide chains, which comprise a first polypeptide chain, a second polypeptide chain, a third polypeptide chain and a fourth polypeptide chain; the first polypeptide chain and the second polypeptide chain have the same amino acid sequence, referred to as "long chain"; the third polypeptide chain and the fourth polypeptide chain have the same amino acid sequence, referred to as "short chain"; the short chain sequentially comprises VH_A-CH1 from the N-terminus to the C-terminus, and the long chain sequentially comprises VL_A-CL-VH_B-L-CH2-CH3 from the N-terminus to the C-terminus; wherein VH_ A and VL_ A are the heavy chain variable region and the light chain variable region of the antigen-binding fragment A respectively, VH_B is the heavy chain variable region of the antigen-binding fragment B, and the L is a linker peptide. In certain embodiments, the linker peptide has an amino acid sequence set forth in SEQ ID NO: 73. In certain embodiments, the antigen-binding fragment A comprises a light chain variable region and a heavy chain variable region, the light chain variable region VL comprises an amino acid sequence set forth in SEQ ID NO: 131, and the heavy chain variable region VH comprises an amino acid sequence set forth in SEQ ID NO: 130; the antigen-binding fragment B comprises a heavy chain variable region, and the heavy chain variable region VH comprises an amino acid sequence set forth in SEQ ID NO: 62. In certain embodiments, the antigen-binding protein comprises two same "short chains" and two same "long chains" (0), the "short chains" comprise an amino acid sequence set forth in SEQ ID NO: 134, and the "long chains" comprise an amino acid sequence set forth in SEQ ID NO: 135.

In the present application, the CDRs may comprise mutations based on the defined sequences. The mutation is an insertion, deletion or substitution of 3, 2 or 1 amino acids on the basis of the amino acid sequences of the VH CDR1, the VH CDR2, the VH CDR3, the VL CDR1, the VL CDR2 and the VL CDR3. In the present application, "amino acid mutation" in the context like "insertion, deletion or substitution of 3, 2 or 1 amino acids" refers to a mutation of an amino acid in the sequence of a variant as compared to the sequence of an original amino acid, including the insertion, deletion or substitution of amino acids on the basis of the original amino acid sequence. An exemplary explanation is that the mutations to the CDRs may comprise 3, 2 or 1 amino acid mutations, and that the same or different numbers of amino acid residues can be optionally selected for the mutations to those CDRs, e.g., 1 amino acid mutation to CDR1, and no amino acid mutation to CDR2 and CDR3.

In the present application, the VH and VL or the polypeptide chain may comprise mutations based on the defined sequences. The mutation is a deletion, substitution or addition of one or more amino acid residues on the defined amino acid sequence, and the amino acid sequence with the mutation has at least 85% sequence homology to the defined amino acid sequence and maintains or improves the binding activity of the antibody or the antigen-binding fragment thereof and the binding protein, wherein the at least 85% sequence homology is preferably at least 90% sequence homology, more preferably at least 95% sequence homology, and most preferably at least 99% sequence homology.

In the present application, the homology generally refers to similarity, likeness or association between two or more sequences. The "percent sequence homology" can be calculated by the following steps: comparing two sequences to be aligned in a comparison window; determining the number of positions at which nucleic acid bases (e.g., A, T, C and G) or amino acid residues (e.g., Ala, Pro, Ser, Thr, Gly, Val, Leu, Ile, Phe, Tyr, Trp, Lys, Arg, His, Asp, Glu, Asn, Gln, Cys and Met) are identical in the two sequences to give the number of matched positions; dividing the number of matched positions by the total number of positions in the comparison window (i.e., the window size); and multiplying the result by 100 to give a percent sequence homology. Alignment for determining the percent sequence homology can be achieved in a variety of ways known in the art, for example, using publicly available computer software such as BLAST, BLAST-2, ALIGN or Megalign (DNASTAR) software. Those skilled in the art can determine suitable parameters for alignment of the sequences, including any algorithms necessary to achieve optimal alignment in a full-length sequence range or target sequence region being compared. The homology can also be determined by the following methods: FASTAand BLAST. For description of the FASTA algorithm, see W. R. Pearson and D. J. Lipman, "Improved Tools for Biological Sequence Comparison", Proc. Natl. Acad. Sci. USA, 85: 2444-2448, 1988; and D. J. Lipman and W. R. Pearson, "Rapid and Sensitive Protein Similarity Searches", Science, 227: 1435-1441, 1989. For description of the BLAST algorithm, see S. Altschul, W. Gish, W. Miller, E. W. Myers and D. Lipman, "A Basic Local Alignment Search Tool", Journal of Molecular Biology, 215: 403-410, 1990.

## Drug conjugate

The protein-drug conjugate in the present application can comprise at least one drug conjugate; the drug conjugate can be linked to a protein moiety in the protein-drug conjugate in a covalent binding form.

The drug conjugate can comprise at least one loaded drug. The loaded drug may comprise an active drug ingredient and/or a labeled molecule.

In the present application, the loaded drug is the form of a class of small molecule compounds or toxins or other drug molecules with pharmaceutical activity, which can be, but are not limited to, small molecule compounds, toxin molecules, antibiotics, oligonucleotides, proteolysis targeting chimeras (PROTACs), affinity ligands, fluorescent groups, nuclide groups, polypeptides, immunomodulatory molecules (e.g., toll-like receptor agonist molecules, STING agonist molecules), etc. A variety of loaded drugs are known in the art. For example, the small molecule compounds generally refer to a class of substances having strong cytotoxicity. Exemplary small molecule compounds can exert such cytotoxic and cytostatic effects by mechanisms including, but not limited to, tubulin binding, DNA binding, inhibition of RNA polymerase, protein synthesis or inhibiting topoisomerase. For example, the small molecule compounds may be a tubulin inhibitor; for example, the tubulin inhibitor may be maytansines (such as DM1 or DM4), auristatins (such as MMAE or MMAF), and the like. For example, the small molecule compound may be a DNA damaging agent; for example, the DNA damaging agent may be calicheamicins, pyrrolobenzodiazepine (PBD), and the like. For example, the proteolysis targeting chimeras (PROTACs) are a class of compounds that can cause the degradation of a target protein by inducing polyubiquitination of the target protein; for example, the PROTAC may be a BET protein degrading agent.

In the present application, the drug conjugate may further comprise at least one linker. For example, the linker may comprise a cleavable linker or a non-cleavable linker. The linker is used to link one or more loaded drugs to antigen-binding proteins. In the present application, the cleavable linker may be a "cleavable" linker favoring the release of drugs. For example, the cleavable linker may include, but is not limited to, an acid-sensitive linker, a protease-sensitive linker, a light-sensitive linker or a disulfide-containing linker.

In the present application, the linker can have a functional group capable of reacting with free cysteine present on the antigen-binding protein to form a covalent bond. For example, such reactive functional groups may include, but are not limited to, maleimide, haloacetamide, $\alpha$-haloacetyl, activated esters such as succinimidyl ester, 4-nitrophenyl ester, pentafluorophenyl ester, tetrafluorophenyl ester, anhydride, acyl chloride, sulfonyl chloride, isocyanate and isothiocyanate. For example, the linker can have a functional group capable of reacting with an electrophilic group present on the antigen-binding protein. For example, such reactive functional groups may include, but are not limited to, hydrazide, oxime, amino, hydrazine, thiosemicarbazone, hydrazine carboxylate and arylhydrazide.

In the present application, the linker may comprise one or more linker members. A variety of linker members are known in the art, such as maleimidocaproyl ("MC"), maleimidopropionyl ("MP"), valine-citrulline ("val-cit" or "vc") and *p*-aminobenzyloxycarbonyl ("PAB").

In the present application, the drug conjugate is mc-vc-PAB-MMAE (also known as VcMMAE, with a structure as shown in 0, CAS No.: 646502-53-6), which comprises a linker mc-vc-PAB and a loaded drug MMAE.

In the present application, the drug conjugate may further be a BRD4 protein degrading agent (with a structure as shown in 0), which comprises a PROTAC GNE-987 targeting BET and a linker comprising a disulfide.

## Protein-drug conjugate

In another aspect, the present application provides a protein-drug conjugate.

In the present application, the protein-drug conjugate comprises at least one antigen-binding protein moiety and at least one drug conjugate moiety.

In the present application, the antigen-binding protein moiety is an antigen-binding protein that targets a specific antigen and comprises one or more antigen-binding fragments and at least two linker peptides, as described in previous "antigen-binding protein".

In the present application, the drug conjugate comprises at least one loaded drug. The loaded drug can comprise, but is not limited to, a small molecule compound, a toxin molecule, an antibiotic, an oligonucleotide, a proteolysis targeting chimera (PROTAC), an affinity ligand, a fluorescently-labeled groups, a nuclide-labeled group, a polypeptide, an immunomodulatory molecule (e.g., a toll-like receptor agonist), etc.

In the present application, the drug conjugate may further comprise at least one linker.

In the present application, the loaded drug can be coupled to the antigen-binding protein in a covalent bond binding manner through the linker; in the present application, the loaded drug can be coupled to the linker peptide in the antigen-binding protein in a covalent bond binding manner through the linker.

In the present application, the drug conjugate can be linked to the antigen-binding protein through the Cys1 of the first linker peptide and/or the Cys1 of the second linker peptide, thereby producing the protein-drug conjugate.

In the present application, covalent binding with the drug conjugate at Cys1 is achieved by cleaving the disulfide bond between the Cys1 of the first linker peptide and the second linker peptide of the antigen-binding protein to form a reactive sulfhydryl functional group.

In the present application, the one or more antigen-binding fragments do not comprise a functional group capable of affecting the coupling of the drug conjugate and the linker peptide at Cys1; for example, they do not comprise a functional group capable of forming a covalent bond with side chain sulfhydryl of the Cys1 in the linker peptide; for example, they do not comprise a functional group capable of forming a disulfide bond with side chain sulfhydryl of the Cys1 in the linker peptide.

In the present application, the linkage between the drug conjugate and the antigen-binding protein mainly occurs at the Cys1 site of the first linker peptide and/or the second linker peptide, and almost does not occur at other cysteine sites (such as the Cys2 site). For example, the probability of binding the Cys1 of the linker peptide to the drug conjugate is at least 50%, may also be at least 55%, may also be at least 60%, may also be at least 65%, may also be at least 70%, may also be at least 75%, may also be at least 80%, may also be at least 85%, may also be at least 90%, may also be at least 95%, or may also be at least 99%. For example, the probability of binding the Cys2 of the linker peptide to the drug conjugate is at most 50%, may also be at most 45%, may also be at most 40%, may also be at most 35%, may also be at most 30%, may also be at most 25%, may also be at most 20%, may also be at most 15%, may also be at most 10%, or may also be at most 5%.

In the present application, the drug conjugate in the present application is linked by using the Cys1 in the first linker peptide and/or the second linker peptide as a coupling site, so that a protein-drug conjugate with a uniform DAR value can be generated. In certain specific embodiments, a uniform coupling product comprising two drug conjugates (i.e., CAR = 2; when one unit of the drug conjugate only comprises one loaded drug, it is also DAR = 2, also known as D2) is generated. In certain specific embodiments, the protein-drug conjugate molecule with CAR = 2 (or DAR = 2, also known as D2) in the coupling product serves as a main component, and the content of the component with CAR = 2 (or DAR = 2, also known as D2) is at least 50%, may also be at least 55%, may also be at least 60%, may also be at least 65%, may also be at least 70%, may also be at least 75%, may also be at least 80%, may also be at least 85%, may also be at least 90%, or may also be at least 95%. In certain specific embodiments, the coupling product comprises more than 80% of an component with CAR = 2 (or DAR = 2, also known as D2), and the coupling mainly occurs at the Cys1 in the first linker peptide and the second linker peptide; without the step of further purification, a protein-drug conjugate with a uniform DAR value can be obtained.

In the present application, the drug conjugate in the present application is linked by using the cysteine Cys-220 (Eu numbering) in the natural sequence (SEQ ID NO: 73) of the human IgG1 antibody heavy chain hinge region as a coupling site, so that a protein-drug conjugate with a uniform DAR value can be generated. In certain specific embodiments, a uniform coupling product comprising two drug conjugates (i.e., CAR = 2; when one unit of the drug conjugate only comprises one loaded drug, it is also DAR = 2, also known as D2) is generated. In certain specific embodiments, the protein-drug conjugate molecule with CAR = 2 (or DAR = 2, also known as D2) in the coupling product serves as a main component, and the content of the component with CAR = 2 (or DAR = 2, also known as D2) is at least 50%, may also be at least 55%, may also be at least 60%, may also be at least 65%, may also be at least 70%, may also be at least 75%, may also be at least 80%, may also be at least 85%, may also be at least 90%, or may also be at least 95%. In certain specific embodiments, the coupling product comprises more than 80% of an component with CAR = 2 (or DAR = 2, also known as D2), and the coupling mainly occurs at the Cys-220 (Eu numbering) in the human IgG1 hinge region; without the step of further purification, a protein-drug conjugate with a uniform DAR value can be obtained.

In the present application, the protein-drug conjugate may have a structure as shown in 0 or 0.

In the present application, the protein-drug conjugate may be an antibody-drug conjugate (ADC). The present application further provides a protein-drug conjugate with a structure shown in 0 (A) obtained by drug coupling with an antigen-binding protein with a structure shown in 0 (A), having the following structural features:

having the structural features of the antigen-binding protein with the structure as shown in 0(A);
the protein-drug conjugate includes a first linker peptide and a second linker peptide, as well as a first antigen-binding fragment and a second antigen-binding fragment;
amino acid sequences of the first linker peptide and the second linker peptide may be identical or different;
targets of the first antigen-binding fragment and the second antigen-binding fragment may be identical or different;
amino acid sequences of the first antigen-binding fragment and the second antigen-binding fragment may be identical or different;
the protein-drug conjugate further includes a pairing unit, such as an Fc fragment;
the first linker peptide and the second linker peptide include at least two cysteines, named Cys 1 and Cys2 from an N-terminus to a C-terminus, respectively;
sequences of the first linker peptide and/or the second linker peptide may be a human IgG1 hinge region sequence (SEQ ID NO: 73) or a derivative sequence thereof (SEQ ID NOs: 74-105, 145-146), and the Cys1 of the first linker peptide and/or the second linker peptide may be Cys-220 (Eu numbering) of the human IgG1 hinge region sequence or Cys in the derivative sequence corresponding to the Cys-220 (Eu numbering) of the human IgG1 hinge region sequence; and the Cys2 may be Cys-226 (Eu numbering) of the human IgG1 hinge region sequence or Cys in the derivative sequence corresponding to the Cys-226 (Eu numbering) of the human IgG1 hinge region sequence;
the sequences of the first linker peptide and/or the second linker peptide may further be a mouse IgG2c hinge region sequence (SEQ ID NO: 147) or a derivative sequence thereof, the Cys1 of the first linker peptide and/or the second linker peptide may be a first cysteine of the mouse IgG2c hinge region sequence or the derivative sequence thereof, and the Cys2 may be a second cysteine of the mouse IgG2c hinge region sequence or the derivative sequence thereof;
during preparation of the protein-drug conjugate, a disulfide bond (i.e., "disulfide bond Cys2-Cys2" of the antigen-binding protein with the structure shown in 0 (A)) between the Cys2 of the first linker peptide and the Cys2 of the second linker peptide is retained;
during preparation of the protein-drug conjugate, a disulfide bond (i.e., "disulfide bond Cys1-Cys1" of the antigen-binding protein with the structure shown in 0(A)) between the Cys1 of the first linker peptide and the Cys1 of the second linker peptide is cleaved for coupling of the drug conjugate;
the first antigen-binding fragment and/or the second antigen-binding fragment do/does not affect the coupling of the drug conjugate at the Cys1 site of the first linker peptide and/or the second linker peptide;
the first antigen-binding fragment and/or the second antigen-binding fragment do/does not include a functional group capable of affecting the coupling of the drug conjugate on the Cys1 site of the first linker peptide and/or the second linker peptide;
the first antigen-binding fragment and/or the second antigen-binding fragment do/does not include a functional group capable of forming a covalent bond with side chain sulfhydryl of the Cys1; and
the first antigen-binding fragment and/or the second antigen-binding fragment do/does not include a functional group capable of forming a disulfide bond with the Cys1.

The present application further provides a protein-drug conjugate with a structure shown in 0 (B) obtained by drug coupling with an antigen-binding protein with a structure shown in 0 (B), having the following structural features:

the structural features of the antigen-binding protein with the structure as shown in 0 (B); the protein-drug conjugate includes a first linker peptide and a second linker peptide, as well as a first antigen-binding fragment, a second antigen-binding fragment, a third antigen-binding fragment and a fourth antigen-binding fragment;
amino acid sequences of the first linker peptide and the second linker peptide may be identical or different;
the first antigen-binding fragment, the second antigen-binding fragment, the third antigen-binding fragment and/or the fourth antigen-binding fragment may be partially or completely identical or different; for example, the first and second antigen-binding fragments are identical, the third and fourth antigen-binding fragments are identical, the first and third antigen-binding fragments are different, and the second and fourth antigen-binding fragments are different;
the identical or different parts may be targets and/or amino acid sequences binding to the antigen-binding fragments;
the protein-drug conjugate further includes a pairing unit, such as an Fc fragment;
the first linker peptide and the second linker peptide include at least two cysteines, named Cys 1 and Cys2 from an N-terminus to a C-terminus, respectively;
sequences of the first linker peptide and/or the second linker peptide may be a human IgG1 hinge region sequence (SEQ ID NO: 73) or a derivative sequence thereof (SEQ ID NOs: 74-105, 145-146), and the Cys1 of the first linker peptide and/or the second linker peptide may be Cys-220 (Eu numbering) of the human IgG1 hinge region sequence or Cys in the derivative sequence corresponding to the Cys-220 (Eu numbering) of the human IgG1 hinge region sequence; and the Cys2 may be Cys-226 (Eu numbering) of the human IgG1 hinge region sequence or Cys in the derivative sequence corresponding to the Cys-226 (Eu numbering) of the human IgG1 hinge region sequence;
the sequences of the first linker peptide and/or the second linker peptide may further be a mouse IgG2c hinge region sequence (SEQ ID NO: 147) or a derivative sequence thereof, the Cys1 of the first linker peptide and/or the second linker peptide may be a first cysteine of the mouse IgG2c hinge region sequence or the derivative sequence thereof, and the Cys2 may be a second cysteine of the mouse IgG2c hinge region sequence or the derivative sequence thereof;
during preparation of the protein-drug conjugate, a disulfide bond (i.e., "disulfide bond Cys2-Cys2" of the antigen-binding protein with the structure shown in 0 (B)) between the Cys2 of the first linker peptide and the Cys2 of the second linker peptide is retained;
during preparation of the protein-drug conjugate, a disulfide bond (i.e., "disulfide bond Cys1-Cys1" of the antigen-binding protein with the structure shown in 0 (B)) between the Cys1 of the first linker peptide and the Cys1 of the second linker peptide is cleaved for coupling of the drug conjugate;
the first antigen-binding fragment and/or the second antigen-binding fragment and/or the third antigen-binding fragment and/or the fourth antigen-binding fragment do/does not include a functional group capable of affecting the site-specific conjugation of the drug conjugate with the linker peptides at the Cys1 site;
the first antigen-binding fragment and/or the second antigen-binding fragment and/or the third antigen-binding fragment and/or the fourth antigen-binding fragment do/does not include a functional group capable of forming a covalent bond with side chain sulfhydryl of the Cys1; and the first antigen-binding fragment and/or the second antigen-binding fragment and/or the third antigen-binding fragment and/or the fourth antigen-binding fragment do/does not include a functional group capable of forming a disulfide bond with side chain sulfhydryl of the Cys1.

The present application further provides a protein-drug conjugate.

In certain cases, the drug conjugate may include a first polypeptide chain and a second polypeptide chain. The first polypeptide chain may sequentially include a first antigen-binding fragment, a first linker peptide, and a first pairing subunit from an N-terminus to a C-terminus. The second polypeptide chain may sequentially include a second antigen-binding fragment, a second linker peptide, and a second pairing subunit from an N-terminus to a C-terminus. In particular, the first pairing subunit and the second pairing subunit can interact to form a dimer. For example, the first polypeptide chain may sequentially include VH of the first antigen-binding fragment, VL of the first antigen-binding fragment, the first linker peptide (such as the human IgG1 hinge region or mouse IgG2c hinge region or derivative sequence), $CH_2$ and $CH_3$ from the N-terminus to the C-terminus, and the drug conjugate is connected to the first polypeptide chain through the Cys1 (such as Cys220 (EU numbering) of the IgG hinge region) of the first linker peptide. For example, the second polypeptide chain may sequentially include VH of the second antigen-binding fragment, VL of the second antigen-binding fragment, the second linker peptide (such as the human IgG1 hinge region or mouse IgG2c hinge region or derivative sequence), $CH_2$ and $CH_3$ from the N-terminus to the C-terminus, and the drug conjugate is connected to the second polypeptide chain through the Cys1 (such as Cys220 (EU numbering) of the IgG hinge region) of the second linker peptide.

For example, the first polypeptide chain may sequentially include VH of the first antigen-binding fragment, the first linker peptide (such as the human IgG1 hinge region or mouse IgG2c hinge region or derivative sequence), $CH_2$ and $CH_3$ from the N-terminus to the C-terminus, and the drug conjugate is connected to the first polypeptide chain through the Cys1 (such as Cys220 (EU numbering) of the IgG hinge region) of the first linker peptide. For example, the second polypeptide chain may sequentially include VH of the second antigen-binding fragment, the second linker peptide (such as the human IgG1 hinge region or mouse IgG2c hinge region or derivative sequence), $CH_2$ and $CH_3$ from the N-terminus to the C-terminus, and the drug conjugate is connected to the second polypeptide chain through the Cys1 (such as Cys220 (EU numbering) of the IgG hinge region) of the second linker peptide.

For example, the first polypeptide chain may sequentially include a receptor protein soluble extracellular region of the first antigen-binding fragment, the first linker peptide (such as the human IgG1 hinge region or mouse IgG2c hinge region or derivative sequence), $CH_2$ and $CH_3$ from the N-terminus to the C-terminus, and the drug conjugate is connected to the first polypeptide chain through the Cys1 (such as Cys220 (EU numbering) of the IgG hinge region) of the first linker peptide. For example, the second polypeptide chain may sequentially include a receptor protein soluble extracellular region of the second antigen-binding fragment, the second linker peptide (such as the human IgG1 hinge region or mouse IgG2c hinge region or derivative sequence), $CH_2$ and $CH_3$ from the N-terminus to the C-terminus, and the drug conjugate is connected to the second polypeptide chain through the Cys1 (such as Cys220 (EU numbering) of the IgG hinge region) of the second linker peptide.

In certain cases, the protein-drug conjugate may include a first polypeptide chain, a second polypeptide chain, a third polypeptide chain and a fourth polypeptide chain. The first polypeptide chain may sequentially include a third antigen-binding fragment, a first antigen-binding fragment, a first linker peptide, and a first pairing subunit from an N-terminus to a C-terminus. The second polypeptide chain may sequentially include a fourth antigen-binding fragment, a second antigen-binding fragment, a second linker peptide, and a second pairing subunit from an N-terminus to a C-terminus. In particular, the first pairing subunit and the second pairing subunit can interact to form a dimer. The third polypeptide chain may include a third antigen-binding fragment. The fourth polypeptide chain may include a fourth antigen-binding fragment.

For example, the first polypeptide chain may sequentially include VL of the third antigen-binding fragment, CL of the third antigen-binding fragment, VH of the first antigen-binding fragment, the first linker peptide (such as the human IgG1 hinge region or mouse IgG2c hinge region or derivative sequence), $CH_2$ and $CH_3$ from the N-terminus to the C-terminus, and the drug conjugate is connected to the first polypeptide chain through the Cys1 (such as Cys220 (EU numbering) of the IgG hinge region) of the first linker peptide. For example, the second polypeptide chain may sequentially include VL of the fourth antigen-binding fragment, CL of the fourth antigen-binding fragment, VH of the second antigen-binding fragment, the second linker peptide (such as the human IgG1 hinge region or mouse IgG2c hinge region or derivative sequence), $CH_2$ and $CH_3$ from the N-terminus to the C-terminus, and the drug conjugate is connected to the second polypeptide chain through the Cys1 (such as Cys220 (EU numbering) of the IgG hinge region) of the second linker peptide. For example, the third polypeptide chain may sequentially include VH of the third antigen-binding fragment and $CH_1$ of the third antigen-binding fragment from an N-terminus to a C-terminus. For example, the fourth polypeptide chain may sequentially include VH of the fourth antigen-binding fragment and $CH_1$ of the fourth antigen-binding fragment from an N-terminus to a C-terminus. For example, the third antigen-binding fragment and/or the fourth antigen-binding fragment may be Fab.

## Nucleic acid, vector and host cell

In another aspect, the present application provides one or more nucleic acid molecules that can encode the protein-drug conjugate described herein. For example, each of the one or more nucleic acid molecules can encode the complete protein-drug conjugate, or a part thereof (such as one or more of HCDR1-3, LCDR1-3, VL, VH, polypeptide chains or heavy chains).

The nucleic acid molecule described herein may be isolated. For example, it may be produced or synthesized by the following methods: (i) *in vitro* amplification, such as amplification by polymerase chain reaction (PCR); (ii) cloning and recombination; (iii) purification, such as separation by enzymatic digestion and gel electrophoresis fractionation; or (iv) synthesis, such as chemical synthesis.

For example, the isolated nucleic acid may be a nucleic acid molecule prepared by recombinant DNA techniques.

In another aspect, the present application provides one or more vectors including the one or more nucleic acid molecules described herein. Each vector may include the one or more nucleic acid molecules. In addition, the vector may further include other genes, such as marker genes that allow selection of the vector in an appropriate host cell and under appropriate conditions. In addition, the vector may further include expression control elements that allow proper expression of the coding region in an appropriate host. Such control elements are well known to those skilled in the art and may include, for example, promoters, ribosome binding sites, enhancers and other control elements for regulating gene transcription or mRNA translation. For example, the expression control sequence is a regulable element. The specific structure of the expression control sequence may vary depending on the function of the species or cell type, but generally includes a 5' non-transcribed sequence and 5' and 3' non-transcribed sequences involved in transcription and translation initiation, respectively, such as TATA boxes, capping sequences, CAAT sequences, etc. For example, the 5' non-transcribed expression control sequence may include a promoter region, which may include a promoter sequence for transcribing and controlling functional linkage of nucleic acids. The expression control sequence may further include an enhancer sequence or an upstream activator sequence. In the present application, appropriate promoters may include, for example, promoters for SP6, T3 and T7 polymerases, human U6RNA promoters, CMV promoters, and artificial hybrid promoters thereof (such as CMV), wherein a portion of the promoter can fuse with a portion of other cell protein (such as human GAPDH, glyceraldehyde-3-phosphate dehydrogenase) gene promoters, and may or may not include additional introns. The one or more nucleic acid molecules in the present application can be operably connected to the expression control element.

The vector may include, for example, a plasmid, a cosmid, a virus, a phage or other vectors commonly used in, for example, genetic engineering. For example, the vector is an expression vector. For example, the expression vector may include a eukaryotic cell expression vector and/or a prokaryotic cell expression vector.

In another aspect, the present application provides a host cell, which may include the one or more nucleic acid molecules and/or the one or more vectors described herein. For example, each type of or each host cell may include one type of or one nucleic acid molecule or vector described herein. In certain embodiments, each type of or each host cell may include multiple (e.g., two or more) or multiple types (e.g., two or more) of nucleic acid molecules or vectors described herein. For example, the vector described herein can be introduced into the host cell described herein, e.g., a prokaryotic cell (for example, bacterial cell), mammalian eukaryotic cell or other eukaryotic cells, such as a plant-derived cell or a fungal or yeast cell. The vector described herein can be introduced into the host cell described herein based on methods known in the art, such as electroporation, lipofectine transfection and lipofectamin transfection. For example, the host cell may be COS, CHO, NSO, sf9, sf21, DH5a, BL21 (DE3) or TG1. For example, the prokaryotic cell may be selected from *E. coli* cells such as TG1 and BL21. For example, the eukaryotic cell may be selected from CHO cell, CHO-K1 cell, CHOZN cell, CHO-S cell, ExpiCHO-S cell, NS/0 cell, HEK293 cell, HEK293-T cell, HEK293-F cell or HEK293-6E cell.

## Method, pharmaceutical composition and use

In another aspect, the present application further provides a method for preparing the protein-drug conjugate, including covalently binding the drug conjugate with a specific cysteine (e.g., Cys1 of the first linker peptide and/or Cys1 of the second linker peptide) of the linker peptide, to obtain the protein-drug conjugate described herein. For example, the preparation method may be a process of drug coupling based on reactive sulfhydryl functional groups of free cysteines at specific sites, the preparation method can produce a protein-drug conjugate with coupling site specificity and homogeneity. For another example, in certain specific embodiments, the preparation method can produce a highly-homogeneous protein-drug conjugate with CAR=2 (one antigen-binding protein carrying two conjugates).

The preparation method may include a combination of one or more steps consisting of multiple implementation steps such as "reduction", "reoxidation", "coupling" and "purification". For example, the preparation method may include steps such as "reduction", "coupling" and "purification"; for another example, the preparation method may include steps such as "reduction" and "coupling". The implementation steps may include one or more reaction processes. During the reaction process, reaction conditions such as a reaction temperature, reaction time, a type and pH of a reaction buffer, a type and an amount of usage of a reducing agent, a type and an amount of usage of a conjugate molecule, etc., may have a significant impact on the results of the preparation method, for example on the efficiency of drug coupling and the quality of final product. The reaction process may further include combinations of different reaction condition parameters; for example, the reaction process of reducing a disulfide bond can be combined with multiple parameter conditions such as different types of reducing agents, different amounts of usage of reducing agents, different reduction reaction temperatures, different reaction buffers, different pH, and different reaction time.

In certain specific embodiments, the step of "reduction" of the preparation method may use dithiothreitol (DTT) or Tris(2-carboxyethyl)phosphine (TCEP) as a reducing agent.

In certain specific embodiments, in the step of "reduction" of the preparation method, the amount of usage of the reducing agent is about 1 to 50 molar multiples, for example, about 1 to 30 molar multiples, about 1 to 20 molar multiples, about 1 to 10 molar multiples, about 1 to 7 molar multiples, about 1 to 6 molar multiples, about 1 to 5 molar multiples, about 1.5 to 6 molar multiples, or about 1.5 to 5 molar multiples.

In certain specific embodiments, in the step of "reduction" of the preparation method, the reaction time is about 1 to 17 hours, for example, about 1 to 10 hours, about 1 to 7 hours, about 1 to 5 hours, about 1 to 4 hours, about 1 to 3 hours, or about 1.5 to 2 hours.

In certain specific embodiments, in the step of "reduction" of the preparation method, the reaction temperature is about 0 °C to 40 °C, for example, 0 °C to 37 °C, about 0 °C to 30 °C, about 20 °C to 37 °C, about 20 °C to 30 °C, or room temperature (about 25 °C to 30 °C). For example, the reaction temperature is 0 °C, room temperature (25 °C to 30 °C) or 37 °C.

In certain specific embodiments, in the step of "reduction" of the preparation method, the pH of the reaction buffer is about 4.0 to 9.0, about 5.0 to 8.0, or about 5.0 to 7.0.

In certain specific embodiments, the step of "reduction" of the preparation method may use TCEP as a reducing agent. The amount of usage of the reducing agent is 1 to 7 molar multiples, the reaction time is 1 to 3 hours, the reaction temperature is 23 °C to 30 °C, and the pH of the reaction buffer is 5.0 to 7.0.

In certain specific embodiments, the step of "reduction" of the preparation method may use dithiothreitol (DTT) or Tris(2-carboxyethyl)phosphine (TCEP) as a reducing agent. The amount of usage of the reducing agent is 1 to 50 molar multiples, the reaction time is 1 to 17 hours, the reaction temperature is 0 °C to 40 °C, and the pH of the reaction buffer is 5.0 to 8.0. Preferably, the reducing agent is TCEP. The amount of usage of the TCEP may be 1 to 7 molar multiples, the reaction time may be 1 to 3 hours, the pH of the reaction buffer is 5.0 to 7.0, and the reaction temperature is 0 °C or the room temperature (25 °C to 30 °C) or 37 °C. More preferably, the amount of usage of the reducing agent TCEP may be 1.5 to 6 molar multiples, the reaction time may be 1.5 to 2 hours, the pH of the reaction buffer is 5.0 to 6.0, and the reaction temperature is 0 °C or the room temperature (25 °C to 30 °C) or 37 °C.

In certain specific embodiments, the step of "coupling" of the preparation method may use mc-vc-PAB-MMAE (0) as a drug conjugate.

In certain specific embodiments, the step of "coupling" of the preparation method may use a BRD4 protein degrading agent (0) as a drug conjugate.

In certain specific embodiments, in the step of "coupling" of the preparation method, the amount of usage of the drug conjugate is about 1 to 50 molar multiples, for example, about 1 to 30 molar multiples, about 1 to 20 molar multiples, about 10 to 15 molar multiples, about 1 to 10 molar multiples, about 1 to 7 molar multiples, or about 3 to 7 molar multiples.

In certain specific embodiments, in the step of "coupling" of the preparation method, the reaction time is about 0.5 to 10 hours, for example, about 0.5 to 10 hours, about 0.5 to 3 hours, about 0.5 to 2 hours, about 1 to 2 hours, or about 0.5 to 1 hour.

In certain specific embodiments, in the step of "coupling" of the preparation method, the reaction temperature is about 0 °C to 40 °C, for example, 0 °C to 37 °C, about 0 °C to 30 °C, about 20 °C to 37 °C, about 20 °C to 30 °C, or room temperature (about 25 °C to 30 °C). For example, the reaction temperature is the room temperature (25 °C to 30 °C).

In certain specific embodiments, in the step of "coupling" of the preparation method, the pH of the reaction buffer is about 4.0 to 9.0, about 5.0 to 8.0, or about 5.0 to 7.0.

In certain specific embodiments, the step of "coupling" of the preparation method may use mc-vc-PAB-MMAE (0) as a drug conjugate. The amount of usage of the drug conjugate is 3 to 7 molar multiples, the reaction time is 0.5 to 2 hours, the reaction temperature is 23 °C to 30 °C, and the pH of the reaction buffer is 5.0 to 7.0.

In certain specific embodiments, the step of "coupling" of the preparation method may use mc-vc-PAB-MMAE (0) as a drug conjugate. The amount of usage of the drug conjugate is 1 to 50 molar multiples, the reaction time is 0.5 to 10 hours, the reaction temperature is 0 °C to 40 °C, and the pH of the reaction buffer is 5.0 to 8.0. Preferably, the amount of usage of the mc-vc-PAB-MMAE may be 3 to 18 molar multiples, the reaction time may be 0.5 to 3 hours, the pH of the reaction buffer is 5.0 to 7.0, and the reaction temperature is the room temperature (25 °C to 30 °C). More preferably, the amount of usage of the mc-vc-PAB-MMAE may be 3 to 7 molar multiples, the reaction time may be 0.5 hour, the pH of the reaction buffer is 5.0 to 6.0, and the reaction temperature is the room temperature (25 °C to 30 °C).

In certain specific embodiments, the step of "coupling" of the preparation method may use a BRD4 protein degrading agent (0) as a drug conjugate. The amount of usage of the drug conjugate is 10 to 15 molar multiples, the reaction time is 1 to 3 hours, the reaction temperature is 23 °C to 30 °C, and the pH of the reaction buffer is 7.0 to 9.0.

In certain specific embodiments, the preparation method may further provide a combination of steps including "reduction" and "coupling", as well as a combination of reaction condition parameters. A preferred combination of the reaction condition parameters may be as follows: a reaction buffer is a buffer with pH of 6.0 containing 20 mM His-HCl, the amount of usage of the reducing agent TCEP is 1.5 to 6.0 molar multiples, the reduction reaction time is 1.5 to 2.0 hours, the reduction reaction temperature is room temperature (25 °C to 30 °C), a conjugate is mc-vc-PAB-MMAE, the amount of usage of the conjugate is 3.0 to 7.0 molar multiples, the coupling reaction time is 0.5 hour, and the coupling reaction temperature is the room temperature (25 °C to 30 °C).

In certain specific embodiments, the preparation method may further provide a step of "purification". The step of "purification" uses hydrophobic interaction chromatography (HIC) to separate respective components in a coupling reaction product to obtain certain specific component, for example, a protein-drug conjugate with CAR=2 obtained by using HIC to purify and separate the coupling product.

In certain specific embodiments, the preparation method can further increase the content of a single component in the coupling product, reducing the need for further purification; more preferably, after the step of coupling, a highly-homogeneous coupling product with a purity greater than 90% and CAR=2 can be obtained without an additional purification step.

In certain specific embodiments, the preparation method may further provide a combination of steps including "reduction" and "coupling", as well as a combination of reaction condition parameters; under the combination of the reaction steps and reaction condition parameters, the disulfide bond of the first cysteine (Cys1) of the linker peptide can be effectively cleaved while the other disulfide bonds remain intact, so that the Cys1 becomes almost the only free cysteine residue and serves as a specific coupling site.

In another aspect, the present application provides a pharmaceutical composition, which may include the protein-drug conjugate and a pharmaceutically acceptable vector. For example, the pharmaceutical composition may further include other antigen-binding proteins or therapeutic agents.

In another aspect, the present application provides use of the protein-drug conjugate and/or the pharmaceutical composition in preparation of a drug for diagnosing, preventing and/or treating diseases. For example, the drug can be used for treating tumors or other diseases.

In another aspect, the present application provides use of the protein-drug conjugate, in combination with other therapies or drugs, in preparation of a drug, wherein the drug can be used for treating tumors or other diseases. For example, the other therapies or drugs are selected from: chemotherapy, radiotherapy, miRNA, and oligonucleotides.

In another aspect, the present application provides a method for detecting a specific antigen in vitro or in vivo, which may include detection by using the protein-drug conjugate and/or the pharmaceutical composition. In certain applicable cases, the method may be for non-diagnostic purposes.

In another aspect, the present application provides an administration device for administering the protein-drug conjugate or the pharmaceutical composition.

## Examples

The present invention is further illustrated by the following examples, which are not intended to limit the present invention. The examples do not include detailed descriptions of conventional methods, such as those methods for constructing vectors and plasmids, methods for inserting genes encoding proteins into such vectors and plasmids, or methods for introducing plasmids into host cells. Such methods are well known to those of ordinary skill in the art and are described in numerous publications. Experimental procedures without specified conditions in the following examples are performed in accordance with conventional procedures and conditions, or in accordance with instructions.

### Example 1 Preparation of Antibodies

The Harbour HCAb mouse (Harbour Antibodies BV, WO2010/109165A2) is a transgenic mouse carrying an immune repertoire of human immunoglobulins, capable of producing heavy chain-only antibodies that are only half the size of conventional IgG antibodies. The antibodies produced by the mouse have human antibody heavy chain variable domains and mouse constant domains. After a VH sequence of a heavy-chain antibody targeting a specific target is obtained, conventional molecular biology methods are used to fuse and express the VH sequence and the human IgG antibody heavy chain Fc sequence (preferably a sequence containing CH2 and CH3 regions of the human IgG1 hinge region set forth in SEQ ID NO: 72) to obtain a fully human recombinant HCAb antibody molecule.

The Harbour H2L2 mouse (Harbour Antibodies BV) is a transgenic mouse carrying an immune repertoire of human immunoglobulins that produces antibodies with intact human antibody variable domains and rat constant domains. An antibody targeting a specific target is screened by using hybridoma technique, single B cell sorting technique, or other techniques. The VL and VH sequences of the antibody were fused to the corresponding human κ light chain constant region and IgG1 heavy chain constant region sequences and expressed to obtain recombinant fully human antibody molecules.

### Example 1.1 Acquisition of fully human anti-CTLA4 HCAb antibodies

Harbour HCAb mice were subjected to multiple rounds of immunization with a soluble recombinant human CTLA4 protein (ACRO Biosystems, #CT4-H5229). When the titer of the CTLA4-specific antibody in the serum of mice was detected to reach a certain level, spleen cells of the mice were taken, from which B cells were isolated, and the CD138-positive plasma cells were sorted using a mouse plasma cell isolation kit (Miltenyi, #130-092-530). The human VH gene was amplified from plasma cells using conventional molecular biology techniques, and the amplified human VH gene fragments were constructed into mammalian cell expression plasmid pCAG vectors encoding the sequence of the heavy chain Fc region of the human IgG1 antibody. Mammalian host cells (e.g., human embryonic kidney cell HEK293) were transfected with the plasmids and allowed to express antibodies to obtain a supernatant with fully human HCAb antibodies. Positive HCAb antibodies were identified by testing the supernatant with HCAb antibodies for binding to recombinant human CTLA4 proteins by ELISA. Those HCAb antibodies were further identified, and several candidate HCAb antibody molecules were preferentially selected according to parameters such as the binding ability to human CTLA4, the binding ability to cynomolgus monkey CTLA4, and ability to inhibit the binding of CTLA4 to B7-1. The candidate HCAb antibody molecules were then subjected to sequence analysis and optimization to obtain several variant sequences. The VH sequence of the HCAb antibody and the human IgG1 heavy chain hinge region and Fc sequence were fused and expressed to obtain fully human recombinant HCAb antibody molecules. The recombinant fully human anti-CTLA4 HCAb antibodies are listed in Table 0-1.

**Example 1.2 Acquisition of fully human anti-BCMA HCAb antibodies**

Harbour HCAb mice were subjected to multiple rounds of immunization with a soluble recombinant human BCMA-ECD-Fc fusion protein (ACRO Biosystems, #BC7-H82F0). The antigenic protein was mixed with an immunoadjuvant to form an immunogenic reagent, which was then injected subcutaneously via the groin or intraperitoneally. In each round of immunization, each mouse received a total injection dose of 100 $\mu$L. In the first round of immunization, each mouse received an immunization with an immunogenic reagent prepared by mixing 50 $\mu$g of antigenic protein with complete Freund's adjuvant (Sigma, #F5881) in a 1:1 volume ratio. In each subsequent round of booster immunization, each mouse received an immunization with an immunogenic reagent prepared by mixing 25 $\mu$g of antigenic protein with Sigma Adjuvant System adjuvant (Sigma, #S6322). The interval between rounds of booster immunization was at least two weeks. In general, there are no more than five rounds of booster immunizations. The immunization was performed at days 0, 14, 28, 42, 56 and 70; and the antibody titer in serum of mice was determined at days 49 and 77. The last round of booster immunization was performed at a dose of 25 $\mu$g of antigenic protein per mouse 5 days before the isolation of Harbour HCAb mouse splenic B cells.

When the titer of the BCMA-specific antibody in the serum of mice was detected to reach a certain level, spleen cells of the mice were taken, from which B cells were isolated, and the CD138-positive plasma cells and BCMA antigen-positive B cell populations were sorted using a BD FACSAria™ III cell sorter. RNA was extracted and reversely transcribed into cDNA, and human VH gene was amplified by PCR. The amplified VH gene fragment was constructed into a mammalian cell expression plasmid pCAG vector encoding the sequence of the heavy chain Fc domain of the human IgG1 antibody. Mammalian host cells (such as human embryonic kidney cell HEK293) were transfected with the plasmid for expression, and the expressed HCAb antibody supernatant was screened with recombinant human BCMA-Fc, Avitag recombinant protein (ACRO Biosystems, #BC7-H82F0) through Mirrorball (SPT Labtech, mirrorball® fluorescence cytometer). The obtained positive monoclonal antibody supernatant was further identified by flow cytometry FACS. The binding ability of the antibody supernatant to cells such as an HEK293T cell strain HEK293T/hBCMA (Beijing KYinno, KC-0233) highly expressing human BCMA, an HEK293T cell strain HEK293T/cyno BCMA (Beijing KYinno, KC-0979) highly expressing cynomolgus monkey BCMA, and a cell line NCI-H929 (ATCC, CRL-9068) highly expressing human BCMA was tested by FACS. Several positive candidate HCAb antibody molecules were obtained by multiple rounds of screening. The nucleotide sequences encoding the variable domains of the antibody molecules were obtained through conventional sequencing means. The VH sequence of the HCAb antibody and the human IgG1 heavy chain hinge region and Fc sequence were fused and expressed to obtain fully human recombinant HCAb antibody molecules.

The CDR regions of the variable region VH of the anti-BCMAHCAb antibody PR001046 were subjected to two rounds of site-directed mutagenesis to obtain mutants with improved binding affinity for BCMA, e.g., PR001046_R2_4G10 (i.e., PR004433). The recombinant fully human anti-BCMAHCAb antibodies are listed in Table 0-1.

**Example 1.3 Acquisition of fully human anti-MSLN HCAb antibodies**

Harbour HCAb mice were subjected to multiple rounds of immunization with a soluble recombinant human MSLN protein (ACRO Biosystems, #MSN-H5223). Screening was performed in a manner similar to that described in 0, and fully human anti-MSLN HCAb antibodies were obtained. The recombinant fully human anti-MSLN HCAb antibodies are listed in Table 0-1.

**Example 1.4 Acquisition of fully human anti-5T4 HCAb antibodies**

Harbour HCAb mice were subjected to multiple rounds of immunization with a soluble recombinant human 5T4 protein (NovoProtein, #C678). Screening was performed in a manner similar to that described in 0, and fully human anti-5T4 HCAb antibodies were obtained. The recombinant fully human anti-5T4 HCAb antibodies are listed in Table 0-1.

**Example 1.5 Acquisition of anti-ROR1 antigen-binding proteins**

Harbour H2L2 mice were subjected to multiple rounds of immunization with a soluble recombinant human ROR1 protein (AcroBiosystems, #RO1-H5250). Screening was performed in a manner similar to that described in 0 and in combination with phage display, and anti-ROR1 antibody clones 1015M2-H4 were obtained. A single chain variable region fragment scFv was prepared from the VH and VL of 1015M2-H4 by using overlap PCR; then, the scFv and a sequence containing a restriction endonuclease BamHI cleavage site (corresponding to the amino acid sequence GGGAS) as well as a human IgG heavy chain Fc (including a human IgG1 hinge region as well as $CH_2$ and $CH_3$ regions, as a sequence set forth in SEQ ID NO: 72) were fused and expressed to obtain an anti-ROR1 antigen-binding protein molecule PR002129 with an scFv-Fc dimer structure. Anti-ROR1 antigen-binding proteins are listed in Table 0-1.

**Example 1.6 Expression and purification of antibodies**

In this example, a general method for preparing antibodies in mammalian host cells (e.g., human embryonic kidney cell HEK293 or Chinese hamster ovary CHO cells and cells derived therefrom) by such techniques as transient transfection and expression, and affinity capture and separation was described. This method is applicable to an antibody of interest comprising an Fc region. The antibody of interest may consist of one or more protein polypeptide chains, and may be derived from one or more expression plasmids.

The amino acid sequences of the polypeptide chains of the antibody were converted into nucleotide sequences by codon optimization. The encoding nucleotide sequences were synthesized and cloned into expression vectors compatible with the host cell. The mammalian host cells were transfected simultaneously with plasmids encoding the polypeptide chains of the antibody in a specific ratio, and the recombinant protein with correct folding and assembly of polypeptide chains could be obtained by the conventional recombinant protein expression and purification techniques. Specifically, FreeStyle™ 293-F cells (Thermo, #R79007) were expanded in FreeStyle™ F17 Expression Medium (Thermo, #A1383504). Before the transient transfection, the cells were adjusted to a concentration of (6-8) $\times$ $10^5$ cells/mL, and cultured in a shaker at 37 °C with 8% $CO_2$ for 24 h to make a concentration of 1.2 $\times$ $10^6$ cells/mL. 30 mL of cultured cells were taken. Plasmids encoding the polypeptide chains of the antibody were mixed in a certain ratio, and a total of 30 μg of the plasmids (the ratio of the plasmids to cells was 1 μg: 1 mL) were dissolved in 1.5 mL of Opti-MEM reduced serum medium (Thermo, #31985088). The resulting mixture was filtered through a 0.22 μm filter membrane for sterilization. Then, 1.5 mL of Opti-MEM was dissolved in 120 μL of 1 mg/mL PEI (Polysciences, #23966-2), and the mixture was left to stand for 5 min. PEI was slowly added to the plasmid, and the mixture was incubated at room temperature for 10 min. The mixed solution of plasmid and PEI was slowly added dropwise while shaking the culture flask, and the cells were cultured in a shaker at 37 °C with 8% $CO_2$ for 5 days. Cell viability was measured after 5 days. The culture was collected and centrifuged at 3,300 g for 10 min, and then the supernatant was collected and centrifuged at high speed to remove impurities. A gravity column (Bio-Rad, #7311550) containing MabSelect™ (GE Healthcare, #71-5020-91) was equilibrated with a PBS buffer (pH 7.4) and rinsed with 2-5 column volumes of PBS. The column was loaded with the supernatant sample, and rinsed with 5-10 column volumes of PBS buffer, followed by 0.1 M glycine at pH 3.5 to elute the target protein. The eluate was adjusted to neutrality with Tris-HCl at pH 8.0, and concentrated and buffer exchanged into PBS buffer or a buffer with other ingredients with an ultrafiltration tube (Millipore, #UFC901024) to obtain a purified solution of the recombinant protein. Finally, the purified protein solution was determined for concentration using NanoDrop (Thermo, NanoDrop™ One), subpackaged and stored for later use.

The antigen-binding proteins used in the present application are summarized in Table 0-1, and the sequence numbers SEQ ID NOs of the corresponding polypeptide chains and the amino acid sequences in the CDR region are listed in Table 0-2, Table 0-3, Table 0-4 and Table 0-5.

**Table 0-1. Some exemplary antigen-binding proteins used in the present application.**

| Target | Clone No. | Protein No. | Sequence source |
|---|---|---|---|
| CTLA4 | CL5v3 | PR000184 | 0 |
| CTLA4 | CL24 | PR000020 | 0 |
| MSLN | 1018P004B9 | PR000759 | 0 |

(continued)

| Target | Clone No. | Protein No. | Sequence source |
|---|---|---|---|
| BCMA | 1005P63B7 | PR001046 | 0 |
| 5T4 | 1041P014H2 | PR004432 | 0 |
| BCMA | PR001046_R2_4G10 | PR004433 | 0 |
| ROR1 | 1015M2-H4 | PR002129 | 0 |
| BCMA | 269A37917 | PR000453 | Patent WO2017025038A1 |
| CD47 | ALX148 | PR006345 | Patent US10829771B2 |

**Table 0-2. Sequence numbers of HCAb antibodies in the present application.**

| Protein No. | Heavy chain | VH | HCDR1 | HCDR2 | HCDR3 |
|---|---|---|---|---|---|
| PR000020 | 64 | 55 | 7 | 22 | 38 |
| PR000184 | 65 | 56 | 8 | 23 | 39 |
| PR000759 | 67 | 58 | 10 | 25 | 41 |
| PR001046 | 68 | 59 | 11 | 26 | 42 |
| PR004432 | 69 | 61 | 13 | 28 | 44 |
| PR004433 | 70 | 62 | 14 | 29 | 42 |
| PR000453 | 66 | 57 | 9 | 24 | 40 |

**Table 0-3. Sequence numbers of antigen-binding proteins of scFv-Fc structure in the present application.**

| Protein No. | Polypeptide chain | VL | VH | LCDR1 | LCDR2 | LCDR3 | HCDR1 | HCDR2 | HCDR3 |
|---|---|---|---|---|---|---|---|---|---|
| PR002129 | 71 | 63 | 60 | 49 | 51 | 53 | 12 | 27 | 43 |

**Table 0-4. Sequence numbers of soluble receptor fusion proteins in the present application.**

| Protein No. | Polypeptide chain |
|---|---|
| PR006345 | 149 |

**Table 0-5. Sequence numbers of bispecific antigen-binding proteins in the present application.**

| Protein No. | Polypeptide chain 1 | Polypeptide chain 2 |
|---|---|---|
| PR005744 | 134 | 135 |
| 2129/4433 | 70 | 71 |

### Example 2 Analysis Methods

### Example 2.1 Analysis of protein purity and polymers by SEC-HPLC (size-exclusion chromatography-high performance liquid chromatography)

In this example, analytical size-exclusion chromatography (SEC) was used to analyze the protein sample for purity and polymer form. An analytical chromatography column TSKgel G3000SWxl (Tosoh Bioscience, #08541, 5 μm, 7.8 mm × 30 cm) was connected to a high performance liquid chromatography HPLC (Agilent Technologies, Agilent 1260 Infinity II) and equilibrated with a PBS buffer at room temperature for at least 1 h. A proper amount of the protein sample (at least 10 μg) was filtered through a 0.22 μm filter membrane and then injected into the system, and an HPLC program was set: the sample was passed through the chromatography column with PBS buffer at a flow rate of 1.0 mL/min for a maximum of 25 min. The detection wavelength was 280 nm. Operation was performed at room temperature. After being recorded, the chromatogram was integrated using ChemStation software and relevant data were calculated. An analysis was generated, with the retention time of the components with different molecular sizes in the sample reported.

**Example 2.2 Analysis of protein purity, hydrophobicity and coupling product components by HIC-HPLC (hydrophobic interaction chromatography-high performance liquid chromatography)**

In this example, analytical hydrophobic interaction chromatography (HIC) was used to analyze the protein sample for purity and hydrophobicity. An analytical chromatography column TSKge1 Buty1-NPR (Tosoh Bioscience, #14947, 4.6 mm × 3.5 cm, 2.5 μm) was connected to a high performance liquid chromatography (HPLC, model: Agilent Technologies, Agilent 1260 Infinity II) and equilibrated with a PBS buffer at room temperature for at least 1 h. The program for HPLC was set: a linear gradient from 100% buffer A(25 mM phosphate buffer, 1.5 M ammonium sulfate ((NH4)2SO4), pH 7.0-7.2) to 100% buffer B (20 mM phosphate buffer, 20% isopropyl alcohol (IPA), pH 7.0-7.2) over about 22 min; flow rate: 0.7 mL/min; protein sample concentration: 1 mg/mL; and injection volume: 20 μL. The detection wavelength was 280 nm. Operation was performed at room temperature. After being recorded, the chromatogram was integrated using ChemStation software and relevant data were calculated. An analysis was generated, with the retention time of the components with different molecular sizes in the sample reported; the number of coupling molecules carried by different components of the coupling product could be inferred by different retention time.

**Example 2.3 Analysis of protein purity, hydrophobicity and coupling product components by RP-HPLC (reversed phase-high performance liquid chromatography)**

In this example, analytical reversed phase high performance liquid chromatography (RP) was used to analyze the protein sample for purity and hydrophobicity. Those skilled in the art can use different models of high performance liquid chromatography and analytical chromatography columns to establish similar analytical methods according to the technical instructions and through experimental exploration. Only representative methods are listed in this example.

In certain specific embodiments, for example in 0 of the present invention, an analytical chromatography column Zorbax RRHD 300-Diphenyl (Agilent Technologies, #858750-944, 2.1 × 100 mm, 1.8 μm) was connected to a high performance liquid chromatography (HPLC, model: Agilent Technologies, Agilent 1260 Infinity II) and the system was equilibrated in accordance with instructions. The program for HPLC was set: a linear gradient from 100% buffer A (1% trifluoroacetic acid TFA dissolved in water) to 100% buffer B (1% trifluoroacetic acid TFA dissolved in acetonitrile) over 60 min; flow rate: 0.35 mL/min; and operation temperature: 50 °C. The detection wavelength was 280 nm. After being recorded, the chromatogram was integrated using ChemStation software and relevant data were calculated. An analysis was generated, with the retention time of the components with different molecular sizes in the sample reported.

In certain other specific embodiments, for example in 0 of the present invention, an analytical chromatography column PLRP-S 1000A (Agilent Technologies, #PL1912-1502, 2.1 × 50 mm, 5.0 μm) was connected to a high performance liquid chromatography (HPLC, model: Agilent Technologies, Agilent 1260 Infinity II) and the system was equilibrated in accordance with instructions. The program for HPLC was set: a linear gradient from 100% buffer A (0.05% trifluoroacetic acid TFA dissolved in water) to 100% buffer B (0.05% trifluoroacetic acid TFA dissolved in acetonitrile) over 40 min; flow rate: 0.25 mL/min; and operation temperature: 60 °C. The detection wavelength was 280 nm. After being recorded, the chromatogram was integrated using ChemStation software and relevant data were calculated. An analysis was generated, with the retention time of the components with different molecular sizes in the sample reported. Then, the number of coupling molecules carried by different components of the coupling product could be inferred by different retention time.

**Example 2.4 Determination of molecular weight by LC-MS (liquid chromatography-mass spectrometry)**

In this example, liquid chromatography-mass spectrometry (LC-MS) was used to analyze the protein sample for molecular weight. Those skilled in the art can use different models of liquid chromatography (LC) and mass spectrometer (MS) to establish similar analytical methods according to the technical instructions and through experimental exploration. Only representative methods are listed in this example.

**Sample preprocessing**

In certain specific embodiments, determination of the molecular weight of a protein sample required desaccharification on the sample to remove modifications such as saccharide chains, for example, the sample was processed with glycosidase PNGase F (NEB, #P0705L) at 37 °C for at least 4 h. For example, in all examples of the present invention, when the molecular weight of protein samples was determined, unless otherwise emphasized, the protein samples to be tested were subjected to desaccharification by the glycosidase PNGase F.

In certain specific embodiments, determination of the reduced molecular weight of a protein sample required reduction on the sample, for example, the sample was processed with 10 nM dithiothreitol (DTT) at 37 °C for 10 min. For example, in all examples of the present invention, the samples after reduction were called reduced samples, and the samples without reduction were called non-reduced samples.

**Setting of LC conditions**

In certain specific embodiments, a chromatography column bioZen 3.6 μm Intact C4 (Phenomenex, #00F-4767-AN, 2.1 × 150 mm, 3.6 μm) was connected to an ultra-high performance liquid chromatography (UPLC, model: Agilent Technologies, Agilent 1290 UPLC) and the system was equilibrated in accordance with instructions. Buffer A was 0.1% formic acid FA dissolved in water, and buffer B was 0.1% formic acid FA dissolved in acetonitrile. Injection: about 1 μg; flow rate: 0.3 mL/min; and operation temperature: 80 °C. When reduced samples were tested, buffer A and buffer B were mixed within 12 min to form a linear gradient: using 10-60% B for the first 8 min and 80% B for the last 4 min. When non-reduced samples were tested, buffer A and buffer B were mixed within 9 min to form a linear gradient: using 15-60% B for the first 6 min and 80% B for the last 3 min.

In certain other specific embodiments, a chromatography column ACQUITY UPLC Protein BEH C4 (Waters, # 186004495, 2.1 × 50 mm, 1.7 μm, 300 Å) was connected to an ultra-high performance liquid chromatography (UPLC, model: Waters Acquity UPLC) and the system was equilibrated in accordance with instructions. Buffer A was 0.1% formic acid FA dissolved in water, and buffer B was 0.1% formic acid FA dissolved in acetonitrile. Injection: about 10 μL; flow rate: 0.2-0.5 mL/min; and operation temperature: 80 °C. The detection wavelength was 214 nm. When reduced samples were tested, buffer A and buffer B were mixed within 20 min to form a linear gradient: using 25-90% B for the first 14 min and 25% B for the last 6 min. When non-reduced samples were tested, buffer A and buffer B were mixed within 8 min to form a linear gradient: using 5-95% B for the first 4 min and 95 -5% B for the last 4 min.

**Setting of MS conditions**

In certain specific embodiments, the model of a mass spectrometer used was AB Sceix X500B, and the operating mode thereof was TOF-MS; when reduced samples were tested, a scanning range was 600-4000 m/z; when non-reduced samples were tested, a scanning range was 900-6000 m/z. Original mass spectrums were deconvolved by Sceix OS software to obtain molecular weight maps.

In certain other specific embodiments, the model of a mass spectrometer used was Waters Xevo G2-XS Q-TOF, and a molecular weight scanning range thereof was 500-4000 Da. Original mass spectrums were deconvolved by Water MaxEnt or Agilent MassHunter BioConfirm software.

**LC-MS combination**

In multiple specific embodiments of the present application, the combination set of different LC and MS conditions was used to achieve LC-MS combination for determining the molecular weight of protein samples. For example, in 0 and 0, the combination of conditions used was (chromatography column: ACQUITY UPLC Protein BEH C4; LC instrument: Waters Acquity UPLC; MS instrument: Waters Xevo G2-XS Q-TOF). For another example, in 0, 0, 0, 0, 0, 0, 0, 0 and 0, the combination of conditions used was (chromatography column: bioZen 3.6 μm Intact C4; LC instrument: Agilent 1290 UPLC; MS instrument: AB Sceix X500B). For another example, in 0 and 0, the combination of conditions used was (chromatography column: Agilent PLRP-S 1000A; LC instrument: Agilent 1290 UPLC; MS instrument: Waters Xevo G2-XS Q-TOF).

**Example 2.5 Determination of molecular stability and molecular aggregation of proteins by Uncle**

Unchained Labs (Uncle) is a multifunctional one-stop protein stability analysis platform that characterizes protein stability by total fluorescence, static light scattering (SLS) and dynamic light scattering (DLS) assay methods. The parameters of melting temperature (Tm), aggregation temperature (Tagg) and particle size (diameter) can be obtained simultaneously for the same group of samples. In this example, an "Tm & Tagg with optional DLS" application of Uncle was selected for the determination. 9 μL of sample was added to a Uni tube, and the temperature was set to gradually increase from 25 °C to 95 °C at a gradient of 0.3 °C/min. Four acquisitions of data were performed at the beginning and end of DLS assay, each for 5 s. After the experimental run was finished, the Tm value of each sample was calculated according to a barycentric mean (BCM) formula using the Uncle analysis software, the Tagg value was calculated from a curve (aggregation curve) of fluorescence intensity of SLS at the wavelength of 266 nm or 473 nm, and the particle size and dispersion of the samples were calculated from DLS related functions.

**Example 2.6 Determination of melting temperatures of proteins by DSC (differential scanning calorimetry)**

In this example, Malvern VP-capillary DSC instrument was used to analyze the protein sample for melting temperature (Tm) by differential scanning calorimetry (DSC). Samples were diluted with a buffer to a detection concentration (0.5 mg/mL) and placed in a DSC sample cell, and the buffer was placed in a reference cell. Identical isothermal heating control was conducted on the DSC sample cell and the reference cell, with a scanning temperature range of 25-100 °C, a scanning speed of 1 °C/min, and an equilibrium time of 3 min before scanning, and each sample to be tested was analyzed once. A peak plot composed of the heat compensating for the temperature difference in the sample pool is the thermodynamic curve of samples, and the peak temperature of the curve is the Tm value of samples. Protein samples containing multiple domains may exhibit multiple peaks in their curves due to different stability in different domains.

**Example 2.7 Determination of melting temperatures of proteins by DSF (differential scanning fluorimetry)**

Differential scanning fluorimetry (DSF) is a commonly used high-throughput method for determining the thermostability of proteins. In this method, changes in the fluorescence intensity of the dye that binds to unfolded protein molecules were monitored using a real-time quantitative fluorescence PCR instrument to reflect the denaturation process of the protein and thus to reflect the thermostability of the protein. In this example, the melting temperature (Tm) of a protein molecule was measured by DSF. 10 μg of protein was added to a 96-well PCR plate (Thermo, #AB-0700/W), followed by the addition of 2 μL of 100× diluted dye SYPROTM (Invitrogen, #2008138), and then the mixture in each well was brought to a final volume of 40 μL by adding buffer. The PCR plate was sealed, placed in a real-time quantitative fluorescence PCR instrument (Bio-Rad CFX96 PCR System), and incubated at 25 °C for 5 min, then at a temperature gradually increased from 25 °C to 95 °C at a gradient of 0.2 °C/0.2 min, and at a temperature decreased to 25 °C at the end of the test. The FRET scanning mode was used and data analysis was performed using Bio-Rad CFX Maestro software to calculate the Tm of the sample.

**Example 3 Identification of Formation of Disulfide Bonds at Cys-220 Site**

As shown in 0, a human IgG1 antibody is a tetramer structure containing two identical heavy chains and two identical light chains. Two interchain disulfide bonds are formed between the two heavy chains, and an interchain disulfide bond is formed between one heavy chain and the corresponding light chain; several sulfhydryls formed after the natural interchain disulfide bonds of these antibodies are reduced become available cysteine coupling sites. 0 (B) shows a human IgG1 hinge region sequence (SEQ ID NO: 73), which contains three cysteines, with corresponding sites Cys-220, Cys-226 and Cys-229 according to Eu numbering, respectively; the Cys-226 and Cys-229 in the two heavy chain hinge regions each form a disulfide bond between heavy chains, while the Cys-220 forms a disulfide bond between the heavy chain and the light chain with Cys-214 near the C' terminus of the light chain.

0 shows a structure of a heavy-chain antibody HCAb with a human IgG1 antibody hinge region sequence and a difference in a hinge region disulfide bond structure between human IgG1 and HCAb. Due to the absence of light chains, Cys-220 located in the hinge region is clearly unable to form a disulfide bond with the light chain Cys-214. There are two possibilities for the state of Cys-220 as shown in 0: (I) Cys-220 does not form a disulfide bond between heavy chains, and its side chain sulfhydryl (-SH) will form a disulfide bond with a sulfhydryl-containing molecule (such as glutathione) in the environment (such as cytoplasm); (II) like Cys-226 or Cys-229, Cys-220 forms an interchain disulfide bond between two heavy chains, so three disulfide bonds will be formed in the hinge region.

In this example, the formation of a disulfide bond at the Cys-220 site in several antigen-binding proteins containing Cys-220 (including heavy-chain antibody HCAb, single chain antibody scFv-Fc and other structures) was researched.

**Example 3.1 Research of Cys-220 in HCAb PR000020**

In this example, different experimental methods were used to identify the state of Cys-220 in HCAb PR000020 (see Table 0-1 for molecular information).

**Identification of Cys-220 by LC-MS molecular weight analysis**

If Cys-220 does not form a disulfide bond between heavy chains (hypothesis 1), but instead its side chain sulfhydryl forms a disulfide bond with other sulfhydryl-containing molecules in the environment, the non-reduced molecular weight (i.e. complete molecular weight) of the HCAb will be greater than the theoretical molecular weight at the time of forming the disulfide bond between heavy chains (hypothesis 2). According to hypothesis 2, the theoretical non-reduced molecular weight of PR000020 is 77,640 Da; according to hypothesis 1, its non-reduced molecular weight may be 77,878 Da (with an increase of 2 cysteines (+119 Da × 2)) or 78,250 Da (with an increase of 2 glutathione (+305 Da × 2)).

Purified HCAb PR000020 protein was desaccharified with glycosidase PNGase F, and its non-reduced molecular weight was determined by the method (LC instrument: Waters Acquity UPLC; MS instrument: Waters Xevo G2-XS Q-TOF) in 0, with a result of 77,643 Da (see 0 for deconvolution molecular weight map), which was almost consistent with the predicted molecular weight according to hypothesis 2. From this, it could be inferred that Cys-220 formed the disulfide bond between heavy chains.

**Table 0-1. Different hypothetical molecular weights of HCAb PR000020**

| PR000020molecular weight | Hypothesis 1 | Hypothesis 2 | Experimental molecular weight |
|---|---|---|---|
| | Cys-220 does not form a disulfide bond between heavy chains, but forms a disulfide bond with a sulfhydryl-containing molecule (such as glutathione) in cells. | Cys-220 forms a disulfide bond between heavy chains. | |
| Reduced molecular weight (Da) | 38,823 | 38,823 | 38,826 |
| Non-reduced molecular weight (Da) | 77,878 ~ 78,250 | **77,640** | **77,643** |

**Identification of Cys-220 by Papain cleavage analysis**

Papain can specifically hydrolyze a peptide bond between His-224 and Thr-225 in a hinge region (see Endo, S., & Arata, Y. (1985).). Purified HCAb PR000020 protein was cleaved with Papain. As shown in 0 (A), if Cys-220 does not form a disulfide bond between heavy chains (hypothesis 1), the hydrolysis of HCAb by Papain will result in a fragment of about 13 KDa (a single VH domain) and an Fc fragment of about 50 KDa; if Cys-220 forms a disulfide bond between heavy chains (hypothesis 2), the hydrolysis of HCAb by Papain will result in a fragment of about 27 KDa (two VH domains connected together by the disulfide bond of Cys-220) and an Fc fragment of about 50 KDa. 0 (B) shows the results of non-reduced SDS-PAGE of a Papain cleavage product; the Papain cleavage product of HCAb (corresponding to lanes 3 and 4) has an obvious band near 28 KDa, but does not have a band near 14 KDa, thus confirming hypothesis 2, that it, Cys-220 forms a disulfide bond between heavy chains.

In summary, both LC-MS molecular weight analysis and Papain cleavage analysis have demonstrated that Cys-220 forms a disulfide bond between heavy chains.

**Example 3.2 Research of Cys-220 in scFv-Fc PR002129**

In this example, the state of Cys-220 in scFv-Fc PR002129 (Table 0-1) was researched. An amino acid sequence of PR002129 is set forth in SEQ ID NO: 71, and includes a human IgG1 hinge region sequence (SEQ ID NO: 73). PR002129 is a dimer structure containing heavy chain Fc, with each polypeptide chain (heavy chain) including a single chain variable region fragment scFv, a linker peptide sequence and a human IgG1 heavy chain Fc sequence; the scFv is derived from the VH and VL sequences of anti-ROR1 antibody 1015M2-H4, and the linker peptide sequence includes a human IgG1 hinge region sequence and a GGGAS sequence (to introduce appropriate restriction endonuclease sites).

In PR002129 and other similar scFv-Fc structures, there are also two possibilities for the state of Cys-220 as shown in 0 (A): the formation or non-formation of an interchain disulfide bond. If Cys-220 does not form a disulfide bond between heavy chains, its side chain sulfhydryl (-SH) will form a disulfide bond with a sulfhydryl-containing molecule (such as cysteine or glutathione) in the environment, so that the actual molecular weight thereof is significantly greater than the theoretical molecular weight.

**Identification of Cys-220 by LC-MS molecular weight analysis**

Purified PR002129 protein was desaccharified with glycosidase PNGase F, and its non-reduced molecular weight was determined by the method (LC instrument: Agilent 1290 UPLC; MS instrument: AB Sceix X500B) in 0 (0 (B) shows the deconvolution molecular weight map), with a result of 104,510 Da. Compared to different hypothetical predicted molecular weights (Table 0-2), it is inferred that Cys-220 does not link other sulfhydryl-containing molecules, so Cys-220 forms a disulfide bond between heavy chains.

**Table 0-2. LC-MS analysis of non-reduced molecular weights of PR002129**

| Component | Theoretical value (Da) | Experimental value (Da) | Deviation (Da) |
|---|---|---|---|
| PR002129 | 104,508 | 104,510 | +2 |
| PR002129 linking 2 cysteines | 104,746 | | |
| PR002129 linking 2 glutathiones | 105,118 | | |

**Example 3.3 Analysis of hinge region sequence and disulfide bond of Cys-220**

Hinge region sequences of different human IgG isotypes are known, and hinge region structures and disulfide bonds have also been fully studied, for example, human IgG1 forms an interchain disulfide bond by the last Cys of a light chain (i.e., Cys-214 encoded by Eu) and the fifth Cys of a heavy chain (i.e., Cys-220 encoded by Eu), while IgG2, IgG3 and IgG4 form an interchain disulfide bond by the last Cys of the light chain and the third Cys of the heavy chain (see Liu, H. et al., mAbs (2012), 4(1), 17-23).

The inventor of the present application further analyzed the crystal structure of IgG1 (PDB accession No. 1HZH), and found that Cys-220 forms a stable disulfide bond with the light chain Cys-214, and the positions of Cβ atoms of Cys-220 in the two heavy chain hinge regions are relatively far apart, and it is inferred from the IgG1 structure that it is difficult to form a disulfide bond between the two Cys-220. However, in another aspect, the previous research has verified the fact that Cys-220 forms a disulfide bond between heavy chains in structures lacking light chains. Therefore, it is inferred that: Cys-220 can form a disulfide bond between heavy chains like Cys-226 or Cys-229, but its disulfide bond stability may be weaker than that of Cys-226 or Cys-229. Therefore, optimization can enable the disulfide bond of Cys-220 to be specifically cleaved while keeping other disulfide bonds unchanged; in this way, Cys-220 may provide an anchor point for site-specific conjugation of cysteine.

The inventor of the present application further studied the IgG antibody hinge region sequences and disulfide bond structures (0) of different species, and found that there are significant differences in hinge region sequences among humans, mice and alpacas, with different hinge region sequence lengths and different numbers of Cys contained (0 (A)). The inventor analyzed the disulfide bond structure in the hinge region using the known IgG full-length antibody crystal structures, for example, 0 (B) shows a human IgG1 hinge region (PDB accession No. 1HZH); 0 (C) shows a human IgG4 hinge region (PDB accession No. 5DK3); 0 (D) shows a mouse IgG1 hinge region (PDB accession No. 1IGY); 0 (E) shows a mouse IgG2a hinge region (PDB accession No. 1IGT). In the known crystal structures, only the hinge regions of human IgG1 and mouse IgG1 contribute to disulfide bonds between a heavy chain and a light chain. The human IgG1 forms a disulfide bond with the light chain by using Cys-220 (Eu numbering) as a first Cys in the hinge region, and the mouse IgG1 forms a disulfide bond with the light chain by using Cys-235 (numbered by residue) as a first Cys in the hinge region; however, the difference is that in the hinge region of human IgG1, the first Cys (Cys-220, Eu numbering) and the second Cys (Cys-226, Eu numbering) are separated by 5 other types of amino acid residues, while in the hinge region of mouse IgG1, the first Cys (Cys-235, numbered by residue) and the second Cys (Cys-237, numbered by residue) are separated by 1 other type of amino acid residue.

The inventor of the present application further inferred that the Cys-220 of the human IgG1 heavy chain can form weaker disulfide bonds between heavy chains when lacking the light chain, which may be related to the distance between Cys-220 and Cys-226 (i.e., the distance between the first Cys and the second Cys); the first Cys and the second Cys may need to be separated by a certain number of other types of amino acid residues in order to make the two Cys form different disulfide bonds with stability (or bond energy) differed. In subsequent examples, the spacing between two Cys and the variant linker peptide sequence derived from the hinge region sequence were further studied, as well as the use of drug coupling using the first Cys. In the hinge region sequence shown in 0 (A), only the number and arrangement of Cys in the hinge region of mouse IgG2c are similar to those of human IgG1. It can be inferred that the first Cys in the hinge region sequence of mouse IgG2c may have similar abilities to Cys-220 in the hinge region of human IgG1.

**Example 3.4 The lack of disulfide bonds in Cys-220 does not affect molecular stability**

This example is to study the influence of the disulfide bond of Cys-220 on molecular structural stability. In this example, several HCAb molecules were selected to prepare their C220S mutant derived molecules (by mutating the Cys-220 in the hinge region into serine Ser to disrupt the formation of its disulfide bond), or the method in 0 is used to prepare a drug-derivative conjugate (ADC); the Uncle method in 0 or the DSC method in 0 was then used to determine the melting temperature (Tm) of molecules to characterize its thermostability. The HCAb molecule is from Table 0-1.

0 shows DSC thermodynamic analysis curve graphs of HCAb PR000184 and the derived molecule thereof; 0 shows DSC thermodynamic analysis curve graphs of HCAb PR000453 and the derived molecule thereof; 0 shows Uncle thermodynamic analysis curve graphs of HCAb PR004432 and the derived molecule thereof; the results are summarized in Table 0-3, in which the minimum melting temperature (Tm1) for each sample is listed. It can be seen from this that C220S mutation has no obvious effect on the thermostability of molecular structures; moreover, the coupling of Cys-220 does not cause significant changes in thermostability.

**Table 0-3. Melting temperatures of HCAb and derived molecules thereof, $Tm_1$ being the minimum melting temperature.**

| Target | VH clone No. | Molecule name | Mutation/modification | Sample No. | Tm Determination methods | Tmi (°C) |
|---|---|---|---|---|---|---|
| CTLA4 | CL5v3 | PR000184 | | 170922005 | DSC | 58.5 |
| CTLA4 | CL5v3 | PR000184(C220S) | C220S mutation | YYX20180628 | DSC | 58.4 |
| BCMA | 269A37917 | PR000453 | | ZW180411003 | DSC | 68.5 |
| BCMA | 269A37917 | PR000453(C220S) | C220S mutation | YYX20180628 | DSC | 68.6 |
| 5T4 | 1041P014H2 | PR004432 | | C20200427-0507PB0507 | Uncle | 70.66 |
| 5T4 | 1041P014H2 | PR004432(C220S) | C220S mutation | YYX20191018 | Uncle | 70.76 |
| 5T4 | 1041P014H2 | PR004432-ADC | MMAE coupling | 200527 | Uncle | 70.43 |

It can be seen from this that the lack of disulfide bonds in Cys-220 does not affect molecular stability.

**Example 4 Coupling Reaction and Purification**

**Example 4.1 Coupling reaction**

The drug coupling process based on cysteine residues includes a combination of one or more steps consisting of different reaction processes such as "reduction", "reoxidation", and "coupling". During these reaction processes, reaction conditions such as a reaction temperature, reaction time, a type and pH of a reaction buffer, a type and an amount of usage of a reducing agent, a type and an amount of usage of a conjugate molecule, etc., may have a significant impact on the efficiency of the whole coupling reaction and the quality of final product. These reaction conditions include combinations of different parameters, and in this example, only representative methods are listed; those skilled in the art can also establish a similar coupling reaction process by combining different parameter conditions and through experimental exploration according to the method taught in this example.

**Reduction**

In this example, strong reducing agent dithiothreitol (DTT) or Tris(2-carboxyethyl)phosphine (TCEP) with moderate reduction ability was tried to be used as a reducing agent. The amount of usage of the reducing agent was × molar multiples (x = 1 to 50), the reaction time for reduction was y hours (y = 1 to 17), the reaction temperature was 0 °C to 40 °C, and the pH of the reaction buffer was 4.0 to 9.0.

**Reoxidation**

In this example, an oxidant dehydroascorbic acid (DHAA) may further be tried to be used for sample processing.

**Coupling**

In this example, a drug conjugate tried to be used was mc-vc-PAB-MMAE (also known as VcMMAE, with a molecular structure shown in 0, with a molecular weight of 1315.78 Da; Manufacturer: Levena Biopharma, Art. No.: SET0201), and the compound included an active maleimide reaction group that could undergo addition reaction with sulfhydryl to form a stable thioether bond. In this example, the amount of usage of the drug conjugate tried was z molar multiples (z = 1 to 20), the reaction time for coupling was h hours (h = 0.5 to 3), the reaction temperature was 25 °C to 30 °C, and the pH was 4.0 to 9.0. After the coupling was completed, the excess drug conjugates were removed from the product through a desalting column and the product was replaced into an appropriate buffer for storage.

In multiple specific embodiments of the present application, a variety of coupling reaction conditions and steps may be used, including but not limited to combinations of one or more steps in the method in this example.

**Example 4.2 Purification of coupling products by HIC (hydrophobic interaction chromatography)**

In this example, the hydrophobic interaction was used to separate respective components in a coupling product. A sample obtained by coupling was purified by AKTA pure chromatography system (Cytiva Life Sciences, AKTA pure) using ToyoScreen Phenyl-600M hydrophobic interaction chromatography column (Tosoh Bioscience, #21892). Within 30 column volumes, a hydrophobic phase was linearly increased from 0% to 100%, during which each group of peaks was collected separately. The collected components were subjected to HIC-HPLC detection (the method in 0), followed by merging, concentration, and liquid exchange to obtain coupling products.

**Example 5 Coupling and Analysis of HCAb PR000020**

This example studied the preparation of an antibody-drug conjugate with uniform DAR values using cysteine coupling to Cys-220 site by anti-CTLA4 HCAb antibody PR000020 (SEQ ID NO: 64) and mc-vc-PAB-MMAE.

**Example 5.1 Optimization of coupling reaction conditions**

In this example, firstly, the method in 0 was used to produce and purify HCAb PR000020 recombinant protein; then, the method in 0 was used to couple the purified HCAb PR000020 under multiple combination conditions of different reduction, reoxidation and coupling conditions (Table 0-1), to try to obtain preferred reaction conditions. A reducing agent used was Tris(2-carboxyethyl)phosphine (TCEP) or dithiothreitol (DTT); an oxidant was dehydroascorbic acid (DHAA);

a drug conjugate was mc-vc-PAB-MMAE (Manufacturer: Levena Biopharma, Art. No.: SET0201). HCAb PR000020 was coupled using 6 groups of conditions in parallel, and trastuzumab (the 7th group of experiment) was used as the IgG1 control. In this experiment, the pH of reduction or coupling reaction was 6.5 to 7.0, and the temperature was room temperature (25 °C to 30 °C). The components of coupling products in each group of experiments were analyzed by the HIC-HPLC analysis method described in 0, and listed in Table 0-2.

**Table 0-1. Different coupling reaction conditions tried**

| Experiment # | Antibody | Reduction conditions | | | Reoxidation conditions | | Coupling conditions | |
|---|---|---|---|---|---|---|---|---|
| | | Reducing agent | Molar multiples of reducing agent | Reaction time | Molar multiples of oxidant | Reaction time | Molar multiples of conjugate | Reaction time |
| 1# | PR000020 | TCEP | 2 x | 4 h | 50x | 4 h | 7 x | 2.5 h |
| 2# | PR000020 | DTT | 50 x | 17 h | 50× | 3h | 7 x | 1 h |
| 3# | PR000020 | TCEP | 10 x | 17 h | None | None | 7 x | 2 h |
| 4# | PR000020 | DTT | 50 x | 17 h | None | None | 7 x | 2 h |
| 5# | PR000020 | TCEP | 1.5 x | 3 h | None | None | 7 x | 1.5 h |
| 6# | PR000020 | TCEP | 2.5 x | 3 h | None | None | 10 x | 1.5 h |
| 7# | trastuzumab | TCEP | 2.75 x | 3 h | None | None | 7 x | 1.5 h |

**Table 0-2. HIC-HPLC analysis results of products under different coupling reaction conditions**

| Experiment # | Antibody | HIC-HPLC analysis results |
|---|---|---|
| 1# | PR000020 | After the processes of reduction and reoxidation, there is no sulfhydryl produced that can be used for the reaction, and coupling cannot occur. |
| 2# | PR000020 | |
| 3# | PR000020 | All sulfhydryls of 6 cysteines in the hinge region are used for the reaction and coupled to the compound; but the product is uneven and unstable. |
| 4# | PR000020 | |
| 5# | PR000020 | The main product is HCAb (DAR = 2) coupled with 2 compounds, accounting for 92%. |
| 6# | PR000020 | The main product is HCAb (DAR = 2) coupled with 2 compounds, accounting for 76%. |
| 7# | trastuzumab | Trastuzumab-MMAE coupling product with an average DAR value of 4.1. |

In combination with Table 0-1 and Table 0-2, the main product produced under experimental conditions 5 and 6 is an antibody-drug conjugate coupled with 2 compound molecules (DAR = 2). Therefore, the optimized coupling reaction conditions preliminarily determined are as follows: TCEP with moderate reduction capacity is selected as a reducing agent, the amount of usage of the reducing agent is 1.5 to 2.5 molar multiples, the temperature of the reduction reaction is 25 °C to 30 °C, the pH for reaction is 6.5 to 7.0, and the reaction time is 3 h; the amount of usage of the drug conjugate mc-vc-PAB-MMAE used for coupling is 7 to 10 molar multiples, the temperature thereof is 25 °C to 30 °C, the pH thereof is 6.5 to 7.0, and the reaction time thereof is 1 to 2 h.

After the coupling was completed, the excess drug conjugates were removed from the product through a desalting column, and the HIC method in 0 was used to purify the coupling product to obtain a main product with DAR = 2.

**Example 5.2 Analysis of coupling product**

In this example, techniques such as high performance liquid chromatography and mass spectrometry were utilized to quantitatively analyze the purified recombinant HCAb PR000020 and a coupling product PR000020-ADC thereof (including a product after coupling and a product after one-step HIC purification) to determine the components of the coupling product.

The HIC-HPLC method described in 0 was used to analyze the above samples. 0 and the table below respectively shows HIC-HPLC analysis results of HCAb PR000020 and a coupling product PR000020-ADC thereof: (A) before HCAb coupling; (B) after coupling and before purification; (C) after coupling and one-step HIC purification. Table 0-3 shows that the sample purity before HCAb PR000020 coupling is as high as 95%; Table 0-4 shows that in the product of HCAb PR000020 coupled with cytotoxic compounds in 0, 75% of the components are coupled with two compounds (DAR = 2); Table 0-5 shows that in the product obtained after one-step HIC purification of the coupling product (0), 99% of the components are coupled with 2 compounds.

**Table 0-3. HIC-HPLC analysis results of HCAb PR000020 (lot.# HSP302)**

| Retention time (min) | Peak area% | Peak instructions |
|---|---|---|
| 7.22 | 95.76 | HCAb main peak |
| 8.26 | 4.00 | Antibody impurities |
| 9.56 | 0.24 | Antibody impurities |

**Table 0-4. HIC-HPLC analysis results of sample (lot.# HSP302-170814) before PR000020-ADC purification**

| Retention time (min) | Peak area% | Peak instructions |
|---|---|---|
| 7.53 | 6.87 | HCAb naked antibody peak |
| 8.53 | 1.61 | DAR = 1 component peak |

(continued)

| Retention time (min) | Peak area% | Peak instructions |
|---|---|---|
| 9.54 | 75.15 | DAR = 2 component peak |
| 11.25 | 3.29 | Polymer impurities or DAR > 2 component peak |
| 12.14 | 13.07 | Unreacted cytotoxic compound peak |

**Table 0-5. HIC-HPLC analysis results of sample (lot.# HSP302-170814PA0815) after PR000020-ADC purification**

| Retention time (min) | Peak area% | Peak instructions |
|---|---|---|
| 7.73 | 0.31 | HCAb naked antibody peak |
| 9.44 | 99.69 | DAR = 2 component peak |

The RP-HPLC method (using an analytical chromatography column Zorbax RRHD 300-Diphenyl) in 0 was used to analyze the above samples. 0 and the table below respectively shows RP-HPLC analysis results of HCAb PR000020 and a coupling product PR000020-ADC thereof: (A) before HCAb coupling; (B) after coupling and before purification; (C) after coupling and one-step HIC purification. Table 0-6 shows that the sample purity before HCAb PR000020 coupling is as high as 99%; Table 0-7 shows that in the product of HCAb PR000020 coupled with cytotoxic compounds in 0, 75% of the components are coupled with two compounds (DAR = 2); Table 0-8 shows that in the product obtained after one-step HIC purification of the coupling product (0), 96% of the components are coupled with 2 compounds.

**Table 0-6. RP-HPLC analysis results of HCAb PR000020 (lot.# HSP302)**

| Retention time (min) | Peak area% | Peak instructions |
|---|---|---|
| 35.46 | 99.06 | HCAb main peak |
| 36.99 | 0.94 | Antibody impurities |

**Table 0-7. RP-HPLC analysis results of sample (lot.# HSP302-170814) before PR000020-ADC purification**

| Retention time (min) | Peak area% | Peak instructions |
|---|---|---|
| 35.48 | 8.71 | HCAb naked antibody peak |
| 36.22 | 2.89 | DAR = 1 component peak |
| 37.10 | 75.88 | DAR = 2 component peak |
| 38.34 | 1.12 | Coupling product impurity peak |
| 39.39 | 7.86 | Unreacted cytotoxic compound peak |
| 41.96 | 3.54 | Unknown |

**Table 0-8. RP-HPLC analysis results of sample (lot.# HSP302-170814PA0815) after PR000020-ADC purification**

| Retention time (min) | Peak area% | Peak instructions |
|---|---|---|
| 35.68 | 0.73 | HCAb naked antibody peak |
| 36.54 | 3.09 | DAR = 1 component peak |
| 37.22 | 96.18 | DAR = 2 component peak |

The method (LC instrument: Waters Acquity UPLC; MS instrument: Waters Xevo G2-XS Q-TOF) in 0 was used to analyze the molecular weight of the sample after purification of the coupling product PR000020-ADC. Before LC-MS analysis, the sample to be tested was desaccharified. 0 respectively shows mass spectrometry deconvolution processing maps of molecular weight analysis of a coupling product PR000020-ADC: (A) non-reduced samples; (B) reduced samples. The theoretical values of the complete molecular weight (non-reduced molecular weight) and heavy chain molecular weight (reduced molecular weight) of PR000020 were calculated by using its amino acid sequence; the molecular weight of the conjugate mc-vc-PAB-MMAE was 1315.78 Da; the theoretical molecular weights of the components coupled with different numbers of compounds were calculated. The peaks and corresponding molecular weights in the maps were analyzed, and the peak area was calculated through integration to infer the content of the corresponding components (as shown in the table below). Table 0-9 shows that the component with DAR = 2 is a main ingredient; correspondingly, as shown in Table 0-10, in the reduced samples, nearly 90% of the heavy chain coupling has one compound (+1 D).

**Table 0-9. LC-MS analysis of non-reduced molecular weight of sample after PR000020-ADC purification**

| Component | Theoretical value (Da) | Experimental value (Da) | Deviation (Da) | Content |
|---|---|---|---|---|
| PR000020 naked antibody | 77,640 | | | |
| PR000020 DAR=1 | 78,956 | | | |
| PR000020 DAR=2 | 80,272 | 80,281 | +9 | Main ingredient |
| PR000020 DAR=3 | 81,588 | | | |

**Table 0-10. LC-MS analysis of reduced molecular weight of sample after PR000020-ADC purification**

| Component | Theoretical value (Da) | Experimental value (Da) | Deviation (Da) | Content |
|---|---|---|---|---|
| PR000020 heavy chain | 38,823 | 38,827 | +4 | 9.5 % |
| PR000020 heavy chain + 1 D | 40,139 | 40,145 | +6 | 87.6 % |
| PR000020 heavy chain + 2 D | 41,455 | 41,460 | +5 | 2.9 % |

Based on the results of various analytical methods above, it can be inferred that by using cysteine for coupling, the main ingredient in the coupling product is a component with DAR = 2. After further HIC purification, a highly-homogeneous product with a purity greater than 90% and DAR = 2 can be obtained.

**Example 5.3 Analysis of compound coupling site by LC-MS peptide map**

In this example, LC-MS was used to analyze ADC coupling sites. In short, the purified samples of uncoupled HCAb PR000020 and the coupling product PR000020-ADC were reduced using dithiothreitol (DTT); then, the active sulfhydryl of cysteine was blocked with iodoacetamide (IAM); next, the sample was cleaved with a trypsin digestive enzyme; the peptide fragment of an enzyme cleavage product was analyzed by LC-MS. Finally, the obtained data underwent library search using software Peaks Studio (Bioinformatics Solutions Inc.), and the drug coupling sites were determined finally by setting the possibility of coupling drugs occurring on cysteine Cys as variable modifications, in combination with the original secondary mass spectrum information, and by extracted ion chromatogram (XIC) for comparison.

If Cys reacts with mc-vc-PAB-MMAE to form a stable thioether bond, then the Cys site cannot form IAM modification through the aforementioned reduction alkylation method. There are 9 Cys on each heavy chain of HCAb PR000020 (numbered in accordance with the order positions of amino acid sequences, respectively, C23, C96, C124, C130, C133, C165, C225, C271, C329), where C124 corresponds to 220 according to Eu numbering. After analysis, in uncoupled PR000020 samples, all 9 Cys, except C225, are not detected due to the front and back dense distribution of pancreatin cleavage sites, and it is detected that Cys at the remaining 8 sites are IAM-modified at a higher degree due to reduction alkylation. The list of peptide fragments containing IAM-modified Cys (Cys-IAM) identified in uncoupled samples is listed in 0 (C). In the coupling product PR000020-ADC samples, a total of 1 drug coupling site, C124 (i.e., Cys-220 according to Eu numbering), is detected, while it is detected that the other 8 Cys are all IAM-modified. The list of peptide fragments containing IAM-modified Cys (Cys-IAM) identified in coupling samples is listed in 0 (B), and these peptide fragments are labeled with a gray background on the amino acid sequence of PR000020-ADC in 0 (A). It can be seen from this that in the PR000020-ADC samples, C124 (i.e., Cys-220) does not undergo IAM modification. When using Peaks Studio for library search, the possibility of a drug conjugate (mc-vc-PAB-MMAE, with a mass difference of 1315.78 Da) occurring on Cys was set as variable modification. The library search results show that only the peptide fragment PHGSDIWGQGTMVTVSSEPKSC#DK (# represents a modification site) containing C124 (i.e., Cys-220) is identified to be modified (i.e., drug coupling) in the coupling samples. The secondary mass spectrum of the peptide fragment is shown in 0 (m/z = 966.2409, z = 4, RT = 83.77 min). Since mc-vc-PAB-MMAE would be fragmentated (a characteristic fragment structure as shown in 0(B)) during the experimental process, resulting in the lack of y ions, the presence of characteristic fragment ions could be observed at the low-quality terminus of the secondary map of the peptide fragment, thus further confirming the coupling modification of C124 (i.e., Cys-220). By comparing the extracted ion chromatogram (XIC, 0) of the peptide fragment in the PR000020-ADC sample and the PR000020 monoclonal antibody sample, it was found that the signal of the peptide fragment undergoing drug coupling (RT = 83.77 min) was only detected in the ADC sample, while no corresponding signal was detected in the monoclonal antibody sample as a control. This further proved that the cysteine residue was a drug coupling site.

In summary, the analysis of PR000020-ADC coupling product by LC-MS peptide map confirms that drug coupling occurs at the site Cys-220.

### Example 5.4 Binding to cells highly expressing CTLA4

This example was to study the activity of HCAb antibody PR000020 and a coupling product PR000020-ADC thereof binding to cells highly expressing CTLA4.

The binding ability of the antibody molecules to an HEK293T cell strain HEK293/hCTLA4 (constructed by Harbour BioMed) highly expressing human CTLA4 and other cells was determined by flow cytometry FACS. Specifically, the HEK293/hCTLA4 cells were digested and resuspended in a DMEM medium, with the cell density adjusted to 1 × $10^6$ cells/mL. HEK293 cells not expressing CTLA4 were used as a negative control. Thereafter, the cells were seeded in a 96-well V-bottom plate (Corning, #3894) at 100 μL/well and centrifuged at 4 °C for 5 min, and the supernatant was discarded. Then, the serially diluted antibody molecules were added to the 96-well plate at 100 μL/well, and the mixtures were well mixed. The antibody molecules could be serially diluted 3-fold from the highest final concentration of 500 nM to obtain a total of 12 concentrations. The cells were incubated at 4 °C for 1 h away from light. Then, the cells in each well were rinsed twice with 100 μL of pre-cooled FACS buffer (PBS buffer containing 0.5% BSA) and centrifuged at 500 g at 4 °C for 5 min, and then the supernatant was discarded. Thereafter, a fluorescent secondary antibody (Alexa Fluor 488 anti-human IgG Fc, Biolegend, #409322, diluted in a 1:1000 ratio) was added at 100 μL/well, and the plate was incubated at 4 °C for 1 h away from light. Then, the cells in each well were then rinsed twice with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

As shown in 0, both the antibody PR000020 and the purified coupling product PR000020-ADC thereof have strong specific binding activity to the HEK293/hCTLA4 cells.

### Example 6 Coupling and Analysis of HCAb PR000759

This example studied the preparation of an antibody-drug conjugate with a uniform DAR value using cysteine coupling to Cys-220 site by an anti-MSLN HCAb antibody PR000759 (SEQ ID NO: 67) and mc-vc-PAB-MMAE.

### Example 6.1 Optimization of coupling reaction conditions

Based on the preliminarily determined optimized coupling reaction conditions obtained in 0, further optimization was made for the antibody molecule. As shown in Table 0-1, the purified HCAb PR000759 recombinant protein was coupled using 5 groups of conditions in parallel; TCEP was selected as a reducing agent, and the selected drug conjugate was mc-vc-PAB-MMAE. The amount of usage of the drug conjugate used for coupling was 5 to 18 molar multiples, and the reaction time was 1 to 2 h. In this experiment, the pH of reduction or coupling reaction was 6.5 to 7.0, and the temperature was room temperature (25 °C to 30 °C). The components of coupling products in each group of experiments were analyzed by the HIC-HPLC analysis method described in 0, and listed in Table 0-1. In this example, the coupling reaction conditions of experimental group #2 could achieve the maximum proportion of DAR = 2 components ('D2 %' in Table 0-1). After the coupling was completed, the excess drug conjugates were removed from the product through a desalting column, and the HIC method in 0 was used to purify the coupling product to obtain a main product with DAR = 2.

**Table 0-1. Coupling reaction conditions of HCAb PR000759**

| Experiment # | HCAb concentration | Amount of usage of TCEP | Amount of usage of VcMMAE | HIC-HPLC analysis results of coupling product | | |
|---|---|---|---|---|---|---|
| | | | | Average DAR | Naked antibody % | D2 % |
| 1 | 5.0 mg/ml | 1.2 x | 5.0 x | 1.15 | 43.2% | 50.3% |
| 2 | 5.0 mg/ml | 1.5 x | 5.0 x | 1.83 | 18.7% | 65.2% |
| 3 | 5.0 mg/ml | 5.0 x | 18.0 x | 2.44 | 8.6% | 52.6% |
| 4 | 5.0 mg/ml | 2.0 x | 5.0 x | 1.52 | 34.6% | 37.0% |
| 5 | 5.0 mg/ml | 2.0 x | 5.0 x | 1.83 | 25.0% | 40.4% |

**Example 6.2 Analysis of coupling product**

In this example, techniques such as high-performance liquid chromatography and mass spectrometry were utilized to quantitatively analyze the purified recombinant HCAb PR000759 and a coupling product PR000759-ADC thereof (including a product after coupling and a product after one-step HIC purification) to determine the components of the coupling product.

The SEC-HPLC method described in 0 and the HIC-HPLC method described in 0 were used to analyze the above samples. 0 and Table 0-2 show SEC-HPLC analysis results of samples before HCAb PR000759 recombinant protein coupling, with a purity as high as 95%. 0 and Table 0-3 show HIC-HPLC analysis results of a product obtained after one-step HIC purification (0) of a coupling product PR000759-ADC, showing the product with 94% of the components that are coupled with 2 compounds (DAR = 2).

**Table 0-2. SEC-HPLC analysis results of HCAb PR000759 (lot.# HSP307A PA1222 F3-4)**

| Retention time (min) | Peak area% | Peak instructions |
|---|---|---|
| 5.68 | 3.14 | |
| 7.22 | 1.43 | |
| 8.38 | 95.43 | HCAb main peak |

**Table 0-3. HIC-HPLC analysis results of sample (lot.# HSP307A-181229 MIX) after purification of PR000759-ADC**

| Retention time (min) | Peak area% | Peak instructions |
|---|---|---|
| 7.39 | 0.20 | HCAb naked antibody peak |
| 8.40 | 2.12 | DAR = 1 component peak |
| 9.44 | 93.91 | DAR = 2 component peak |
| 10.73 | 1.29 | Impurities or DAR > 2 component peak |
| 11.34 | 2.47 | |

The method described in 0 was used to analyze the molecular weights of HCAb PR000759 and the sample after purification of PR000759-ADC. Before LC-MS analysis, the sample to be tested was desaccharified. 0 shows mass spectrometry deconvolution processing maps of molecular weight analysis: (A) non-reduced samples of PR000759; (B) non-reduced samples ofPR000759-ADC; (C) reduced samples of PR000759-ADC. According to the process of the molecular weight analysis method described in 0, the molecular weight of the components coupled with different numbers of compounds and the contents thereof in the samples were calculated (see the table below). Table 0-4 shows actually determined molecular weights of HCAb PR000759. Table 0-5 shows that in the non-reduced samples of PR000759-ADC, the component with DAR = 2 is a main ingredient (accounting for 91%), and the average DAR value of PR000759-ADC product is calculated to be 2.10. Correspondingly, as shown in Table 0-6, in the reduced samples of PR000759-ADC, the heavy chain coupled with 1 compound (+1 D) is a main ingredient, and the average DAR value of PR000759-ADC product is calculated to be 1.98.

**Table 0-4. LC-MS analysis of non-reduced molecular weight of HCAb PR000759 (lot.# HSP307A PA1222 F3-4)**

| Component | Theoretical value (Da) | Experimental value (Da) | Deviation (Da) | Content |
|---|---|---|---|---|
| PR000759 monoclonal antibody | 78,645 | 78,648~-78,776 | +3~ +131 * | Main ingredient |
| (* may contain 1 C' -terminal Lys not cleaved (+ 128 Da)) | | | | |

**Table 0-5. LC-MS analysis of non-reduced molecular weight of sample (lot.# HSP307A-181229 MIX) after purification of PR000759-ADC**

| Component | Theoretical value (Da) | Experimental value (Da) | Deviation (Da) | Content |
|---|---|---|---|---|
| PR000759 naked antibody | 78,645 | 78,650 | +5 | 0.8 % |
| PR000759 DAR=1 | 79,961 | 79,971 | +10 | 2.4 % |
| PR000759 DAR=2 | 81,277 | 81,284 | +7 | 90.6 % |
| PR000759 DAR=3 | 82,593 | 82,607 | +14 | 1.8 % |
| PR000759 DAR=4 | 83,909 | 83,922 | +13 | 2.3 % |
| PR000759 DAR=6 | 86,541 | 86,554 | +13 | 2.0 % |

**Table 0-6. LC-MS analysis of reduced molecular weight of sample (lot.# HSP307A-181229 MIX) after purification of PR000759-ADC**

| Component | Theoretical value (Da) | Experimental value (Da) | Deviation (Da) | Content |
|---|---|---|---|---|
| PR000759 heavy chain | 39,324 | 39,330 | +6 | 10.2 % |
| PR000759 heavy chain + 1D | 40,639 | 40,646 | +7 | 83.0 % |
| PR000759 heavy chain + 2D | 41,955 | 41,964 | +9 | 4.5 % |
| PR000759 heavy chain + 3D | 43,271 | 43,280 | +9 | 2.3 % |

Based on the results of various analytical methods above, it can be inferred that by using cysteine for coupling, the main ingredient in the coupling product is a component with DAR = 2. After further HIC purification, a highly-homogeneous product with a purity greater than 90% and DAR = 2 can be obtained.

**Example 6.3 Analysis of compound coupling site by LC-MS peptide map**

In this example, LC-MS was used to analyze ADC coupling sites. The first step was to preprocess a sample PR000759-ADC to be tested. The sample was transferred to a 10 KD ultrafiltration tube, and after three times of ultrafiltration, the sample was replaced to a buffer containing 8 M urea; dithiothreitol (DTT) with a final concentration of 20 mM was then added, and the mixture was reacted at 37 °C for 1 h; subsequently, indole-3-acetic acid (IAA) with a final concentration of 50 mM was added, and the mixture was reacted at room temperature for 30 min away from light; after ultrafiltration again, the sample was replaced to a buffer containing 50 mM ammonium bicarbonate, and trypsin was added in a ratio of sample:enzyme = 50:1, for cleavage at 37 °C overnight. The second step was to analyze the sample to be tested. The sample was identified by LC-MS/MS after trypsin cleavage, and library search was performed using software Peaks Studio (Bioinformatics Solutions Inc.). The parameters were set as follows: trypsin cleavage; allowable fragment ion mass error: 0.05 Da; allowable parent ion mass error: 10 ppm; maximum number of missed cleavages: 2; variable modifications: Carbamidomethylation 57.02, Oxidation (M) 15.99, Deamidation (NQ) 0.98, Pyroglutamate formation (N-term E) -18.01, ADC drug 1315.78.

The results of library search were rigorously Chi-square filtered to obtain a trusted peptide fragment (-10lgP ≥ 20), with a sequence coverage rate of nearly 100%. Three peptide fragments with mc-vc-PAB-MMAE coupling sites were selected from the peptide fragment list (0), and the results showed that PR000759-ADC was identified with coupling modifications on cysteines at site 127 (220 according to Eu numbering), site 136 (229 according to Eu numbering), and site 168 (261 according to Eu numbering) (as shown in bold C in 0). Further verification was conducted on the corresponding secondary maps of these three peptide fragments one by one, confirming the modifications of Δmass = 1315.78 Da occurring on the peptide fragments were highly reliable; in addition, by enlarging the low-quality terminus of the secondary maps, the presence of characteristic fragment ions generated after the fragmentation of mc-vc-PAB-MMAE could be clearly seen, further confirming the coupling modification sites.

In the peptide map analysis, the proportion of each coupling site in the product cannot be accurately quantified; however, in combination with the previous HIC-HPLC and LC-MS molecular weight analysis, it has been demonstrated that the vast majority of coupling occurs at the cysteine at site 127 (i.e., Cys-220) of PR000759 to form a coupling product with DAR = 2.

**Example 7 Coupling and Analysis of HCAb PR001046**

This example studied the preparation of an antibody-drug conjugate with a uniform DAR value using cysteine coupling to Cys-220 site by an anti-BCMAHCAb antibody PR001046 (SEQ ID NO: 68) and mc-vc-PAB-MMAE.

**Example 7.1 Optimization of coupling reaction conditions**

Based on the preliminarily determined optimized coupling reaction conditions obtained in 0, further optimization was made for the antibody molecule. As shown in Table 0-1, the purified HCAb PR001046 recombinant protein was coupled using 6 groups of conditions in parallel; TCEP was selected as a reducing agent, and the selected drug conjugate was mc-vc-PAB-MMAE. The amount of usage of the drug conjugate used for coupling was 5 to 8 molar multiples, and the reaction time was 1 to 3 h. In this experiment, the pH of reduction or coupling reaction was 6.5 to 7.0, and the temperature was room temperature (25 °C to 30 °C). The components of coupling products in each group of experiments were analyzed by the HIC-HPLC analysis method described in 0, and listed in Table 0-1. When the amount of usage of the TCEP was 1.3 to 1.5 molar multiples, the DAR = 2 component yield ('D2%' in Table 0-1) was maximum. After the coupling was completed, the excess drug conjugates were removed from the product through a desalting column, and the HIC method in 0 was used to purify the coupling product to obtain a main product with DAR = 2.

**Table 0-1. Coupling reaction conditions of HCAb PR001046**

| Experiment # | HCAb concentration | Amount of usage of TCEP | Amount of usage of VcMMAE | HIC-HPLC analysis results of coupling product | | |
|---|---|---|---|---|---|---|
| | | | | Average DAR | Naked antibody % | D2 % |
| 1 | 5.0 mg/ml | 1.3 x | 8.0 x | 1.82 | 16.9% | 64.8% |
| 2 | 5.0 mg/ml | 1.5 x | 8.0 x | 1.98 | 12.3% | 64.9% |
| 3 | 5.0 mg/ml | 1.7 x | 8.0 x | 2.06 | 10.7% | 62.8% |
| 4 | 5.0 mg/ml | 2.0 x | 8.0 x | 2.22 | 8.5% | 57.4% |
| 5 | 5.0 mg/ml | 1.5 x | 5.0 x | 1.97 | 12.6% | 65.4% |
| 6 | 2.0 mg/ml | 1.5 x | 5.0 x | 1.77 | 17.9% | 67.1% |

**Example 7.2 Analysis of coupling product**

In this example, techniques such as high-performance liquid chromatography and mass spectrometry were utilized to quantitatively analyze the purified recombinant HCAb PR001046 and a coupling product PR001046-ADC thereof (including a product after coupling and a product after one-step HIC purification) to determine the components of the coupling product.

The SEC-HPLC method described in 0 and the HIC-HPLC method described in 0 were used to analyze the above samples. 0 and Table 0-2 show SEC-HPLC analysis results of samples before HCAb PR001046 recombinant protein coupling, with a purity as high as 98%. 0 and Table 0-3 show HIC-HPLC analysis results of a product obtained after one-step HIC purification (0) of a coupling product PR001046-ADC, showing the product with 88% of the components that are coupled with 2 compounds (DAR = 2).

**Table 0-2. SEC-HPLC analysis results of HCAb PR001046 (lot.# HSP307B PA1222 F11-12)**

| Retention time (min) | Peak area% | Peak instructions |
|---|---|---|
| <7 | 0.28 | |
| 7.31 | 1.59 | - |
| 8.30 | 98.13 | HCAb main peak |

**Table 0-3. HIC-HPLC analysis results of sample (lot.# HSP307B-181229 MIX) after purification of PR001046-ADC**

| Retention time (min) | Peak area% | Peak instructions |
|---|---|---|
| 7.58 | 0.87 | HCAb naked antibody peak |
| 8.58 | 1.20 | DAR = 1 component peak |
| 9.50 | 88.19 | DAR = 2 component peak |
| 10.65 | 3.47 | Impurities or DAR > 2 component peak |
| 11.06 | 6.27 | |

The method described in 0 was used to analyze the molecular weights of HCAb PR001046 and the sample after purification of PR001046-ADC. Before LC-MS analysis, the sample to be tested was desaccharified. 0 shows mass spectrometry deconvolution processing maps of molecular weight analysis: (A) non-reduced samples ofPR001046; (B) non-reduced samples ofPR001046-ADC; (C) reduced samples of PR001046-ADC. According to the process of the molecular weight analysis method described in 0, the molecular weight of the components coupled with different numbers of compounds and the contents thereof in the samples were calculated (see the table below). Table 0-4 shows actually determined molecular weights of HCAb PR001046. Table 0-5 shows that in the non-reduced samples of PR001046-ADC, the component with DAR = 2 is a main ingredient (accounting for 81%), and the average DAR value of PR001046-ADC product is calculated to be 2.39. Correspondingly, as shown in Table 0-6, in the reduced samples of PR001046-ADC, the heavy chain coupled with 1 compound (+1 D) is a main ingredient, and the average DAR value of PR001046-ADC product is calculated to be 2.20.

**Table 0-4. LC-MS analysis of non-reduced molecular weight of HCAb PR001046 (lot.# HSP307B PA1222 F11-12)**

| Component | Theoretical value (Da) | Experimental value (Da) | Deviation (Da) | Content |
|---|---|---|---|---|
| PR001046 monoclonal antibody | 78,735 | 78,738~-78,866 | +3~ +131 * | Main ingredient |
| (* may contain 1 C' -terminal Lys not cleaved (+ 128 Da)) | | | | |

**Table 0-5. LC-MS analysis of non-reduced molecular weight of sample (lot.# HSP307B-181229 MIX) after purification of PR001046-ADC**

| Component | Theoretical value (Da) | Experimental value (Da) | Deviation (Da) | Content |
|---|---|---|---|---|
| PR001046 naked antibody | 78,735 | 78,739 | +4 | 1.3 % |
| PR001046 DAR=1 | 80,051 | 80,057 | +6 | 0.5 % |

(continued)

| Component | Theoretical value (Da) | Experimental value (Da) | Deviation (Da) | Content |
|---|---|---|---|---|
| PR001046 DAR=2 | 81,367 | 81,373 | +6 | 81.2 % |
| PR001046 DAR=3 | 82,683 | 82,691 | +8 | 3.9 % |
| PR001046 DAR=4 | 83,999 | 84,009 | +10 | 6.7 % |
| PR001046 DAR=5 | 85,315 | 85,331 | +16 | 0.4 % |
| PR001046 DAR=6 | 86,631 | 86,643 | +12 | 6.0 % |

**Table 0-6. LC-MS analysis of reduced molecular weight of sample (lot.# HSP307B-181229 MIX) after purification of PR001046-ADC**

| Component | Theoretical value (Da) | Experimental value (Da) | Deviation (Da) | Content |
|---|---|---|---|---|
| PR001046 heavy chain | 39,369 | 39,375 | +6 | 7.8 % |
| PR001046 heavy chain + 1D | 40,685 | 40,692 | +7 | 79.3 % |
| PR001046 heavy chain + 2D | 42,001 | 42,009 | +8 | 8.3 % |
| PR001046 heavy chain + 3D | 43,317 | 43,325 | +8 | 4.7 % |

Based on the results of various analytical methods above, it can be inferred that by using cysteine for coupling, the main ingredient in the coupling product is a component with DAR = 2. After further HIC purification, a highly-homogeneous product with a purity greater than 80% and DAR = 2 can be obtained.

**Example 7.3 Analysis of compound coupling site by LC-MS peptide map**

In this example, the method described in 0 was used to analyze coupling sites of PR001046-ADC.

Three peptide fragments with mc-vc-PAB-MMAE coupling sites were listed in 0, and the results showed that PR001046-ADC was identified with coupling modifications on cysteines at site 127 (220 according to Eu numbering), site 136 (229 according to Eu numbering), and site 168 (261 according to Eu numbering) (as shown in bold C in 0). Further verification was conducted on the corresponding secondary maps of these three peptide fragments one by one, confirming the modifications of Δmass = 1315.78 Da occurring on the peptide fragments were highly reliable; for example, 0 shows the secondary maps of peptide fragments containing C220 coupling sites. In the peptide map analysis, the proportion of each coupling site in the product cannot be accurately quantified; however, in combination with the previous HIC-HPLC and LC-MS molecular weight analysis, it has been demonstrated that the vast majority of coupling occurs at the cysteine at site 127 (i.e., Cys-220) of PR001046 to form a coupling product with DAR = 2.

**Example 7.4 Binding to cells highly expressing BCMA**

This example was to study the activity of HCAb antibody PR001046 and a coupling product PR001046-ADC thereof binding to cells highly expressing BCMA.

The binding ability of the antibody molecules to an HEK293T cell strain HEK293T-hBCMA (Beijing KYinno, KC-0233) highly expressing human BCMA was determined by flow cytometry FACS. Specifically, the cells were digested and resuspended in a DMEM complete medium, and the cell density was adjusted to $1 \times 10^6$ cells/mL. The cells were seeded in a 96-well V-bottom plate (Corning, #3894) at 100 μL/well, followed by the addition of test antibodies diluted in a 3-fold gradient at a concentration that was 2 times the final concentration, each at 100 μL/well. The cells were incubated at 4 °C for 1 h away from light. Thereafter, the cells in each well were rinsed twice with 100 μL of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcγ Fragment Specific, Jackson

ImmunoResearch Inc., #109-545-098, diluted in a 1:500 ratio) was added at 100 μL/well, and the plate was incubated at 4 °C for 30 min away from light. The cells in each well were then rinsed twice with 100 μL of pre-cooled PBS and centrifuged at 500 g at 4 °C for 5 min, and then the supernatant was discarded. Finally, the cells in each well were resuspended in 200 μL of pre-cooled PBS. Fluorescence signal values were read using a BD FACS CANTOII flow cytometer, and the data were processed and analyzed using FlowJo v10 (FlowJo, LLC) software.

The data were processed and analyzed by plotting using GraphPad Prism 8 software, and binding curves of the antibodies to target cells, $EC_{50}$ values and other parameters were obtained through four-parameter nonlinear fitting.

As shown in 0, both the antibody PR001046 and the purified coupling product PR001046-ADC thereof have strong specific binding activity to the HEK293T-hBCMA cells.

**Example 7.5 Cytotoxic killing experiment**

This example was to study the specific cell killing activity of the anti-BCMA antibody coupling product PR001046-ADC against a cell line NCI-H929 (ATCC, CRL-9068) highly expressing human BCMA, without killing activity against SNU-16 cells (ATCC, CRL-5974) not expressing human BCMA.

The NCI-H929 cells and SNU-16 cells were seeded in 96-well plates (Perkin Elmer, #6005225) respectively, with the following seeded cell numbers: 10000 cells/50 μL/well (NCI-H929), 5000 cells/50 μL/well (SNU-16), 10000 NCI-H929 cells and 5000 SNU-16 cells/100 μL/well mixture (NCI-H929 + SNU-16). Then, a test sample serially pre-diluted was added at 50 μL/well, with concentrations of 1 μg/mL, 0.1 μg/mL and 0.01 μg/mL for PR001046 and PR001046-ADC, and concentrations of 10 nM, 1 nM and 0.1 nM for mc-vc-PAB-MMAE. Thereafter, the plates were incubated at 37 °C with 5% $CO_2$ for 72 h, a CellTiter-Glo cell viability detection reagent (Promega, #G7573) was added, then the plates were incubated at room temperature for at least 10 min to obtain stable luminescence signals, and the luminescence values were detected using a microplate reader. The data were processed and analyzed by plotting using a GraphPad Prism 8 software. In this example, multiple groups of different PR001046-ADC samples were used, including samples containing different percentages of DAR = 2 (D2) components before or after purification.

As shown in 0, PR001046-ADC with concentrations of 0.1 μg/mL and above can specifically kill the BCMA-positive cells NCI-H929 and the mixed cells of NCI-H929 and SNU-16, but cannot kill the BCMA-negative cells SNU-16. As shown in 0(A), uncoupled PR001046 or mc-vc-PAB-MMAE compounds cannot effectively kill the cells. In another aspect, as shown in 0(B), the purity of the main ingredient (DAR = 2) of the coupling product does not significantly affect the killing ability.

**Example 8 Coupling and Analysis of HCAb PR004432**

This example studied the preparation of an antibody-drug conjugate with a uniform DAR value using cysteine coupling to Cys-220 site by an anti-5T4 HCAb antibody PR004432 (SEQ ID NO: 69) and mc-vc-PAB-MMAE.

**Example 8.1 Optimization of coupling reaction conditions**

Based on the preliminarily determined optimized coupling reaction conditions obtained in 0, further optimization was made for the antibody molecule. As shown in Table 0-1, the purified HCAb PR004432 recombinant protein was coupled using 2 groups of conditions in parallel; TCEP was selected as a reducing agent, with the amount of usage of the TCEP of 4 molar multiples, and the selected drug conjugate was mc-vc-PAB-MMAE. The amount of usage of the drug conjugate used for coupling was 12 molar multiples, and the reaction time was 1 to 2 h. In this experiment, the pH of reduction or coupling reaction was 6.5 to 7.0, and the temperature was room temperature (25 °C to 30 °C). The components of coupling products in each group of experiments were analyzed by the RP-HPLC analysis method (using an analytical chromatography column PLRP-S 1000A) described in 0, and listed in Table 0-1; the DAR = 2 component yield ('D2 %') was about 60%. After the coupling was completed, the excess drug conjugates were removed from the product through a desalting column, and the HIC method in 0 was used to purify the coupling product to obtain a main product with DAR = 2.

**Table 0-1. Coupling reaction conditions of HCAb PR004432**

| Experiment # | HCAb concentration | Amount of usage of TCEP | Amount of usage of VcMMAE | RP-HPLC analysis results of coupling product | | |
|---|---|---|---|---|---|---|
| | | | | Average DAR | Naked antibody % | D2 % |

(continued)

| Experiment # | HCAb concentration | Amount of usage of TCEP | Amount of usage of VcMMAE | RP-HPLC analysis results of coupling product | | |
|---|---|---|---|---|---|---|
| 20052501 | 5.0 mg/ml | 4.0 x | 12.0 x | 2.1 | | 61.1% |
| 20060301 | 5.0 mg/ml | 4.0 x | 12.0 x | 2.1 | | 62.6% |

**Example 8.2 Analysis of coupling product**

In this example, techniques such as high-performance liquid chromatography and mass spectrometry were utilized to quantitatively analyze the purified recombinant HCAb PR004432 and a coupling product PR004432-ADC thereof (including a product after coupling and a product after one-step HIC purification) to determine the components of the coupling product.

The SEC-HPLC method described in 0 and the RP-HPLC method (using an analytical chromatography column PLRP-S 1000A) described in 0 were used to analyze the above samples. 0 and Table 0-2 show SEC-HPLC analysis results of samples before HCAb PR004432 recombinant protein coupling, with a purity as high as 98%. 0 and Table 0-3 show RP-HPLC analysis results of a product obtained after one-step HIC purification (0) of a coupling product PR004432-ADC, showing the product with 90.6% of the components that are coupled with 2 compounds (DAR = 2).

**Table 0-2. SEC-HPLC analysis results of HCAb PR004432 (lot.# HSP314-01 K20200427-0507PB0507)**

| Retention time (min) | Peak area% | Peak instructions |
|---|---|---|
| 6.90 | 0.59 | |
| 7.32 | 0.76 | |
| 7.70 | 98.64 | HCAb main peak |

**Table 0-3. RP-HPLC analysis results of sample (lot.# HSP31401-ADC-20200609) after purification of PR004432-ADC**

| Retention time (min) | Peak area% | Peak instructions |
|---|---|---|
| 15.55 | 4.48 | HCAb naked antibody peak |
| 16.90 | 3.50 | DAR = 1 component peak |
| 18.15 | 90.63 | DAR = 2 component peak |
| 20.07 | 1.36 | Impurities or DAR > 2 component peak |
| 21.40 | 0.04 | |

The method (LC instrument: Agilent 1290 UPLC; MS instrument: AB Sceix X500B) described in 0 was used to analyze the molecular weight of the sample after purification of PR004432-ADC. Before LC-MS analysis, the sample to be tested was desaccharified. 0 shows mass spectrometry deconvolution processing maps of molecular weight analysis: (A) non-reduced samples of PR004432-ADC; (B) reduced samples of PR004432-ADC. According to the process of the molecular weight analysis method described in 0, the molecular weight of the components coupled with different numbers of compounds and the contents thereof in the samples were calculated (see the table below). Table 0-4 shows that in the non-reduced samples of PR004432-ADC, the component with DAR = 2 is a main ingredient (accounting for 98.3%), and the average DAR value of PR004432-ADC product is calculated to be 1.97. Correspondingly, as shown in Table 0-5, in the reduced samples of PR004432-ADC, the heavy chain coupled with 1 compound (+1 D) is a main ingredient (accounting for 96.5%), and the average DAR value of PR004432-ADC product is calculated to be 1.93.

**Table 0-4. LC-MS analysis of non-reduced molecular weight of sample (lot.# HSP31401-ADC-20200609) after purification of PR004432-ADC**

| Component | Theoretical value (Da) | Experimental value (Da) | Deviation (Da) | Content |
|---|---|---|---|---|
| PR004432 naked antibody | 80,531 | 80,534 ~ 80,549 | +3 ~ +18 | 1.7 % |
| PR004432 DAR=1 | 81,847 | | | |
| PR004432 DAR=2 | 83,163 | 83, 138 ~ 83,397 | -25 ~ +234 * | 98.3 % |
| PR004432 DAR=3 | 84,479 | | | |
| (* may include molecular weight deviations caused by C'-terminus Lys not cleaved (+ 128 Da) or other modifications) | | | | |

**Table 0-5. LC-MS analysis of reduced molecular weight of sample (lot.# HSP31401-ADC-20200609) after purification of PR004432-ADC**

| Component | Theoretical value (Da) | Experimental value (Da) | Deviation (Da) | Content |
|---|---|---|---|---|
| PR004432 heavy chain | 40,266 | 40,199 ~ 40,399 | -6 ~ +133 | 3.5 % |
| PR004432 heavy chain + 1D | 41,582 | 41,448 ~ 41,714 | -134 ~ +132 * | 96.5 % |
| PR004432 heavy chain + 2D | 42,898 | | | |
| (* may include molecular weight deviations caused by C'-terminus Lys not cleaved (+ 128 Da) or other modifications) | | | | |

Based on the results of various analytical methods above, it can be inferred that by using cysteine for coupling, the main ingredient in the coupling product is a component with DAR = 2. After further HIC purification, a highly-homogeneous product with a purity greater than 90% and DAR = 2 can be obtained.

**Example 8.3 Analysis of compound coupling site by LC-MS peptide map**

In this example, the method described in 0 was used to analyze coupling sites of PR004432-ADC. In short, the PR004432-ADC sample was identified by LC-MS/MS after trypsin cleavage, and library search was performed using software BioPharmView (SCIEX) to obtain the data. The parameters for the library search were set as follows: trypsin cleavage; allowable fragment ion mass error: 0.05 Da; allowable parent ion mass error: 10 ppm; maximum number of missed cleavages: 1; variable modifications: Carbamidomethylation 57.02, Oxidation (M) 15.99, Deamidation (NQ) 0.98, mc-vc-PAB-MMAE 1315.78. The relevant target peptide fragments with compound coupling modifications (molecular weight difference of 1315.78) were found and the corresponding secondary maps were checked for further confirmation. The occupancy rate of a coupling site (the proportion of a compound linked to this site) was calculated using the following formula:

$$Coupling\ rate\ \% = \frac{The\ sum\ of\ peak\ areas\ of\ peptide\ fragments\ linked\ to\ the\ compound\ at\ this\ site}{The\ sum\ of\ peak\ areas\ of\ all\ peptide\ fragments\ at\ this\ site}$$

0 shows analysis of compound coupling sites of PR004432-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing SC (+ 1315.78) DK accounted for 97.12%, indicating that 97.12% of the Cys-220 site was coupled with a compound. Similarly, only 1.5% of the Cys-226 or Cys-229 site was coupled.

**Example 8.4 Binding to cells highly expressing ST4**

This example was to study the activity of HCAb antibody PR004432 and a coupling product PR004432-ADC thereof binding to cells highly expressing BCMA.

The binding ability of the antibody molecules to a human breast cancer cell strain HCC1954 (ATCC, CRL-2338) highly expressing human 5T4 was determined by flow cytometry FACS. Specifically, the cell density was adjusted to 1 $\times$ $10^6$ cells/mL. The cells were seeded in a 96-well V-bottom plate (Corning, #3894) at 100 μL/well, followed by the addition of test antibodies diluted in a 3-fold gradient at a concentration that was 2 times the final concentration, each at 100 μL/well. The cells were incubated at 4 °C for 1 h away from light. Thereafter, the cells in each well were rinsed twice with 100 μL of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcy Fragment Specific, Jackson ImmunoResearch Inc., #109-545-098, diluted in a 1:500 ratio) was added at 100 μL/well, and the plate was incubated at 4 °C for 30 min away from light. The cells in each well were then rinsed twice with 100 μL of pre-cooled PBS and centrifuged at 500 g at 4 °C for 5 min, and then the supernatant was discarded. Finally, the cells in each well were resuspended in 200 μL of pre-cooled PBS. Fluorescence signal values were read using a BD FACS CANTOII flow cytometer, and the data were processed and analyzed using FlowJo v10 (FlowJo, LLC) software.

The data were processed and analyzed by plotting using GraphPad Prism 8 software, and binding curves of the antibodies to target cells, $EC_{50}$ values and other parameters were obtained through four-parameter nonlinear fitting.

As shown in 0, both the antibody PR004432 and the purified coupling product PR004432-ADC thereof have strong specific binding activity to the HCC1954 cells.

**Example 8.5 Cytotoxic killing experiment**

This example was to study the cell killing activity of the anti-5T4 antibody coupling product PR004432-ADC against a human breast cancer cell strain HCC1954 (ATCC, CRL-2338) highly expressing human 5T4.

The HCC1954 cells were seeded in a 96-well plate (Perkin Elmer, #6005225) at 4000 cells/50 μL/well and incubated overnight at 37 °C with 5% $CO_2$. Then, a test sample serially pre-diluted was added at 50 μL/well. PR004432, PR004432-ADC and PR004433-ADC were diluted at a 3-fold concentration gradient from the highest final concentration of 30 nM, and mc-vc-PAB-MMAE was diluted at a 3-fold concentration gradient from the highest final concentration of 60 nM. In addition, a well without the test sample was set as a control well. Thereafter, the plates were incubated at 37 °C with 5% $CO_2$ for 72 h, a CellTiter-Glo cell viability detection reagent (Promega, #G7573) was added, then the plates were incubated at room temperature for at least 10 min to obtain stable luminescence signals, and the luminescence values were detected using a microplate reader. The cell viability was calculated based on the luminescence values by the following formula:

$$\text{Cell viability (\%)} = (A / B) \times 100\%$$

In which, A= experimental well (target cells + test sample + medium); B = control well (target cells + medium)

The data were processed and analyzed by plotting using GraphPad Prism 8 software, and binding curves of the antibodies to target cells, $EC_{50}$ values and other parameters were obtained through four-parameter nonlinear fitting.

0 shows the cytotoxic killing of the anti-5T4 HCAb PR004432 and the coupling product PR004432-ADC thereof, the coupling product PR004433-ADC of anti-BCMA HCAb PR004433, the uncoupled cytotoxic compound mc-vc-PAB-MMAE and other test samples against the HCC1954 cells highly expressing human 5T4. Only PR004432-ADC can produce effective and specific cytotoxic killing against HCC1954, and exhibit antibody concentration dependence; mc-vc-PAB-MMAE only has a killing effect at the highest concentration (60 nM), while other molecules cannot produce effective killing against the cells. These results confirm that PR004432-ADC has the high specificity and high efficacy of target dependence.

**Example 9 Coupling and Analysis of HCAb PR004433**

This example studied the preparation of an antibody-drug conjugate with a uniform DAR value using cysteine coupling to Cys-220 site by an anti-BCMAHCAb antibody PR004433 (SEQ ID NO: 70) and mc-vc-PAB-MMAE.

**Example 9.1 Optimization of coupling reaction conditions**

Based on the preliminarily determined optimized coupling reaction conditions obtained in 0, further optimization was made for the antibody molecule. As shown in Table 0-1, the purified HCAb PR004433 recombinant protein was coupled using 6 groups of conditions in parallel; TCEP was selected as a reducing agent, with the amount of usage of the TCEP of 1.7 to 2.1 molar multiples, and the selected drug conjugate was mc-vc-PAB-MMAE. The amount of usage of the drug conjugate used for coupling was 7 molar multiples, and the reaction time was 2 h. In this experiment, the pH of reduction or coupling reaction was 6.5 to 7.0, and the temperature was room temperature (25 °C to 30 °C). The components of coupling products in each group of experiments, particularly the content of the component with DAR = 2 ('D2 %'), were analyzed by the HIC-HPLC analysis method described in 0, and listed in Table 0-1. After the coupling was completed, the excess drug conjugates were removed from the product through a desalting column, and the HIC method in 0 was used to purify the coupling product to obtain a main product with DAR = 2.

**Table 0-1. Coupling reaction conditions of HCAb PR004433**

| Experiment # | HCAb concentration | Amount of usage of TCEP | Amount of usage of VcMMAE | HIC-HPLC analysis results of coupling product | | |
|---|---|---|---|---|---|---|
| | | | | Average DAR | Naked antibody % | D2 % |
| 20041501 | 5.0 mg/ml | 2.0 x | 7.0 x | 2.2 | | 52.4% |
| 20041601 | 5.0 mg/ml | 1.7 x | 7.0 x | 2.1 | | 52.7% |
| 20042101 | 5.0 mg/ml | 2.0 x | 7.0 x | 2.2 | | 61.0% |
| 20050601 | 5.0 mg/ml | 2.1 x | 7.0 x | 2.0 | | 53.9% |
| 20050801 | 5.0 mg/ml | 2.1 x | 7.0 x | 2.0 | | 50.6% |
| 20051201 | 5.0 mg/ml | 2.1 x | 7.0 x | 1.8 | | 56.1% |

**Example 9.2 Analysis of coupling product**

In this example, techniques such as high-performance liquid chromatography and mass spectrometry were utilized to quantitatively analyze the purified recombinant HCAb PR004433 and a coupling product PR004433-ADC thereof (including a product after coupling and a product after one-step HIC purification) to determine the components of the coupling product.

The SEC-HPLC method described in 0 and the HIC-HPLC method described in 0 were used to analyze the above samples. 0 and Table 0-2 show SEC-HPLC analysis results of samples before HCAb PR004433 recombinant protein coupling, with a purity as high as 98%. 0 and Table 0-3 show HIC-HPLC analysis results of a product obtained after one-step HIC purification (0) of a coupling product PR004433-ADC, showing the product with 90.8% of the components that are coupled with 2 compounds (DAR = 2). 0 and the table below show the HIC-HPLC analysis results of HCAb PR004433 and the coupling product PR004433-ADC thereof, respectively: (A) PR004433 before coupling (Table 0-4); (B) after coupling and before purification (Table 0-5); (C) after coupling and one-step HIC purification (Table 0-6).

**Table 0-2. SEC-HPLC analysis results of HCAb PR004433 (lot.# HSP314-02 K20200330-0409PA0409)**

| Retention time (min) | Peak area% | Peak instructions |
|---|---|---|
| < 6.5 | 0.86 | |
| 6.86 | 1.12 | |
| 7.73 | 98.02 | HCAb main peak |

**Table 0-3. HIC-HPLC analysis results of sample (lot.# HSP31402-ADC-20200521) after PR004433-ADC purification**

| Retention time (min) | Peak area% | Peak instructions |
|---|---|---|
| 7.39 | 0.24 | HCAb naked antibody peak |
| 8.50 | 2.07 | DAR = 1 component peak |

(continued)

| Retention time (min) | Peak area% | Peak instructions |
|---|---|---|
| 9.38 | 90.80 | DAR = 2 component peak |
| 11.00 | 5.77 | Impurities or DAR > 2 component peak |
| 11.93 | 1.12 | |

**Table 0-4. HIC-HPLC analysis results of HCAb PR004433 (lot.# HSP314-02 K20200330-0409PA0409)**

| Retention time (min) | Peak area% | Peak instructions |
|---|---|---|
| 7.07 | 0.11 | |
| 7.77 | 99.89 | HCAb main peak |

**Table 0-5. HIC-HPLC analysis results of sample (lot.# HSP31402-20041601) before PR004433-ADC purification**

| Retention time (min) | Peak area% | Peak instructions |
|---|---|---|
| 7.81 | 15.3 | HCAb naked antibody peak |
| 8.84 | 1.17 | DAR = 1 component peak |
| 9.78 | 52.74 | DAR = 2 component peak |
| 11.43 | 17.87 | Impurities or DAR > 2 component peak |
| 12.43 | 12.91 | |

**Table 0-6. HIC-HPLC analysis results of sample (lot.# HSP31402-20041601PA0416) after PR004433-ADC purification**

| Retention time (min) | Peak area% | Peak instructions |
|---|---|---|
| 8.45 | 1.77 | DAR = 1 component peak |
| 9.37 | 93.85 | DAR = 2 component peak |
| 11.02 | 4.38 | Impurities or DAR > 2 component peak |

The method (LC instrument: Agilent 1290 UPLC; MS instrument: AB Sceix X500B) described in 0 was used to analyze the molecular weight of the sample after purification of PR004433-ADC. Before LC-MS analysis, the sample to be tested was desaccharified. 0 shows mass spectrometry deconvolution processing maps of molecular weight analysis: (A) non-reduced samples of PR004433-ADC; (B) reduced samples of PR004433-ADC. According to the process of the molecular weight analysis method described in 0, the molecular weight of the components coupled with different numbers of compounds and the contents thereof in the samples were calculated (see the table below). Table 0-7 shows that in the non-reduced samples of PR004433-ADC, the component with DAR = 2 is a main ingredient (accounting for 98%), and the average DAR value of PR004433-ADC product is calculated to be 2.02. Correspondingly, as shown in Table 0-8, in the reduced samples of PR004433-ADC, the heavy chain coupled with 1 compound (+1 D) is a main ingredient (accounting for 90.5%), and the average DAR value of PR004433-ADC product is calculated to be 2.02.

**Table 0-7. LC-MS analysis of non-reduced molecular weight of sample (lot.# HSP31402-ADC-20200521) after purification of PR004433-ADC**

| Component | Theoretical value (Da) | Experimental value (Da) | Deviation (Da) | Content |
|---|---|---|---|---|
| PR004433 naked antibody | 78,797 | | | 0% |

(continued)

| Component | Theoretical value (Da) | Experimental value (Da) | Deviation (Da) | Content |
|---|---|---|---|---|
| PR004433 DAR=1 | 80,113 | 80,119 | +6 | 0.8 % |
| PR004433 DAR=2 | 81,429 | 81,405 ~ 81,693 | -24 ~ +264 * | 98.1 % |
| PR004433 DAR=4 | 84,065 | 84,070 | +5 | 0.5 % |
| PR004433 DAR=6 | 86,701 | 86,704 | +3 | 0.5 % |
| (* may include molecular weight deviations caused by C'-terminus Lys not cleaved (+ 128 Da) or other modifications | | | | |

**Table 0-8. LC-MS analysis of reduced molecular weight of sample (lot.# HSP31402-ADC-20200521) after purification of PR004433-ADC**

| Component | Theoretical value (Da) | Experimental value (Da) | Deviation (Da) | Content |
|---|---|---|---|---|
| PR004433 heavy chain | 39,400 | 39,403 ~ 39,503 | +3 ~ +103 | 4.7 % |
| PR004433 heavy chain + 1D | 40,716 | 40,585 ~ 40,847 | -131 ~ +131 * | 90.5 % |
| PR004433 heavy chain + 2D | 42,032 | 42,037 ~ 42,164 | +5 ~ +127 | 4.1 % |
| PR004433 heavy chain + 3D | 43,348 | 43,353 | +5 | 0.7 % |
| (* may include molecular weight deviations caused by C'-terminus Lys not cleaved (+ 128 Da) or other modifications) | | | | |

Based on the results of various analytical methods above, it can be inferred that by using cysteine for coupling, the main ingredient in the coupling product is a component with DAR = 2. After further HIC purification, a highly-homogeneous product with a purity greater than 90% and DAR = 2 can be obtained.

### Example 9.3 Analysis of compound coupling site by LC-MS peptide map

In this example, the method described in 0 was used to analyze coupling sites of PR004433-ADC.

0 shows analysis of compound coupling sites of PR004433-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing SC (+ 1315.78) DK accounted for 98%, indicating that 98% of the Cys-220 site was coupled with a compound. Similarly, only 4% of the Cys-226 or Cys-229 site was coupled.

### Example 9.4 Binding to cells highly expressing BCMA

In this example, the method described in 0 was used to study the binding ability of an HCAb antibody PR004433 or a coupling product PR004433-ADC thereof to an HEK293T cell strain HEK293T-hBCMA (Beijing KYinno, KC-0233) highly expressing human BCMA, a cell line NCI-H929 (ATCC, CRL-9068) highly expressing human BCMA, or a cell line SNU-16 (ATCC, CRL-5974) not expressing human BCMA and other cells.

As shown in 0, both PR004433 and PR004433-ADC can have a strong specific binding to the HEK293T-hBCMA cells (0A) and NCI-H929 cells (0B); moreover, PR004433 cannot bind to the BCMA-negative cells SNU-16 (0(C)).

### Example 9.5 Cytotoxic killing experiment

This example was to study the specific cell killing activity of the anti-BCMA antibody coupling product PR004433-ADC against an HEK293T cell strain HEK293T-hBCMA (Beijing KYinno, KC-0233) highly expressing human BCMA and a tumor cell line NCI-H929 (ATCC, CRL-9068) highly expressing human BCMA, without killing activity against HEK293T cells (ATCC, CRL-11268) and SNU-16 (ATCC, CRL-5974) not expressing human BCMA.

The HEK293T-hBCMA, NCI-H929, HEK293T and SNU-16 cells were seeded in 96-well plates (Perkin Elmer, #6005225) respectively, with the following seeded cell number: 2500 cells/50 μL/well (HEK293T-hBCMA), 5000 cells/50 μL/well (NCI-H929), 2500 cells/50 μL/well (HEK293T), and 5000 cells/50 μL/well (SNU-16). Then, the plates were incubated overnight at 37 °C with 5% $CO_2$. Then, a test sample serially pre-diluted was added at 50 μL/well. PR004433, PR004433-ADC and PR004432-ADC were diluted at a 3-fold concentration gradient from the highest final concentration of 10 nM, and mc-vc-PAB-MMAE was diluted at a 3-fold concentration gradient from the highest final concentration of 20 nM. In addition, a well without the test sample was set as a control well. Thereafter, the plates were incubated at 37 °C with 5% $CO_2$ for 72 h, a CellTiter-Glo cell viability detection reagent (Promega, #G7573) was added, then the plates were incubated at room temperature for at least 10 min to obtain stable luminescence signals, and the luminescence values were detected using a microplate reader. The cell viability was calculated based on the luminescence values by the following formula:

$$\text{Cell viability (\%)} = (A / B) \times 100\%$$

In which, A= experimental well (target cells + test sample + medium); B = control well (target cells + medium)

The data were processed and analyzed by plotting using GraphPad Prism 8 software, and binding curves of the antibodies to target cells, $EC_{50}$ values and other parameters were obtained through four-parameter nonlinear fitting.

0 shows the cytotoxic killing of the anti-BCMA HCAb PR004433 and the coupling product PR004433-ADC thereof, the coupling product PR004432-ADC of anti-5T4 HCAb PR004432, the uncoupled cytotoxic compound mc-vc-PAB-MMAE and other test samples against the BCMA-positive cells (HEK293T-hBCMA and NCI-H929) and BCMA-negative cells (HEK293T and SNU-16). Only PR004433-ADC can produce effective and specific cytotoxic killing against the BCMA-positive cells HEK293T-hBCMA (0(A)) and NCI-H929 (0(C)), and exhibit antibody concentration dependence, without cytotoxicity against the BCMA-negative cells; while other molecules cannot produce effective killing against the cells. These results confirms that PR004433-ADC has the high specificity and high efficacy of target dependence.

### Example 10 Optimization of Coupling Reaction Conditions of HCAb PR004433

0 has studied the preparation of an antibody-drug conjugate with a uniform DAR value using cysteine coupling to Cys-220 site by an anti-BCMA HCAb antibody PR004433 (SEQ ID NO: 70) and mc-vc-PAB-MMAE; however, as shown in the experimental process described in 0 and the reaction conditions and results listed in Table 0-1, in the product after one-step coupling, the content of the component with DAR = 2 ('D2 %') was about 50% to 60%, which required one-step HIC purification (the method described in 0) to remove non-main ingredients to obtain high-purity DAR = 2 components.

In this example, the coupling reaction conditions were further optimized, so that in the product after one-step coupling, the content of the component with DAR = 2 was increased, reducing the need for further purification; more preferably, after the coupling step, without the additional purification step, a highly-homogeneous product with a purity greater than 90% and DAR = 2 could be obtained.

### Example 10.1 Optimization of coupling reaction conditions

During the reaction processes such as reduction and coupling, various reaction conditions such as a reaction temperature, reaction time, a type and pH of a reaction buffer, a type and an amount of usage of a reducing agent, a type and an amount of usage of a conjugate molecule, etc., could have a significant impact, therefore, based on the method described in 0, a series of experiments were designed in this example, some of experimental condition parameters were fixed while changing others in an attempt to obtain better reaction conditions. Specifically, in this example, 17 groups of different combinations of reduction and coupling conditions were used to conduct coupling reaction on HCAb PR004433 and analyze the coupling product. Compared to 0, in this example, the effects of parameters such as reaction buffer pH and reduction reaction temperature on coupling were further investigated. In this experiment, the concentration of PR004433 was 5 mg/mL; the reaction buffer was 20 mM His-HCl, with an adjustable pH; the reducing agent was TCEP, with a variable molar equivalent; the reduction reaction temperature and reaction time were variables; the drug conjugate molecule was mc-vc-PAB-MMAE (manufacturer: Levena Biopharma, Cat. No.: SET0201), with a variable molar equivalent; the coupling reaction temperature was room temperature, and the reaction time was 30 min. The 17 groups of experiments and respective reaction condition parameters were listed in Table 0-1. For each group of experiments, the coupling products were subjected to complete molecular weight analysis of the non-reduced samples using the method (LC instrument: Agilent 1290 UPLC; MS instrument: AB Sceix X500B; samples without desaccharification preprocessing) described in 0, and the molecular weights of the components coupled with different numbers of compounds and the contents in the samples thereof were calculated by using the total ion chromatogram (TIC) in the mass spectrum of each sample in combination with the molecular weight analysis process used in 0. The analysis results are listed in Table 0-2. In Table 0-2, "D0 %", "D2 %", "D4 %", and "D6 %" represent the content of components not coupled with a compound (DAR = 0), coupled with 2 compounds (DAR = 2), coupled with 4 compounds (DAR = 4), and coupled with 6 compounds (DAR = 6), respectively, and the average DAR value was calculated accordingly. The coupling products produced in Experiments #8, #9, #11 and #12 had a DAR = 2 component content exceeding 85%, and the average DAR value was also very close to 2.0. The TICs of the mass spectrum of the coupling product samples in these four experiments correspond to (A)-(D) in 0, respectively.

**Table 0-1. Coupling reaction conditions of PR004433**

| Experiment # | PR004433 | Reaction buffer | Reaction reduction conditions | | | Coupling reaction conditions | | |
|---|---|---|---|---|---|---|---|---|
| | Antibody concentration (mg/mL) | 20mM His-HCl, pH= | TCEP (equivalent) | Temperature (°C) | Time (h) | Conjugate (equivalent) | Temperature (°C) | Time (h) |
| #1 | 5 | 5.0 | 1.5 | 37 | 2 | 7.0 | Room temperature | 0.5 |
| #2 | 5 | 5.0 | 1.5 | 37 | 2 | 3.0 | Room temperature | 0.5 |
| #3 | 5 | 5.0 | 1.9 | 37 | 2 | 7.0 | Room temperature | 0.5 |
| #4 | 5 | 5.0 | 1.9 | 37 | 2 | 3.0 | Room temperature | 0.5 |
| #5 | 5 | 5.0 | 2.1 | 37 | 2 | 7.0 | Room temperature | 0.5 |
| #6 | 5 | 5.5 | 1.2 | Room temperature | 2 | 3.0 | Room temperature | 0.5 |
| #7 | 5 | 5.5 | 1.2 | 0 | 2 | 3.0 | Room temperature | 0.5 |
| #8 | 5 | 5.5 | 2.1 | 37 | 2 | 7.0 | Room temperature | 0.5 |
| #9 | 5 | 5.5 | 2.1 | 37 | 2 | 3.0 | Room temperature | 0.5 |
| #10 | 5 | 6.0 | 2.1 | 37 | 2 | 7.0 | Room temperature | 0.5 |
| #11 | 5 | 6.0 | 3.0 | 0 | 1.5 | 3.0 | Room temperature | 0.5 |
| #12 | 5 | 6.0 | 3.0 | Room temperature | 1.5 | 3.0 | Room temperature | 0.5 |
| #13 | 5 | 6.5 | 2.1 | 37 | 2 | 7.0 | Room temperature | 0.5 |
| #14 | 5 | 6.5 | 2.5 | 37 | 2 | 7.0 | Room temperature | 0.5 |
| #15 | 5 | 6.5 | 3.0 | 37 | 2 | 7.0 | Room temperature | 0.5 |
| #16 | 5 | 7.0 | 2.1 | 37 | 2 | 7.0 | Room temperature | 0.5 |
| #17 | 5 | 8.0 | 2.1 | 37 | 2 | 7.0 | Room temperature | 0.5 |

**Table 0-2. Analysis results of respective components of product after coupling with PR004433 (without HIC purification) by LC-MS**

| Experiment # | Analysis results of coupling product | | | | | |
|---|---|---|---|---|---|---|
| | D0 % | D2 % | D4 % | D6 % | Average DAR | Subgraph in 0 |
| #1 | 16.7 % | 78.4 % | 2.2 % | 2.7 % | 1.82 | |
| #2 | 22.3 % | 72.1 % | 2.5 % | 3.0 % | 1.72 | |
| #3 | 7.4 % | 84.0 % | 3.3 % | 5.3 % | 2.13 | |
| #4 | 8.6 % | 81.0 % | 4.3 % | 6.0 % | 2.15 | |
| #5 | 2.1 % | 80.5 % | 6.0 % | 11.4 % | 2.53 | |
| #6 | 36.6 % | 63.4 % | 0.0 % | 0.0 % | 1.27 | |
| #7 | 63.6 % | 36.4 % | 0.0 % | 0.0 % | 0.73 | |
| **#8** | **2.9 %** | **89.1 %** | **3.6 %** | **4.4 %** | **2.19** | **(A)** |
| **#9** | **3.9 %** | **87.3 %** | **4.7 %** | **4.1 %** | **2.18** | **(B)** |
| #10 | 17.1 % | 82.9 % | 0.0 % | 0.0 % | 1.66 | |
| **#11** | **7.6 %** | **89.3 %** | **3.1 %** | **0.0 %** | **1.91** | **(C)** |
| **#12** | **2.8 %** | **93.5 %** | **2.8 %** | **1.0 %** | **2.04** | **(D)** |
| #13 | 34.2 % | 65.8 % | 0.0 % | 0.0 % | 1.32 | |
| #14 | 31.1 % | 68.9 % | 0.0 % | 0.0 % | 1.38 | |
| #15 | 31.3 % | 68.7 % | 0.0 % | 0.0 % | 1.37 | |
| #16 | 80.4 % | 19.6 % | 0.0 % | 0.0 % | 0.39 | |
| #17 | 84.7 % | 15.3 % | 0.0 % | 0.0 % | 0.31 | |

Specifically, the content of DAR = 2 component in the coupling product produced in Experiment #12 exceeded 90%, while the content of other components was extremely low, indicating that HCAb PR004433 can produce high-purity coupling products using the reaction conditions of Experiment #12, and highly-homogeneous products with DAR values determined can be obtained without an additional purification step. This is a significant improvement compared to the coupling method in 0.

For the coupling product produced in Experiment #12, the method described in 0 was further used to analyze the coupling sites of the antibody-drug conjugate molecule. The results showed that 96.22% of the Cys-220 sites were coupled; similarly, only 6% of Cys-226 or Cys-229 sites were coupled. Therefore, the coupling product produced in Experiment #12 is a highly-homogeneous antibody-drug conjugate molecule that is site-specifically coupled to Cys-220. This example fully demonstrates that the inventors of the present application have made multiple attempts and optimizations to various reaction conditions throughout the coupling process, and have found a more optimized combination of reaction conditions. Under some combinations of reaction conditions, the disulfide bond of Cys-220 can be effectively cleaved while the other disulfide bonds remain intact, so that Cys-220 becomes almost the only free cysteine residue and serves as a specific coupling site.

**Example 11 Change in Human IgG1 Hinge Region Sequence**

This example studied the number and type of amino acids by which the first Cys (Cys1) and the second Cys (Cys2) were separated in the linker peptide variant sequence generated based on the natural sequence (SEQ ID NO: 73) in the human IgG1 antibody heavy chain hinge region, as well as the effect on the generation of a coupling product with a uniform CAR value (a conjugate/antigen-binding protein ratio) by using the first Cys as a specific site-specific coupling site.

**Example 11.1 Changing hinge region linker peptide of HCAb PR004433 to generate variant sequence**

In this example, based on HCAb PR004433, different lengths of randomized primers were designed for the hinge region and a series of antigen-binding proteins with different hinge region linker peptides were produced by protein engineering. For example, by using the primers described in Table 0-1, 3, 4, 5, 6, 7, 8, 9, or 10 amino acids could be inserted between Cys1 and Cys2, respectively, to produce a linker peptide sequence set forth in any one of SEQ ID NOs: 74-81. Then, 3 different mutation sequences were randomly selected for each length of linker peptide to further study the different amino acid compositions between Cys1 and Cys2, and the amino acid between Cys1 and Cys2 could not be cysteine. Subsequently, in subsequent examples, antigen-binding proteins (PR004433 variant sequence) with different lengths and amino acid compositions of linker peptides were expressed and purified using the aforementioned method, and coupled with the drug conjugate mc-vc-PAB-MMAE to analyze the components and coupling sites of the coupling product. A series of different lengths of linker peptide sequences randomly produced were listed in Table 0-2. The hinge region sequence of HCAb PR004433 was replaced with the linker peptide sequence in Table 0-2 to produce a variety of antigen-binding protein variant sequences (Table 0-3) derived from PR004433. The linker peptides between the variable region and Fc constant region of these antigen-binding proteins have different lengths and amino acid compositions.

**Table 0-1. Randomized primer list** (in forward primer sequences, N represents any base in A/T/G/C, and K represents any base in G/T)

| Primer | Primer sequence | SEQ ID NO |
|---|---|---|
| Forward primer-3 | 5'-ATTAGGTCACCGTCTCCTCAGAGCCCAAATCTTGTNNKNNKNNKTGCCCACCGTGCCCAGCACCTGAACTCCT -3' | 136 |
| Forward primer-4 | 5'-ATTAGGTCACCGTCTCCTCAGAGCCCAAATCTTGTNNKNNKNNKNNKTGCCCACCGTGCCCAGCACCTGAACTCCT -3' | 137 |
| Forward primer-5 | 5'-ATTAGGTCACCGTCTCCTCAGAGCCCAAATCTTGTNNKNNKNNKNNKNNKTGCCCACCGTGCCCAGCACCTGAACTCCT -3' | 138 |
| Forward primer-6 | 5'-ATTAGGTCACCGTCTCCTCAGAGCCCAAATCTTGTNNKNNKNNKNNKNNKNNKTGCCCACCGTGCCCAGCACCTGAACTCCT -3' | 139 |
| Forward primer-7 | 5'-ATTAGGTCACCGTCTCCTCAGAGCCCAAATCTTGTNNKNNKNNKNNKNNKNNKNNKTGCCCACCGTGCCCAGCACCTGAACTCCT -3' | 140 |

(continued)

| Primer | Primer sequence | SEQ ID NO |
|---|---|---|
| Forward primer-8 | 5'- ATTAGGTCACCGTCTCCTCAGAGCCCAAATCTTGTNNKNNKNNKNNKNNKNNKNNKNNKTGCCCACCGTGCCCAGCACCTGAACTCCT -3' | 141 |
| Forward primer-9 | 5'- ATTAGGTCACCGTCTCCTCAGAGCCCAAATCTTGTNNKNNKNNKNNKNNKNNKNNKNNKNNKTGCCCACCGTGCCCAGCACCTGAACTCCT -3' | 142 |
| Forward primer-10 | 5'- ATTAGGTCACCGTCTCCTCAGAGCCCAAATCTTGTNNKNNKNNKNNKNNKNNKNNKNNKNNKNNKTGCCCACCGTGCCCAGCACCTGAACTCCT -3' | 143 |
| Reverse primer | 5'- ACTCGACTCGAGTCCCCCGGGATG -3' | 144 |

**Table 0-2. Different lengths of linker peptides**

| Identification of linker peptide | Number of amino acids by which Cys1 and Cys2 are separated | Sequence of linker peptide | SEQ ID NO |
|---|---|---|---|
| hIgG1_hinge | 5 | EPKSCDKTHTCPPCP | 73 |
| Hinge3_5 | 3 | EPKSCQPRCPPCP | 82 |
| Hinge3_6 | 3 | EPKSCAGWCPPCP | 83 |
| Hinge3_18 | 3 | EPKSCNVACPPCP | 84 |
| Hinge4_1 | 4 | EPKSCTGKMCPPCP | 85 |
| Hinge4_6 | 4 | EPKSCRDARCPPCP | 86 |
| Hinge4_15 | 4 | EPKSCRGTLCPPCP | 87 |
| Hinge5_2 | 5 | EPKSCQAESRCPPCP | 88 |
| Hinge5_12 | 5 | EPKSCKDYGRCPPCP | 89 |
| Hinge5_13 | 5 | EPKSCSDNHDCPPCP | 90 |
| Hinge6_4 | 6 | EPKSCAPVRPWCPPCP | 91 |
| Hinge6_15 | 6 | EPKSCSGPSTLCPPCP | 92 |
| Hinge6_18 | 6 | EPKSCSAVPRRCPPCP | 93 |
| Hinge7_2 | 7 | EPKSCAVLPEIGCPPCP | 94 |
| Hinge7_3 | 7 | EPKSCWLYISDKCPPCP | 95 |
| Hinge7_19 | 7 | EPKSCSYLFGRVCPPCP | 96 |

(continued)

| Identification of linker peptide | Number of amino acids by which Cys1 and Cys2 are separated | Sequence of linker peptide | SEQ ID NO |
|---|---|---|---|
| Hinge8_8 | 8 | EPKSCMPRLLFHSCPPCP | 97 |
| Hinge8_18 | 8 | EPKSCSSDVVGGRCPPCP | 98 |
| Hinge8_20 | 8 | EPKSCRMTVYGSRCPPCP | 99 |
| Hinge9_5 | 9 | EPKSCLALPGVMSPCPPCP | 100 |
| Hinge9_7 | 9 | EPKSCASMNRGGGGCPPCP | 101 |
| Hinge9_14 | 9 | EPKSCGGGPFLWLVCPPCP | 102 |
| Hinge10_3 | 10 | EPKSCQMSVFARHFPCPPCP | 103 |
| Hinge10_5 | 10 | EPKSCSVGRFGSMLECPPCP | 104 |
| Hinge10_7 | 10 | EPKSCITEWSVRDLQCPPCP | 105 |

**Table 0-3. Replacing hinge region sequence of HCAb PR004433 with various linker peptide sequences to produce different derived variant sequences**

| Antigen-binding protein | Identification of linker peptide | Linker peptide SEQ ID NO | Polypeptide chain SEQ ID NO |
|---|---|---|---|
| PR004433 | hIgG1_hinge | 73 | 70 |
| PR006031 | Hinge3_5 | 82 | 106 |
| PR006032 | Hinge3_6 | 83 | 107 |
| PR006033 | Hinge3_18 | 84 | 108 |
| PR006034 | Hinge4_1 | 85 | 109 |
| PR006035 | Hinge4_6 | 86 | 110 |
| PR006036 | Hinge4_15 | 87 | 111 |
| PR006037 | Hinge5_2 | 88 | 112 |
| PR006038 | Hinge5_12 | 89 | 113 |
| PR006039 | Hinge5_13 | 90 | 114 |
| PR006040 | Hinge6_4 | 91 | 115 |
| PR006041 | Hinge6_15 | 92 | 116 |
| PR006042 | Hinge6_18 | 93 | 117 |
| PR006310 | Hinge7_2 | 94 | 118 |
| PR006311 | Hinge7_3 | 95 | 119 |
| PR006312 | Hinge7_19 | 96 | 120 |
| PR006313 | Hinge8_8 | 97 | 121 |
| PR006314 | Hinge8_18 | 98 | 122 |
| PR006315 | Hinge8_20 | 99 | 123 |
| PR006316 | Hinge9_5 | 100 | 124 |
| PR006317 | Hinge9_7 | 101 | 125 |
| PR006318 | Hinge9_14 | 102 | 126 |
| PR006319 | Hinge10_3 | 103 | 127 |

(continued)

| Antigen-binding protein | Identification of linker peptide | Linker peptide SEQ ID NO | Polypeptide chain SEQ ID NO |
|---|---|---|---|
| PR006320 | Hinge10_5 | 104 | 128 |
| PR006321 | Hinge10_7 | 105 | 129 |

**Example 11.2 Expression and identification of variant molecules derived from HCAb PR004433**

The antigen-binding protein derived from PR004433 in Table 0-3 was expressed and purified according to the method described in 0, and the SEC-HPLC method described in 0 was used to analyze the purity and the DSF method described in 0 was used to determine the minimum melting temperature (Tm1). The results of expression, purification, and thermostability analysis of the antigen-binding proteins are listed in Table 0-4. It can be seen that after the hinge region sequence of HCAb PR004433 is replaced with variant sequences of various linker peptides, and Cys1 and Cys2 are separated by at least 3 other amino acids, the variant sequences derived from PR004433 can be effectively expressed, and the expression yield and thermostability Tm1 of the vast majority of variant molecules are not affected.

**Table 0-4. Expression, purification, and thermostability analysis of PR004433 and variant molecules thereof**

| Antigen-binding protein | Identification of linker peptide | Expression system and volume | SEC-HPLC purity (%) | Yield (mg/L) after first purification | DSF $Tm_1$ (°C) |
|---|---|---|---|---|---|
| PR004433 | hIgG1 hinge | CHO (1000ml) | 98.86 | 35.2 | 55.8 |
| PR006031 | Hinge3_5 | HEK293 (100ml) | 99.05 | 186.6 | 54.8 |
| PR006032 | Hinge3_6 | HEK293 (100ml) | 96.89 | 180.7 | 56.6 |
| PR006033 | Hinge3_18 | HEK293 (100ml) | 95.66 | 218.3 | 54.6 |
| PR006034 | Hinge4_1 | HEK293 (100ml) | 99.65 | 184.8 | 55.2 |
| PR006035 | Hinge4_6 | HEK293 (100ml) | 99.33 | 206.5 | 54.4 |
| PR006036 | Hinge4_15 | HEK293 (100ml) | 99.55 | 202.7 | 54.2 |
| PR006037 | Hinge5_2 | HEK293 (100ml) | 98.95 | 207.5 | 55.4 |
| PR006038 | Hinge5_12 | HEK293 (100ml) | 99.58 | 227.9 | 54.6 |
| PR006039 | Hinge5_13 | HEK293 (100ml) | 99.23 | 106.2 | 55.6 |
| PR006040 | Hinge6_4 | HEK293 (100ml) | 98.65 | 142.4 | 54.6 |
| PR006041 | Hinge6_15 | HEK293 (100ml) | 99.12 | 166.8 | 55.2 |
| PR006042 | Hinge6_18 | HEK293 (100ml) | 99.05 | 167.2 | 54.4 |
| PR006310 | Hinge7_2 | HEK293 (100ml) | 98.68 | 218.0 | 55.0 |
| PR006312 | Hinge7_19 | HEK293 (100ml) | 99.18 | 171.9 | 52.8 |
| PR006313 | Hinge8_8 | HEK293 (100ml) | 97.28 | 79.0 | 49.2 |
| PR006314 | Hinge8_18 | HEK293 (100ml) | 99.34 | 201.9 | 55.2 |
| PR006315 | Hinge8_20 | HEK293 (100ml) | 99.38 | 99.4 | 51.8 |
| PR006316 | Hinge9_5 | HEK293 (100ml) | 99.15 | 89.7 | 53.6 |
| PR006317 | Hinge9_7 | HEK293 (100ml) | 99.33 | 170.6 | 55.0 |
| PR006318 | Hinge9_14 | HEK293 (100ml) | 86.91 | 18.7 | 47.4 |
| PR006319 | Hinge10_3 | HEK293 (100ml) | 100.00 | 114.6 | 52.8 |
| PR006320 | Hinge10_5 | HEK293 (100ml) | 97.80 | 145.4 | 53.6 |
| PR006321 | Hinge10_7 | HEK293 (100ml) | 94.49 | 193.8 | 55.2 |

This example further utilized the LC-MS molecular weight analysis method described in 0 to determine the state of Cys1 in different linker peptide variant sequences. In variant sequences derived from PR004433, there were two possibilities for the state of Cys1 (or Cys-220) as shown in 0: the formation or no formation of an interchain disulfide bond. If Cys1 did not form a disulfide bond between heavy chains, its side chain sulfhydryl (-SH) would form a disulfide bond with a sulfhydryl-containing molecule (such as cysteine or glutathione) in the environment, so that the actual molecular weight thereof was significantly greater than the theoretical molecular weight. Purified antigen-binding proteins were desaccharified with glycosidase PNGase F, and its complete (non-reduced) molecular weight was determined by the method (LC instrument: Agilent 1290 UPLC; MS instrument: AB Sceix X500B) described in 0 to be compared to the corresponding theoretical molecular weight. Table 0-5 shows that the theoretical and measured values of molecular weight are very consistent. Therefore, the Cys1 of the antigen-binding proteins does not link to other sulfhydryl-containing molecules. It can be inferred that when Cys1 and Cys2 are separated by 3 to 10 other amino acids, Cys1 can form a disulfide bond between heavy chains (i.e., a disulfide bond is formed between Cys1 of the first linker peptide and Cys1 of the second linker peptide).

**Table 0-5. LC-MS determination results of complete molecular weight of sample after purification of PR004433 variant molecule**

| Antigen-binding protein | Identification of linker peptide | Theoretical value (Da) | Measured value (Da) | Deviation (Da) |
|---|---|---|---|---|
| PR006031 | Hinge3_5 | 78,395 | 78,395.6 | 0.6 |
| PR006032 | Hinge3_6 | 78,261 | 78,261.4 | 0.4 |
| PR006033 | Hinge3_18 | 78,201 | 78,201.5 | 0.5 |
| PR006034 | Hinge4_1 | 78,467 | 78,467.8 | 0.8 |
| PR006035 | Hinge4_6 | 78,629 | 78,629.9 | 0.9 |
| PR006036 | Hinge4_15 | 78,487 | 78,487.9 | 0.9 |
| PR006037 | Hinge5_2 | 78,775 | 78,776.0 | 1 |
| PR006038 | Hinge5_12 | 78,871 | 78,872.2 | 1.2 |
| PR006039 | Hinge5_13 | 78,769 | 78,770.2 | 1.2 |
| PR006040 | Hinge6_4 | 79,046 | 79,046.7 | 0.7 |
| PR006041 | Hinge6_15 | 78,717 | 78,718.2 | 1.2 |
| PR006042 | Hinge6_18 | 78,966 | 78,966.9 | 0.9 |
| PR006310 | Hinge7_2 | 78,992 | 78,991.6 | -0.4 |
| PR006312 | Hinge7_19 | 79,278 | 79,278.0 | 0 |
| PR006313 | Hinge8_8 | 79,596 | 79,596.3 | 0.3 |
| PR006314 | Hinge8_18 | 79,148 | 79,147.7 | -0.3 |
| PR006316 | Hinge9_5 | 79,364 | 79,364.4 | 0.4 |
| PR006317 | Hinge9_7 | 79,208 | 79,207.8 | -0.2 |
| PR006318 | Hinge9_14 | 79,486 | 79,486.5 | 0.5 |
| PR006319 | Hinge10_3 | 80,035 | 80,035.0 | 0 |
| PR006320 | Hinge10_5 | 79,760 | 79,760.7 | 0.7 |
| PR006321 | Hinge10_7 | 80,089 | 80,088.9 | -0.1 |

This example further utilized the method described in 0 to study the binding ability of the HCAb antibody PR004433 and a variant molecule thereof to NCI-H929 cells (ATCC, CRL-9068) highly expressing human BCMA. As shown in 0, the binding ability of the PR004433 variant molecule to NCI-H929 is almost the same as that of its parent molecule PR004433, indicating that the change of the hinge region (linker peptide) sequence of PR004433 will not affect the binding of an antigen-binding fragment thereof to the target.

**Example 11.3 Analysis of coupling product of variant molecule derived from HCAb PR004433**

This example further utilized the coupling method described in 0 to conduct coupling reaction on a variant molecule derived from PR004433 listed in Table 0-4, to produce different coupling reaction products by changing the amount of usage of a reducing agent TCEP based on the preferred reaction condition parameters obtained in 0. In this experiment, the concentration of variant molecules derived from PR004433 with different linker peptides (PR006031, PR006034, PR006037, PR006040, PR006312, PR006314, PR006317, and PR006320) was 5 mg/mL. A reaction buffer was a buffer with pH of 6.0 containing 20 mM His-HCl, the amount of usage of the reducing agent TCEP varied from 1.5 to 6.0 times molar equivalents, the reduction reaction was carried out at room temperature for 2 h, the amount of usage of a conjugate mc-vc-PAB-MMAE was 7.0 molar multiples, and the coupling reaction was carried out at room temperature for 0.5 h; the coupling product did not undergo the step of HIC purification. Furthermore, for the coupling product, the molecular weight analysis method used in 0 was also utilized to calculate the molecular weight of the components coupled with different numbers of compounds and the content thereof in the sample. The analysis results are listed in Table 0-6. For example, Cys1 and Cys2 of the linker peptide in PR006031 are separated by 3 amino acids, when 2.0 equivalents of TCEP is used as a reducing agent and the above reaction parameters are used for coupling, the content of the DAR = 2 component in the coupling product is 84.3%, and the average DAR value of the coupling product is 2.12; for another example, Cys1 and Cys2 of the linker peptide in PR006320 are separated by 10 amino acids, when 3.0 equivalents of TCEP is used as a reducing agent and the above reaction parameters are used for coupling, the content of the DAR = 2 component in the coupling product is 94.8%, and the average DAR value of the coupling product is 1.99.

**Table 0-6. Analysis results of respective components of coupling product (without HIC purification) of PR004433 variant molecule by LC-MS**

| Antigen-binding protein | Identification of linker peptide | Equivalents of TCEP | D2 % | (D4+D6) % | Average DAR |
|---|---|---|---|---|---|
| **PR006031** | Hinge3_5 | 1.5 | 75.4% | 2.8% | 1.64 |
| | | 2.0 | 84.3% | 9.1% | 2.12 |
| | | 3.0 | 73.6% | 25.1% | 2.65 |
| | | 4.0 | 61.4% | 37.3% | 3.02 |
| | | 5.0 | 49.9% | 47.8% | 3.41 |
| | | 6.0 | 42.8% | 55.2% | 3.69 |
| **PR006034** | Hinge4_1 | 1.5 | 62.7% | 2.2% | 1.53 |
| | | 2 | 69.7% | 6.0% | 1.90 |
| | | 3 | 66.0% | 16.5% | 2.41 |
| | | 4 | 57.2% | 29.0% | 2.94 |
| | | 5 | 50.2% | 36.8% | 3.21 |
| | | 6 | 41.1% | 47.1% | 3.69 |
| **PR006037** | Hinge5_2 | 1.5 | 63.9% | 0.0% | 1.30 |
| | | 2 | 63.0% | 1.9% | 1.36 |
| | | 3 | 72.2% | 4.7% | 1.68 |
| | | 4 | 80.9% | 8.4% | 2.03 |
| | | 5 | 81.9% | 11.6% | 2.20 |
| | | 6 | 80.1% | 15.4% | 2.35 |

(continued)

| Antigen-binding protein | Identification of linker peptide | Equivalents of TCEP | D2 % | (D4+D6) % | Average DAR |
|---|---|---|---|---|---|
| PR006040 | Hinge6_4 | 1.5 | 76.0% | 2.4% | 1.63 |
| | | 2 | 83.0% | 5.6% | 1.91 |
| | | 3 | 82.9% | 13.1% | 2.25 |
| | | 4 | 73.6% | 25.6% | 2.60 |
| | | 5 | 69.3% | 29.8% | 2.71 |
| | | 6 | 60.4% | 38.7% | 2.95 |
| PR006312 | Hinge7_19 | 1.5 | 90.1% | 2.9% | 1.98 |
| | | 2 | 89.4% | 5.6% | 2.10 |
| | | 3 | 84.8% | 13.9% | 2.36 |
| | | 4 | 79.2% | 19.4% | 2.53 |
| | | 5 | 81.6% | 17.4% | 2.52 |
| PR006314 | Hinge8_18 | 1.5 | 51.9% | 3.1% | 1.18 |
| | | 2 | 59.3% | 5.2% | 1.44 |
| | | 3 | 65.0% | 10.8% | 1.84 |
| | | 4 | 72.5% | 17.0% | 2.31 |
| | | 5 | 68.7% | 24.4% | 2.59 |
| PR006317 | Hinge9_7 | 1.5 | 75.4% | 7.7% | 1.90 |
| | | 2 | 78.8% | 15.1% | 2.36 |
| | | 3 | 63.1% | 32.7% | 2.90 |
| | | 4 | 58.3% | 36.9% | 3.21 |
| | | 5 | 44.8% | 49.9% | 3.68 |
| PR006320 | Hinge10_5 | 1.5 | 78.7% | 0.6% | 1.66 |
| | | 2 | 88.4% | 0.9% | 1.87 |
| | | 3 | 94.8% | 1.1% | 1.99 |
| | | 4 | 96.0% | 1.5% | 2.03 |
| | | 5 | 97.1% | 1.6% | 2.03 |

This example illustrated that when Cys1 and Cys2 of the linker peptide were separated by at least 3 other amino acids, Cys1 could form a disulfide bond between heavy chains (i.e., a disulfide bond was formed between Cys1 of the first linker peptide and Cys1 of the second linker peptide, called "Cys1-Cys1"), moreover, Cys2 could also form a disulfide bond between heavy chains (i.e., a disulfide bond was formed between Cys2 of the first linker peptide and Cys2 of the second linker peptide, called "Cys2-Cys2"). However, the stability of the disulfide bond Cys1-Cys1 could be weaker than that of other disulfide bonds. Under some combinations of reaction conditions, the disulfide bond Cys1-Cys1 can be effectively cleaved while the other disulfide bonds (such as Cys2-Cys2) remain intact, so that the Cys1 becomes almost the only free cysteine residue and serves as a specific coupling site. The protein-drug conjugate produced by using the antigen-binding protein containing the linker peptide has a coupling site at Cys1 and a CAR (or DAR) of about 2.

**Example 12 Coupling and Analysis at Mouse IgG2c Hinge Region**

This example studied the preparation of an antibody-drug conjugate using the coupling of the mouse IgG2c antibody heavy chain hinge region (SEQ ID NO: 147) and mc-vc-PAB-MMAE through cysteine. The mouse IgG2c hinge region has three cysteines, and the first cysteine (Cys1) and the second cysteine (Cys2) are separated by 5 other amino acid residues.

On the basis of HCAb PR004433 (SEQ ID NO: 70), the human IgG1 hinge region (SEQ ID NO: 73) was partially replaced with the mouse IgG2c antibody hinge region sequence (SEQ ID NO: 147); that is, the VH (SEQ ID NO: 62) of PR004433 was fused with the mouse IgG2c antibody hinge region (SEQ ID NO: 147) as well as the CH2 and CH3 sequences of the human IgG1 heavy chain constant region and expressed to obtain a new HCAb molecule PR006468 (SEQ ID NO: 148).

**Example 12.1 Coupling and analysis of PR006468**

PR006468 was expressed and purified according to the method described in 0, and the SEC-HPLC method described in 0 was used to analyze the purity. Then, the coupling method described in 0 was further used to conduct coupling reaction on the purified PR006468 recombinant protein.

In this experiment, the concentration of PR006468 was 1.75 mg/mL. A reaction buffer was a buffer with pH of 6.0 containing 20 mM His-HCl, the amount of usage of the reducing agent TCEP was 7.0 times molar equivalents, the reduction reaction was carried out at 37 °C for 2 h, the amount of usage of a conjugate mc-vc-PAB-MMAE was 7.0 molar equivalents, and the coupling reaction was carried out at room temperature for 0.5 h. After the coupling was completed, the excess drug conjugates were removed from the product through a desalting column, and the method described in 0 was used to purify the coupling product PR006468-ADC. The method (LC instrument: Agilent 1290 UPLC; MS instrument: AB Sceix X500B) described in 0 was used to analyze the molecular weight of the sample after purification of PR006468-ADC. Before LC-MS analysis, the sample to be tested was desaccharified. 0 shows a mass spectrometry deconvolution processing map of non-reduced molecular weight analysis of PR006468-ADC. According to the process of the molecular weight analysis method described in 0, the molecular weight of the components coupled with different numbers of compounds and the contents thereof in the samples were calculated. Table 0-1 shows that in the non-reduced samples of PR006468-ADC, the component with DAR = 2 is a main ingredient (accounting for 95.5%), and the average DAR value of PR006468-ADC product is calculated to be 2.09.

**Table 0-1. LC-MS analysis of non-reduced molecular weight of sample (lot.# CY20210203) after purification of PR006468-ADC**

| Component | Theoretical value (Da) | Experimental value (Da) | Deviation (Da) | Content |
|---|---|---|---|---|
| PR006468 naked antibody | 80,091 | | | 0% |
| PR006468 DAR=2 | 82,723 | 81,965 ~ 83,675 | -758 ~ +952 [*1] | 95.5 % |
| PR006468 DAR=4 | 85,354 | 84,624 | -730 [*2] | 4.5 % |
| [*1] O-glycosylation may be present (+947 Da); [*2] partial fragmentation may occur in mc-vc-PAB-MMAE (-762 Da). | | | | |

**Example 12.2 Analysis of compound coupling site by LC-MS peptide map**

In this example, the method described in 0 was used to analyze coupling sites of PR006468-ADC.

0 shows analysis of compound coupling sites of PR006468-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing C (+ 1315.78) PPLK accounted for 90.8% (0 shows the secondary map of this peptide fragment), indicating that 90.8% of the Cys1 site in the mouse hinge region was coupled with a compound. Similarly, only 5.8% of the Cys2 site was coupled.

Therefore, the mouse IgG2c hinge region sequence can also be used for site-specific cysteine coupling as described in the present invention.

**Example 12.3 Binding to cells highly expressing BCMA**

In this example, the method described in 0 was used to study the binding ability of antigen-binding proteins PR006468 (including a mouse IgG2c hinge region), PR004433 (including a human IgG1 hinge region) and coupling products PR006468-ADC and PR004433-ADC thereof to tumor cells NCI-H929 (ATCC, CRL-9068) highly expressing human BCMA.

As shown in 0, PR006468 and PR004433 have the similar binding ability. Accordingly, PR006468-ADC and PR004433-ADC also have the similar binding ability. This indicates that replacing the human IgG1 hinge region sequence in the antigen-binding protein with the mouse IgG2c hinge region sequence will not affect the target-binding ability of the antigen-binding proteins and the coupling products thereof.

**Example 12.4 Cytotoxic killing experiment**

In this example, the method described in 0 was used to study the specific cell killing activity of antigen-binding proteins PR006468 (including a mouse IgG2c hinge region), PR004433 (including a human IgG1 hinge region) and coupling products PR006468-ADC and PR004433-ADC thereof against tumor cells NCI-H929 (ATCC, CRL-9068) highly expressing human BCMA.

As shown in 0, PR006468-ADC and PR004433-ADC both produce effective and specific cytotoxic killing against NCI-H929, and exhibit antibody concentration dependence, while other molecules cannot produce effective killing against the cells; and the target cell killing efficacy of PR006468-ADC and PR004433-ADC is almost identical. This also indicates that coupling products with mouse IgG2c hinge region sequences can also retain original functions.

**Example 13 Coupling and Analysis of scFv-Fc PR002129**

This example studied the preparation of an antibody-drug conjugate with a uniform DAR value using cysteine coupling to Cys-220 site by an anti-ROR1 antigen-binding protein PR002129 (SEQ ID NO: 71) and mc-vc-PAB-MMAE. The sequence of PR002129 was from 0 including a human IgG1 hinge region sequence.

**Example 13.1 Optimization of coupling reaction conditions**

Based on the preliminarily determined coupling reaction conditions obtained in 0, and by combining the preferred reaction condition parameters obtained in 0, the antigen-binding protein was coupled. As shown in Table 0-1, the purified scFv-Fc PR002129 recombinant protein was coupled under multiple groups of conditions in parallel. The components of coupling products in each group of experiments, particularly the content of the component with DAR = 2 ('D2 %'), were analyzed by the HIC-HPLC analysis method described in 0. After the coupling was completed, the excess drug conjugates were removed from the product through a desalting column; and optionally, the method described in 0 was used to purify the coupling product to obtain a main product with DAR = 2. The reaction conditions used in Experiment #20210202-2 were as follows: a reaction buffer was a buffer with pH of 6.0 containing 20 mM His-HCl, the amount of usage of the reducing agent TCEP was 2.3 times molar equivalents, the reduction reaction was carried out at room temperature for 2 h, the amount of usage of a conjugate mc-vc-PAB-MMAE was 7.0 molar equivalents, and the coupling reaction was carried out at room temperature for 0.5 h.

**Table 0-1. Coupling reaction conditions of PR002129**

| Experiment # | Protein concentration | Amount of usage of TCEP | Amount of usage of VcMMAE | HIC-HPLC analysis results of coupling product | | |
|---|---|---|---|---|---|---|
| | | | | Average DAR | Naked antibody % | D2 % |
| 20081401 | 3.0 mg/ml | 2.2 x | 7.0 x | | 18.5% | 59.2% |
| 20210202-1 | 6.5 mg/ml | 1.9 x | 7.0 x | | | 67.0% |
| 20210202-2 | 6.5 mg/ml | 2.3 x | 7.0 x | 2.07 | 10.3% | 73.2% |
| 20210202-3 | 6.5 mg/ml | 2.5 x | 7.0 x | | | 68.0% |

**Example 13.2 Analysis of coupling product**

In this example, techniques such as high-performance liquid chromatography and mass spectrometry were utilized to quantitatively analyze the purified recombinant PR002129 and a coupling product PR002129-ADC thereof (including a product after coupling and a product after one-step HIC purification) to determine the components of the coupling product.

The HIC-HPLC method described in 0 was used to analyze the above samples. 0 and the table below show HIC-HPLC analysis results of a coupling product PR002129-ADC of PR002129 respectively: (A) after coupling and before purification (Table 0-2); (B) after coupling and one-step HIC purification (Table 0-3). Before HIC purification, the coupling product PR002129-ADC had a DAR = 2 component accounting for 73.2%, and the calculated average DAR value was 2.07; after one-step HIC purification, 97.9% of the components had DAR = 2.

**Table 0-2. HIC-HPLC analysis results of sample (lot.# CY20210202) before PR002129-ADC purification**

| Retention time (min) | Peak area% | Peak instructions |
|---|---|---|
| 11.86 | 10.28 | DAR = 0 component peak |
| 13.34 | 1.36 | DAR = 1 component peak |
| 15.54 | 73.2 | DAR = 2 component peak |
| 19.99 | 15.17 | DAR = 4 component peak |

**Table 0-3. HIC-HPLC analysis results of sample (lot.# HSP31405-20200820) after purification of PR002129-ADC**

| Retention time (min) | Peak area% | Peak instructions |
|---|---|---|
| 8.30 | 97.9 | DAR = 2 component peak |

The method (LC instrument: Agilent 1290 UPLC; MS instrument: AB Sceix X500B) described in 0 was used to analyze the molecular weight of the sample before and after purification of PR002129-ADC. Before LC-MS analysis, the sample to be tested was desaccharified. 0 shows mass spectrometry deconvolution processing maps of molecular weight analysis: (A) non-reduced molecular weights of samples before HIC purification; (B) reduced molecular weights of samples after HIC purification; (C) non-reduced molecular weights of samples after HIC purification. According to the process of the molecular weight analysis method described in 0, the molecular weight of the components coupled with different numbers of compounds and the contents thereof in the samples were calculated (see the table below). Table 0-4 shows that in the non-reduced samples of PR002129-ADC before HIC purification, the component with DAR = 2 accounts for 74.1%, and the average DAR value is calculated to be 2.03. Table 0-5 shows that in the non-reduced samples of PR002129-ADC after purification, the component with DAR = 2 is a main ingredient; correspondingly, as shown in Table 0-6, in the reduced samples, the heavy chain coupled with 1 compound (+1 D) is a main ingredient (accounting for 88%), and the average DAR value of PR002129-ADC product is calculated to be 1.9.

**Table 0-4. LC-MS analysis of non-reduced molecular weight of sample (lot.# CY20210202) before purification of PR002129-ADC**

| Component | Theoretical value (Da) | Experimental value (Da) | Deviation (Da) | Content |
|---|---|---|---|---|
| PR002129 naked antibody | 104,508 | 104,479 ~ 104,510 | -29 ~ +2 | 13.8 % |
| PR002129 DAR=2 | 107,140 | 107,145 ~ 107,306 | +5 ~ +166 [*1] | 74.1 % |
| PR002129 DAR=4 | 109,771 | 109,780 | + 9 | 9.0 % |
| PR002129 heavy chain +3D [*2] | 56,202 | 56,206 | + 4 | 3.1 % |
| [*1] some saccharification (+162 Da) modifications may be present; [*2] due to all three disulfide bonds between chains are cleaved and exist in the form of a single heavy chain under denaturation conditions, the heavy chain +3D corresponds to a component with DAR = 6. | | | | |

**Table 0-5. LC-MS analysis of non-reduced molecular weight of sample (lot.# HSP31405-20200820) after purification of PR002129-ADC**

| Component | Theoretical value (Da) | Experimental value (Da) | Deviation (Da) | Content |
|---|---|---|---|---|
| PR002129 naked antibody | 104,508 | | | |
| PR002129 DAR=1 | 105,824 | 105,848 | + 24 * | |
| PR002129 DAR=2 | 107,140 | 107,163 | + 23 * | Main |
| (* molecular weight deviation caused by oxidation modification of certain groups may be included) | | | | |

**Table 0-6. LC-MS analysis of reduced molecular weight of sample (lot.# HSP31405-20200820) after purification of PR002129-ADC**

| Component | Theoretical value (Da) | Experimental value (Da) | Deviation (Da) | Content |
|---|---|---|---|---|
| PR002129 heavy chain | 52,255 | 52,258 | + 3 | 10 % |
| PR002129 heavy chain +1D | 53,571 | 53,575 | + 4 | 88 % |
| PR002129 heavy chain +2D | 54,887 | 54,892 | + 5 | 2 % |

Based on the results of various analytical methods above, it can be inferred that by using cysteine for coupling, the main ingredient in the coupling product is a component with DAR = 2. After further HIC purification, a highly-homogeneous product with a purity of about 90% and DAR = 2 can be obtained.

**Example 13.3 Analysis of compound coupling site by LC-MS peptide map**

In this example, the method described in 0 was used to analyze coupling sites of PR002129-ADC.

0 shows analysis of compound coupling sites of PR002129-ADC by using LC-MS peptide maps, including 3 screened peptide fragments with coupling sites and corresponding coupling site coverage rates. All peptide fragments containing Cys sites and undergoing coupling

modifications were analyzed, and the secondary map corresponding to each peptide fragment was verified one by one to confirm that the coupling modifications they undergone were highly reliable. For example, the peptide fragment containing SC (+ 1315.78) DK accounted for 88%, indicating that 88% of the Cys-220 site was coupled with a compound.

**Example 13.4 Binding to cells highly expressing ROR1**

This example was to study the activity of an antigen-binding protein PR002129 and a coupling product PR002129-ADC thereof binding to cells highly expressing ROR1.

The binding ability of the antigen-binding protein to tumor cells PanC-1 (ATCC, CRL-1469) highly expressing human ROR1 was determined by flow cytometry FACS. Specifically, the cells were digested and resuspended in a DMEM complete medium, and the cell density was adjusted to 1 $\times$ $10^6$ cells/mL. The cells were seeded in a 96-well V-bottom plate (Corning, #3894) at 100 $\mu$L/well, followed by the addition of test binding proteins diluted in a 3-fold gradient at a concentration that was 2 times the final concentration, each at 100 $\mu$L/well. The cells were incubated at 4 °C for 1 h away from light. Thereafter, the cells in each well were rinsed twice with 100 $\mu$L of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcγ Fragment Specific, Jackson ImmunoResearch Inc., #109-545-098, diluted in a 1:500 ratio) was added at 100 $\mu$L/well, and the plate was incubated at 4 °C for 30 min away from light. The cells in each well were then rinsed twice with 100 $\mu$L of pre-cooled PBS and centrifuged at 500 g at 4 °C for 5 min, and then the supernatant was discarded. Finally, the cells in each well were resuspended in 200 $\mu$L of pre-cooled PBS. Fluorescence signal values were read using a BD FACS CANTOII flow cytometer, and the data were processed and analyzed using FlowJo v10 (FlowJo, LLC) software.

The data were processed and analyzed by plotting using GraphPad Prism 8 software, and binding curves of the antibodies to target cells, $EC_{50}$ values and other parameters were obtained through four-parameter nonlinear fitting.

As shown in 0, both PR002129 and the coupling product PR002129-ADC thereof have strong specific binding activity to the PanC-1 cells, with similar binding ability.

**Example 14 Coupling and Analysis of SIRPa-Fc fusion protein**

In this example, the drug coupling method of the present application was applied to non-antibody fusion proteins, to conduct site-specific coupling on the fusion proteins using the first Cys of the linker peptide (such as Cys-220 of a human IgG1 hinge region). This is a brand new attempt to extend drug coupling beyond antibodies, but to any fusion protein as long as it includes a structure of the linker peptide; this indicates that the drug coupling method of the present application has universality.

ALX148 is a high-affinity variant fusion protein of human SIRPa (Kauder SE et al., PLoS ONE (2018) 13(8): e0201832), and its sequence is disclosed in Patent US10829771B2. Compared with the wild-type sequence of human SIRPa, the affinity of ALX148 to CD47 is increased by about 3000 times.

In this example, the SIRPa variant partial sequence in ALX148 was fused with a sequence containing a human IgG1 hinge region and Fc (SEQ ID NO: 72) and expressed to obtain the SIRPa-Fc fusion protein PR006345 (SEQ ID NO: 149). Thereafter, coupling and analysis were conducted on PR006345.

**Example 14.1 Optimization of coupling reaction conditions**

This example utilized the coupling method described in 0 to conduct coupling reaction on the SIRPa-Fc fusion protein PR006345, to produce different coupling reaction products by changing the amount of usage of a reducing agent TCEP based on the preferred reaction condition parameters obtained in 0. In this experiment, the concentration of PR006345 was 4.1 mg/mL, a reaction buffer was a buffer with pH of 6.0 containing 20 mM His-HCl, the amount of usage of the reducing agent TCEP varied from 1.5 to 3.0 times molar equivalents, the reduction reaction was conducted at room temperature for 2 h, the amount of usage of a conjugate mc-vc-PAB-MMAE was 7.0 molar multiples, and the coupling reaction was conducted at the room temperature for 0.5 h; the coupling product did not undergo the step of HIC purification. Furthermore, for the coupling product, the molecular weight analysis method used in 0 was also used to calculate the molecular weight of the components coupled with different numbers of compounds and the contents thereof in the samples. The analysis results are listed in Table 0-1.

**Table 0-1. Analysis results of respective components of coupling product of fusion protein PR006345 under different reaction conditions (without HIC purification) by LC-MS**

| Experiment # | Equivalents of TCEP | D2 % | Average DAR |
|---|---|---|---|
| 1 | 1.5 | 88% | 1.89 |
| 2 | 2.0 | 89% | 2.23 |
| 3 | 3.0 | 74% | 2.91 |

The coupling product produced in Experiment #2 was determined using the LC-MS molecular weight analysis method to have a DAR = 2 component content of 89%, and the average DAR value of the coupling product was 2.23.

**Example 14.2 Analysis of coupling product**

In this example, techniques such as high-performance liquid chromatography and mass spectrometry were utilized to analyze the SIRPa-Fc fusion protein PR006345 and the coupling product PR006345-ADC (without HIC purification) produced in Experiment #2, to determine the components of the coupling product.

The HIC-HPLC method described in 0 was used to analyze the above samples. 0 and Table 0-2 show the HIC-HPLC analysis results of unpurified coupling product PR006345-ADC; the component with DAR = 2 accounts for 75.4%, and the calculated average DAR value is 1.83. The method (LC instrument: Agilent 1290 UPLC; MS instrument: AB Sceix X500B) described in 0 was used to analyze the molecular weight of the unpurified coupling product PR006345-ADC. Before LC-MS analysis, the sample to be tested was desaccharified. 0 shows a mass spectrometry deconvolution processing map of molecular weight analysis. According to the process of the molecular weight analysis method described in 0, the molecular weight of the components coupled with different numbers of compounds and the contents thereof in the samples were calculated (Table 0-3); the component with DAR = 2 accounted for 89%, and the calculated average DAR value was 2.23.

**Table 0-2. HIC-HPLC analysis results of sample (lot.# CY20210202) before PR006345-ADC purification**

| Retention time (min) | Peak area% | Peak instructions |
|---|---|---|
| 10.36 | 14.66 | DAR = 0 component peak |
| 12.27 | 2.67 | DAR = 1 component peak |
| 14.82 | 75.37 | DAR = 2 component peak |
| 19.68 | 7.30 | DAR = 4 component peak |

**Table 0-3. LC-MS analysis of non-reduced molecular weight of sample (lot.# CY20210202) before purification of PR006345-ADC**

| Component | Theoretical value (Da) | Experimental value (Da) | Deviation (Da) | Content |
|---|---|---|---|---|
| PR006345 DAR=0 | 78,640 | 78,640 | 0 | 3 % |
| PR006345 DAR=2 | 81,272 | 81,152 ~81,439 | -120 ~ +167 [*1] | 89 % |
| PR006345 DAR=4 | 83,904 | 83,910 | +6 | 2 % |
| PR006345 heavy chain +3D [*2] | 43,268 | 43,272 | +4 | 7 % |
| [*1] some saccharification (+162 Da) modifications may be present; [*2] due to all three disulfide bonds between chains are cleaved and exist in the form of a single heavy chain under denaturation conditions, the heavy chain +3D corresponds to a component with DAR = 6. | | | | |

Based on the results of various analytical methods above, it can be inferred that by using cysteine for coupling, the main ingredient in the coupling product of the SIRPa-Fc fusion protein is a component with DAR = 2.

**Example 14.3 Analysis of compound coupling site by LC-MS peptide map**

In this example, the method described in 0 was used to analyze coupling sites of PR006345-ADC.

0 shows analysis of compound coupling sites of PR006345-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing SC (+ 1315.78) DK accounted for 92.45%, indicating that 92.45% of the Cys-220 site was coupled with a compound.

**Example 14.4 Binding to cells highly expressing CD47**

This example was to study the activity of SIRPa-Fc fusion protein PR006345 and a coupling product PR006345-ADC thereof binding to cells highly expressing CD47.

The binding ability of the antigen-binding protein to CHOK1-hCD47 cells (GenScript, M00581) highly expressing human CD47 was determined by flow cytometry FACS. Specifically, the cells were digested and resuspended in a DMEM complete medium, and the cell density was adjusted to 1 $\times$ $10^6$ cells/mL. The cells were seeded in a 96-well V-bottom plate (Corning, #3894) at 100 $\mu$L/well, followed by the addition of test binding proteins diluted in a 3-fold gradient at a concentration that was 2 times the final concentration, each at 100 $\mu$L/well. The cells were incubated at 4 °C for 1 h away from light. Thereafter, the cells in each well were rinsed twice with 100 $\mu$L of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcy Fragment Specific, Jackson ImmunoResearch Inc., #109-545-098, diluted in a 1:500 ratio) was added at 100 $\mu$L/well, and the plate was incubated at 4 °C for 30 min away from light. The cells in each well were then rinsed twice with 100 $\mu$L of pre-cooled PBS and centrifuged at 500 g at 4 °C for 5 min, and then the supernatant was discarded. Finally, the cells in each well were resuspended in 200 $\mu$L of pre-

cooled PBS. Fluorescence signal values were read using a BD FACS CANTOII flow cytometer, and the data were processed and analyzed using FlowJo v10 (FlowJo, LLC) software.

The data were processed and analyzed by plotting using GraphPad Prism 8 software, and binding curves of the antibodies to target cells, $EC_{50}$ values and other parameters were obtained through four-parameter nonlinear fitting.

As shown in 0, both PR006345 and the coupling product PR006345-ADC thereof have strong specific binding activity to CD47, with similar binding ability.

**Example 15 Coupling and Analysis of Bispecific Antigen-Binding Protein PR005744**

In this example, a tetravalent bispecific Fab-HCAb structure as shown in 0 was constructed using the antigen-binding fragment VH and antigen-binding fragment Fab, and the drug coupling method of the present application was applied to the structure to conduct site-specific coupling using the first Cys of the linker peptide (such as Cys-220 of a human IgG1 hinge region).

The structure belongs to a bispecific symmetric structure as shown in 0(B), where one antigen-binding fragment is Fab and the other antigen-binding fragment is VH. Although the structure has two different polypeptide chains (called short and long chains), the short chain does not form a disulfide bond with the first Cys (Cys-220) on the linker peptide of the long chain. Therefore, under some combinations of reaction conditions, the disulfide bond of the Cys can be effectively cleaved while the other disulfide bonds remain intact, so that the Cys can serve as a site-specific coupling site.

**Example 15.1 Construction and preparation of PR005744**

In this example, the VH sequence (SEQ ID NO: 62) of anti-BCMA HCAb PR004433 and the VH sequence (SEQ ID NO: 130) and VL sequence (SEQ ID NO: 131) of an anti-BCMA IgG antibody PR000892 were used to construct a bispecific (dual binding epitope) antigen-binding protein PR005744 with a tetravalent Fab-HCAb structure as shown in 0. The sequence of PR000892 is disclosed in Invention Patent CN111234020B. The sequence numbers for PR004433, PR000892, and PR005744 are listed in Table 0-1 and Table 0-2.

**Table 0-1. Sequence number table of monoclonal antibodies used in this example.**

| Antigen-binding protein | Type | VH | VL | Heavy chain | Light chain |
|---|---|---|---|---|---|
| PR004433 | HCAb | 62 | | 70 | |
| PR000892 | IgG1 | 130 | 131 | 132 | 133 |

**Table 0-2. Sequence number table of bispecific antibody constructed in this example.**

| Antigen-binding protein | Structure | Short chain | Long chain |
|---|---|---|---|
| PR005744 | Fab-HCAb | 134 | 135 |

The antigen-binding protein PR005744 was prepared using the method described in 0. Thereafter, its ability to bind to BCMA and its internalization ability on the NCI-H929 cells (ATCC, CRL-9068) highly expressing BCMA were tested.

**Example 15.2 Internalization effect of PR005744**

This example was to study the killing against cells expressing human BCMA mediated by internalization of BCMA-targeted antigen-binding proteins. Specifically, NCI-H929 (ATCC, CRL-9068) cells were seeded in a 96-well plate (Beyotime, # FT018) at $2 \times 10^5$ cells/well; then, 200 nM test antigen-binding proteins diluted in an FACS buffer was added; then, the plate was incubated at 4 °C for 1 h; thereafter, samples were taken and incubated at 37 °C for various periods of time (e.g., 30 min, 1 h, 2 h, and 4 h); then, the cells were centrifuged and resuspended, and incubated at 4 °C for 30 min after a fluorescent secondary antibody (Jackson ImmunoResearch Inc., #109-545-098) was added. Finally, fluorescence signal values were read using a flow cytometer, and the data were processed and analyzed using FlowJo v10 (FlowJo, LLC) software. The MFI of the fluorescence signal at 0 min (T0) of incubation at 37 °C was taken as a baseline, and the MFIs of samples at different incubation times were subtracted from the baseline of T0 and the relative reduction was calculated to reflect the efficiency of internalization of the antigen-binding proteins. The data were processed and analyzed by plotting using a GraphPad Prism 8 software.

As shown in 0, the bispecific antigen-binding protein PR005744 exhibited strong internalization; it allowed more than 60% of BCMA to be internalized within 30 min.

**Example 15.3 Analysis of coupling product of PR005744**

Further, the method described in 0 or 0 was used to conduct coupling on PR005744. In this experiment, a reaction buffer was 20 mM His-HCl with pH of 6.0; a reducing agent was TCEP, with variable molar equivalent; the reduction reaction was conducted at room temperature for 2 h, the amount of usage of a conjugate mc-vc-PAB-MMAE was 7.0 molar multiples, and the coupling reaction was conducted at the room temperature for 0.5 h. For each group of experiments, the coupling products were subjected to complete molecular weight analysis of the non-reduced samples using the method (LC instrument: Agilent 1290 UPLC; MS instrument: AB Sceix X500B) described in 0, and the molecular weights of the components coupled with different numbers of compounds and the contents in the samples thereof were calculated by using the total ion chromatogram or deconvolution map in the mass spectrum of each sample. The analysis results are listed in Table 0-3. For example, when using the combination of the reaction condition parameters (a reaction buffer was a buffer with pH of 6.0 containing 20 mM His-HCl, the amount of usage of the reducing agent TCEP was 3.5 molar multiples, the reduction reaction was conducted at room temperature for 2 h, the amount of usage of the conjugate mc-vc-PAB-MMAE was 7.0 molar multiples, and the coupling reaction was conducted at the room temperature for 0.5 h), about 84% of the components in the coupling product were coupled with 2 drug conjugates (that is, DAR = 2, D2 %).

**Table 0-3. Analysis results of respective components of product after coupling of PR005744 (without HIC purification) by LC-MS**

| Equivalents of TCEP | D0 % | D2 % | D4 % | D6 % | Average DAR |
|---|---|---|---|---|---|
| 2.0 | 23.1 % | 71.8 % | 4.0 % | 1.1 % | 1.66 |
| 2.5 | 21.2 % | 72.6 % | 5.1 % | 1.1 % | 1.72 |
| 3.5 | 6.4 % | 84.1 % | 7.1 % | 2.4 % | 2.11 |

**Example 15.4 Analysis of compound coupling site by LC-MS peptide map**

In this example, the method described in 0 was used to analyze coupling sites of PR005744-ADC.

0 shows analysis of compound coupling sites of PR005744-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing SC (+ 1315.78) DK accounted for 99.07%, indicating that almost all Cys-220 sites were coupled with compounds. This confirms that drug coupling specifically occurs at the first Cys (i.e., Cys-220) of the linker peptide in PR005744.

**Example 15.5 Binding to cells highly expressing BCMA**

In this example, the method described in 0 was used to study the binding ability of PR005744 and a coupling product PR005744-ADC thereof to tumor cells NCI-H929 (ATCC, CRL-9068) highly expressing human BCMA.

As shown in 0, both PR005744 and the coupling product PR005744-ADC thereof can specifically bind to the NCI-H929 cells, with similar binding ability.

**Example 15.6 Cytotoxic killing experiment**

In this example, the method described in 0 was used to study the specific cell killing activity of PR005744 and a coupling product PR005744-ADC thereof against tumor cells NCI-H929 (ATCC, CRL-9068) highly expressing human BCMA.

As shown in 0, PR005744-ADC produces effective and specific cytotoxic killing against NCI-H929, and exhibits concentration dependence, while PR005744 cannot produce effective killing against the cells.

**Example 16 Coupling and Analysis of HCAb PR004433-PROTAC**

This example studied the preparation of an antibody-drug conjugate using cysteine coupling to Cys-220 site by an anti-BCMAHCAb antibody PR004433 (SEQ ID NO: 70) and a proteolysis targeting chimera (PROTAC).

The PROTACs are a class of compounds that can cause the degradation of a target protein by inducing polyubiquitination of the target protein. The mechanism of action of the PROTACs is very different from traditional small cytotoxic molecule compounds such as MMAE, and their molecular structures are more complex. At present, although the PROTACs have good efficacy against many targets, their tissue selectivity is relatively limited and it is difficult to distinguish different cell types. There are literature reports that coupling the PROTACs to HER2-binding antibodies can enhance the selectivity of the PROTACs, so that they can only degrade targets in HER2-positive tumor cells (Maneiro MA et al., ACS Chem Biol., 2020;15(6):1306-1312).

In this example, the PROTAC used was a BRD4 protein degrading agent (with a structure as shown in 0), which comprises a PROTAC GNE-987 targeting BET and a linker comprising a disulfide. The PROTAC served as a drug conjugate, and the drug coupling method of the present application was used to conduct site-specific coupling on an HCAb antibody PR004433. This is a brand new attempt to extend the drug conjugate beyond traditional cytotoxic compounds such as MMAE, but to other loaded drug types; this indicates that the drug coupling method of the present application has universality.

### Example 16.1 Coupling reaction and analysis of PROTAC

In this example, the coupling method described in 0 and further changed conditions were used to conduct coupling reaction on HCAb PR004433. The drug conjugate was a BRD4 protein degrading agent compound (with a structure as shown in 0 and molecular weight of 1306.58 Da; manufacturer: MedChemExpress, Cat. No.: HY-129938), which was abbreviated as PROTAC in this example. In the coupling experiment, the concentration of PR004433 was 5.0 mg/mL, a reaction buffer was a buffer with pH of 6.0 containing 20 mM His-HCl, the amount of usage of a reducing agent TCEP was 3.0 molar equivalents, and the reduction reaction was conducted at room temperature for 2 h; after the reduction reaction, the product was replaced with a 30 kd ultrafiltration tube into a buffer with pH of 8.0 containing 50 mM Tris-HCl; the amount of usage of the conjugate PROTAC was 10.0 to 15.0 molar equivalents, and the coupling reaction was conducted at the room temperature for 2 h; the coupling product (PR004433-PROTAC) did not undergo the step of HIC purification.

During the coupling reaction, the methylsulfonyl ($CH_3SO_2$) of the PROTAC molecule was cleaved, exposing a reactive sulfhydryl, and then forming a disulfide bond with the reactive sulfhydryl on the antibody. Therefore, coupling each PROTAC to the antibody increased the total molecular weight by 1226 Da.

Furthermore, the method (LC instrument: Agilent 1290 UPLC; MS instrument: AB Sceix X500B) described in 0 was used to analyze the molecular weight of the coupling product PR004433-PROTAC. Before LC-MS analysis, the sample to be tested was desaccharified. 0 shows a mass spectrometry deconvolution processing map of non-reduced molecular weight analysis of PR004433-PROTAC. Based on this, the molecular weight of the components coupled with different numbers of PROTACs and the contents thereof in the samples were calculated. Table 0-1 shows that in the non-reduced samples of PR004433-PROTAC, the component with DAR = 2 is a main ingredient (accounting for 66.4%), and the average DAR value of the coupling product is calculated to be 1.6.

**Table 0-1. LC-MS analysis of non-reduced molecular weight of sample (lot.# CY20210203) before purification of PR004433-PROTAC**

| Component | Theoretical value (Da) | Experimental value (Da) | Deviation (Da) | Content |
|---|---|---|---|---|
| PR004433 naked antibody | 78,797 | 78,798 | + 1 | 28.4 % |
| PR004433 DAR=2 | 81,249 | 81,225 81,414 | -24 ~ +165 [*] | 66.4 % |
| PR004433 DAR=4 | 83,701 | 83,708 | + 7 | 3.5 % |
| [*] some saccharification (+ 162 Da) modifications may be present. | | | | |

### Example 16.2 Binding to cells highly expressing BCMA

In this example, the method described in 0 was used to study the binding ability of HCAb PR004433 and a coupling product PR004433-PROTAC thereof to tumor cells NCI-H929 (ATCC, CRL-9068) highly expressing human BCMA.

As shown in 0, both PR004433 and the coupling product PR004433-PROTAC thereof can specifically bind to the NCI-H929 cells.

**Example 17 Coupling and Analysis of Bispecific Antigen-Binding Protein 2129/4433**

In this example, the VH sequence (SEQ ID NO: 62) of anti-BCMA HCAb PR004433 and the scFv sequence (having VH set forth in SEQ ID NO: 60 and VL set forth in SEQ ID NO: 63) of an anti-ROR1 antibody PR002129 were used to construct a bivalent bispecific antigen-binding protein 2129/4433 with an asymmetric structure as shown in 0. The drug coupling method of the present application was applied to the structure, to conduct site-specific coupling using the first Cys of the linker peptide (such as Cys-220 of a human IgG1 hinge region).

The structure belongs to the structure as shown in 0(A), but a first antigen-binding fragment and a second antigen-binding fragment thereof have different structures and amino acid sequences. The first antigen-binding fragment is a VH structure, and the second antigen-binding fragment is an scFv structure, so it is an asymmetric structure comprising two different polypeptide chains. The first polypeptide chain comprises a first linker peptide (containing a human IgG1 hinge region sequence), and the second polypeptide chain comprises a second linker peptide (containing a human IgG1 hinge region sequence). The first Cys (Cys-220) on the first linker peptide and the first Cys (Cys-220) on the second linker peptide form a disulfide bond. Therefore, under some combinations of reaction conditions, the disulfide bond of the Cys-220 can be effectively cleaved while the other disulfide bonds remain intact, so that the Cys can serve as a site-specific coupling site.

**Example 17.1 Construction and preparation of 2129/4433**

A plasmid encoding PR004433 polypeptide chain (SEQ ID NO: 70) and a plasmid encoding PR002129 polypeptide chain (SEQ ID NO: 71) were co-transfected, and expressed and undergone one-step affinity purification according to the method described in 0. The product had a PR004433 homodimer (4433/4433), a PR002129 homodimer (2129/2129), and PR004433 and PR002129 heterodimers (2129/4433, 0) formed by each chain. The heterodimer protein 2129/4433 was a target protein in this experiment. Due to the different molecular weights and isoelectric points of these three components (4433/4433, 2129/2129, 2129/4433) (Table 0-1), the product could be further separated using methods such as ion exchange chromatography or size-exclusion chromatography to obtain the purified heterodimer target protein 2129/4433.

Furthermore, the LC-MS molecular weight analysis method described in 0 was used to confirm the formation of a disulfide bond between heavy chains between Cys-220 on 2129-chain and Cys-220 on 4433-chain of the heterodimer protein 2129/4433.

**Table 0-1. Calculation of molecular weights and isoelectric points of different dimer components**

| Component | Theoretical molecular weight value (Da) | Predicted isoelectric point value |
|---|---|---|
| 2129/2129 | 104,508 | 9.40 |
| 4433/4433 | 78,797 | 7.14 |
| 2129/4433 | 91,652 | 8.92 |

**Example 17.2 Analysis of coupling product of 2129/4433**

The method described in 0 was used to couple the purified heterodimer protein 2129/4433. In this experiment, the concentration of 2129/4433 was 3.2 mg/mL, a reaction buffer was a buffer with pH of 6.0 containing 20 mM His-HCl, the amount of usage of the reducing agent TCEP was 2.1 to 2.5 molar equivalents, the reduction reaction was carried out at room temperature for 2 h, the amount of usage of a conjugate mc-vc-PAB-MMAE was 7.0 molar equivalents, and the coupling reaction was carried out at the room temperature for 0.5 h. After the coupling was completed, the excess drug conjugates were removed from the product through a desalting column, to obtain a coupling product 2129/4433-ADC. Optionally, the HIC method described in 0 was further used to purify the coupling product.

Then, the HIC-HPLC method described in 0 was used to analyze the 2129/4433-ADC sample without HIC purification. 0 and Table 0-2 show the HIC-HPLC analysis results of the samples before purification of 2129/4433-ADC: before HIC purification, the component with DAR = 2 accounts for 68.7%, and the calculated average DAR value is 2.26.

**Table 0-2. HIC-HPLC analysis results of sample (lot.# CY20210202) before purification of coupling product 2129/4433-ADC**

| Retention time (min) | Peak area% | Peak instructions |
|---|---|---|
| 12.88 | 7.05 | DAR = 0 component peak |

(continued)

| Retention time (min) | Peak area% | Peak instructions |
|---|---|---|
| 15.65 | 3.05 | DAR = 1 component peak |
| 17.12 | 68.72 | DAR = 2 component peak |
| 20.81 | 18.43 | DAR = 4 component peak |

### Example 17.3 Analysis of compound coupling site by LC-MS peptide map

In this example, the method described in 0 was used to analyze coupling sites of 2129/4433-ADC.

0 shows analysis of compound coupling sites of samples (the component with DAR = 2 accounts for 100%) after HIC purification of 2129/4433-ADC by using LC-MS peptide maps, including 3 screened peptide fragments with coupling sites and corresponding coupling site coverage rates. All peptide fragments containing Cys sites and undergoing coupling modifications were analyzed, and the secondary map corresponding to each peptide fragment was verified one by one to confirm that the coupling modifications they undergone were highly reliable. For example, the peptide fragment containing SC (+ 1315.78) DK accounted for 99.6% (0 shows the secondary map of this peptide fragment), indicating that almost all Cys-220 sites were coupled with compounds.

### Example 18 Coupling of Immunomodulatory Agonist Molecules

Toll-like receptor 9 (TLR9) is an important receptor molecule widely expressed in immune cells such as dendritic cells and macrophages, playing an important role in human innate immunity. CpG oligonucleotide is a natural agonist ligand of TLR9, and the TLR9 can activate immune cells by binding to CpG. In this example, the CpG oligonucleotide was coupled to the antigen-binding protein in the aforementioned examples by cysteine through a linker containing a maleimide group or sulfhydryl to obtain a coupling product. For example, anti-5T4 HCAb PR004432 and the CpG oligonucleotide CpG-1826 containing a 3'-terminus sulfhydryl modification (sequence 5'-tccatgacgttcctgacgtt-3') were coupled to the Cys1 site (e.g., Cys-220) of the linker peptide using the method described in the present invention to prepare a drug conjugate PR004432-CpG; an anti-ROR1 binding protein PR002129 and CpG were coupled to the Cys-220 site to prepare a drug conjugate PR002129-CpG; a SIRPa-Fc fusion protein PR006345 and CpG were coupled to the Cys-220 site to prepare a drug conjugate PR006345-CpG. These CpG drug conjugates specifically identify antigen targets on tumors, use Fc receptors on immune cells in tumor microenvironment to mediate internalization and activate TLR9 through CpG, and further activate immune cells, thereby improving the anti-tumor effect using multiple mechanisms.

The foregoing detailed explanation is provided by way of explanation and example, and is not intended to limit the scope of the appended claims. Various modifications of the embodiments currently listed in the present application will be apparent to those of ordinary skill in the art, and are intended to be within the scope of the appended claims and their equivalents.

## Claims

1. A protein-drug conjugate, comprising an antigen-binding protein moiety and a drug conjugate moiety, the antigen-binding protein moiety comprising one or more antigen-binding fragments, and at least two linker peptides directly or indirectly connected with the antigen-binding fragment; the at least two linker peptides comprising a first linker peptide and a second linker peptide, wherein the first linker peptide comprises a first cysteine Cys1 and a second cysteine Cys2, and the drug conjugate moiety is coupled to the first linker peptide through the Cys1 and the second linker peptide comprises a first cysteine Cys1 and a second cysteine Cys2, and the drug conjugate moiety is coupled to the second linker peptide through the Cys1.

2. The protein-drug conjugate according to claim 1, wherein the Cys2 of the first linker peptide is connected to the Cys2 of the second linker peptide through a disulfide bond.

3. The protein-drug conjugate according to claim 1 or 2, wherein the one or more antigen-binding fragments do not comprise a functional group capable of affecting coupling of a drug conjugate with the first linker peptide and/or the second linker peptide at the Cys1, or a conjugate formed by the functional group and other drug conjugates, and the affection is manifested in significantly reducing a coupling probability of the drug conjugate at a Cys1 site.

4. The protein-drug conjugate according to any one of claims 1 to 3, wherein the one or more antigen-binding fragments do not comprise a functional group capable of forming a disulfide bond with the Cys1 of the first linker peptide and/or the Cys1 of the second linker peptide, or a conjugate formed by the functional group and other drug conjugates.

5. The protein-drug conjugate according to any one of claims 1 to 4, wherein the one or more antigen-binding fragments do not comprise a cysteine capable of forming a disulfide bond with the Cys1 of the first linker peptide and/or the Cys1 of the second linker peptide, or a conjugate formed by the cysteine and other drug conjugates.

6. The protein-drug conjugate according to any one of claims 1 to 5, wherein the first linker peptide and the second linker peptide are identical or different.

7. The protein-drug conjugate according to any one of claims 1 to 6, wherein the Cys1 and the Cys2 of the first linker peptide are separated by at least any 3 amino acids other than cysteine, and/or the Cys1 and the Cys2 of the second linker peptide are separated by at least 3 amino acids other than cysteine.

8. The protein-drug conjugate according to any one of claims 1 to 7, wherein the first linker peptide and/or the second linker peptide comprises an amino acid sequence set forth in Cys1-(X)n-Cys2 from an N-terminus to a C-terminus, wherein n is an integer greater than or equal to 3, and X is any amino acid other than cysteine.

9. The protein-drug conjugate according to any one of claims 1 to 8, wherein the first linker peptide and/or the second linker peptide are/is derived from an antibody hinge region sequence or a derivative sequence thereof.

10. The protein-drug conjugate according to claim 9, wherein the antibody hinge region is an IgG hinge region.

11. The protein-drug conjugate according to claim 9 or 10, wherein the antibody hinge region is a human IgG1 hinge region (SEQ ID NO: 73) or a mouse IgG2c hinge region (SEQ ID NO: 147).

12. The protein-drug conjugate according to any one of claims 1 to 11, wherein the Cys1 is Cys-220 according to EU numbering in the human IgG1 hinge region, or a corresponding cysteine, or the Cys1 is a first cysteine in the mouse IgG2c hinge region.

13. The protein-drug conjugate according to any one of claims 1 to 12, wherein sequences of the first linker peptide and the second linker peptide are each independently an amino acid sequence set forth in any one of SEQ ID NOs: 73-105 and 145-147.

14. The protein-drug conjugate according to any one of claims 1 to 13, wherein the plurality of antigen-binding fragments are connected to the same linker peptide or respectively connected to the different linker peptides.

15. The protein-drug conjugate according to any one of claims 1 to 14, wherein the plurality of antigen-binding fragments can be partially or completely identical or different from each other.

16. The protein-drug conjugate according to any one of claims 1 to 15, wherein the plurality of antigen-binding fragments comprise a first antigen-binding fragment and a second antigen-binding fragment.

17. The protein-drug conjugate according to any one of claims 1 to 16, wherein the plurality of antigen-binding fragments further comprise a third antigen-binding fragment and/or a fourth antigen-binding fragment.

18. The protein-drug conjugate according to any one of claims 1 to 17, wherein the one or more antigen-binding fragments are capable of each independently being a VHH domain, a VH, a VL, an Fab, an ScFv, a receptor protein soluble extracellular region, a ligandin, a lipocalin, a neuronal cell adhesion molecule NCAM, a fibronectin and/or a designed ankyrin repeat protein DARPins.

19. The protein-drug conjugate according to any one of claims 1 to 18, wherein the one or more antigen-binding fragments are capable of specifically binding to a tumor antigen or a non-tumor antigen.

20. The protein-drug conjugate according to claim 19, wherein the tumor antigen comprises CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG (TMPRSS2 ETS fusion gene), NA17, PAX3, androgen receptor (AR), Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, Cadherin17, CD32b, EGFRvIII, GPNMB, GPR64, HER3, LRP6, LYPD8, NKG2D, SLC34A2, SLC39A6, SLITRK6, GUCY2C, 5T4 and/or TACSTD2.

**21.** The protein-drug conjugate according to any one of claims 1 to 20, wherein the one or more antigen-binding fragments each independently comprise HCDR1, HCDR2, and HCDR3, and the antigen-binding fragments comprise amino acid sequences selected from any one of (1) HCDR1: SEQ ID NO: 7, HCDR2: SEQ ID NO: 22, HCDR3: SEQ ID NO: 38; (2) HCDR1: SEQ ID NO: 10, HCDR2: SEQ ID NO: 25, HCDR3: SEQ ID NO: 41; (3) HCDR1: SEQ ID NO: 11, HCDR2: SEQ ID NO: 26, HCDR3: SEQ ID NO: 42; (4) HCDR1: SEQ ID NO: 13, HCDR2: SEQ ID NO: 28, HCDR3: SEQ ID NO: 44; (5) HCDR1: SEQ ID NO: 14, HCDR2: SEQ ID NO: 29, HCDR3: SEQ ID NO: 42; (6) HCDR1: SEQ ID NO: 9, HCDR2: SEQ ID NO: 24, HCDR3: SEQ ID NO: 40; and (7) HCDR1: SEQ ID NO: 8, HCDR2: SEQ ID NO: 23, HCDR3: SEQ ID NO: 39.

**22.** The protein-drug conjugate according to any one of claims 1 to 21, wherein the one or more antigen-binding fragments each independently comprise a VH, and the VH comprises an amino acid sequence set forth in any one of SEQ ID NOs: 55-59, 61 and 62.

**23.** The protein-drug conjugate according to any one of claims 1 to 22, wherein the one or more antigen-binding fragments each independently comprise HCDR1, HCDR2, HCDR3, LCDR1, LCDR2 and LCDR3, and the HCDR1, HCDR2, HCDR3, LCDR1, LCDR2 and LCDR3 respectively and sequentially comprise amino acid sequences set forth in SEQ ID NO: 12, SEQ ID NO: 27, SEQ ID NO: 43, SEQ ID NO: 49, SEQ ID NO: 51 and SEQ ID NO: 53.

**24.** The protein-drug conjugate according to any one of claims 1 to 23, wherein the one or more antigen-binding fragments comprise at least one pair of VH and VL, and the pair of VH and VL respectively comprises amino acid sequences set forth in SEQ ID NO: 60 and SEQ ID NO: 63; or the one or more antigen-binding fragments comprise at least one pair of VH and VL and/or another type of VH, and the pair of VH and VL respectively comprises amino acid sequences set forth in SEQ ID NO: 130 and SEQ ID NO: 131, and the another type of VH comprises an amino acid sequence set forth in SEQ ID NO: 62.

**25.** The protein-drug conjugate according to any one of claims 1 to 24, further comprising a pairing unit moiety directly or indirectly connected with the linker peptide, wherein the pairing unit comprises at least a first pairing subunit and a second pairing subunit, and the first pairing subunit can interact with the second pairing subunit through a covalent bond or a non-covalent bond.

**26.** The protein-drug conjugate according to claim 25, wherein the pairing unit is an antibody Fc fragment or a mutant Fc fragment.

**27.** The protein-drug conjugate according to claim 25 or 26, wherein an N-terminus of the first pairing subunit is connected to a C-terminus of the first linker peptide, and an N-terminus of the second pairing subunit is connected to a C-terminus of the second linker peptide; or the N-terminus of the first pairing subunit is connected to the C-terminus of the second linker peptide, and the N-terminus of the second pairing subunit is connected to the C-terminus of the first linker peptide.

**28.** The protein-drug conjugate according to any one of claims 1 to 27, comprising an amino acid sequence set forth in any one of SEQ ID NOs: 64-71, 106-129, 148 and 149, or a combination of amino acid sequences set forth in SEQ ID NOs: 134 and 135.

**29.** The protein-drug conjugate according to any one of claims 1 to 28, wherein the drug conjugate moiety comprises two drug conjugates respectively connected to the first linker peptide and the second linker peptide, and the drug conjugate comprises a loaded drug and optionally a linker.

**30.** The protein-drug conjugate according to claim 29, wherein the loaded drug comprises a small molecule compound, an oligonucleotide, a proteolysis targeting chimera (PROTAC), an affinity ligand, a fluorescently-labeled group, a nuclide-labeled group, a toxin molecule, an antibiotic molecule, an immunomodulator and/or a polypeptide.

**31.** The protein-drug conjugate according to claim 29 or 30, wherein the linker comprises a cleavable linker or a non-cleavable linker.

**32.** The protein-drug conjugate according to any one of claims 29 to 31, wherein the cleavable linker comprises a protease cuttable linker.

**33.** The protein-drug conjugate according to any one of claims 1 to 32, wherein the drug conjugate comprises mc-vc-PAB-MMAE and/or PROTAC.

**34.** The protein-drug conjugate according to any one of claims 1 to 33, being an antibody-drug conjugate.

**35.** A method for preparing the protein-drug conjugate according to any one of claims 1 to 34, comprising covalently binding the drug conjugate with a reactive sulfhydryl of the Cys1 on the first linker peptide and/or the second linker peptide.

**36.** A method for preparing a protein-drug conjugate, the protein-drug conjugate comprising an antigen-binding protein moiety and a drug conjugate moiety, the antigen-binding protein moiety comprising one or more antigen-binding fragments, and at least two linker peptides directly or indirectly connected with the antigen-binding fragments, the two linker peptides comprising a first linker peptide and a second linker peptide, wherein the first linker peptide comprises a first cysteine Cys1 and a second cysteine Cys2, and the second linker peptide comprises a first cysteine Cys1 and a second cysteine Cys2, and the method comprises binding the drug conjugate moiety with the first linker peptide through the Cys1 of the first linker peptide, and/or binding the drug conjugate moiety with the second linker peptide through the Cys1 of the second linker peptide.

**37.** The method according to claim 36, wherein the one or more antigen-binding fragments do not comprise a functional group capable of affecting coupling of a drug conjugate with the first linker peptide and/or the second linker peptide at the Cys1, or a conjugate formed by the functional group and other drug conjugates, and the affection is manifested in significantly reducing a coupling probability of the drug conjugate at a Cys1 site.

**38.** The method according to any of claims 36 to 37, wherein the one or more antigen-binding fragments do not comprise a functional group capable of forming a disulfide bond with the Cys1 of the first linker peptide and/or the Cys1 of the second linker peptide, or a conjugate formed by the functional group and other drug conjugates.

**39.** The method according to any one of claims 36 to 38, wherein the one or more antigen-binding fragments do not comprise a cysteine capable of forming a disulfide bond with the Cys1 of the first linker peptide and/or the Cys1 of the second linker peptide, or a conjugate formed by the cysteine and other drug conjugates.

**40.** The method according to any one of claims 36 to 39, wherein the first linker peptide and the second linker peptide are identical or different.

**41.** The method according to any one of claims 36 to 40, wherein the first linker peptide and/or the second linker peptide comprises an amino acid sequence set forth in Cys1-(X)n-Cys2 from an N-terminus to a C-terminus, wherein n is an integer greater than or equal to 3, and X is any amino acid other than cysteine.

**42.** The method according to any one of claims 36 to 41, wherein the first linker peptide and/or the second linker peptide are/is derived from an antibody hinge region sequence or a derivative sequence thereof.

**43.** The method according to claim 42, wherein the derivative sequence is obtained by modifying the antibody hinge region sequence, and the modification does not involve changes in Cys1 and Cys2 in the antibody hinge region.

**44.** The method according to claim 43, wherein the modification is adjusting the type or number of amino acids between Cys1 and Cys2 in the antibody hinge region sequence, and/or adding flexible linker amino acids before Cys1.

**45.** The method according to any one of claims 42 to 44, wherein the antibody hinge region is an IgG hinge region.

**46.** The method according to any one of claims 42 to 45, wherein the antibody hinge region is a human IgG1 hinge region (SEQ ID NO: 73) or a mouse IgG2c hinge region (SEQ ID NO: 147).

**47.** The method according to any one of claims 36 to 46, wherein the Cys1 is Cys-220 according to EU numbering in the human IgG1hinge region, or a corresponding cysteine, or the Cys1 is a first cysteine in the mouse IgG2c hinge region.

**48.** The method according to any one of claims 36 to 47, wherein sequences of the first linker peptide and the second linker peptide are each independently an amino acid sequence set forth in any one of SEQ ID NOs: 73-105 and 145-147.

**49.** The method according to any one of claims 36 to 48, comprising steps of reduction, coupling, and optionally purification.

**50.** The method according to claim 49, wherein the reduction comprises cleaving a disulfide bond formed between the Cys1 of the first linker peptide and/or the Cys1 of the second linker peptide in the antigen-binding protein moiety under a condition of a reducing agent to form a reactive sulfhydryl, while a disulfide bond formed between the Cys2 of the first linker peptide and the Cys2 of the second linker peptide is retained.

**51.** The method according to claim 49 or 50, wherein the reduction comprises using DTT and/or TCEP as a reducing agent; preferably, the reducing agent is TCEP.

**52.** The method according to claim 50 or 51, wherein an amount of usage of the reducing agent is 1 to 50 molar multiples; preferably, the amount of usage of the reducing agent is 1 to 6 molar multiples.

**53.** The method according to any one of claims 49 to 52, wherein reaction time for the reduction is 1 to 17 hours; preferably, the reaction time for the reduction is 1.5 to 3 hours; further preferably, the reaction time for the reduction is 2 hours.

**54.** The method according to any one of claims 49 to 53, wherein a reaction temperature for the reduction is 0 °C to 40 °C; preferably, the reaction temperature for the reduction is 0 °C or room temperature (25 °C to 30 °C) or 37 °C; further preferably, the reaction temperature for the reduction is room temperature (25 °C to 30 °C).

**55.** The method according to any one of claims 49 to 54, wherein a pH of a reaction buffer for the reduction is 5.0 to 8.0; preferably, the pH of the reaction buffer for the reduction is 5.0 to 7.0; further preferably, the pH of the reaction buffer for the reduction is 5.0 to 6.0.

**56.** The method according to any one of claims 49 to 55, wherein the coupling comprises covalently binding the drug conjugate with a reactive sulfhydryl of the Cys1 on the first linker peptide and/or the Cys1 on the second linker peptide.

**57.** The method according to any one of claims 36 to 56, wherein the drug conjugate comprises mc-vc-PAB-MMAE or PROTAC.

**58.** The method according to any one of claims 36 to 57, wherein an amount of usage of the drug conjugate mc-vc-PAB-MMAE is 1 to 50 molar multiples; preferably, the amount of usage of the drug conjugate is 3 to 18 molar multiples; further preferably, the amount of usage of the drug conjugate is 3 to 7 molar multiples.

**59.** The method according to any one of claims 36 to 58, wherein an amount of usage of the drug conjugate PROTAC is 10 to 15 molar multiples.

**60.** The method according to any one of claims 49 to 59, wherein reaction time for the coupling is 0.5 to 10 hours; preferably, the reaction time for the coupling is 0.5 to 3 hours; further preferably, the reaction time for the coupling is 0.5 to 2 hours.

**61.** The method according to any one of claims 49 to 60, wherein a reaction temperature for the coupling is 0 °C to 40 °C; preferably, the reaction temperature for the coupling is room temperature (25 °C to 30 °C).

**62.** The method according to any one of claims 49 to 61, wherein a pH of a reaction buffer for the coupling is 5.0 to 8.0;

preferably, the pH of the reaction buffer for the coupling is 5.0 to 7.0; further preferably, the pH of the reaction buffer for the coupling is 5.0 to 6.0.

**63.** The method according to any one of claims 36 to 62, wherein a purity of the prepared protein-drug conjugate is greater than 80%.

**64.** A pharmaceutical composition, comprising the protein-drug conjugate according to any one of claims 1 to 34 and a pharmaceutically acceptable carrier.

**65.** A method for preventing and/or treating diseases, comprising administering the protein-drug conjugate according to any one of claims 1 to 34 and/or the pharmaceutical composition according to claim 64, wherein the protein-drug conjugate and/or the pharmaceutical composition are optionally in combination with other therapies or drugs; the diseases are tumors or other diseases.

**66.** Use of the protein-drug conjugate according to any one of claims 1 to 34 and/or the pharmaceutical composition according to claim 64 in preparation of a drug, wherein the drug is used for treating tumors or other diseases.

**67.** Use of the protein-drug conjugate according to any one of claims 1 to 34 and/or the pharmaceutical composition according to claim 64, in combination with other therapies or drugs, in preparation of a drug, wherein the drug is used for treating tumors or other diseases.

**68.** Use according to claim 78 or 80, wherein the other therapies or drugs are selected from: chemotherapy, radiotherapy, miRNA and oligonucleotides.

**69.** The method or the use according to any one of claims 65 to 64, wherein the tumor is selected from any one or more of: lymphoma, multiple myeloma, breast cancer, ovarian cancer, kidney cancer, endometrial cancer, melanoma, pancreatic cancer, lung cancer, stomach cancer, liver cancer, mesothelioma, esophageal cancer, head and neck cancer, bile duct cancer, gallbladder cancer, bladder cancer, thymus cancer and colorectal cancer.

**70.** A method for diagnosing a specific disease, comprising using the protein-drug conjugate according to any one of claims 1 to 34 and/or the pharmaceutical composition according to claim 64.

**71.** A method for detecting a specific target, comprising using the protein-drug conjugate according to any one of claims 1 to 34 and/or the pharmaceutical composition according to claim 64, wherein preferably the detection is for non-disease diagnostic purposes.

**72.** A detection kit, comprising the protein-drug conjugate according to any one of claims 1 to 34 and/or the pharmaceutical composition according to claim 64, and optionally instructions for use.

**73.** An administration device, comprising the protein-drug conjugate according to any one of claims 1 to 34 and/or the pharmaceutical composition according to claim 64, and a device for administering the protein-drug conjugate and/or the pharmaceutical composition.

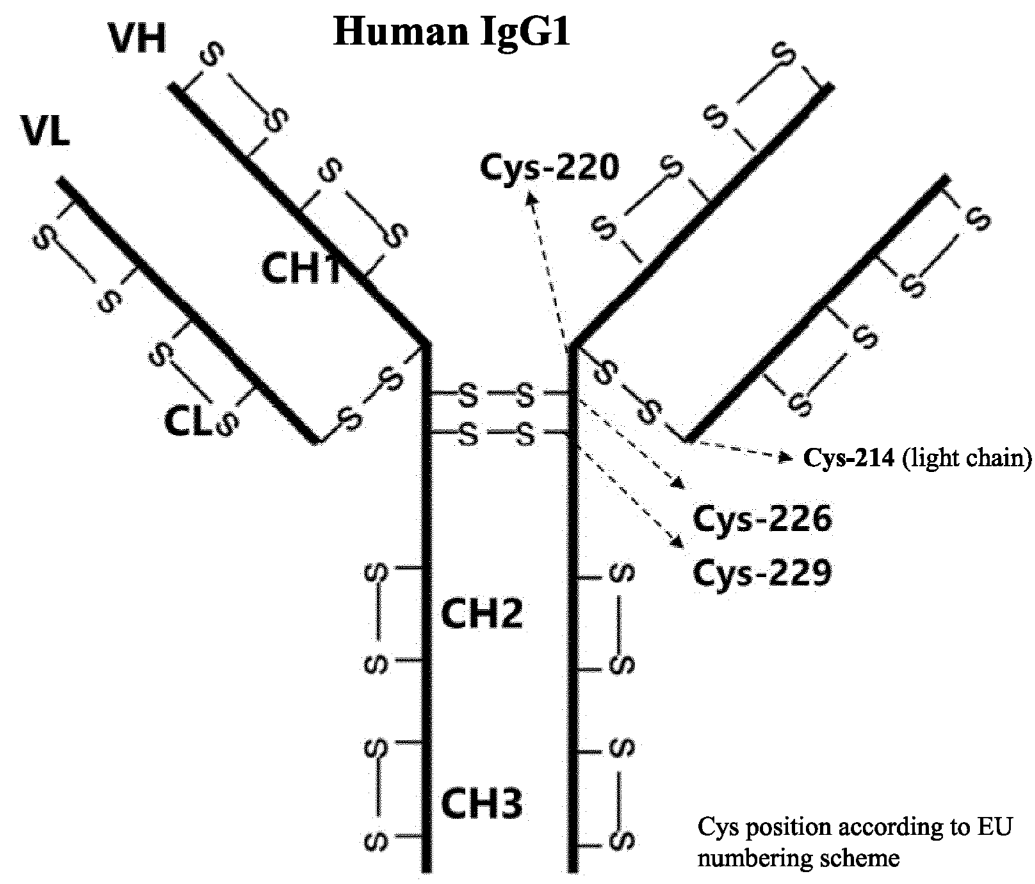
Human IgG1
VH
VL
CH1
CL
Cys-220
Cys-214 (light chain)
Cys-226
Cys-229
CH2
CH3
Cys position according to EU numbering scheme

FIG. 1

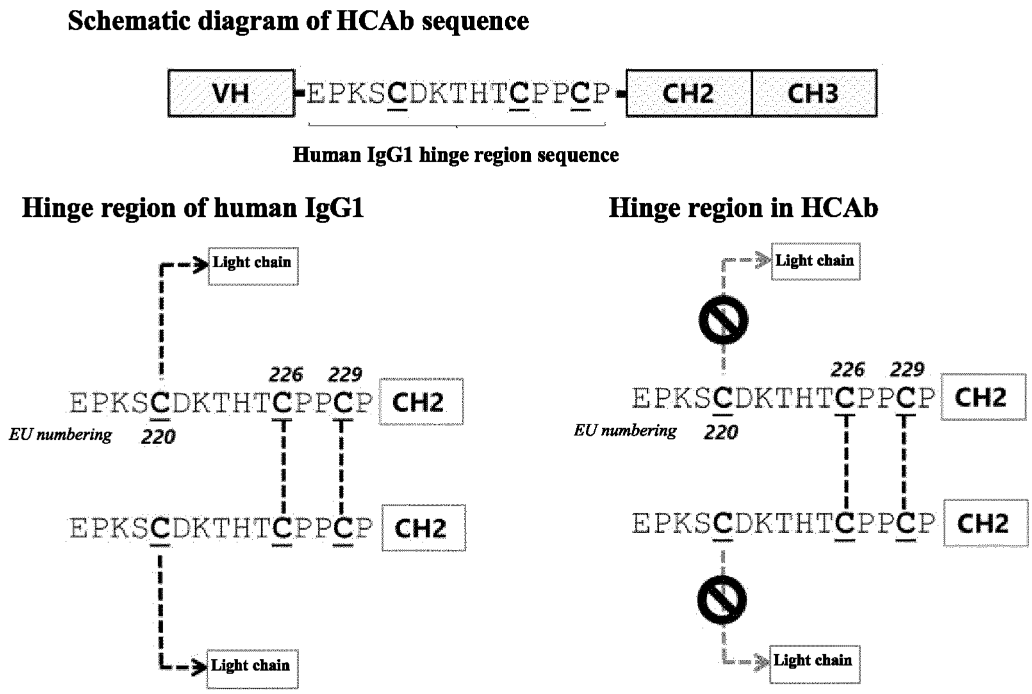
Schematic diagram of HCAb sequence
VH
EPKSCDKTHTCPPCP
CH2
CH3
Human IgG1 hinge region sequence
Hinge region of human IgG1
Hinge region in HCAb
Light chain
226 229
EPKSCDKTHTCPPCP
CH2
EU numbering 220
Light chain

FIG. 2

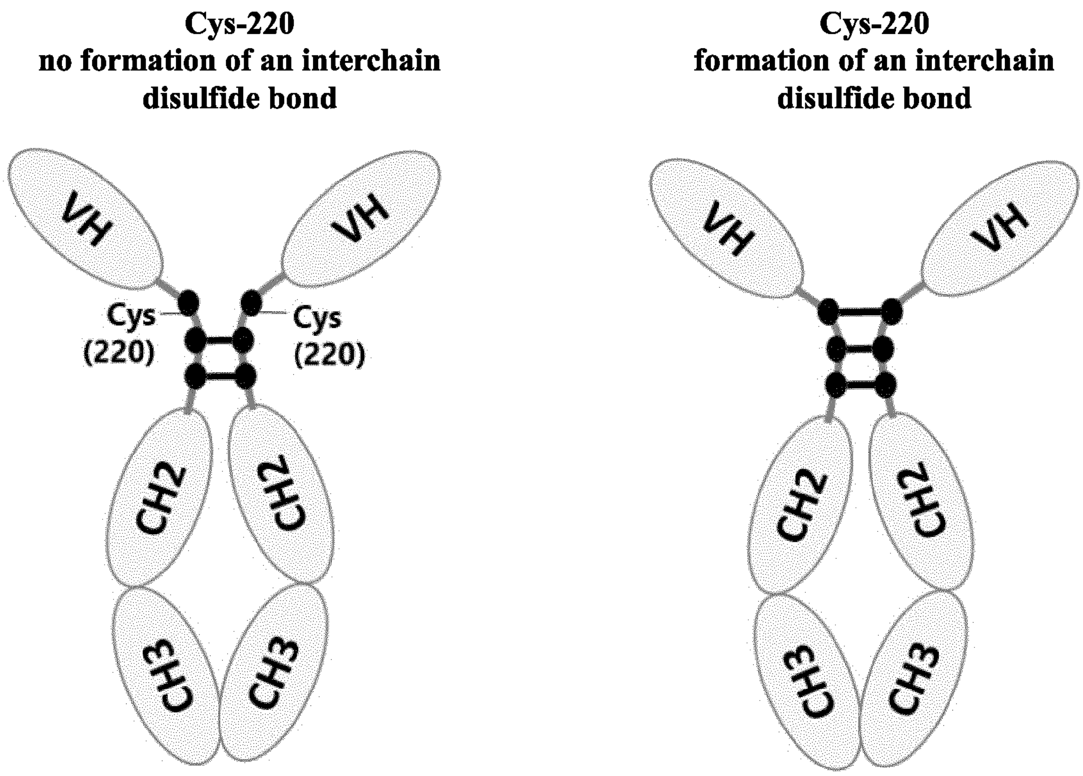
Cys-220
no formation of an interchain
disulfide bond
Cys-220
formation of an interchain
disulfide bond
VH
VH
Cys
(220)
Cys
(220)
CH2
CH2
CH3
CH3
VH
VH
CH2
CH2
CH3
CH3

FIG. 3

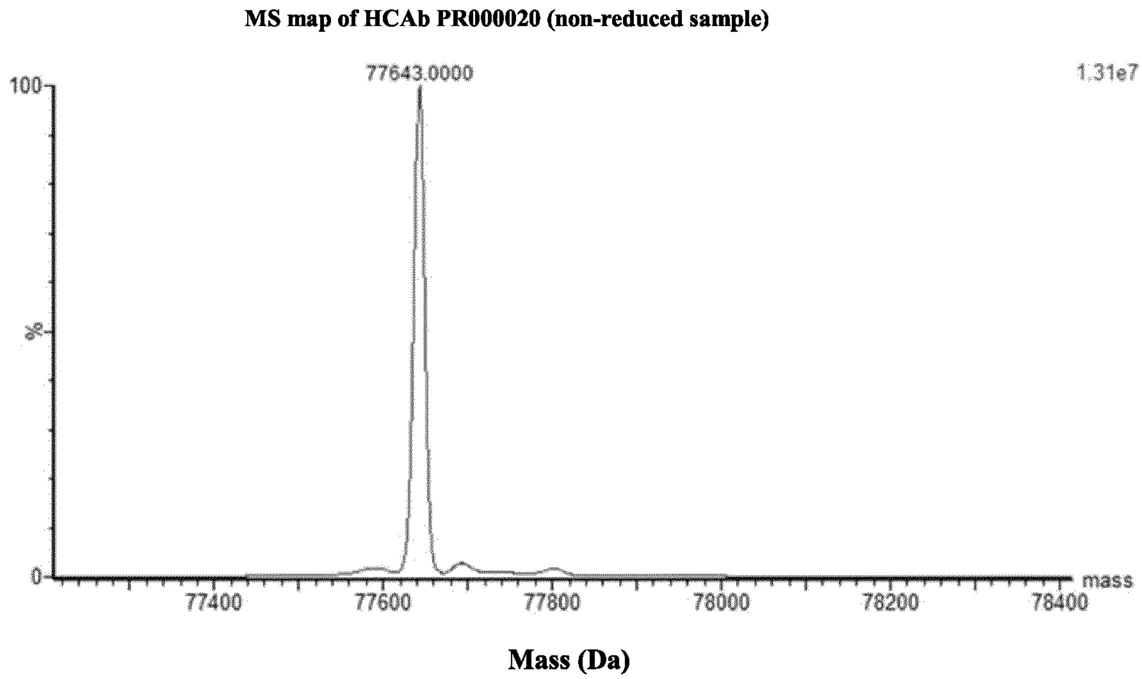
MS map of HCAb PR000020 (non-reduced sample)
77643.0000
1.31e7
100
%
0
77400
77600
77800
78000
78200
78400
mass
Mass (Da)

FIG. 4

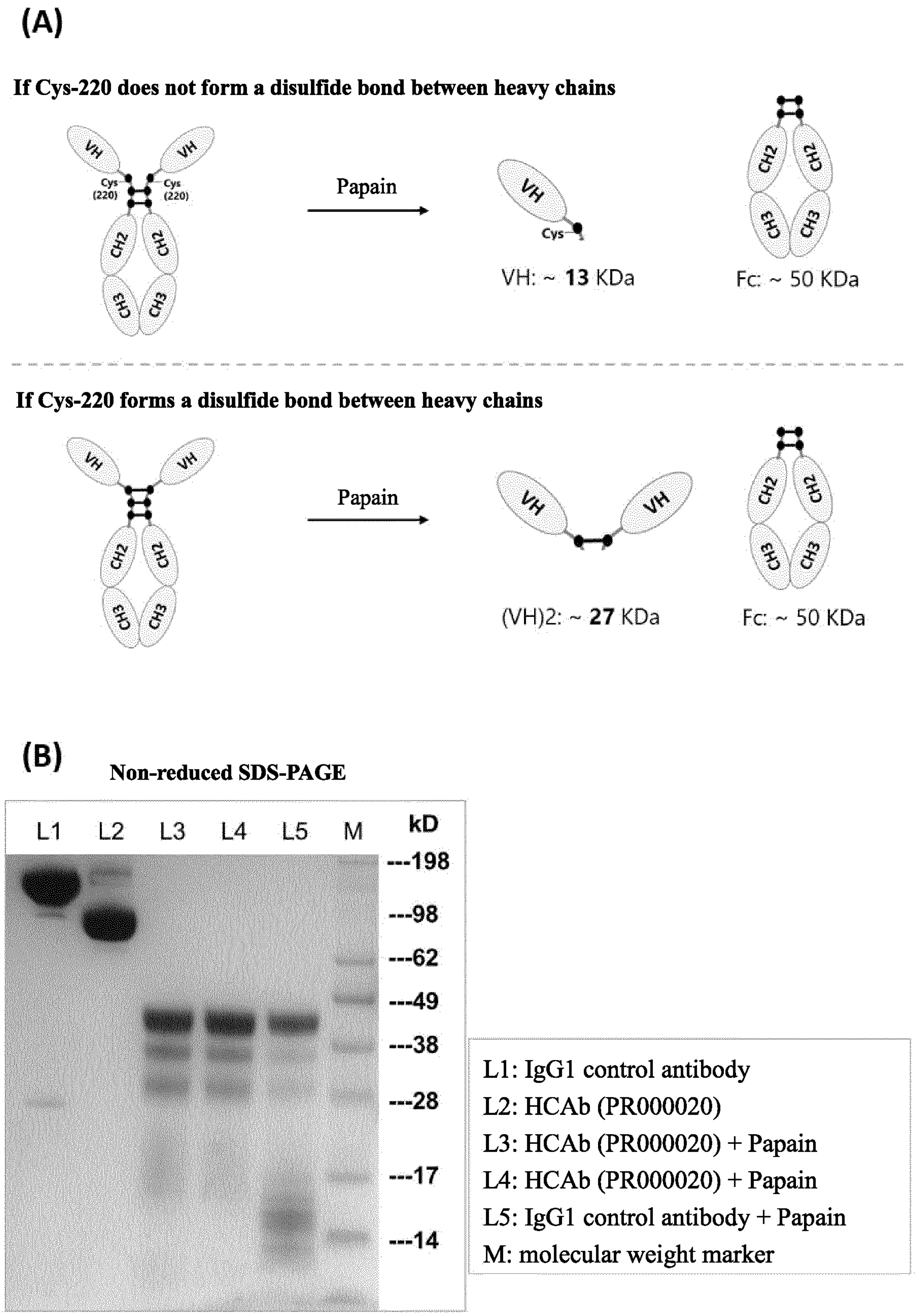
(A)
If Cys-220 does not form a disulfide bond between heavy chains
VH
VH
Cys (220)
Cys (220)
CH2
CH2
CH3
CH3
Papain
VH
Cys
VH: ~ 13 KDa
CH2
CH2
CH3
CH3
Fc: ~ 50 KDa
If Cys-220 forms a disulfide bond between heavy chains
VH
VH
CH2
CH2
CH3
CH3
Papain
VH
VH
(VH)2: ~ 27 KDa
CH2
CH2
CH3
CH3
Fc: ~ 50 KDa
(B)
Non-reduced SDS-PAGE
L1
L2
L3
L4
L5
M
kD
---198
---98
---62
---49
---38
---28
---17
---14
L1: IgG1 control antibody
L2: HCAb (PR000020)
L3: HCAb (PR000020) + Papain
L4: HCAb (PR000020) + Papain
L5: IgG1 control antibody + Papain
M: molecular weight marker

FIG. 5

(A)

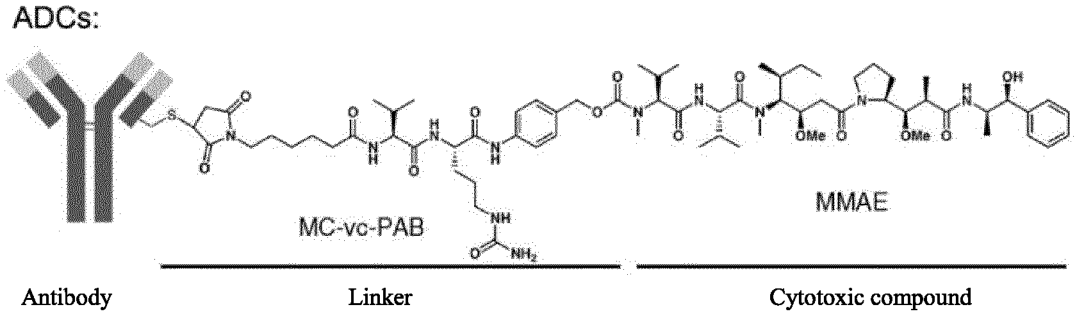
ADCs:
MC-vc-PAB
MMAE
Antibody
Linker
Cytotoxic compound

(B)

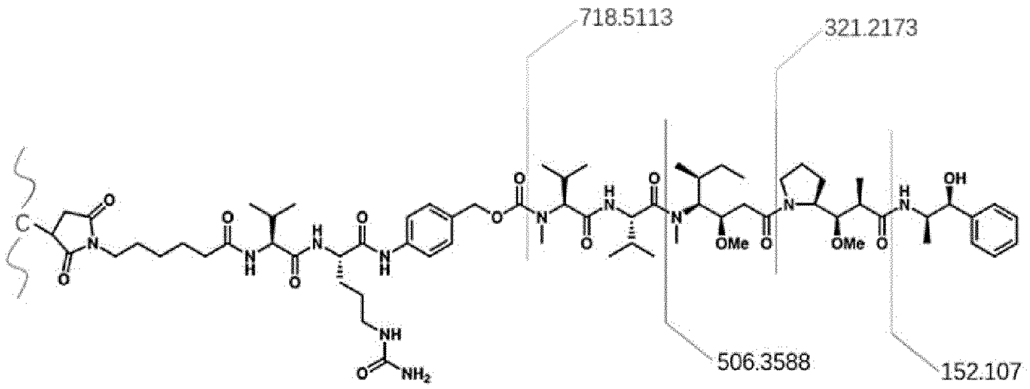
718.5113
321.2173
506.3588
152.107

FIG. 6

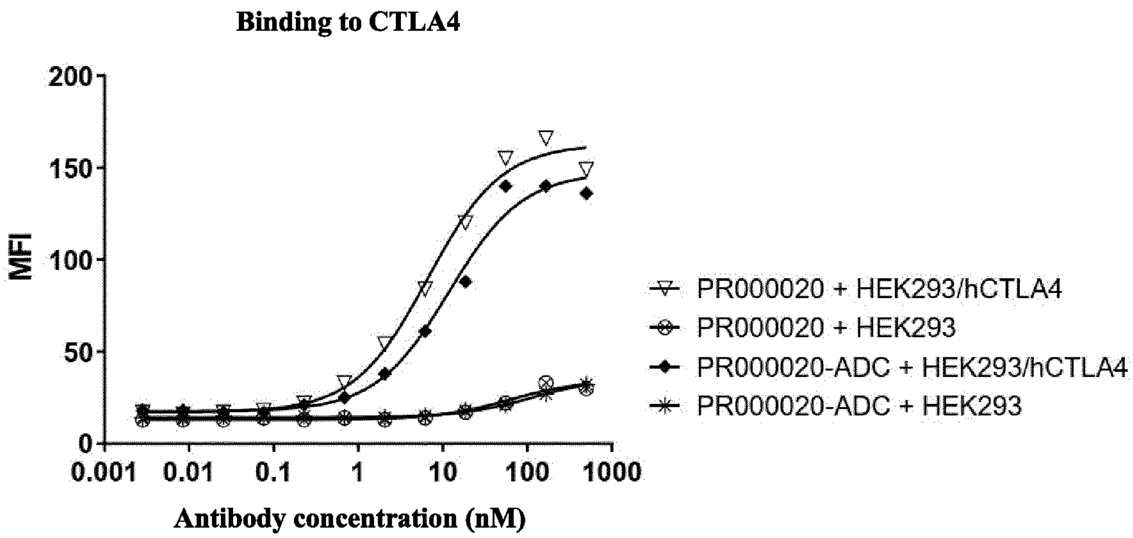
Binding to CTLA4
200
150
100
50
0
MFI
0.001
0.01
0.1
1
10
100
1000
Antibody concentration (nM)
PR000020 + HEK293/hCTLA4
PR000020 + HEK293
PR000020-ADC + HEK293/hCTLA4
PR000020-ADC + HEK293

FIG. 7

(A)

HCAb PR000020 (lot.# HSP302), HIC-HPLC

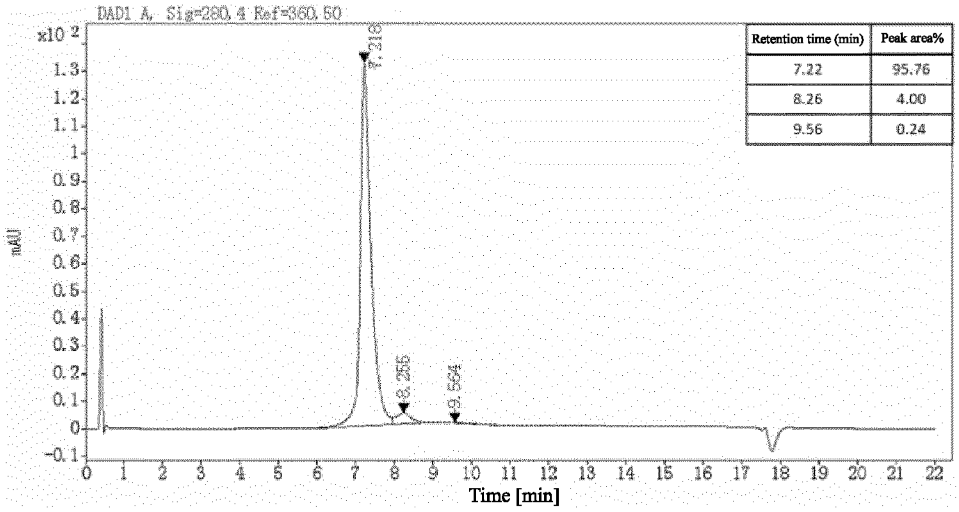


(B)

(Lot.# HSP302-170814) before purification of PR000020-ADC, HIC-HPLC

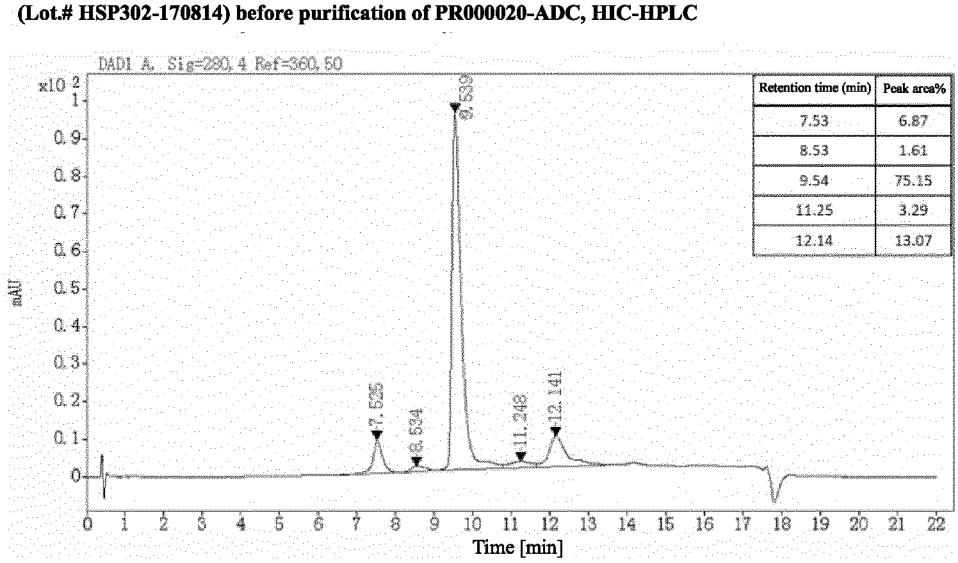


(C)

(Lot.# HSP302-170814PA0815) after purification of PR000020-ADC, HIC-HPLC

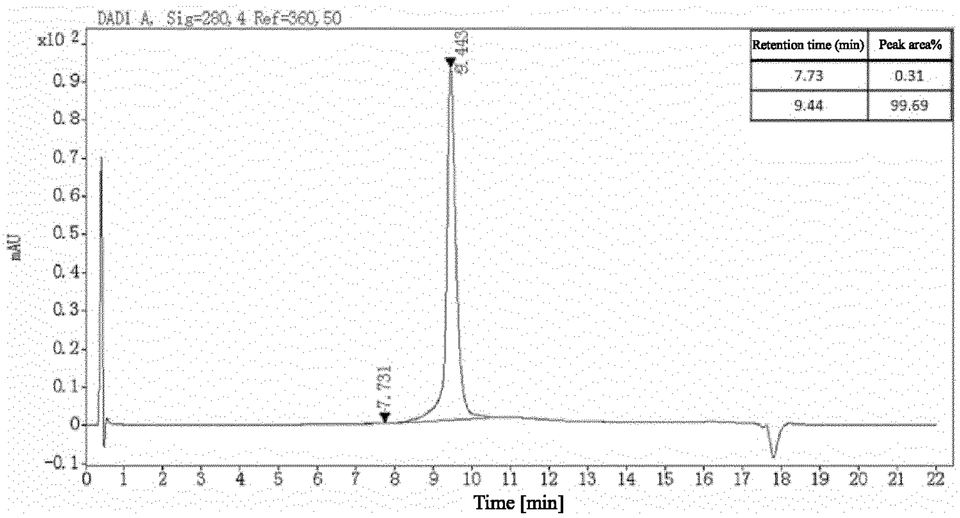


FIG. 8

(A)

HCAb PR000020 (lot.# HSP302), RP-HPLC

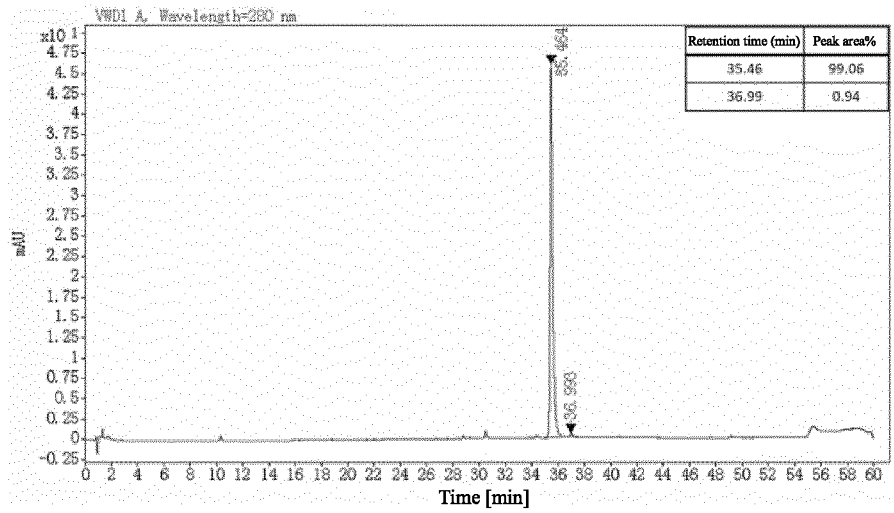

| Retention time (min) | Peak area% |
|---|---|
| 35.46 | 99.06 |
| 36.99 | 0.94 |

(B)

(Lot.# HSP302-170814) before purification of PR000020-ADC, RP-HPLC

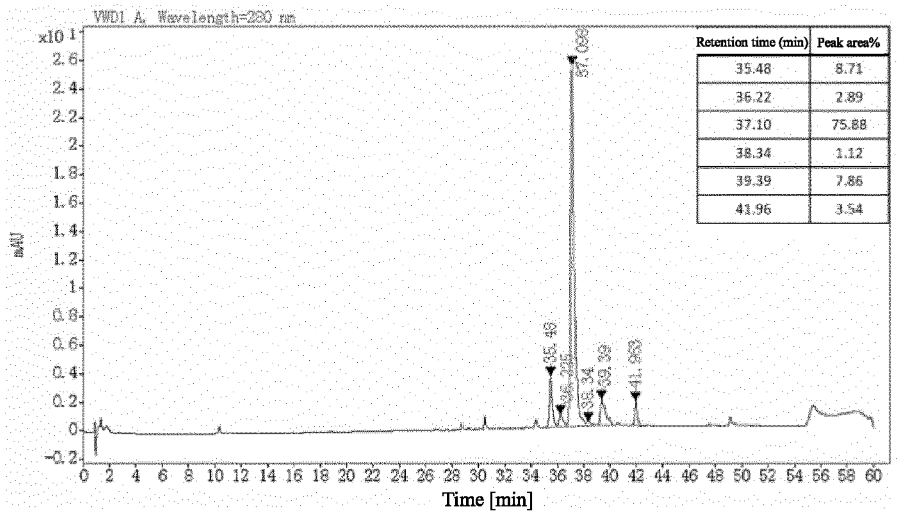

| Retention time (min) | Peak area% |
|---|---|
| 35.48 | 8.71 |
| 36.22 | 2.89 |
| 37.10 | 75.88 |
| 38.34 | 1.12 |
| 39.39 | 7.86 |
| 41.96 | 3.54 |

(C)

(Lot.# HSP302-170814PA0815) after purification of PR000020-ADC, RP-HPLC

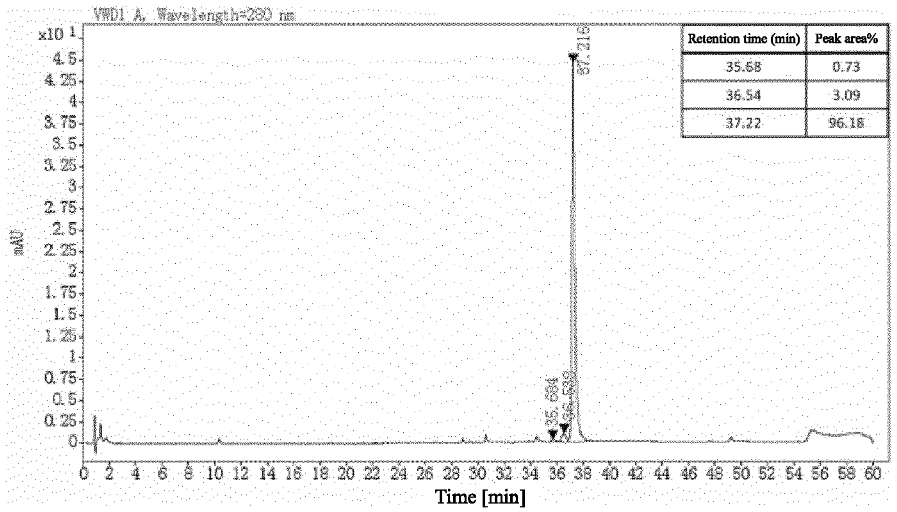

| Retention time (min) | Peak area% |
|---|---|
| 35.68 | 0.73 |
| 36.54 | 3.09 |
| 37.22 | 96.18 |

FIG. 9

(A)

**MS map of PR000020-ADC (non-reduced sample)**

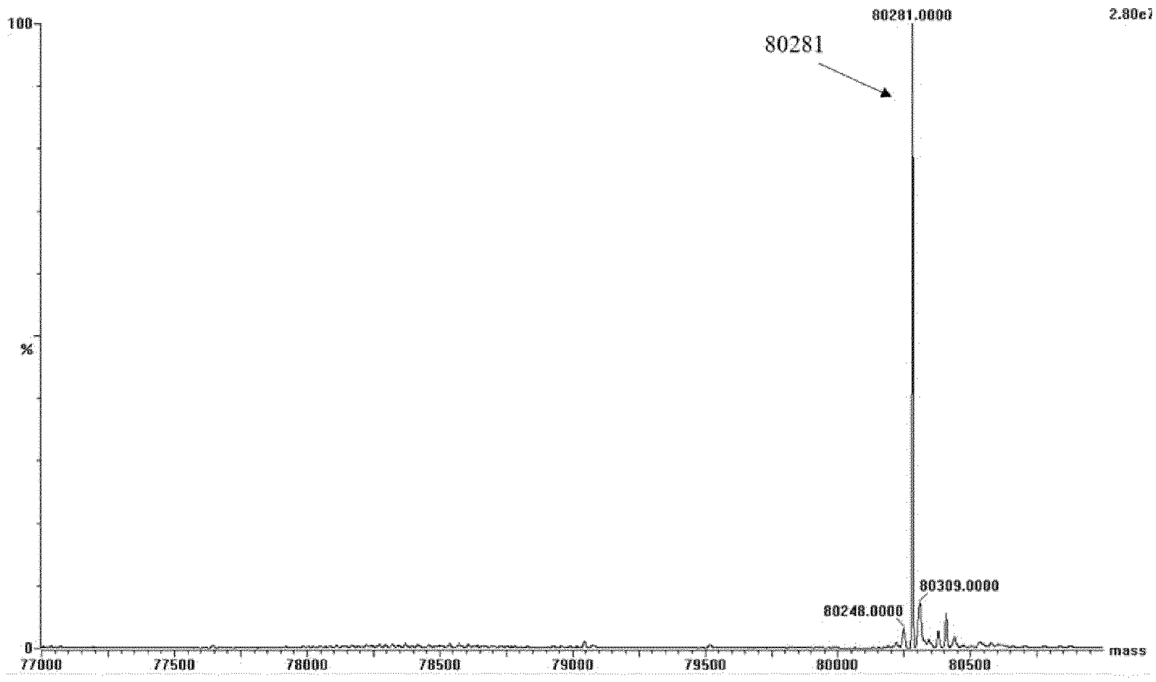


**Mass (Da)**

(B)

**MS map of PR000020-ADC (reduced sample)**

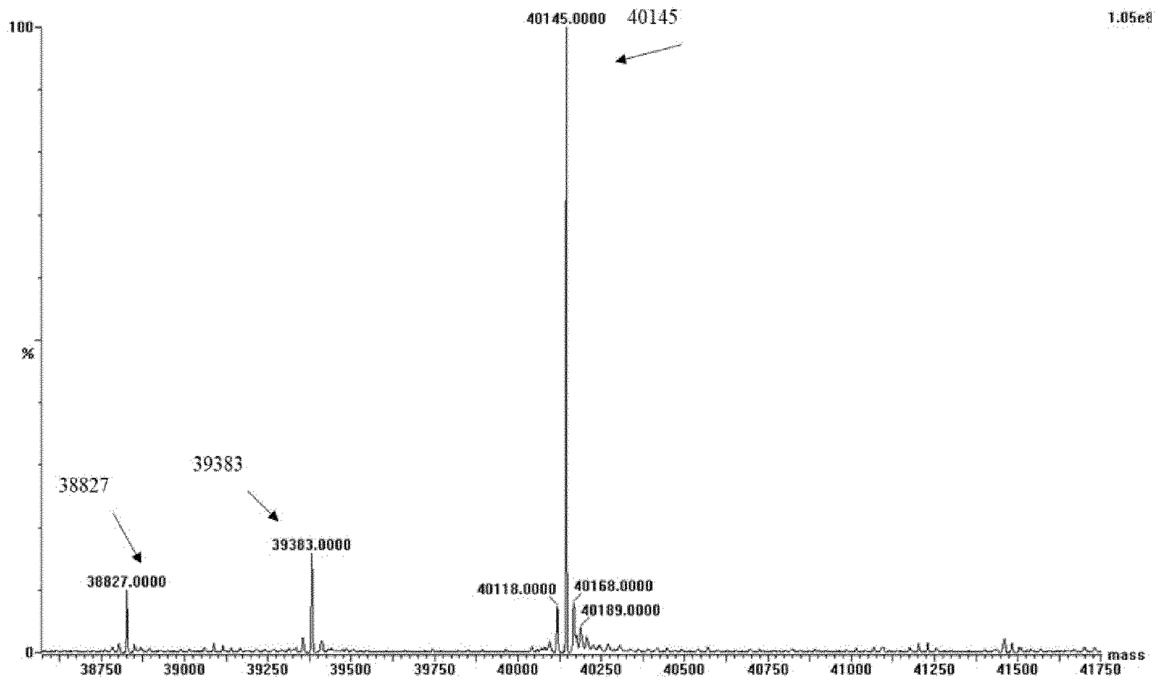


**Mass (Da)**

**FIG. 10**

**(A)**

SEVQLVETGGGLIQPGGSLRLSCAASGLNVSRHYLSWVRQAPGKGLEWVAVIYS
GGGIEYVDSVKGRFTISRDNANNTLYLQMRSLRTEDSAVYYCARAIPRPHGSDI
WGQGTMVTVSSEPKSCDKTHTCPPCPAPELLGGPSVFLFPPKPKDTLMISRTPE
VTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQG
NVFSCSVMHEALHNHYTQKSLSLSPGK

**(B)**

**Polypeptide fragments containing Cys-IAM in PR000020-ADC sample**

| Peptide | -10lgP | Mass | ppm | m/z | z | RT | AScore |
|---|---|---|---|---|---|---|---|
| R.LSC(+57.02)AASGLN(+.98)VSRHYLSWVR.Q | 44.5 | 2176.08 | 1.3 | 545.0292 | 4 | 54.89 | C3:Carbamidomethylation:1000.00;N9:Deamidation (NQ):1000.00 |
| R.SLRTEDSAVYYC(+57.02)AR.A | 62.98 | 1689.78 | 0.9 | 845.8971 | 2 | 32.27 | C12:Carbamidomethylation:1000.00 |
| K.THTC(+57.02)PPC(+57.02)PAPELLGGPSVFLFPPKPKD.T | 77.06 | 2958.48 | 1.3 | 987.1676 | 3 | 67.82 | C4:Carbamidomethylation:1000.00;C7:Carbamidomethylation:1000.00 |
| T.C(+57.02)VVVDVSHEDPEVK.F | 67.15 | 1610.76 | -0.5 | 806.3874 | 2 | 34.74 | C1:Carbamidomethylation:1000.00 |
| R.VVSVLTVLHQDWLN(+.98)GKEYKC(+57.02)K.V | 23.85 | 2516.31 | 1.3 | 504.2699 | 5 | 60.98 | N14:Deamidation (NQ):11.12;C20:Carbamidomethylation:1000.00 |
| R.EEMTKNQVSLTC(+57.02)LVK.G | 67.92 | 1778.89 | -1 | 890.4517 | 2 | 44.65 | C12:Carbamidomethylation:1000.00 |
| R.WQQGNVFSC(+57.02)SVM(+15.99)HEALHNHYTQK.S | 75.57 | 2816.25 | 1.8 | 564.2592 | 5 | 42.96 | C9:Carbamidomethylation:1000.00;M12:Oxidation (M):1000.00 |

**(C)**

**Polypeptide fragments containing Cys-IAM in PR000020 sample**

| Peptide | -10lgP | Mass | ppm | m/z | z | RT | AScore |
|---|---|---|---|---|---|---|---|
| R.LSC(+57.02)AASGLN(+.98)VSR.H | 62.36 | 1234.598 | 2 | 618.3073 | 2 | 31.34 | C3:Carbamidomethylation:1000.00;N9:Deamidation (NQ):1000.00 |
| R.TEDSAVYYC(+57.02)AR.A | 61.23 | 1333.561 | 0.3 | 667.7879 | 2 | 28.85 | C9:Carbamidomethylation:1000.00 |
| K.SC(+57.02)DKTHTC(+57.02)PPC(+57.02)PAPELLGGPSVFLFPPK.P | 51.68 | 3108.487 | 1.2 | 778.13 | 4 | 65.97 | C2:Carbamidomethylation:1000.00;C8:Carbamidomethylation:1000.00;C11:Carbamidome |
| K.THTC(+57.02)PPC(+57.02)PAPELLGGPSVF.L | 68.56 | 2035.95 | 1.5 | 1018.984 | 2 | 60.96 | C4:Carbamidomethylation:1000.00;C7:Carbamidomethylation:1000.00 |
| R.TPEVTC(+57.02)VVVDVSHED.P | 71.26 | 1684.761 | 1.4 | 843.3891 | 2 | 49.5 | C6:Carbamidomethylation:1000.00 |
| K.NQVSLTC(+57.02)LVK.G | 53.29 | 1160.622 | 0.7 | 581.3188 | 2 | 46.87 | C7:Carbamidomethylation:1000.00 |
| R.WQQGNVFSC(+57.02)SVMHEALHNHYTQK.S | 82.25 | 2800.26 | 1.5 | 1401.139 | 2 | 48.19 | C9:Carbamidomethylation:1000.00 |

Definitions of column names in the table above:

- Peptide: polypeptide fragment amino acid sequence
- -101gP: -101gP fraction of this polypeptide
- Mass: mass of single isotope
- ppm: molecular weight error
- m/z: mass-to-charge ratio
- z: charge number
- RT: retention time
- PTM: modification
- Ascore: fraction of modification (PTM) of this polypeptide

**FIG. 11**

HCAb PR000759 (lot.# HSP307A PA1222 F3-4), SEC-HPLC

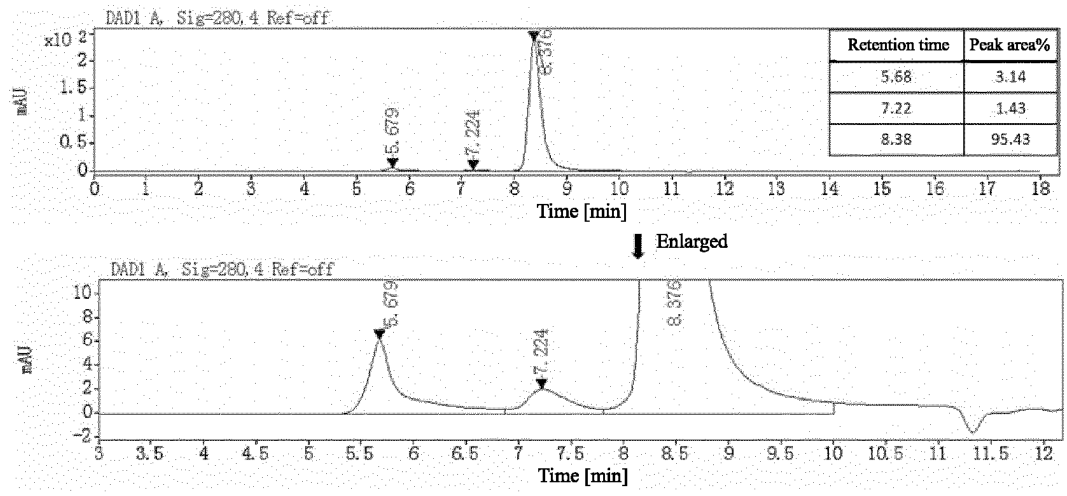


**FIG. 12**

**(Lot.# HSP307A-181229 MIX) after purification of PR000759-ADC, HIC-HPLC**

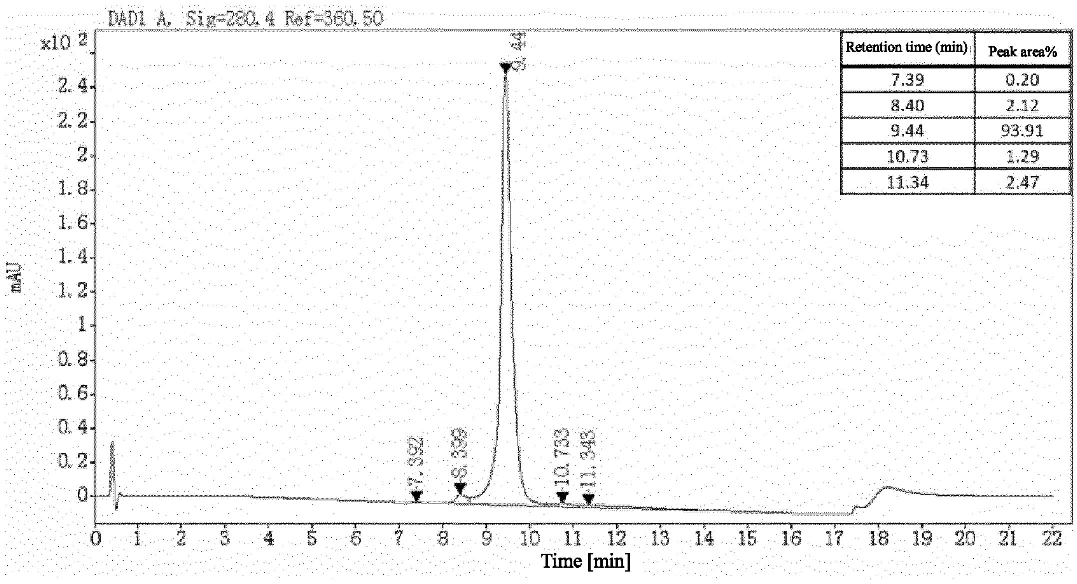


**FIG. 13**

EVQLVESGGGVVQPGRSLRLSCAASGFTFSSFAFHWVRQAPGKGLEGVAVIWYDGRNENYADSVKGRFTISRDNSK
NMLYLQMNSLRAEDTAVYYCARGRGVLGDTRAFDIWGQGTMVTVSSEPKS**C**DKTHT**C**PP**C**PAPELLGGPSVFLFPP
KPKDTLMISRTPEVT**C**VVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK

| Peptide | -10lgP | Mass | ppm | m/z | RT | PTM |
|---|---|---|---|---|---|---|
| SC(+1315.78)DKTHTC(+57.02)PPC(+57.02)PAPELLGGPSVFLFPPKPK | 41.21 | 4592.3926 | -1 | 1149.1042 | 61.71 | ADC Drug 1316; Carbamidomethylation |
| THTC(+57.02)PPC(+1315.78)PAPELLGGPSVFLFPPKPK | 51.27 | 4102.208 | -3.2 | 1026.556 | 61.7 | Carbamidomethylation; ADC Drug 1316 |
| TPEVTC(+1315.78)VVVDVSHEDPEVK | 50.84 | 3396.7778 | -0.3 | 1133.2662 | 61.74 | ADC Drug 1316 |

**FIG. 14**

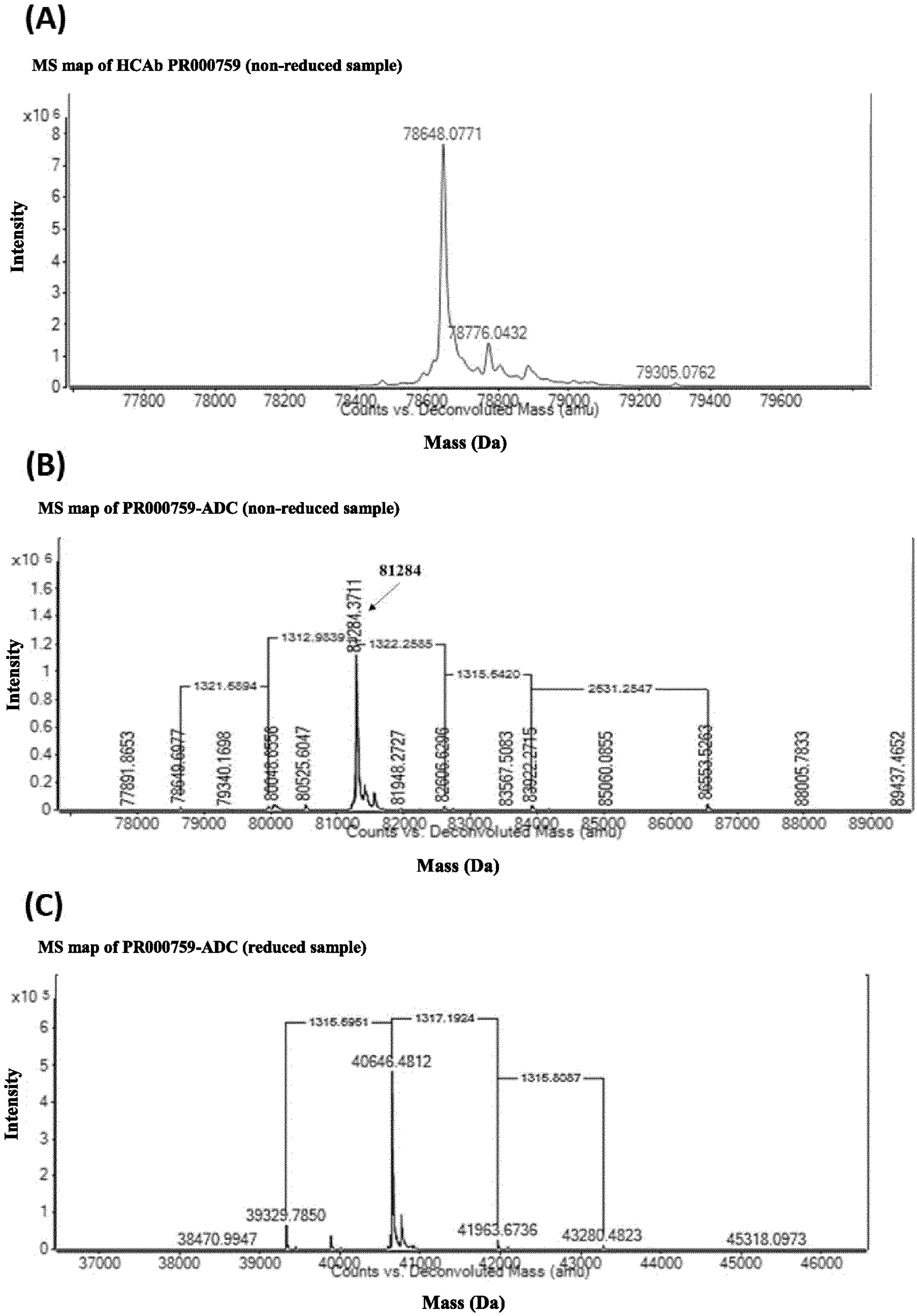
(A)
MS map of HCAb PR000759 (non-reduced sample)
Intensity
x10 6
78648.0771
78776.0432
79305.0762
Counts vs. Deconvoluted Mass (amu)
Mass (Da)
(B)
MS map of PR000759-ADC (non-reduced sample)
Intensity
x10 6
81284
81284.3711
1321.5894
1312.9539
1322.2585
1315.5420
2531.2547
77891.8653
78649.6077
79340.1698
80048.6556
80525.6047
81948.2727
82606.6296
83567.5083
83922.2715
85060.0855
86553.5263
88005.7833
89437.4652
Counts vs. Deconvoluted Mass (amu)
Mass (Da)
(C)
MS map of PR000759-ADC (reduced sample)
Intensity
x10 5
1315.6951
1317.1924
1315.8087
40646.4812
39329.7850
38470.9947
41963.6736
43280.4823
45318.0973
Counts vs. Deconvoluted Mass (amu)
Mass (Da)

FIG. 15

HCAb PR001046 (lot.# HSP307B PA1222 F11-12), SEC-HPLC

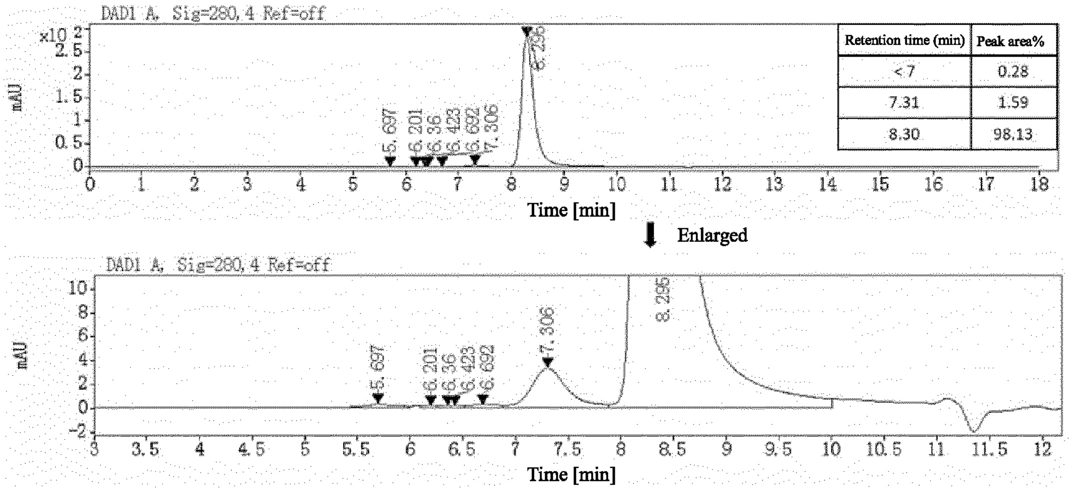


**FIG. 16**

**(Lot.# HSP307B-181229 MIX) after purification of PR001046-ADC,**

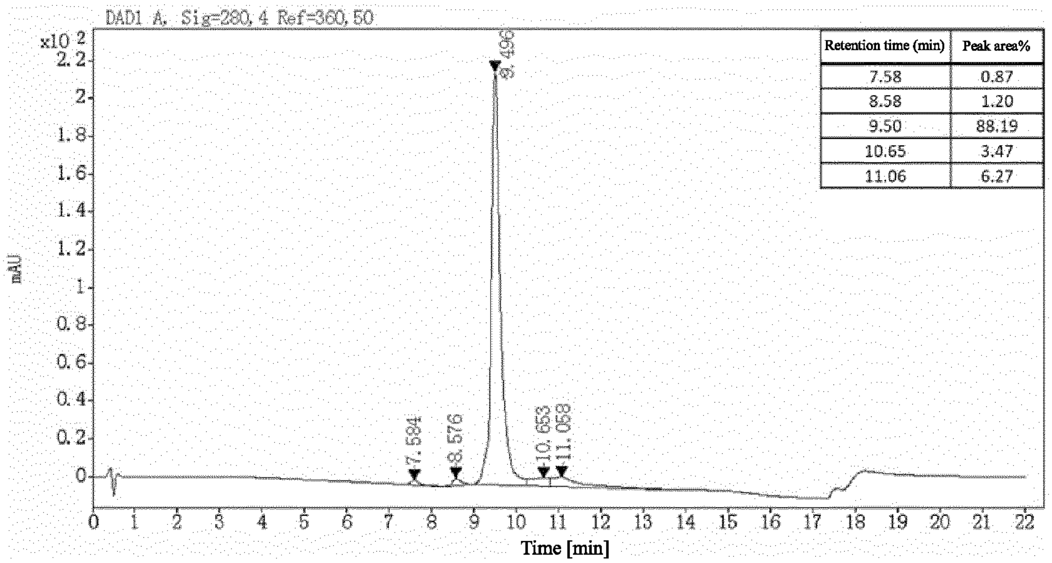


**FIG. 17**

EVQLVETGGGLIQPGGSLRLSCAASGFTVSDNYMTWVRQAPGKGLEWVSVIFSGGNTYYADSVKGRFTISRDNAKN
TLYLQMNSLRAEDTALYYCARRNYDDTRGTDVFDIWGQGTMVTVSSEPKS**C**DKTHT**C**PP**C**PAPELLGGPSVFLFPP
KPKDTLMISRTPEVT**C**VVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK

| Peptide | -10lgP | Mass | ppm | m/z | RT | PTM |
|---|---|---|---|---|---|---|
| TPEVTC(+1315.78)VVVDVSHEDPEVK | 40.62 | 3396.7778 | -3.5 | 850.1988 | 61.75 | ADC Drug 1316 |
| SC(+1315.78)DKTHTC(+57.02)PPC(+57.02)PAPELLGGPSVFLFPPKPK | 36.64 | 4592.3926 | 7.7 | 1149.1143 | 61.71 | ADC Drug 1316; Carbamidomethylation |
| THTC(+57.02)PPC(+1315.78)PAPELLGGPSVFLFPPKPK | 48.56 | 4102.208 | 2.4 | 1026.5618 | 61.7 | Carbamidomethylation; ADC Drug 1316 |

**FIG. 18**

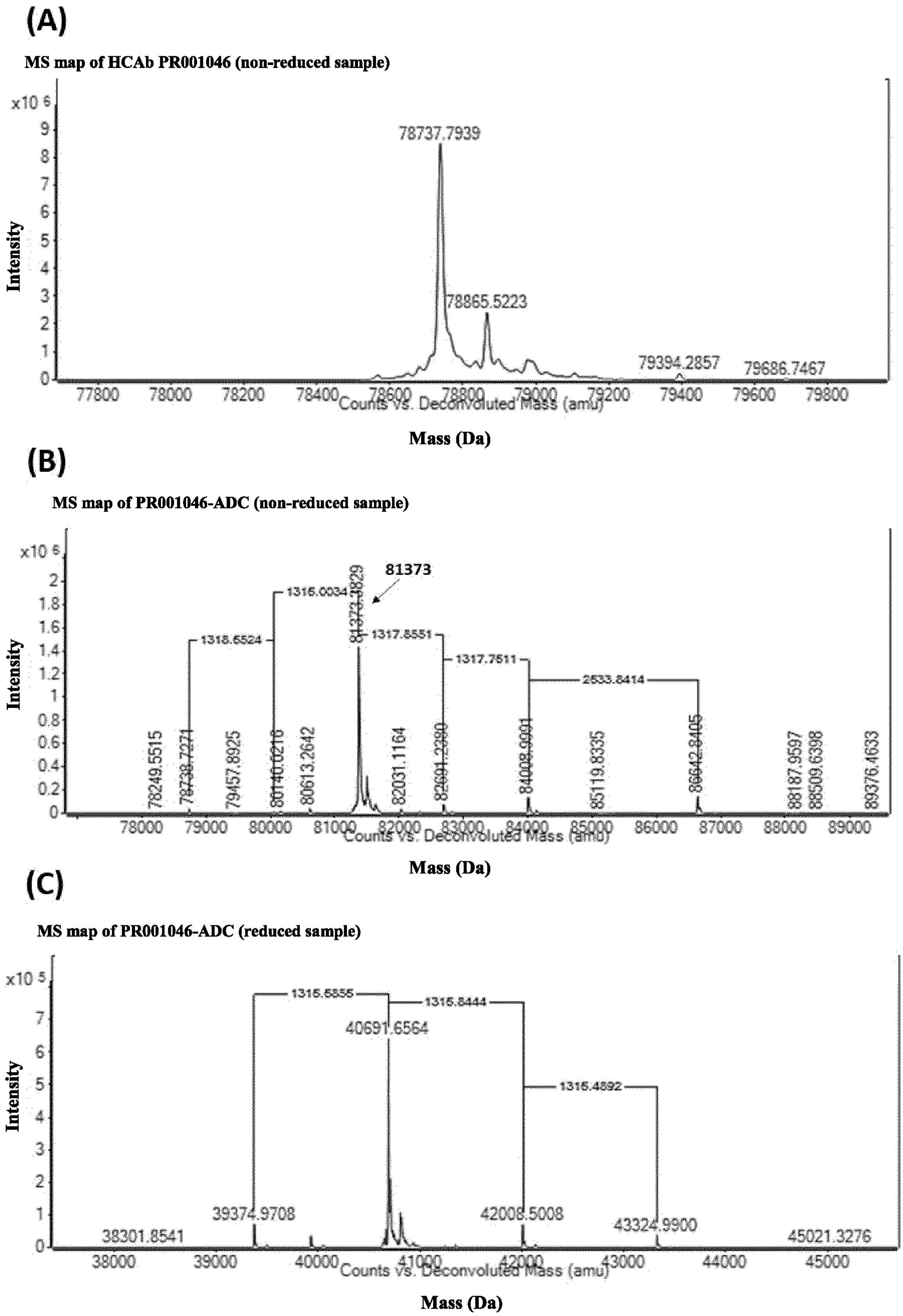
(A)
MS map of HCAb PR001046 (non-reduced sample)
x10 6
Intensity
78737.7939
78865.5223
79394.2857
79686.7467
Counts vs. Deconvoluted Mass (amu)
Mass (Da)
(B)
MS map of PR001046-ADC (non-reduced sample)
x10 6
Intensity
81373
81373.3829
1316.0034
1318.6524
1317.8551
1317.7511
2533.8414
78249.5515
78738.7271
79457.8925
80140.0216
80613.2642
82031.1164
82691.2380
84008.9901
85119.8335
86642.8405
88187.9597
88509.6398
89376.4633
Counts vs. Deconvoluted Mass (amu)
Mass (Da)
(C)
MS map of PR001046-ADC (reduced sample)
x10 5
Intensity
1316.6855
1315.8444
1316.4892
40691.6564
38301.8541
39374.9708
42008.5008
43324.9900
45021.3276
Counts vs. Deconvoluted Mass (amu)
Mass (Da)

FIG. 19

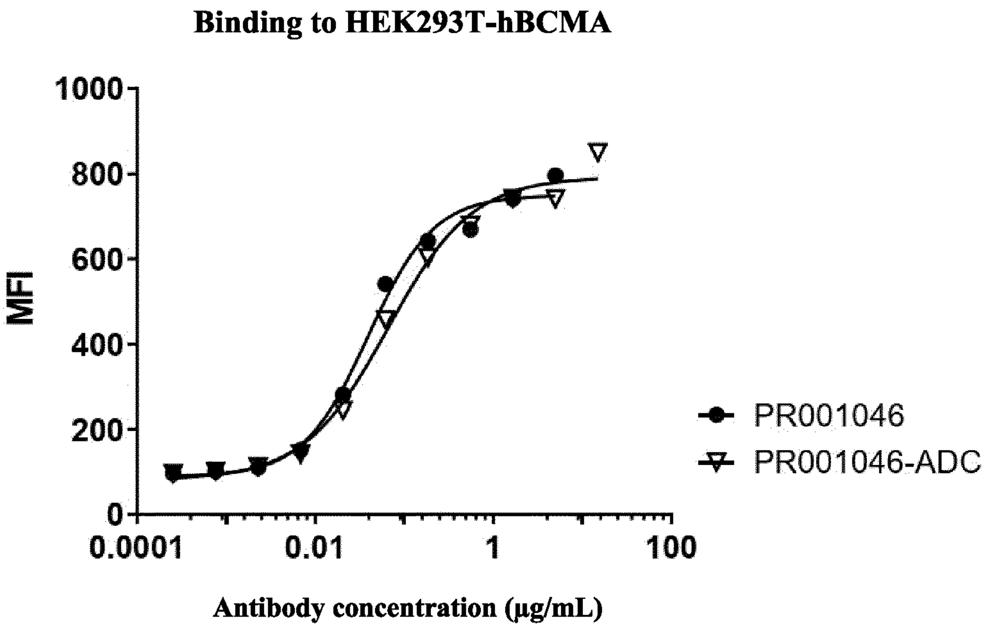
Binding to HEK293T-hBCMA
1000
800
600
400
200
0
MFI
0.0001
0.01
1
100
Antibody concentration (μg/mL)
PR001046
PR001046-ADC

FIG. 20

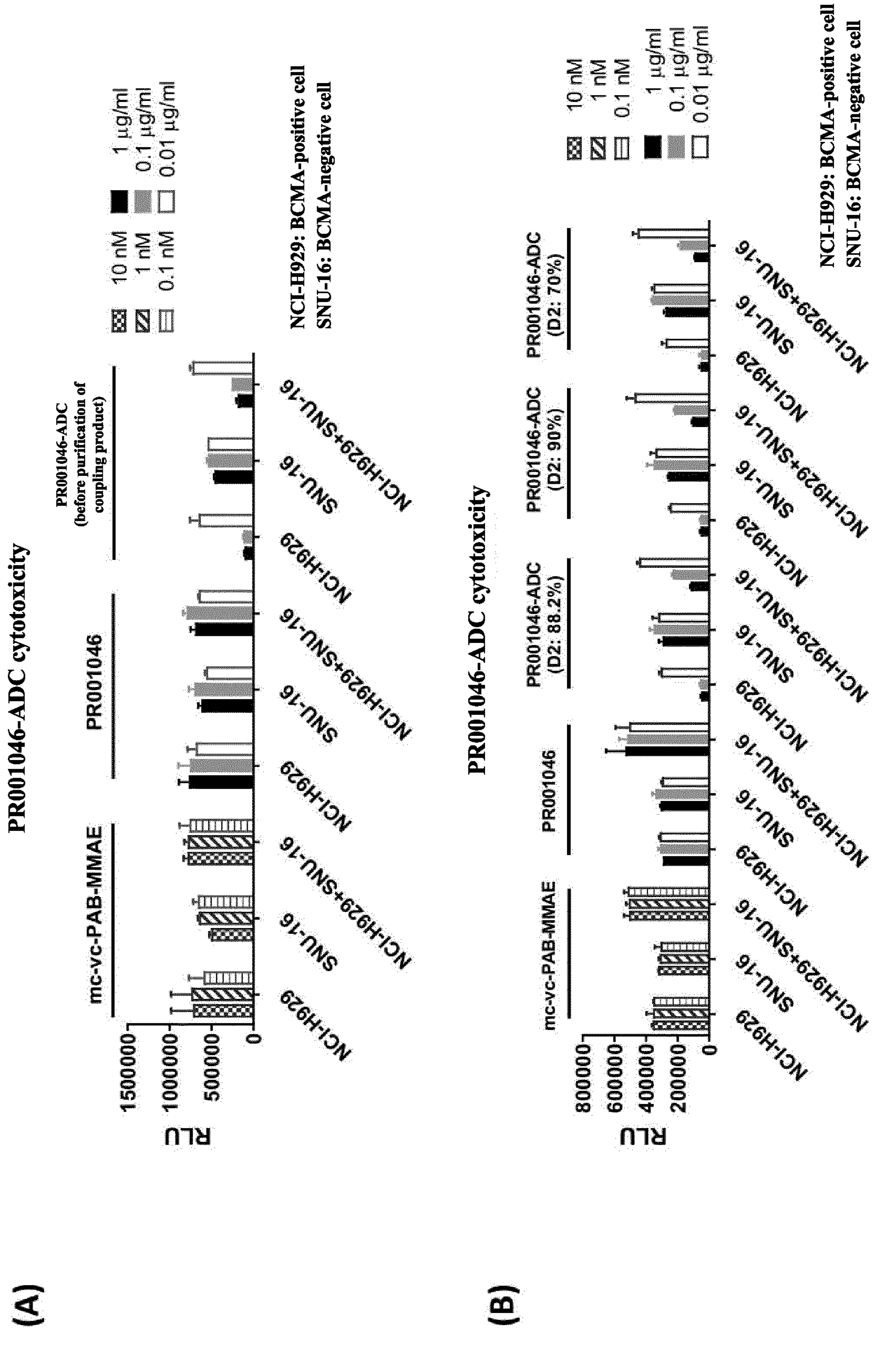
(A)
PR001046-ADC cytotoxicity
RLU
1500000
1000000
500000
0
mc-vc-PAB-MMAE
PR001046
PR001046-ADC
(before purification of
coupling product)
NCI-H929
SNU-16
NCI-H929+SNU-16
10 nM
1 nM
0.1 nM
1 µg/ml
0.1 µg/ml
0.01 µg/ml
NCI-H929: BCMA-positive cell
SNU-16: BCMA-negative cell
(B)
PR001046-ADC cytotoxicity
RLU
800000
600000
400000
200000
0
mc-vc-PAB-MMAE
PR001046
PR001046-ADC
(D2: 88.2%)
PR001046-ADC
(D2: 90%)
PR001046-ADC
(D2: 70%)
NCI-H929
SNU-16
NCI-H929+SNU-16
10 nM
1 nM
0.1 nM
1 µg/ml
0.1 µg/ml
0.01 µg/ml
NCI-H929: BCMA-positive cell
SNU-16: BCMA-negative cell

FIG. 21

**HCAb PR004432 (lot.# HSP314-01 K20200427-0507PB0507), SEC-HPLC**

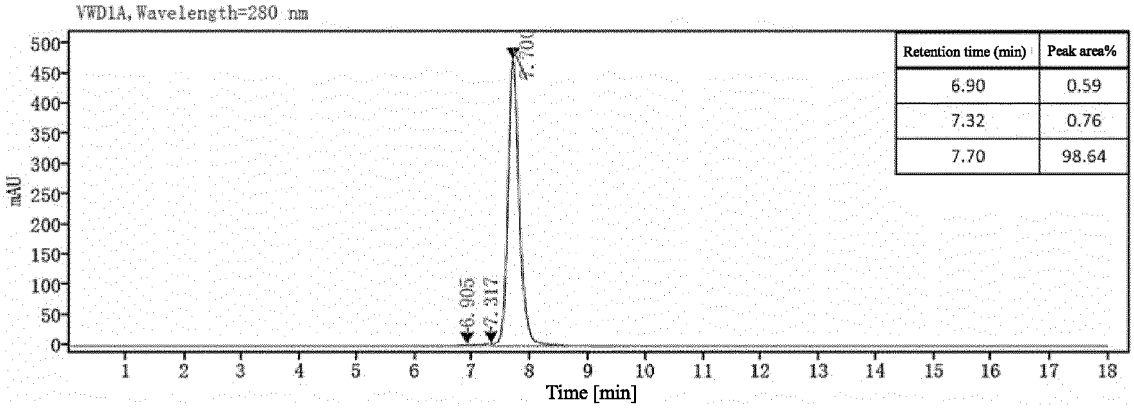


**FIG. 22**

**(Lot.# HSP31401-ADC-20200609) after purification of PR004432-ADC, RP-HPLC**

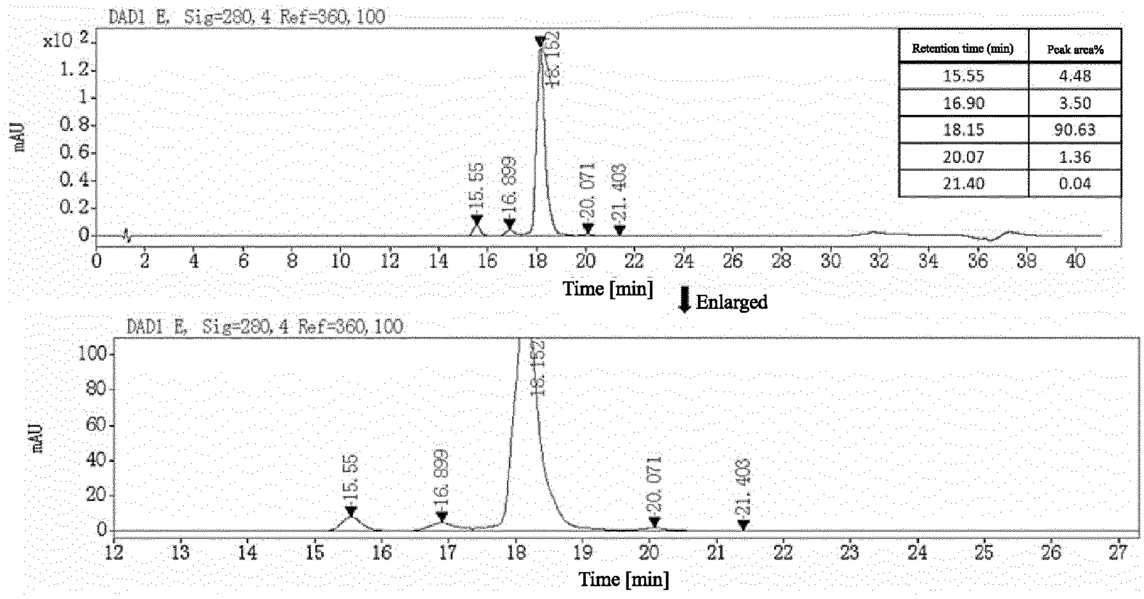


**FIG. 23**

EVQLQQSGPGLVKPSQTLSLTCAISGDSVSNNYAAWNWIRQSPSRGLEWLGRTYYRSKWYNDYAVS
VKSRITINPDTSKNQFSLQLNRVTPEDTAVYYCARDNYYGSGSPPVDALDIWGQGTMVTVSSEP
**97.12%** **1.49%**
KS**C**DKTHT**C**PP**C**PAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPGK

- SC(+1315.78)DK
- SC(+1315.78)DKTHTCPPCPAPELLGGPSVFLFPPK
- THTCPPCPAPELLGGPSVFLFPPK

| | Attribute Name | Value |
|---|---|---|
| 1 | MMAE "SCDK" | 97.12 % |
| 2 | MMAE "THTCPPCPAPELLGGPS... | 1.49 % |

**FIG. 24**

(A)

**MS map of PR004432-ADC (non-reduced sample)**

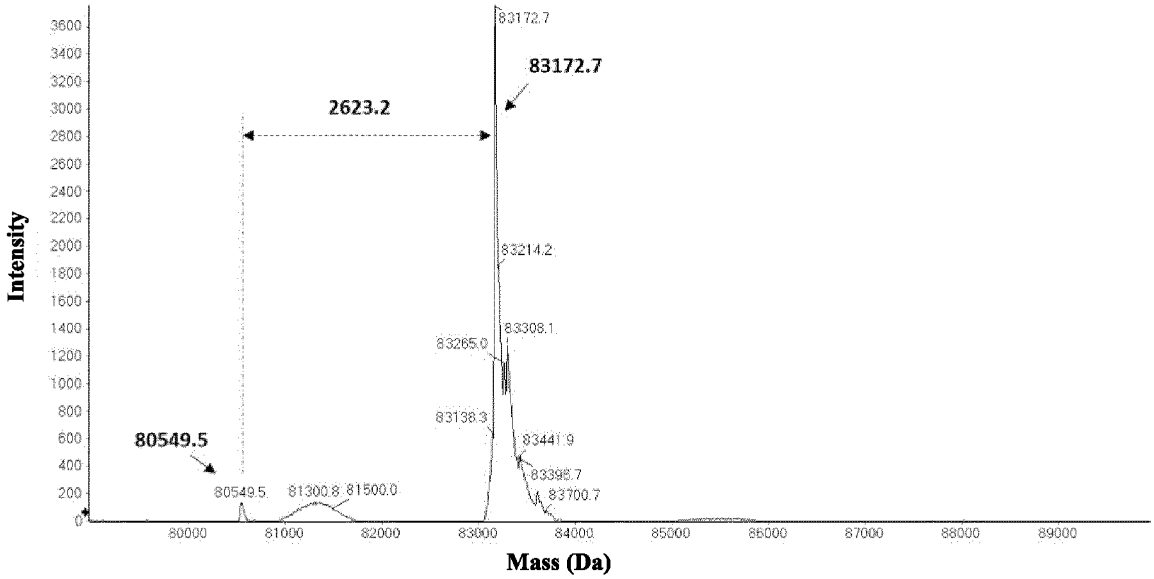


(B)

**MS map of PR004432-ADC (reduced sample)**

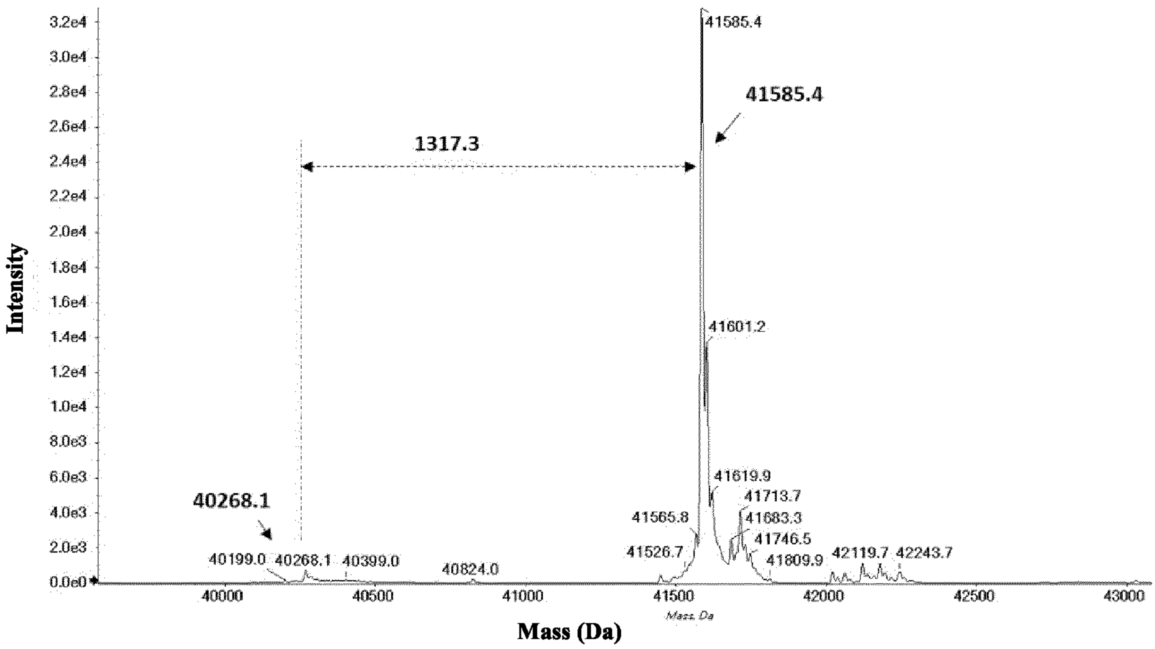


**FIG. 25**

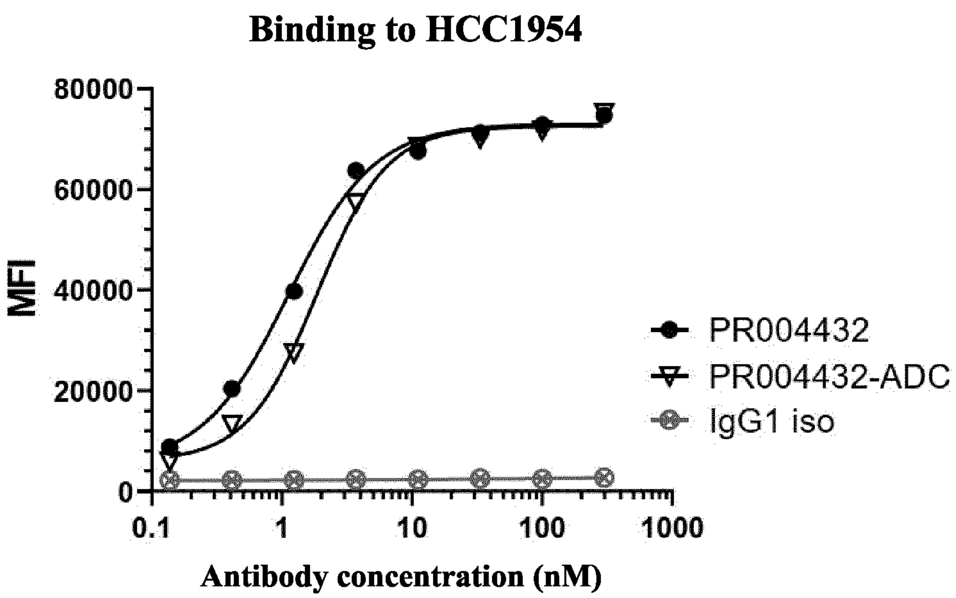
Binding to HCC1954
80000
60000
40000
20000
0
MFI
0.1
1
10
100
1000
Antibody concentration (nM)
PR004432
PR004432-ADC
IgG1 iso

FIG. 26

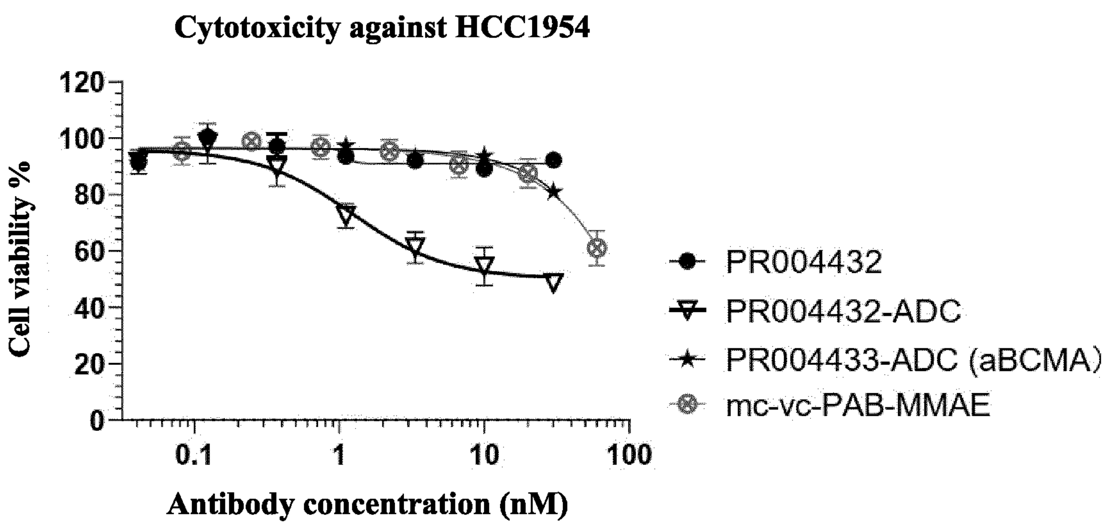
Cytotoxicity against HCC1954
120
100
80
60
40
20
0
Cell viability %
0.1
1
10
100
Antibody concentration (nM)
PR004432
PR004432-ADC
PR004433-ADC (aBCMA)
mc-vc-PAB-MMAE

FIG. 27

**HCAb PR004433 (lot.# HSP314-02 K20200330-0409PA0409), SEC-HPLC**

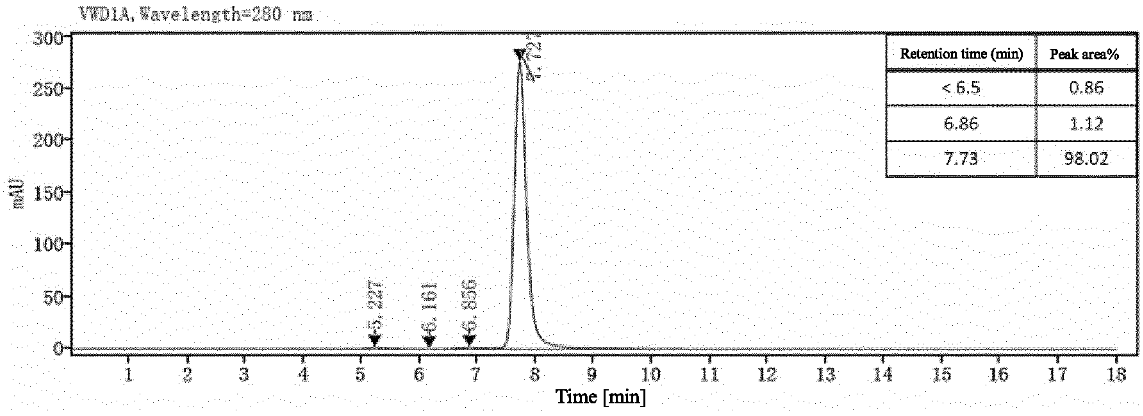


| Retention time (min) | Peak area% |
|---|---|
| < 6.5 | 0.86 |
| 6.86 | 1.12 |
| 7.73 | 98.02 |

**FIG. 28**

**(Lot.# HSP31402-ADC-20200521) after purification of PR004433-ADC, HIC-HPLC**

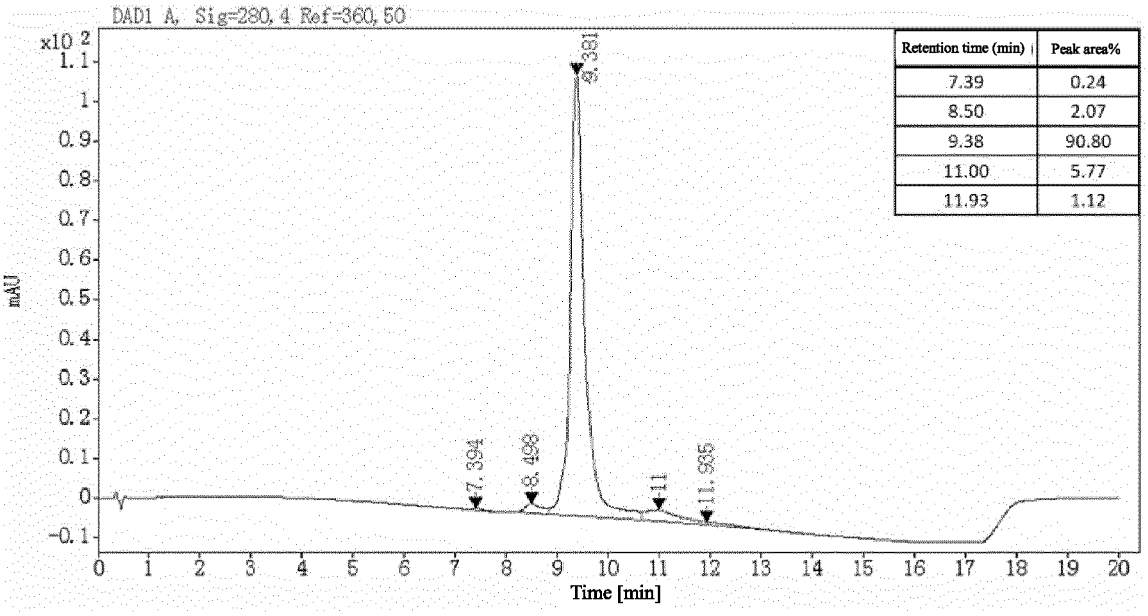


| Retention time (min) | Peak area% |
|---|---|
| 7.39 | 0.24 |
| 8.50 | 2.07 |
| 9.38 | 90.80 |
| 11.00 | 5.77 |
| 11.93 | 1.12 |

**FIG. 29**

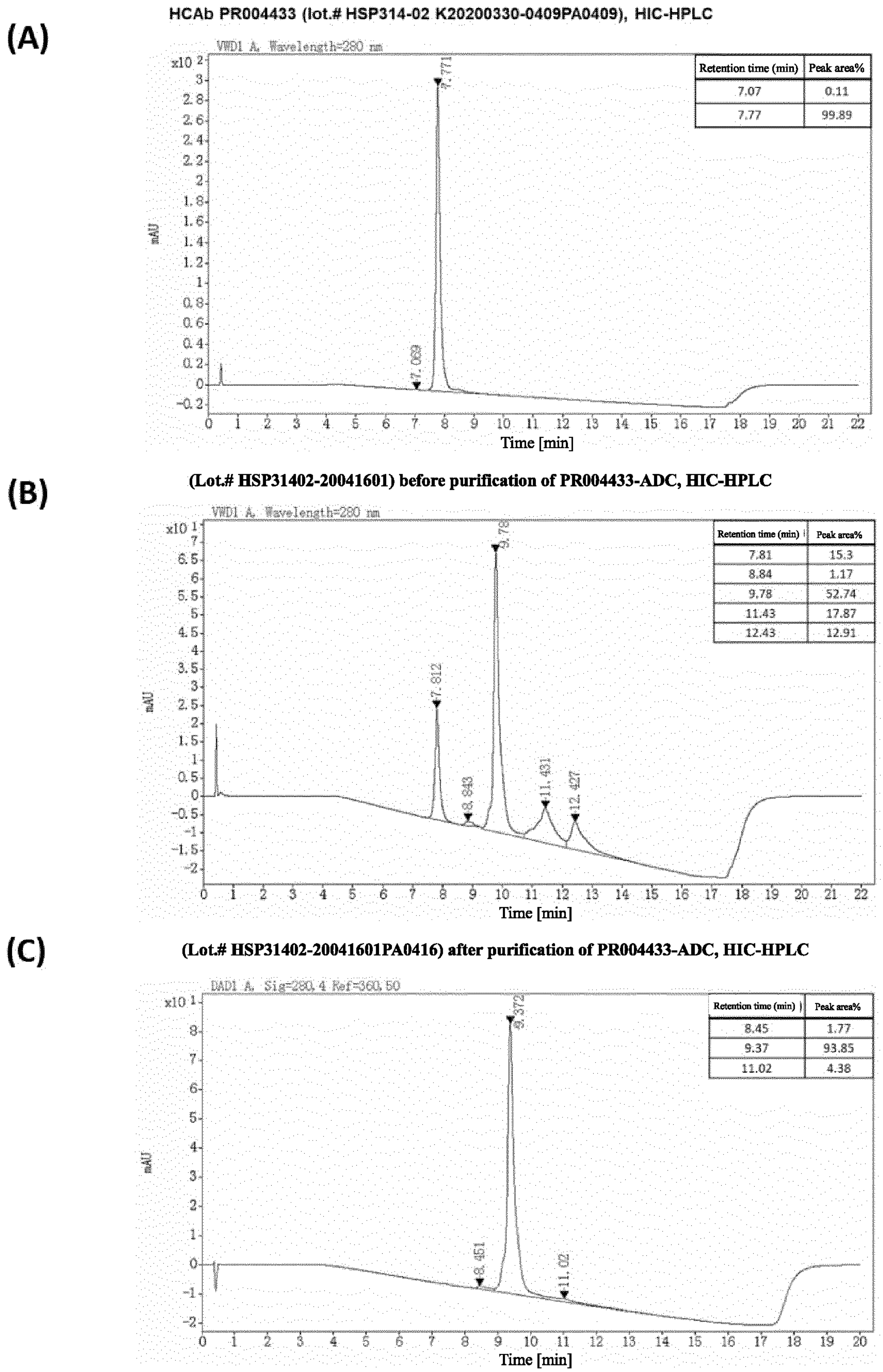
(A)
HCAb PR004433 (lot.# HSP314-02 K20200330-0409PA0409), HIC-HPLC
VWD1 A, Wavelength=280 nm
Retention time (min) | Peak area%
7.07 | 0.11
7.77 | 99.89
7.771
7.069
mAU
Time [min]
(B)
(Lot.# HSP31402-20041601) before purification of PR004433-ADC, HIC-HPLC
VWD1 A, Wavelength=280 nm
Retention time (min) | Peak area%
7.81 | 15.3
8.84 | 1.17
9.78 | 52.74
11.43 | 17.87
12.43 | 12.91
7.812
8.843
9.78
11.431
12.427
mAU
Time [min]
(C)
(Lot.# HSP31402-20041601PA0416) after purification of PR004433-ADC, HIC-HPLC
DAD1 A, Sig=280,4 Ref=360,50
Retention time (min) | Peak area%
8.45 | 1.77
9.37 | 93.85
11.02 | 4.38
9.372
8.451
11.02
mAU
Time [min]

FIG. 30

(A)

**MS map of PR004433-ADC (non-reduced sample)**

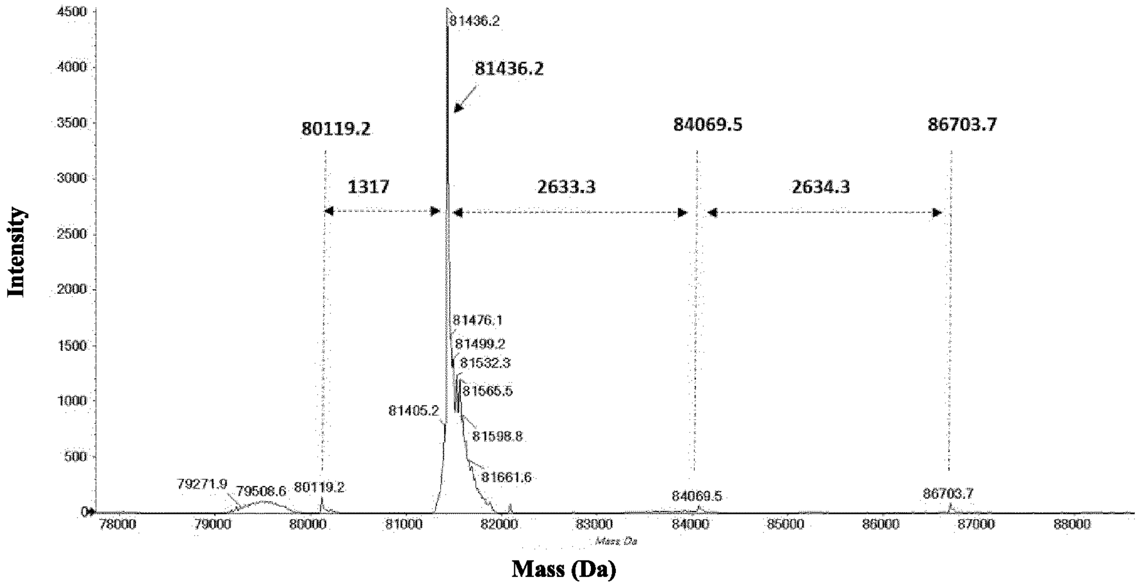


(B)

**MS map of PR004433-ADC (reduced sample)**

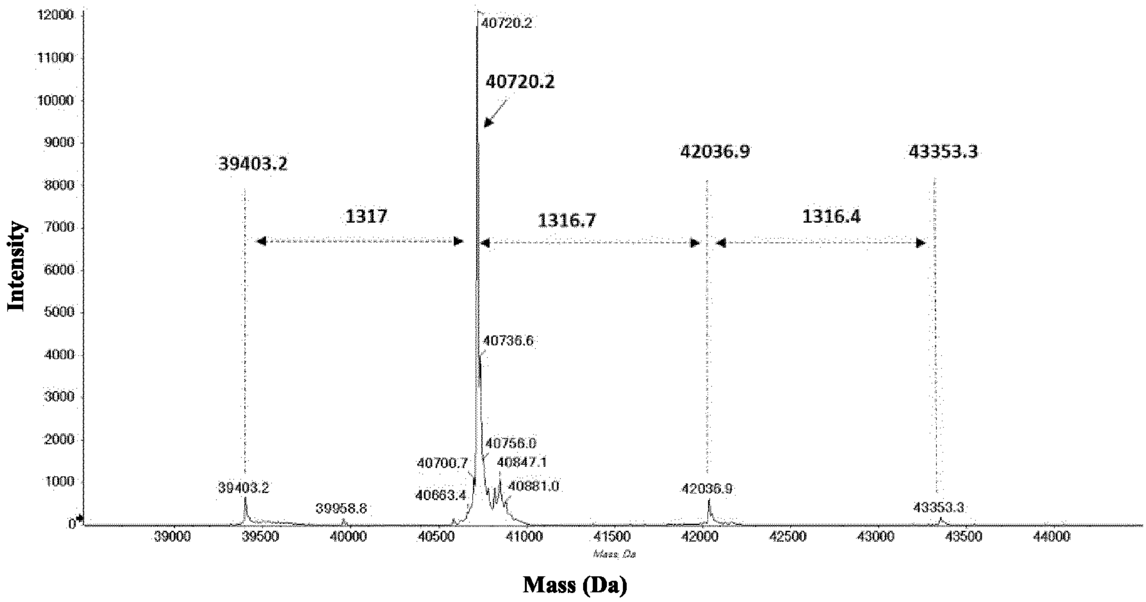


**FIG. 31**

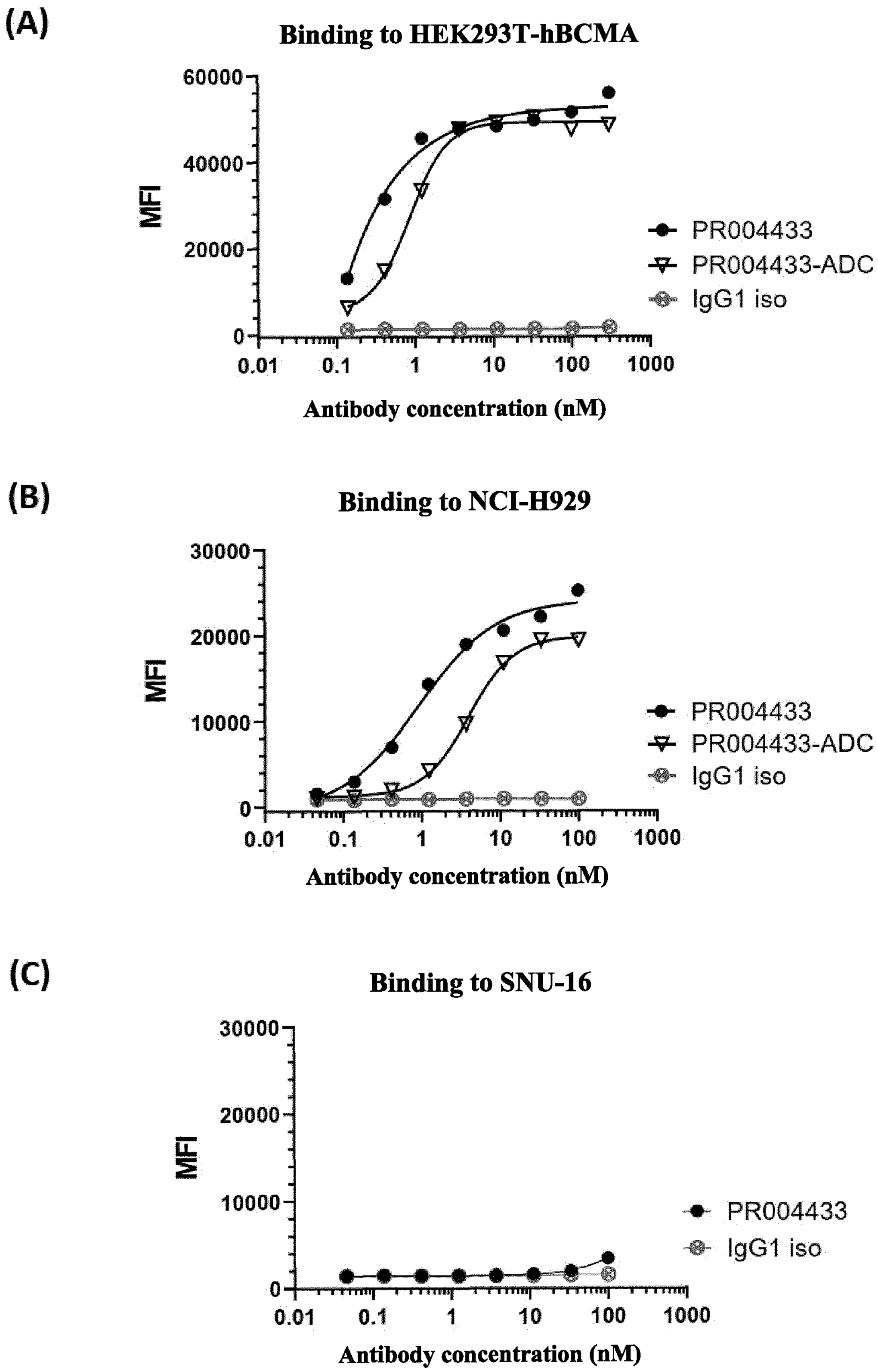
(A)
Binding to HEK293T-hBCMA
MFI
60000
40000
20000
0
0.01
0.1
1
10
100
1000
Antibody concentration (nM)
PR004433
PR004433-ADC
IgG1 iso
(B)
Binding to NCI-H929
MFI
30000
20000
10000
0
0.01
0.1
1
10
100
1000
Antibody concentration (nM)
PR004433
PR004433-ADC
IgG1 iso
(C)
Binding to SNU-16
MFI
30000
20000
10000
0
0.01
0.1
1
10
100
1000
Antibody concentration (nM)
PR004433
IgG1 iso

FIG. 32

(A)

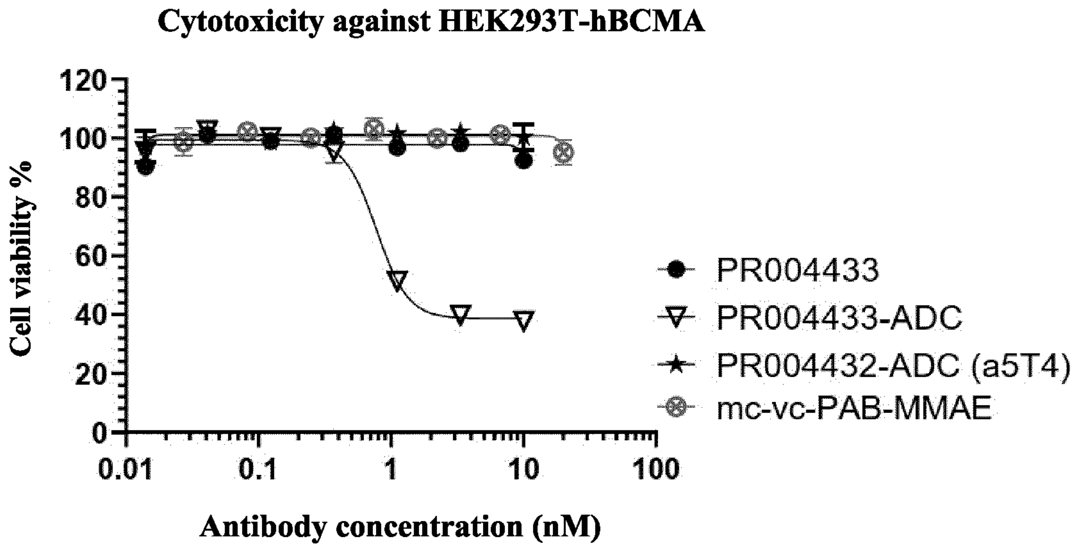


(B)

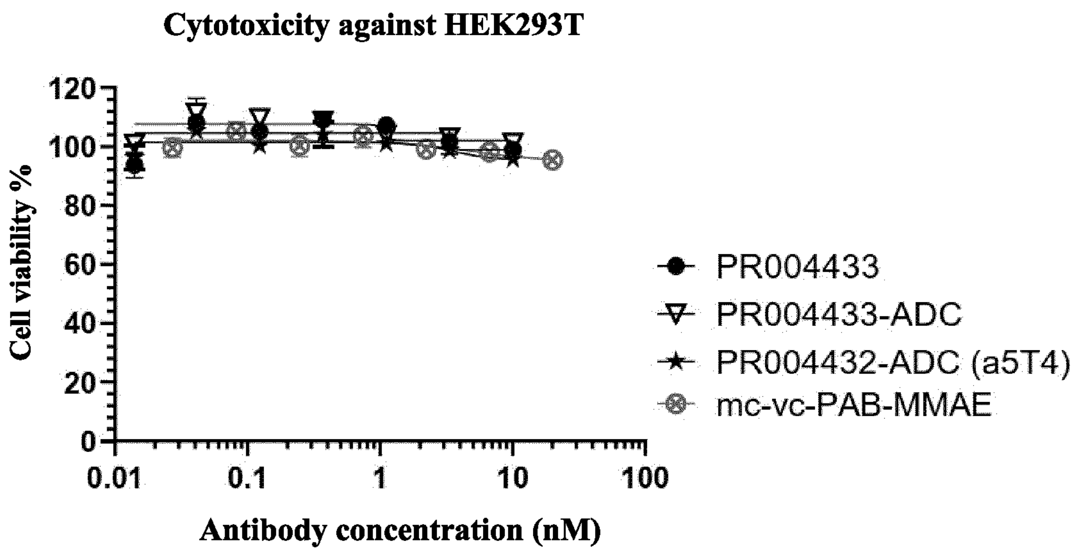


(continued on next page)

(continued from previous page)

(C)

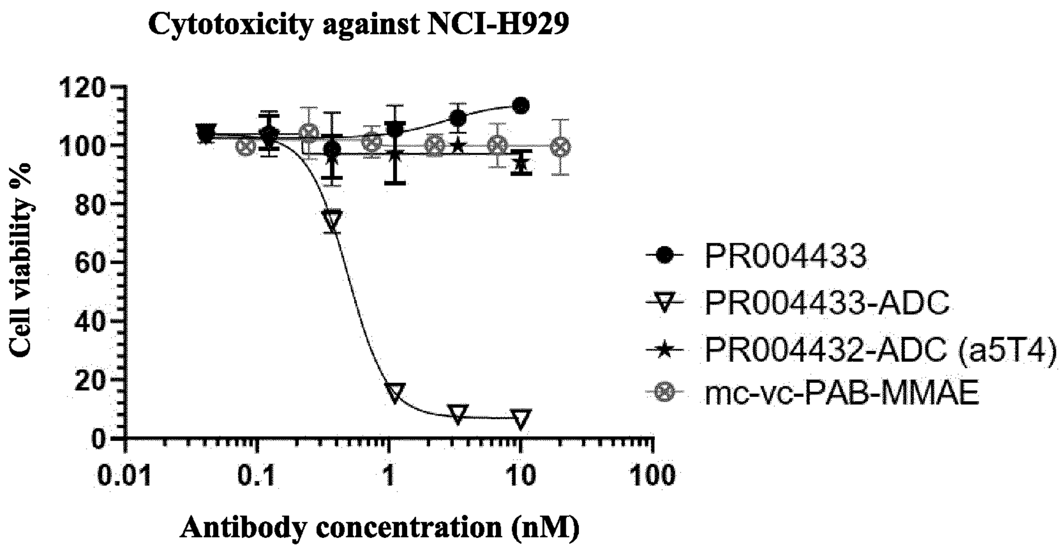


(D)

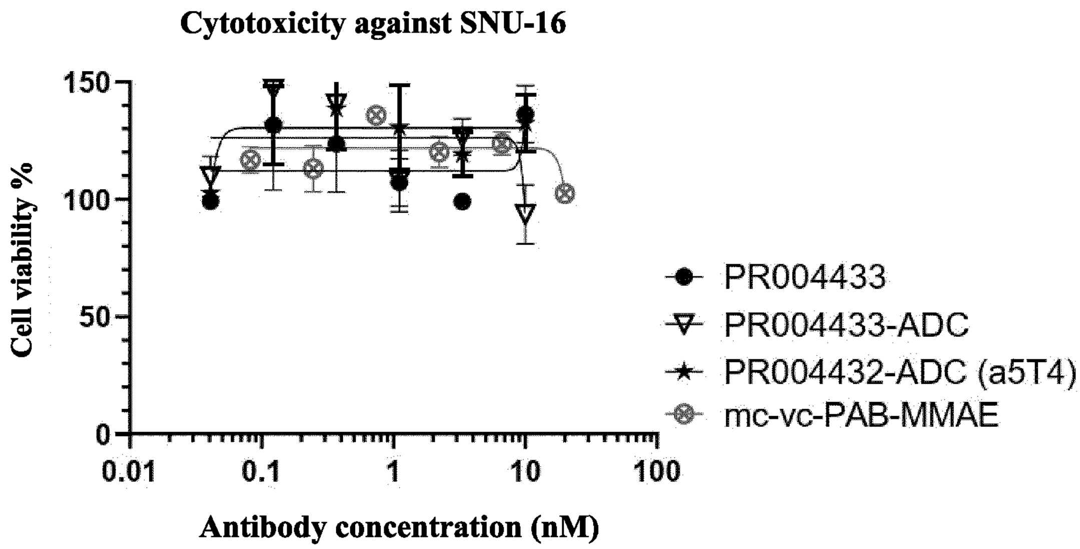


FIG. 33

EVQLVETGGGLIQPGGSLRLSCAASGFTVSDSYMTWVRQAPGKGLEWVSVIFSGGRTYYSDSV
KGRFTISRDNAKNTLYLQMNSLRAEDTALYYCARRNYDDTRGTDVFDIWGQGTMVTVSSEPK
**98%** **4%**
S**C**DKTHT**C**PP**C**PAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK

- SC(+1315.78)DK
- SC(+1315.78)DKTHTCPPCPAPELLGGPSVFLFPPK
- THTCPPCPAPELLGGPSVFLFPPK

| | Attribute Name | Value |
|---|---|---|
| 1 | MMAE "SCDK" | 98.03 % |
| 2 | MMAE "THTCPPCPAPELLGGPSVFLFPPKPK" | 4.01 % |

FIG. 34

**PR000020-ADC peptide fragment 'PHGSDIWGQGTMVTVSSEPKSC (+ 1315.78) DK' secondary MS map**

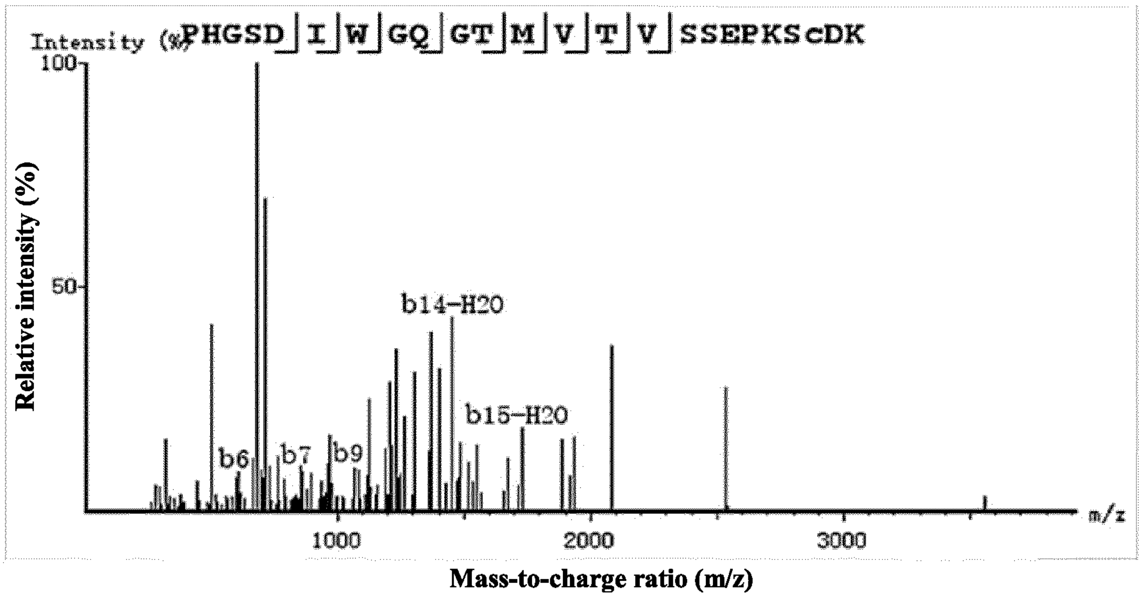


FIG. 35

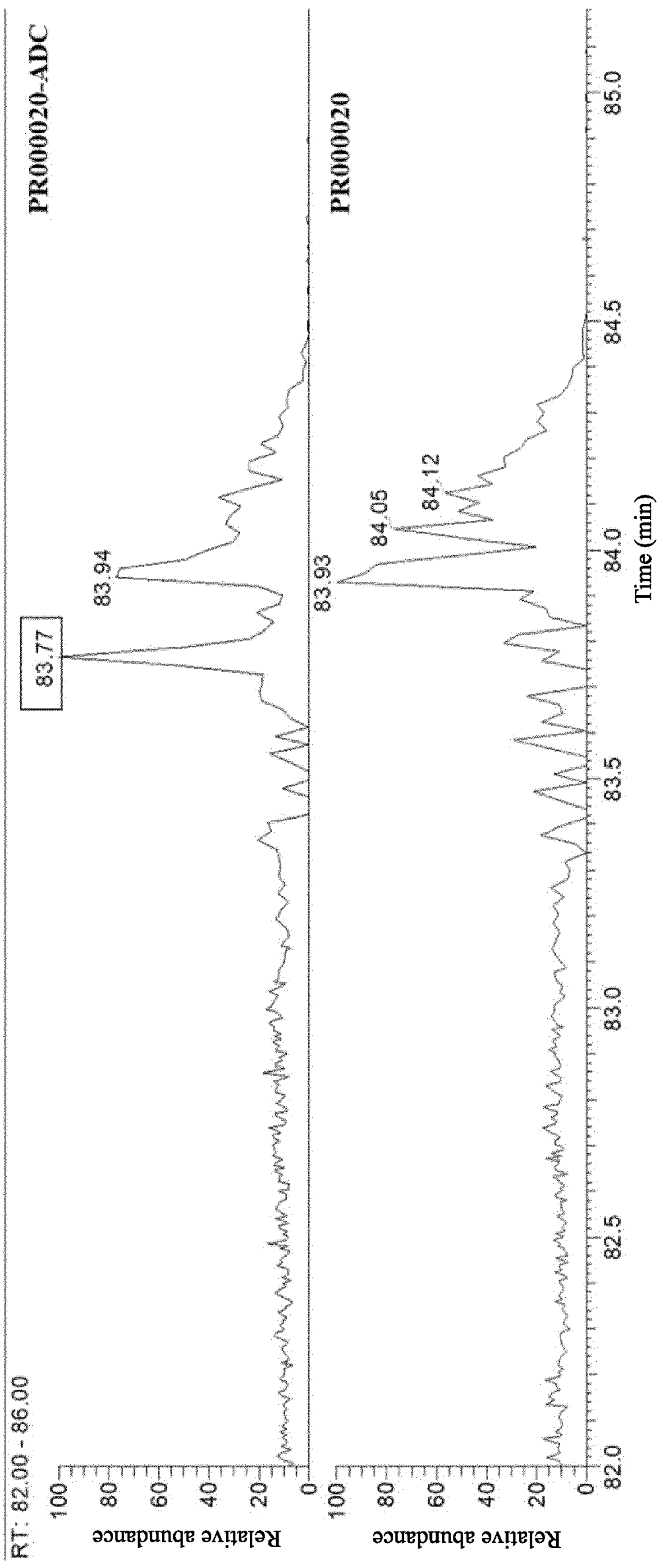

RT: 82.00 - 86.00
PR000020-ADC
83.77
83.94
PR000020
83.93
84.05
84.12
Relative abundance
100
80
60
40
20
0
82.0
82.5
83.0
83.5
84.0
84.5
85.0
Time (min)


FIG. 36

(A)

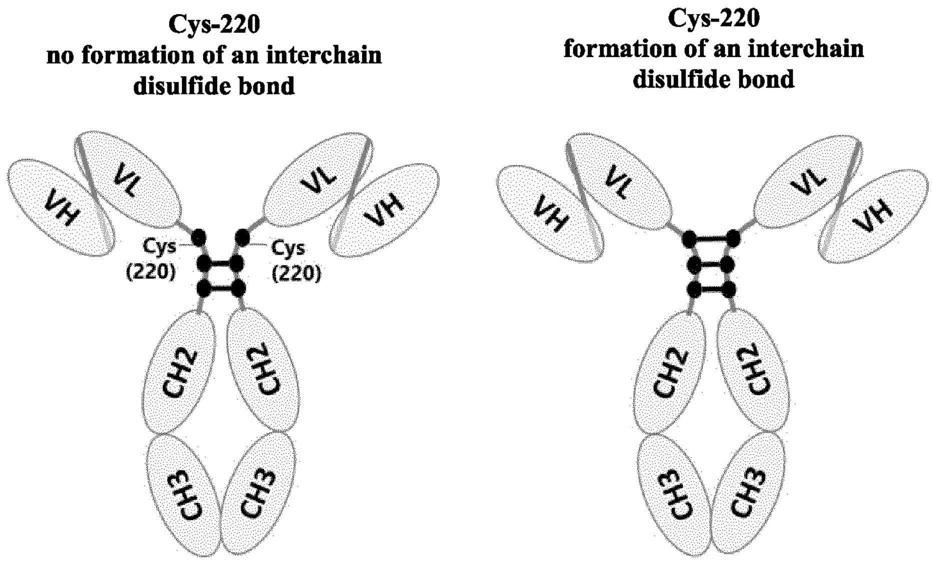


(B)

MS map of PR002129 (non-reduced sample)

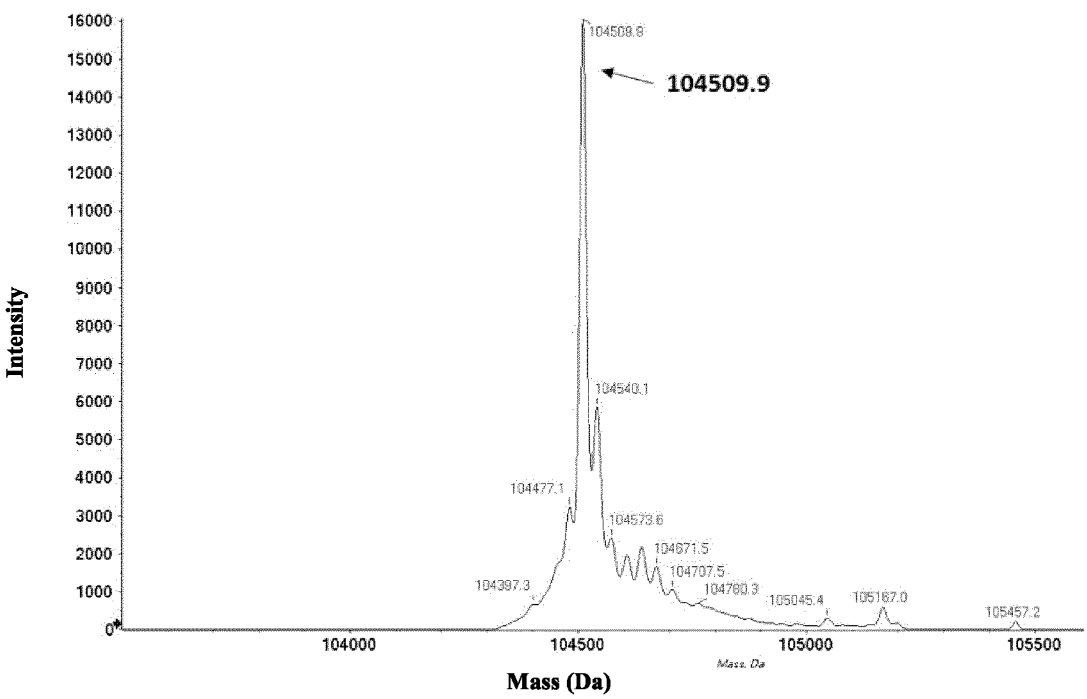


FIG. 37

(A)

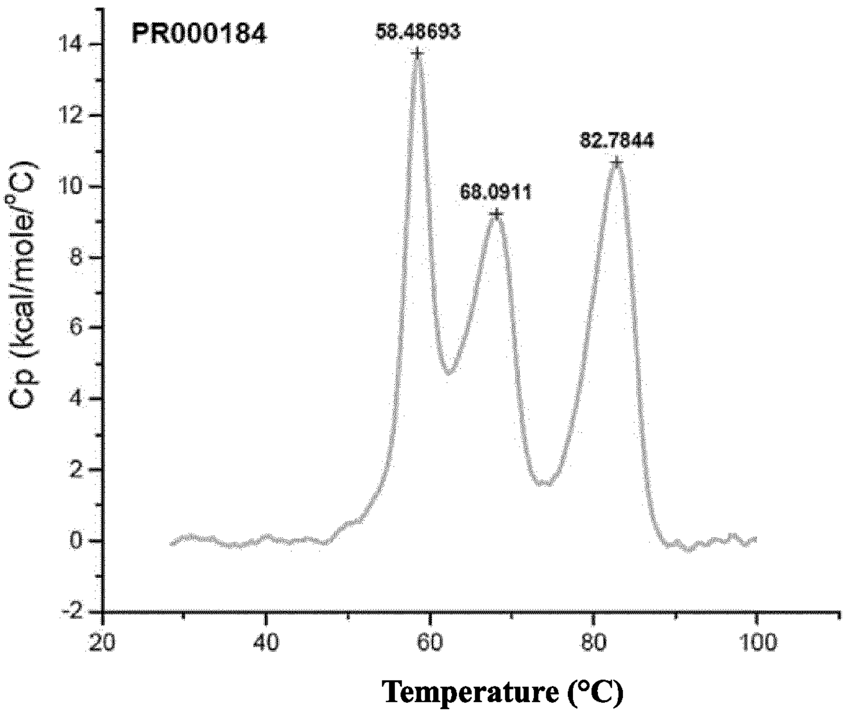
PR000184
58.48693
68.0911
82.7844
Cp (kcal/mole/°C)
14
12
10
8
6
4
2
0
-2
20
40
60
80
100
Temperature (°C)

(B)

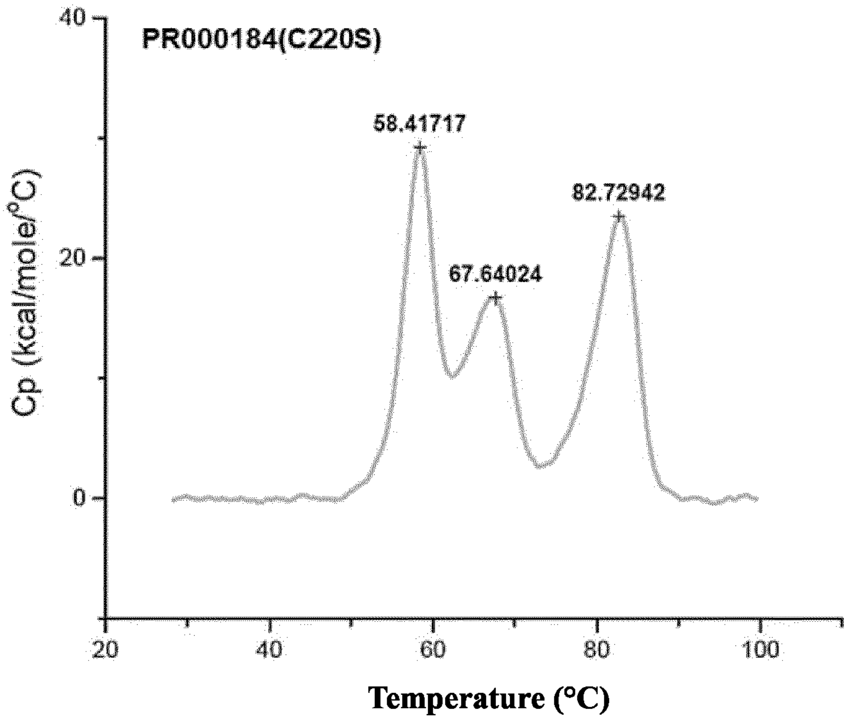
PR000184(C220S)
58.41717
67.64024
82.72942
Cp (kcal/mole/°C)
40
20
0
20
40
60
80
100
Temperature (°C)

FIG. 38

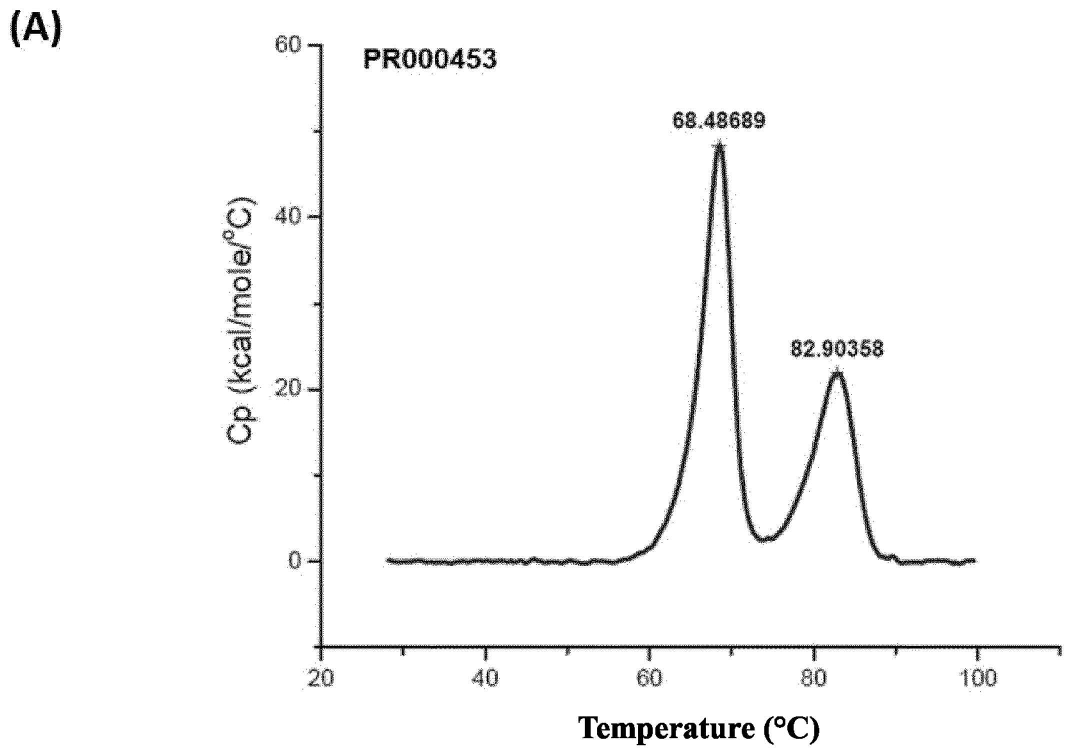
(A)
PR000453
68.48689
82.90358
Cp (kcal/mole/°C)
60
40
20
0
20
40
60
80
100
Temperature (°C)

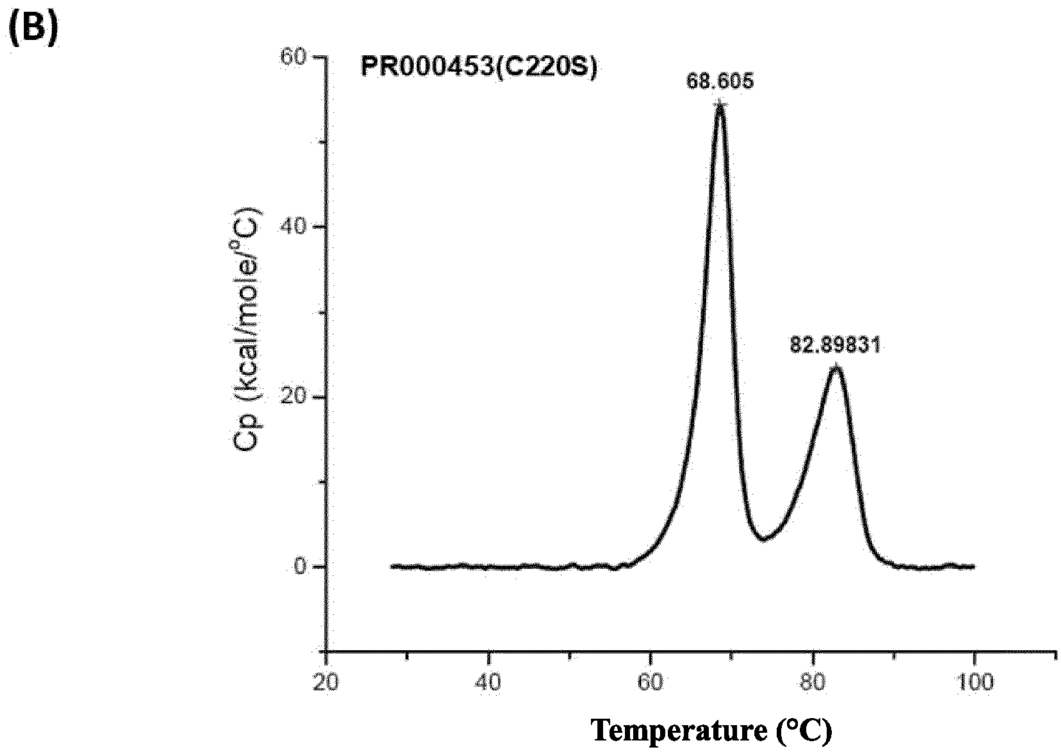
(B)
PR000453(C220S)
68.605
82.89831
Cp (kcal/mole/°C)
60
40
20
0
20
40
60
80
100
Temperature (°C)

FIG. 39

(A)

(B)

(C)

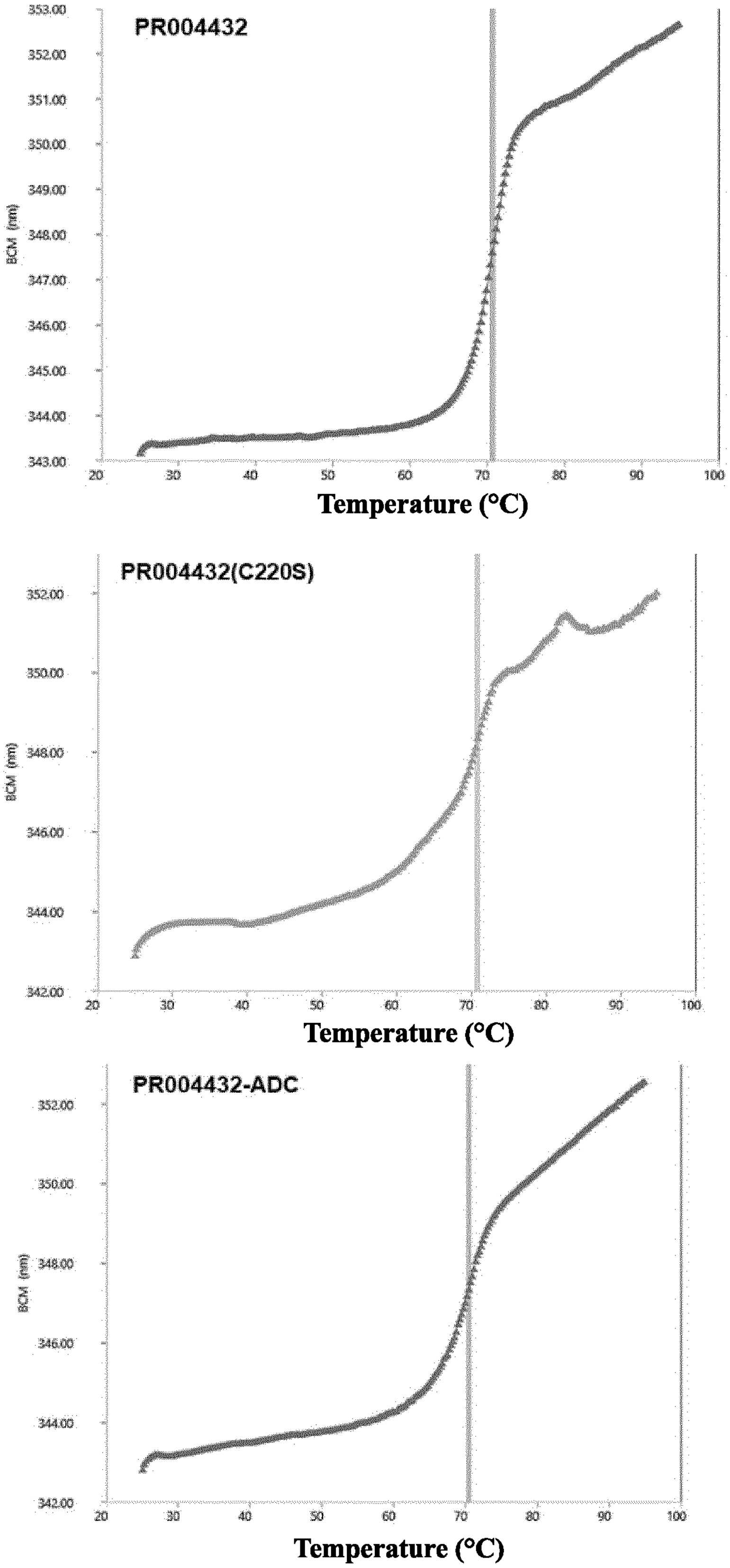
PR004432
BCM (nm)
Temperature (°C)
PR004432(C220S)
BCM (nm)
Temperature (°C)
PR004432-ADC
BCM (nm)
Temperature (°C)

FIG. 40

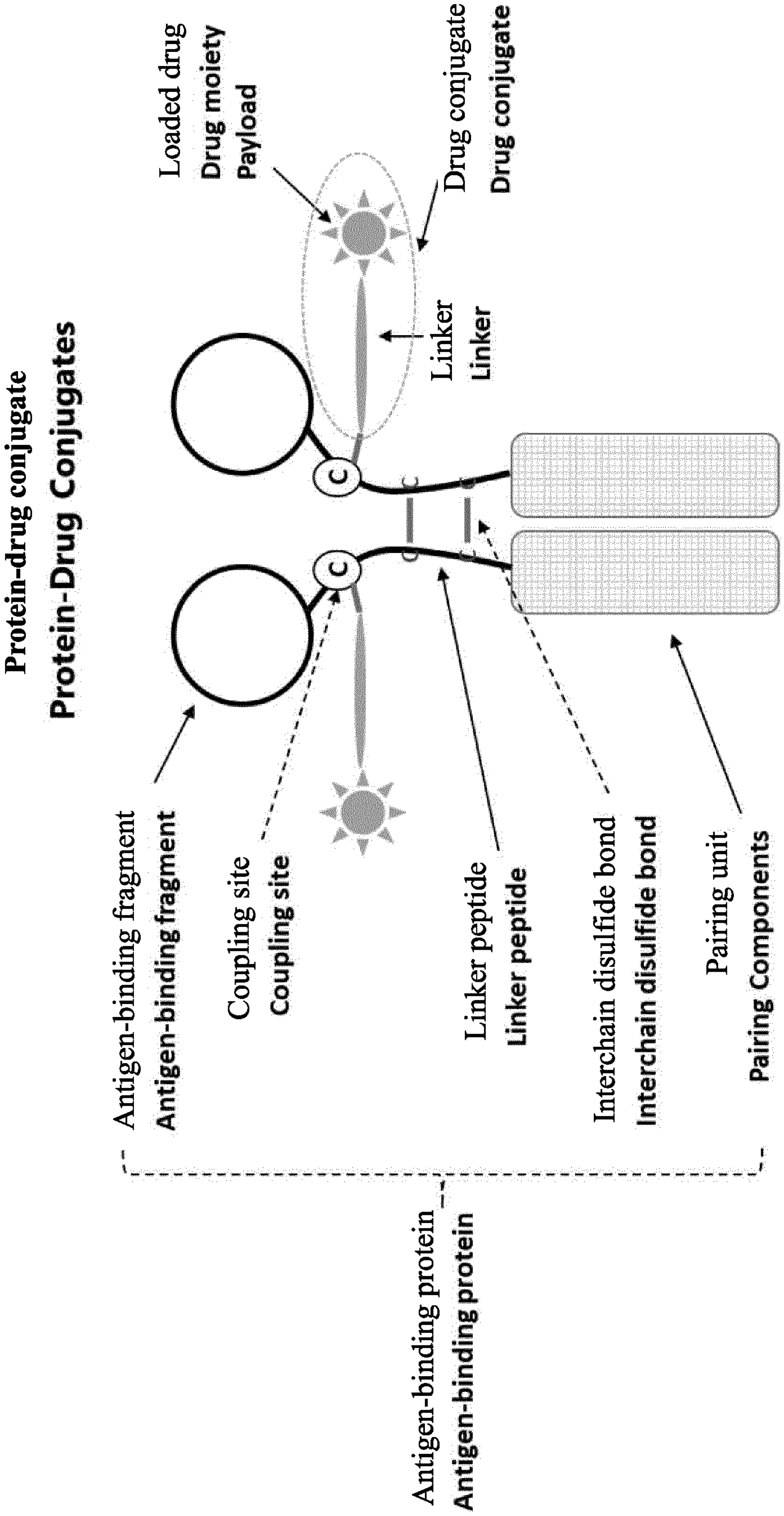
Protein-drug conjugate
Protein-Drug Conjugates
Loaded drug
Drug moiety
Payload
Drug conjugate
Drug conjugate
Linker
Linker
Antigen-binding fragment
Antigen-binding fragment
Coupling site
Coupling site
Linker peptide
Linker peptide
Interchain disulfide bond
Interchain disulfide bond
Pairing unit
Pairing Components
Antigen-binding protein
Antigen-binding protein
C
C

FIG. 41

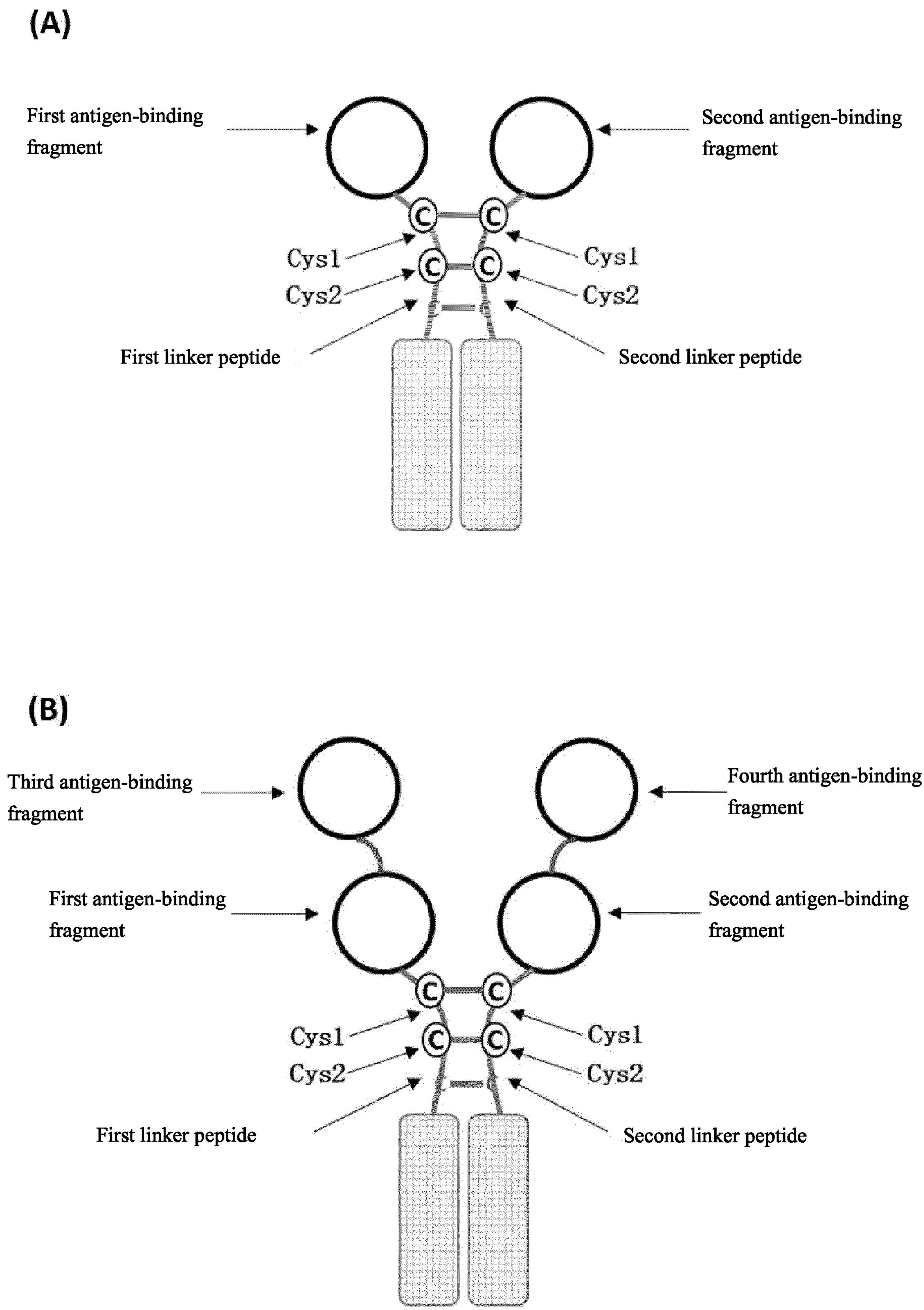
(A)
First antigen-binding fragment
Second antigen-binding fragment
Cys1
Cys2
Cys1
Cys2
First linker peptide
Second linker peptide
(B)
Third antigen-binding fragment
Fourth antigen-binding fragment
First antigen-binding fragment
Second antigen-binding fragment
Cys1
Cys2
Cys1
Cys2
First linker peptide
Second linker peptide

FIG. 42

(A)

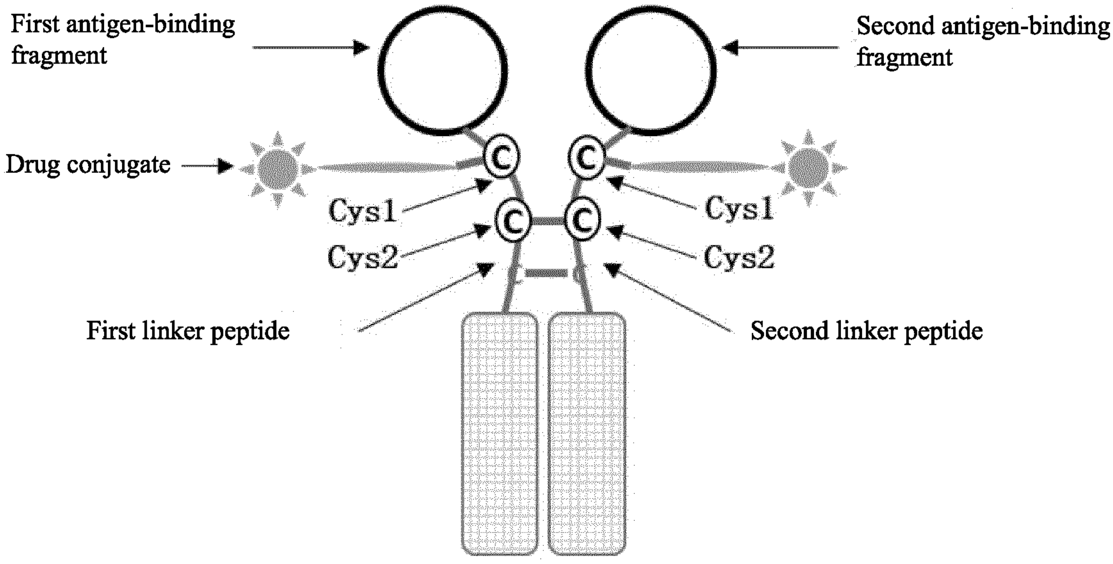
First antigen-binding fragment
Second antigen-binding fragment
Drug conjugate
Cys1
Cys2
Cys1
Cys2
First linker peptide
Second linker peptide

(B)

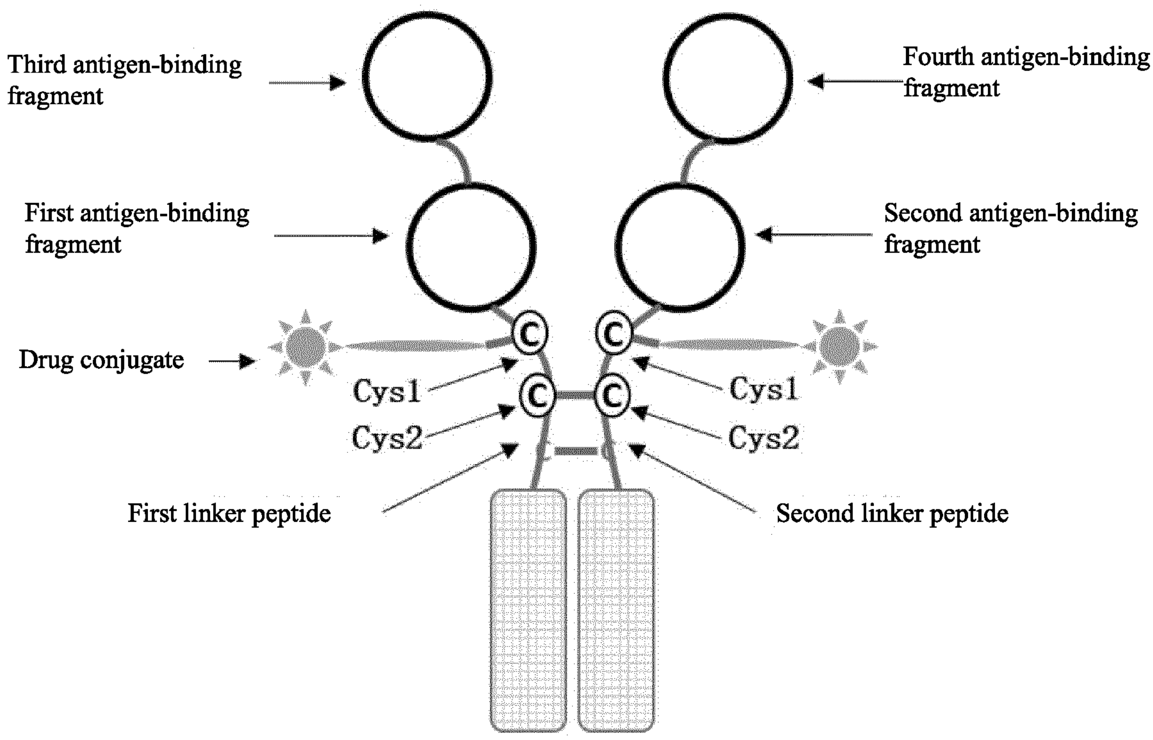
Third antigen-binding fragment
Fourth antigen-binding fragment
First antigen-binding fragment
Second antigen-binding fragment
Drug conjugate
Cys1
Cys2
Cys1
Cys2
First linker peptide
Second linker peptide

**FIG. 43**

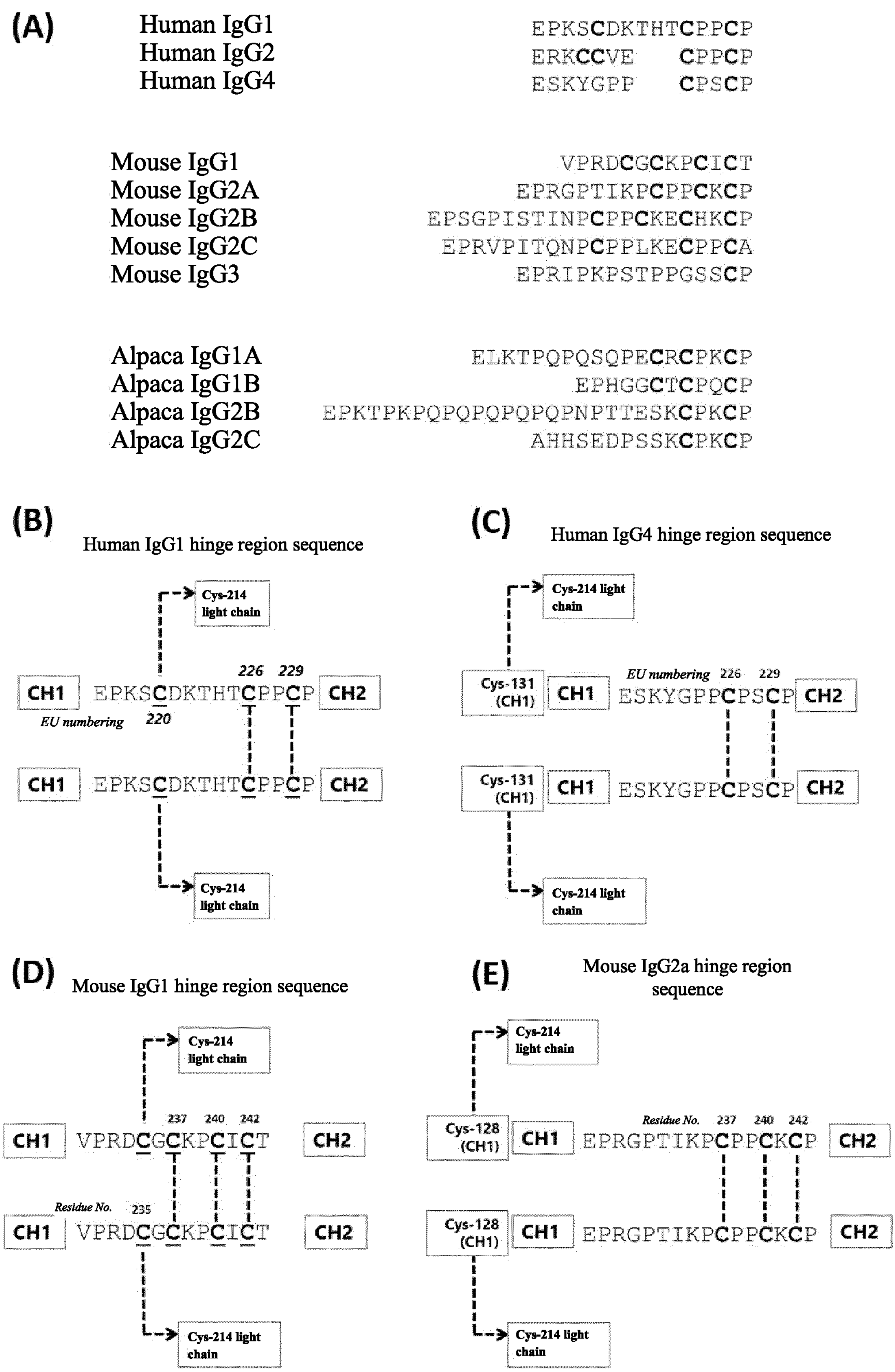
(A)
Human IgG1 EPKSCDKTHTCPPCP
Human IgG2 ERKCCVE CPPCP
Human IgG4 ESKYGPP CPSCP
Mouse IgG1 VPRDCGCKPCICT
Mouse IgG2A EPRGPTIKPCPPCKCP
Mouse IgG2B EPSGPISTINPCPPCKECHKCP
Mouse IgG2C EPRVPITQNPCPPLKECPPCA
Mouse IgG3 EPRIPKPSTPPGSSCP
Alpaca IgG1A ELKTPQPQSQPECRCPKCP
Alpaca IgG1B EPHGGCTCPQCP
Alpaca IgG2B EPKTPKPQPQPQPQPQPNPTTESKCPKCP
Alpaca IgG2C AHHSEDPSSKCPKCP
(B)
Human IgG1 hinge region sequence
Cys-214 light chain
226 229
CH1 EPKSCDKTHTCPPCP CH2
EU numbering 220
CH1 EPKSCDKTHTCPPCP CH2
Cys-214 light chain
(C)
Human IgG4 hinge region sequence
Cys-214 light chain
EU numbering 226 229
Cys-131 (CH1) CH1 ESKYGPPCPSCP CH2
Cys-131 (CH1) CH1 ESKYGPPCPSCP CH2
Cys-214 light chain
(D)
Mouse IgG1 hinge region sequence
Cys-214 light chain
237 240 242
CH1 VPRDCGCKPCICT CH2
Residue No. 235
CH1 VPRDCGCKPCICT CH2
Cys-214 light chain
(E)
Mouse IgG2a hinge region sequence
Cys-214 light chain
Residue No. 237 240 242
Cys-128 (CH1) CH1 EPRGPTIKPCPPCKCP CH2
Cys-128 (CH1) CH1 EPRGPTIKPCPPCKCP CH2
Cys-214 light chain

**FIG. 44**

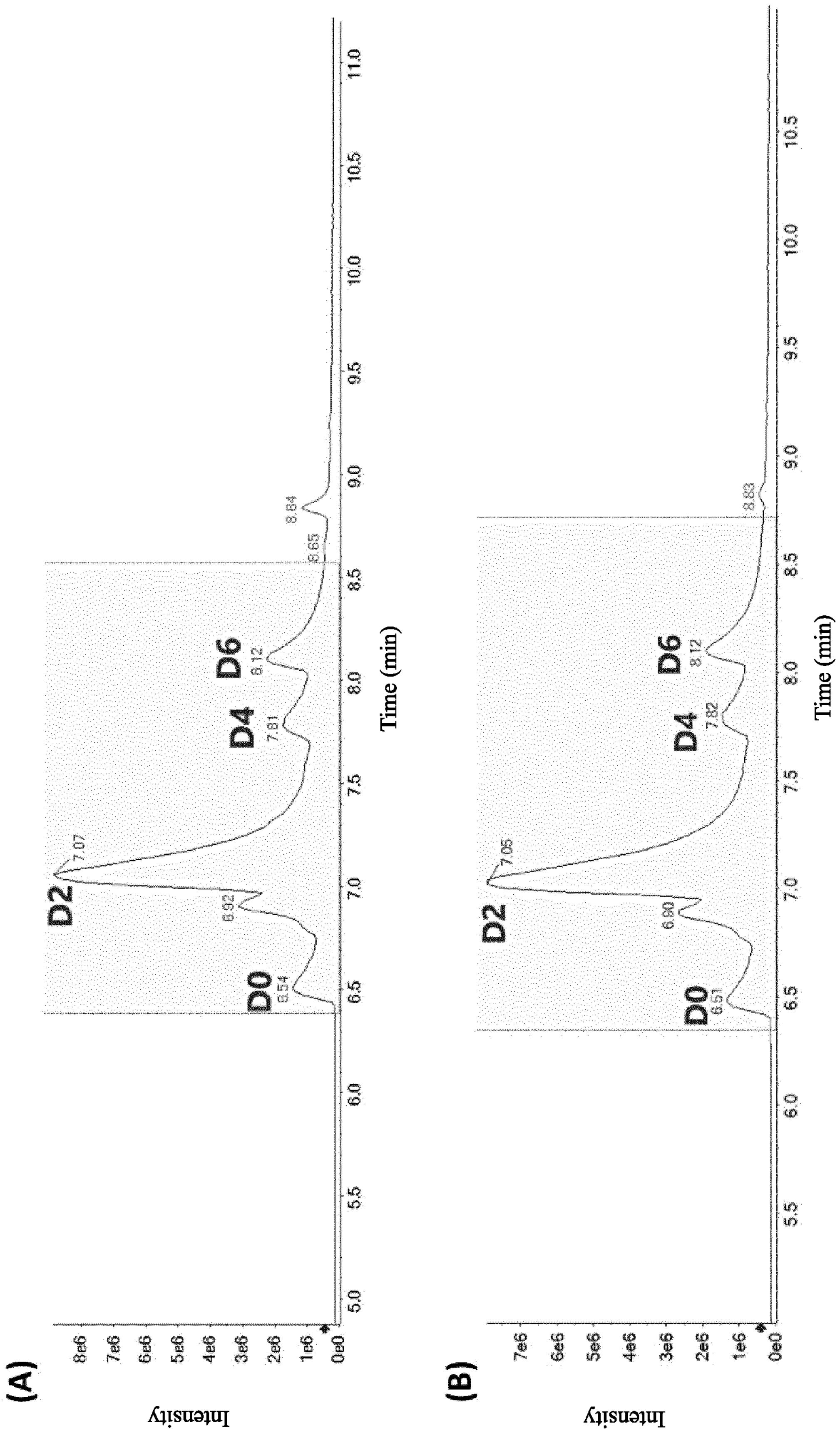


(continued on next page)

(continued from previous page)

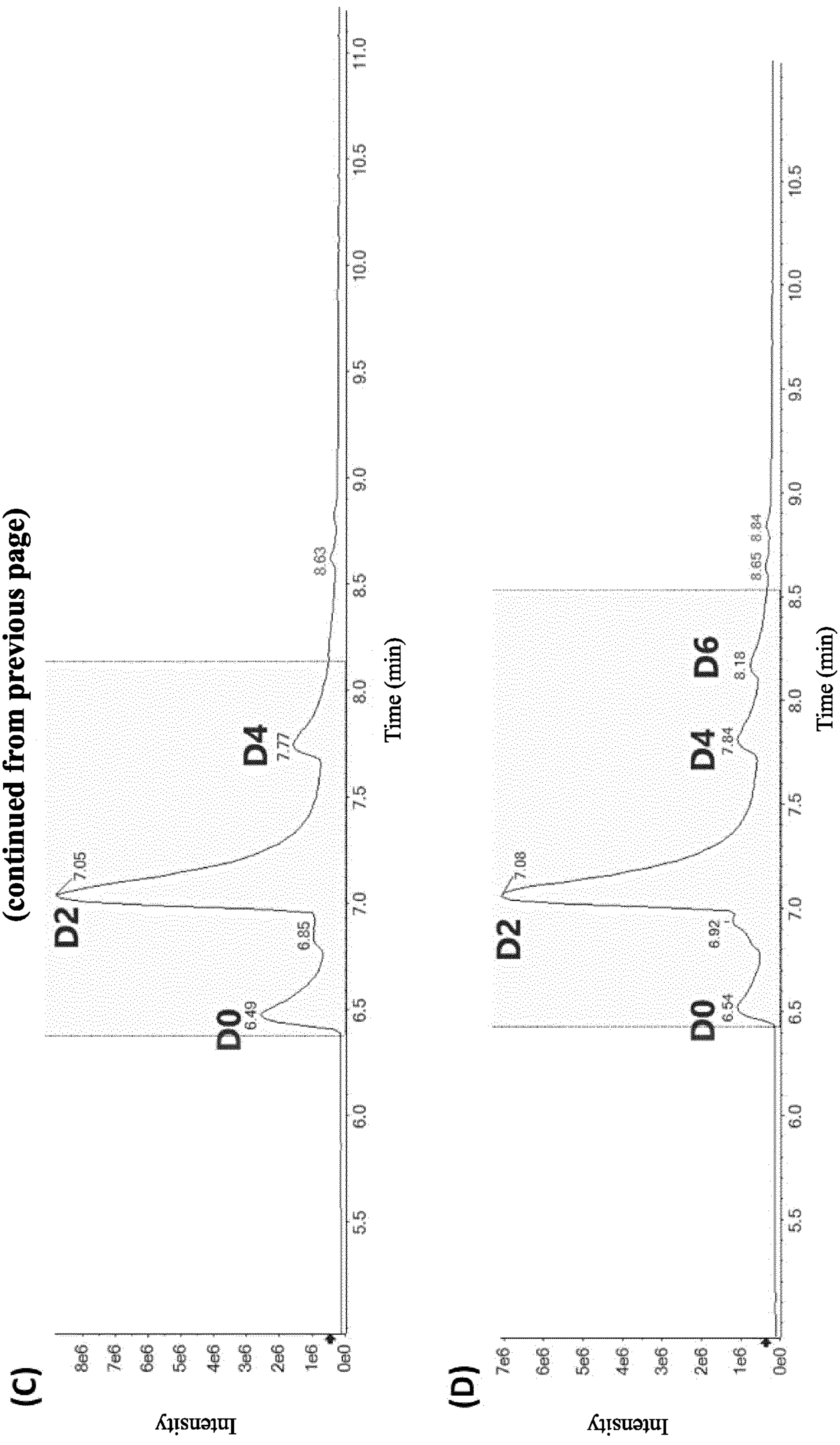


FIG. 45

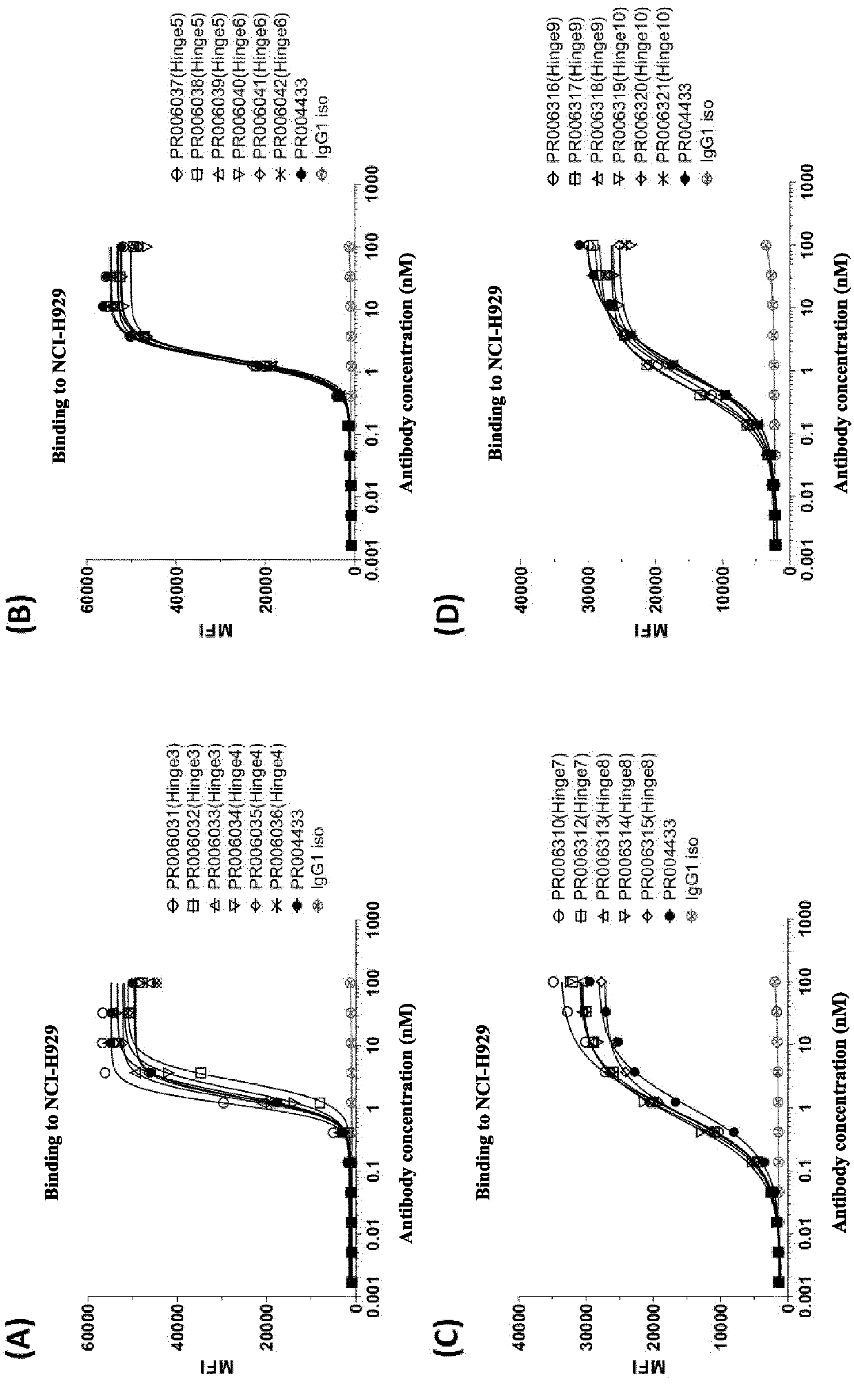
(A)
Binding to NCI-H929
MFI
Antibody concentration (nM)
PR006031(Hinge3)
PR006032(Hinge3)
PR006033(Hinge3)
PR006034(Hinge4)
PR006035(Hinge4)
PR006036(Hinge4)
PR004433
IgG1 iso
(B)
Binding to NCI-H929
MFI
Antibody concentration (nM)
PR006037(Hinge5)
PR006038(Hinge5)
PR006039(Hinge5)
PR006040(Hinge6)
PR006041(Hinge6)
PR006042(Hinge6)
PR004433
IgG1 iso
(C)
Binding to NCI-H929
MFI
Antibody concentration (nM)
PR006310(Hinge7)
PR006312(Hinge7)
PR006313(Hinge8)
PR006314(Hinge8)
PR006315(Hinge8)
PR004433
IgG1 iso
(D)
Binding to NCI-H929
MFI
Antibody concentration (nM)
PR006316(Hinge9)
PR006317(Hinge9)
PR006318(Hinge9)
PR006319(Hinge10)
PR006320(Hinge10)
PR006321(Hinge10)
PR004433
IgG1 iso

FIG. 46

**MS map of PR006468-ADC (non-reduced sample)**

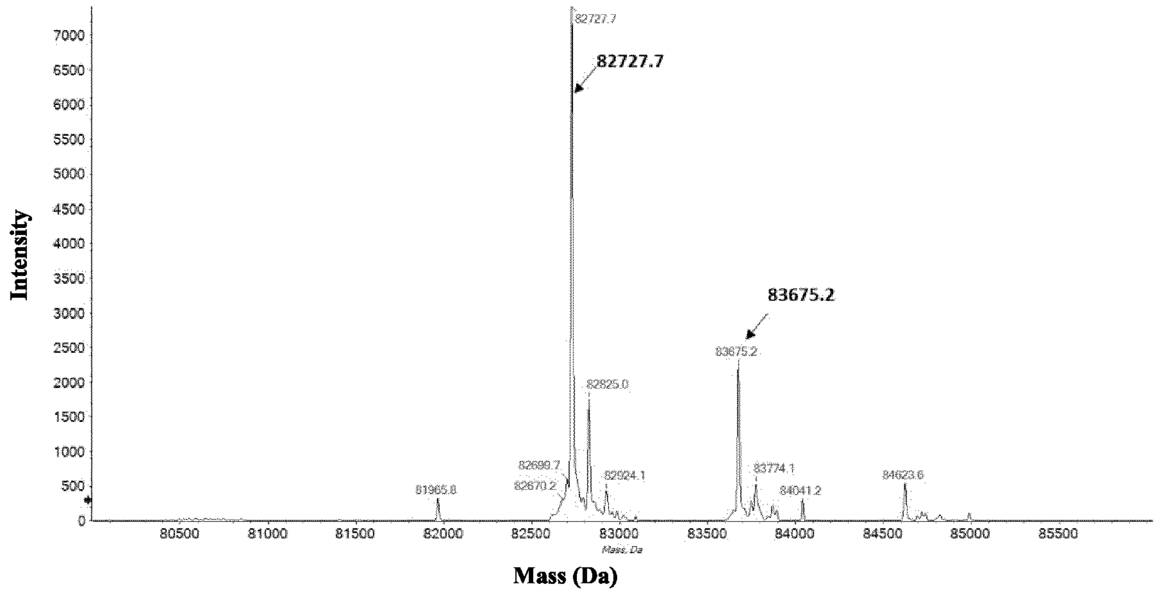


**FIG. 47**

EVQLVETGGGLIQPGGSLRLSCAASGFTVSDSYMTWVRQAPGKGLEWVSVIFSGGRTYYS
DSVKGRFTISRDNAKNTLYLQMNSLRAEDTALYYCARRNYDDTRGTDVFDIWGQGTMVTV
**90.8%** **5.81%**
SSEPRVPITQNP**C**PPLKE**C**PP**C**AAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSLSPGK

1. VPITQNPC(+1315.78)PPLK
2. VPITQNPC(+1315.78)PPLKECPPCAPELLGGPSVFLFPPKPK
3. EC(+1315.78)PPCAPELLGGPSVFLFPPKPK

| | Attribute Name | Value |
|---|---|---|
| 1 | MMAE@CPPLK | 90.80 % |
| 2 | MMAE@ECPPC | 5.81 % |

**FIG. 48**

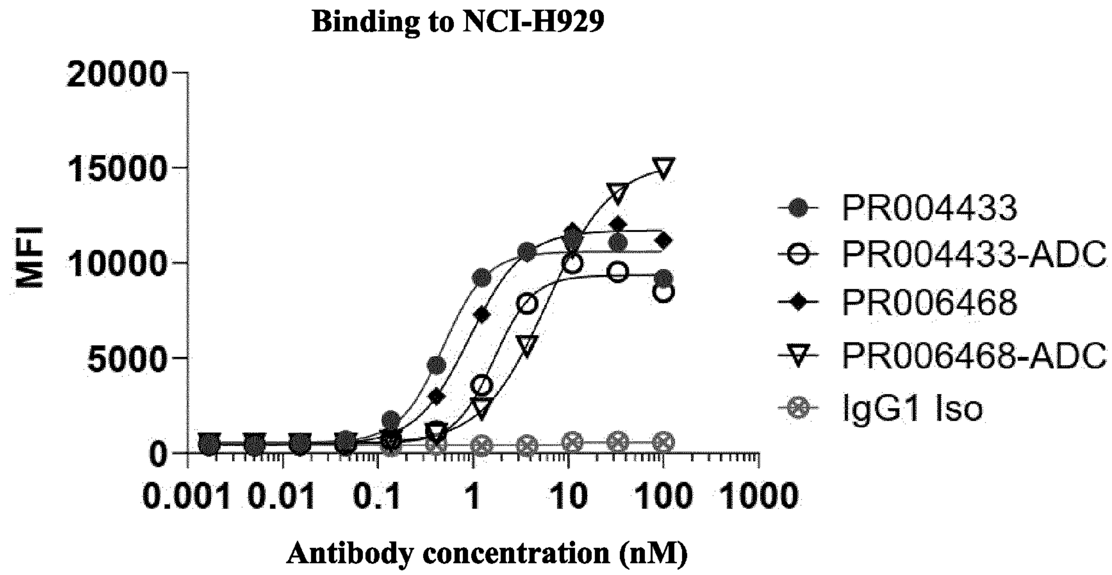
Binding to NCI-H929
20000
15000
10000
5000
0
MFI
0.001
0.01
0.1
1
10
100
1000
Antibody concentration (nM)
PR004433
PR004433-ADC
PR006468
PR006468-ADC
IgG1 Iso

FIG. 49

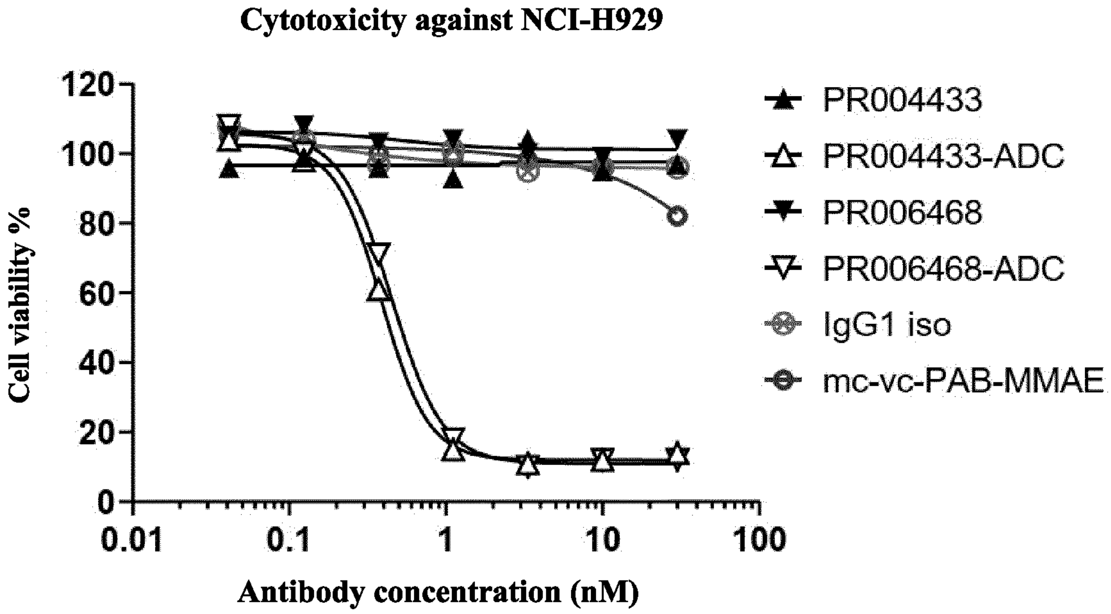
Cytotoxicity against NCI-H929
120
100
80
60
40
20
0
Cell viability %
0.01
0.1
1
10
100
Antibody concentration (nM)
PR004433
PR004433-ADC
PR006468
PR006468-ADC
IgG1 iso
mc-vc-PAB-MMAE

FIG. 50

(A)

**(Lot.# CY20210202) before purification of PR002129-ADC, HIC-HPLC**

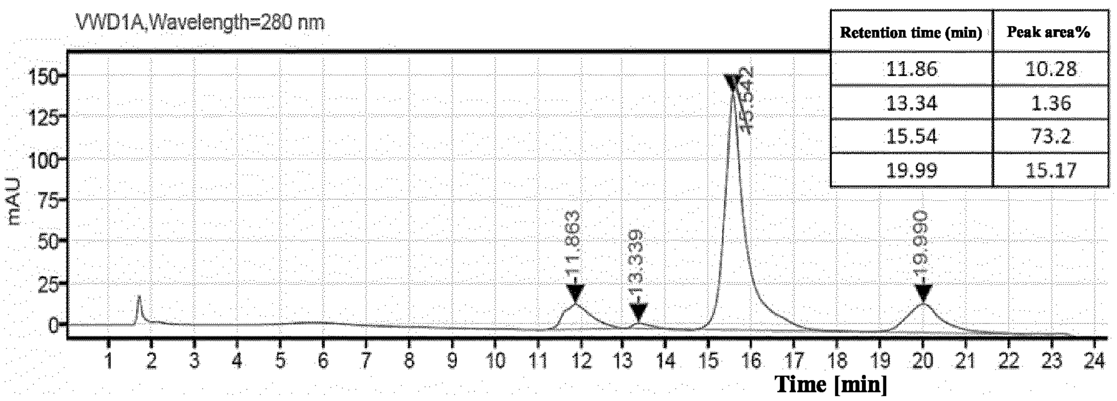


| Retention time (min) | Peak area% |
|---|---|
| 11.86 | 10.28 |
| 13.34 | 1.36 |
| 15.54 | 73.2 |
| 19.99 | 15.17 |

(B)

**(Lot.# HSP31405-20200820) after purification of PR002129-ADC, HIC-HPLC**

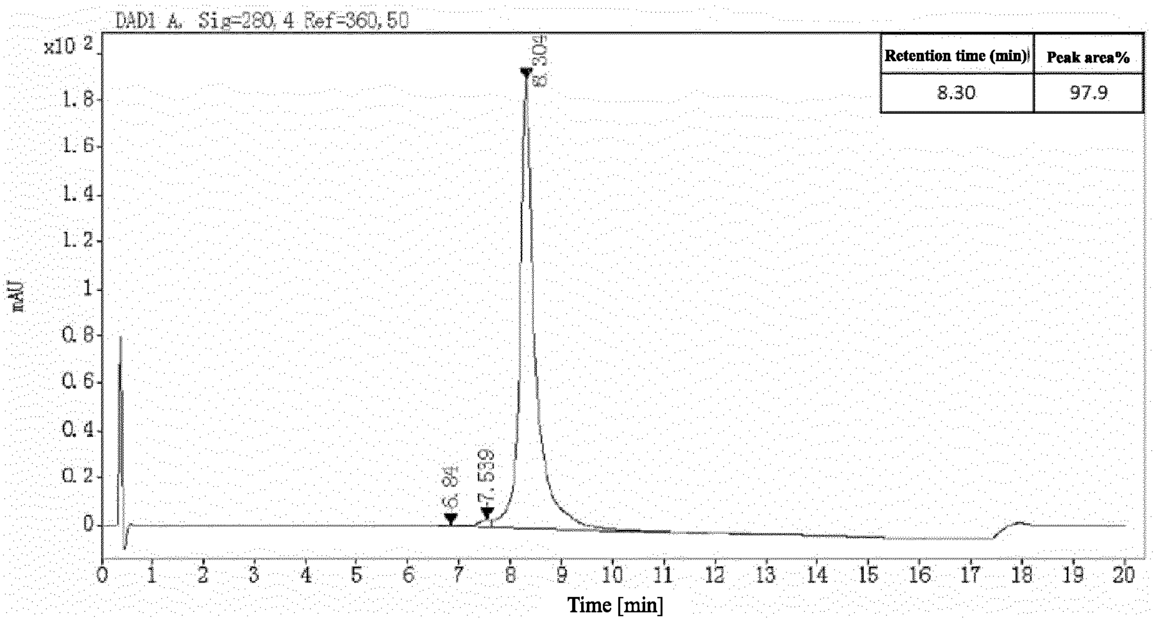


| Retention time (min) | Peak area% |
|---|---|
| 8.30 | 97.9 |

**FIG. 51**

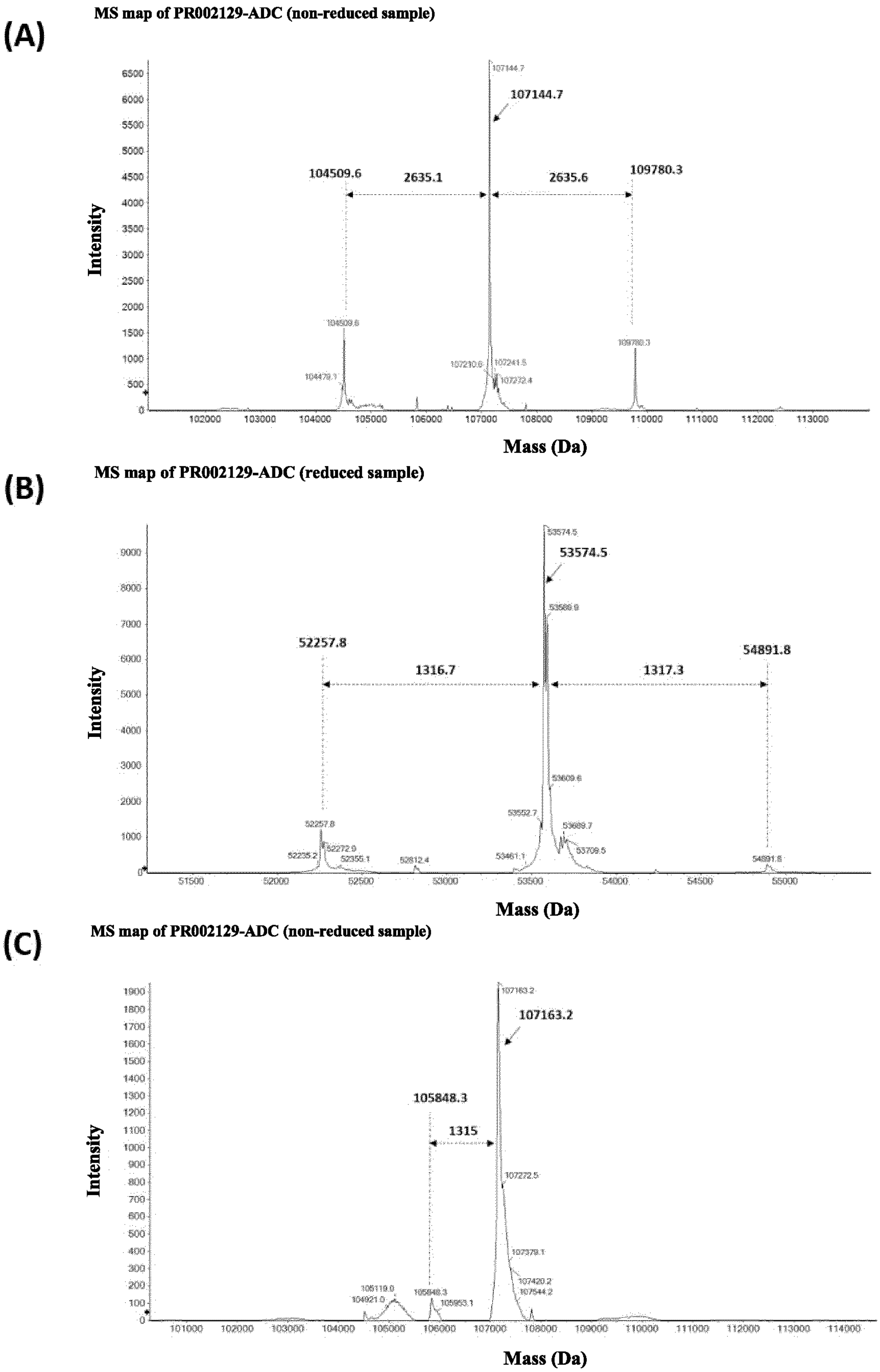
(A)
MS map of PR002129-ADC (non-reduced sample)
Intensity
Mass (Da)
107144.7
104509.6
2635.1
2635.6
109780.3
(B)
MS map of PR002129-ADC (reduced sample)
Intensity
Mass (Da)
53574.5
52257.8
1316.7
1317.3
54891.8
(C)
MS map of PR002129-ADC (non-reduced sample)
Intensity
Mass (Da)
107163.2
105848.3
1315

FIG. 52

```
QVQLVQSGGGVVQPGRSLRLSCAASGFTFSSYGMHWVRQAPGKGLEWVAVIWYDGSNKDYADSVKGRFTISRD
TSKNTLYLQMNSLRAEDTAVYYCAREQARSFDLWGRGTLVTVSSGGGGSGGGGSGGGGSDIQLTQSPYSLSAS
VGDRVTITCRASQTINNWLAWYQQKPGKAPKLLIYKASSLESGVPSRFSGSGSGTEFTLTISSLQP
                                        87.9%     7.5%
DDFATYYCQQYKLYSPMYTFGQGTKVEIKGGGASEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK
```

1. SC(+1315.78)DK
2. SC(+1315.78)DKTHTCPPCPAPELLGGPSVFLFPPK
3. SCDKTHTCPPC(+1315.78)PAPELLGGPSVFLFPPK

**FIG. 53**

**Binding to PanC-1**

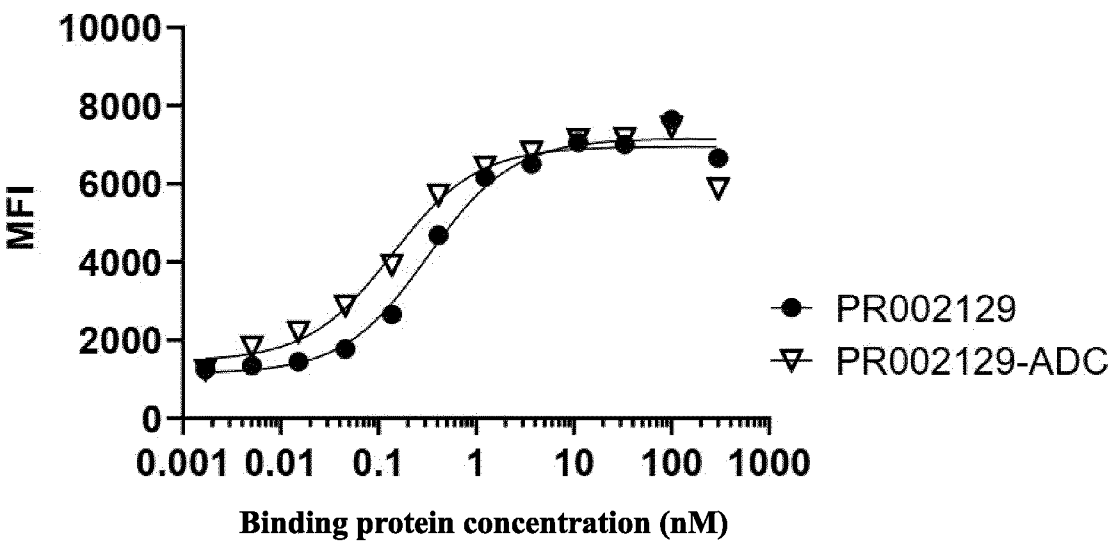


**FIG. 54**

(Lot.# CY20201229) before purification of PR006345-ADC, HIC-HPLC

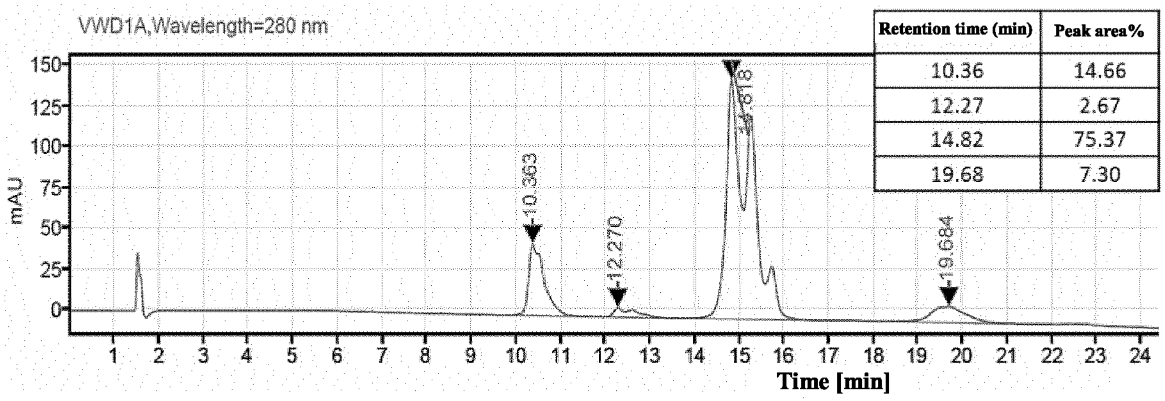


| Retention time (min) | Peak area% |
|---|---|
| 10.36 | 14.66 |
| 12.27 | 2.67 |
| 14.82 | 75.37 |
| 19.68 | 7.30 |

FIG. 55

MS map of PR006345-ADC (non-reduced sample)

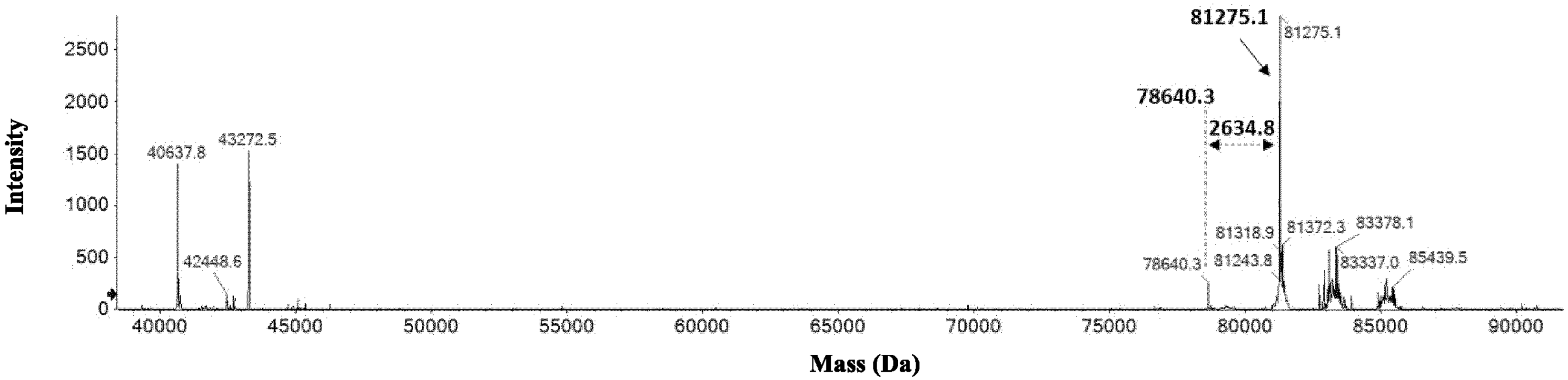


FIG. 56

```
EEELQIIQPDKSVLVAAGETATLRCTITSLFPVGPIQWFRGAGPGRVLIYNQRQGPFPRV
TTVSDTTKRNNMDFSIRIGNITPADAGTYYCIKFRKGSPDDVEFKSGAGTELSVRAKPSG
         92.45%  1.36%
GGASEPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
```

1. SC(+1315.78)DK
2. SC(+1315.78)DKTHTCPPCPAPELLGGPSVFLFPPK
3. THTC(+1315.78)PPCPAPELLGGPSVFLFPPK

| | Attribute Name | Value |
|---|---|---|
| 1 | MMAE@SCDK | 92.45 % |
| 2 | MMAE@THTCPPC | 1.36 % |

FIG. 57

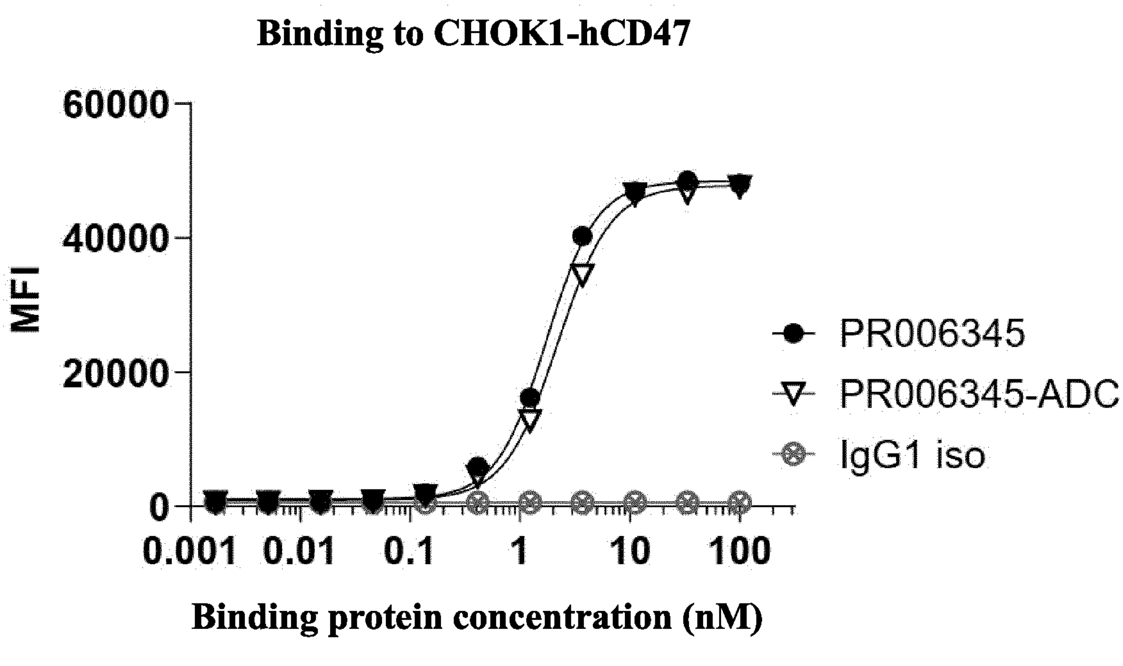


FIG. 58

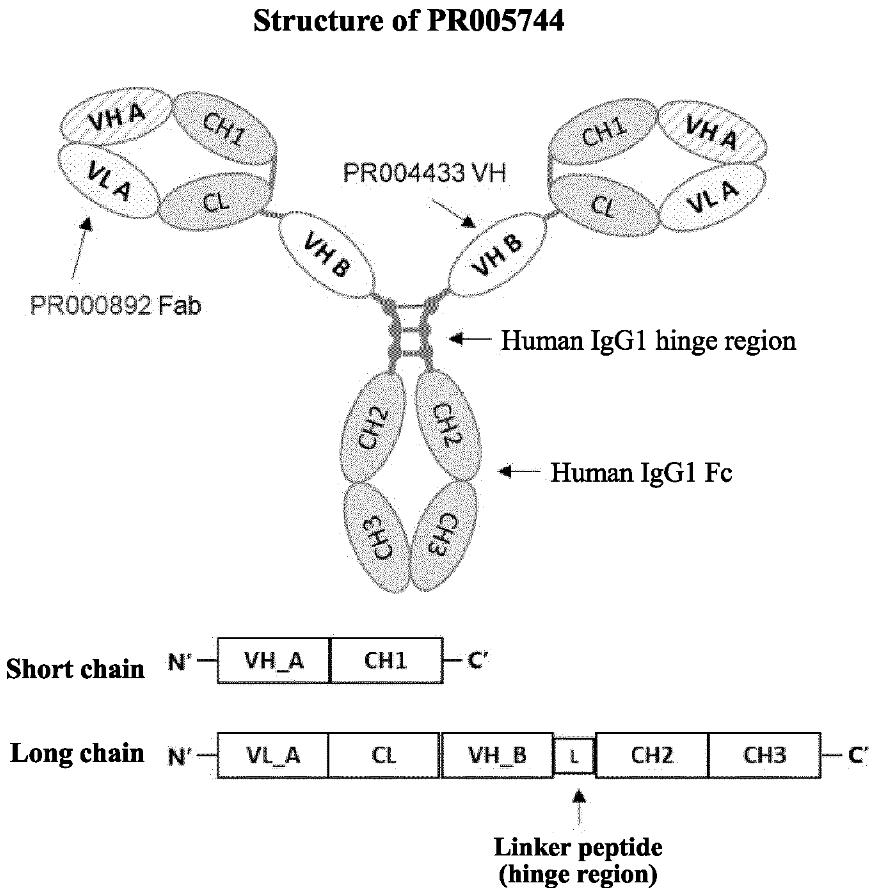
Structure of PR005744
VH A
CH1
VL A
CL
VH B
PR004433 VH
PR000892 Fab
Human IgG1 hinge region
CH2
CH3
Human IgG1 Fc
Short chain
N'
VH_A
CH1
C'
Long chain
VL_A
CL
VH_B
L
CH2
CH3
Linker peptide
(hinge region)

FIG. 59

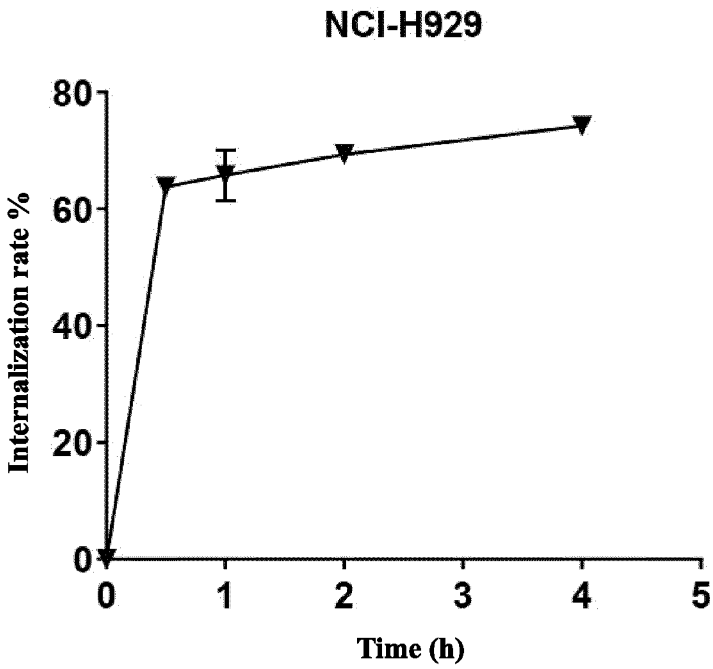
NCI-H929
Internalization rate %
80
60
40
20
0
0
1
2
3
4
5
Time (h)

FIG. 60

**PR005744 long chain:**

DIQMTQSPSTLSASVGDRVTITCRASQSISSWLAWYQQKPGKAPKLLIYKASSLESGVPS
RFSGSGSGTEFTLTISSLQTDDFATYYCQQYNSYLFTFGQGTKLEIKRTVAAPSVFIFPP
SDEQLKSGTASVVCLLNNFYPREAKVQWKVDNALQSGNSQESVTEQDSKDSTYSLSSTLT
1.36%
LSKADYEKHKVYACEVTHQGLSSPVTKSFNRGECEVQLVETGGGLIQPGGSLRLSCAASG
FTVSDSYMTWVRQAPGKGLEWVSVIFSGGRTYYSDSVKGRFTISRDNAKNTLYLQMNSLR
99.07% 4.15%
AEDTALYYCARRNYDDTRGTDVFDIWGQGTMVTVSSEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK

**PR005744 short chain:**

QVQLVESGGGVVQPGRSLRLSCAATGFTFSSYGMYWVRQAPGKGLEWVAAIWNDGSNNYY
ADSVKGRFTISRDDSKNTLNLQMNSLRAEDTAMYYCARDRLPMASLRYFDWLGVMDAWGQ
GTSVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSC

1. SC(+1315.78)DK
2. THTC(+1315.78)PPCPAPELLGGPSVFLFPPK
3. GEC(+1315.78)EVQLETGGGLIQPGGSLR

| | Attribute Name | Value |
|---|---|---|
| 1 | MMAE@HC GECEVQ | 1.36 % |
| 2 | MMAE@HC SCDK | 99.07 % |
| 3 | MMAE@HC THTCPPC | 4.15 % |

**FIG. 61**

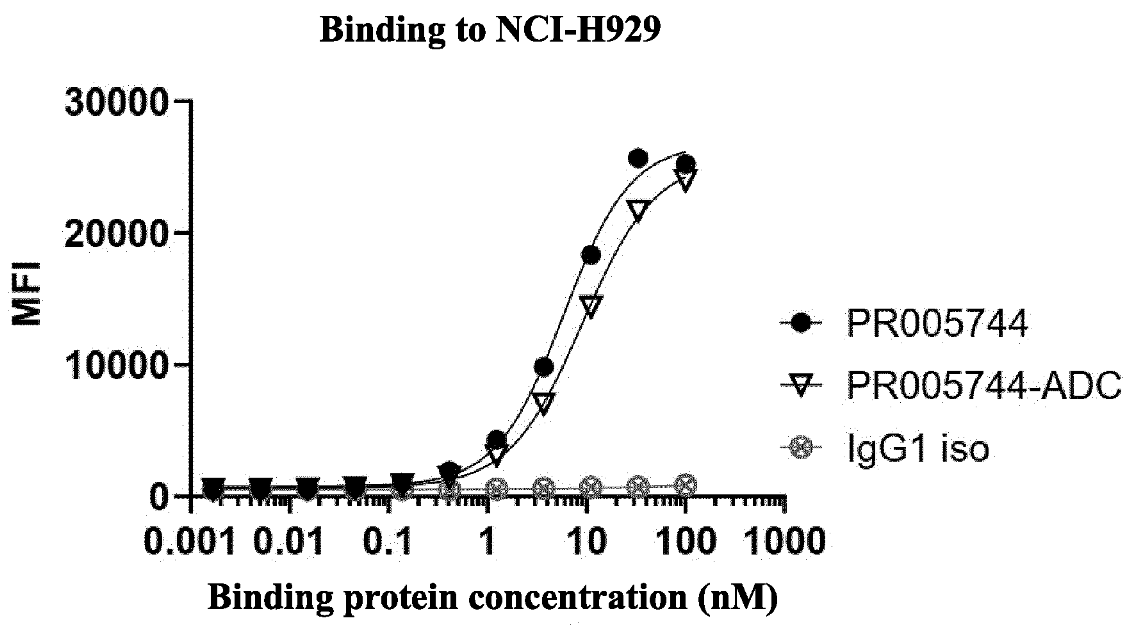


**FIG. 62**

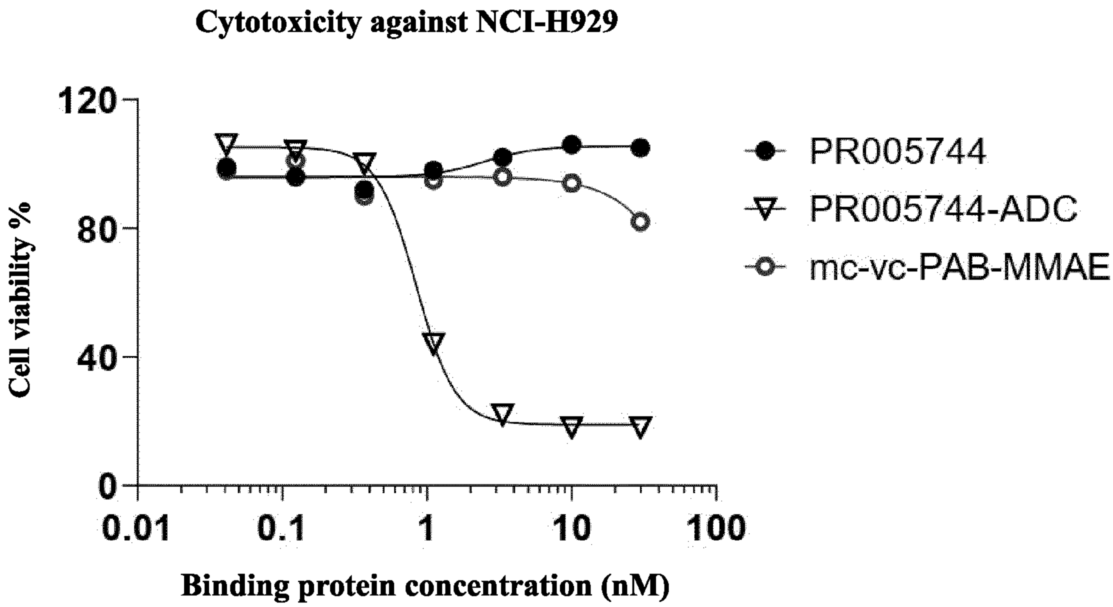


FIG. 63

$C_{62}H_{77}F_2N_9O_{12}S_4$

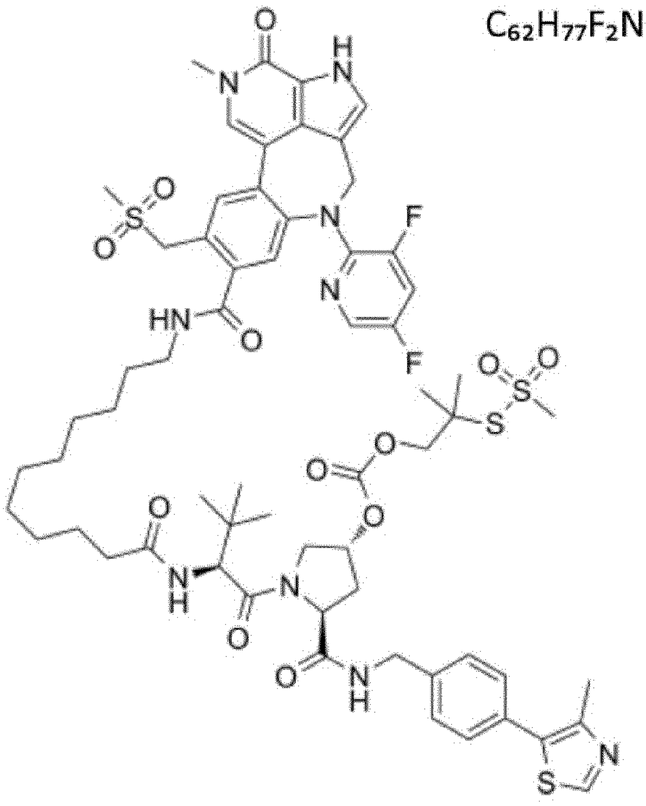

FIG. 64

MS map of PR004433-PROTAC (non-reduced sample)

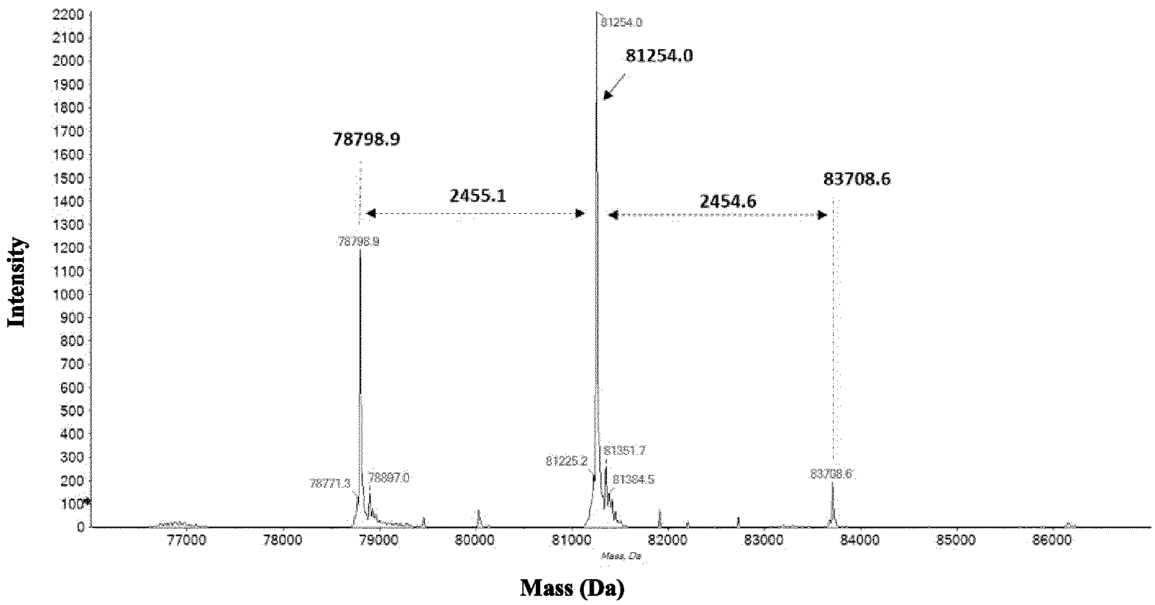


FIG. 65

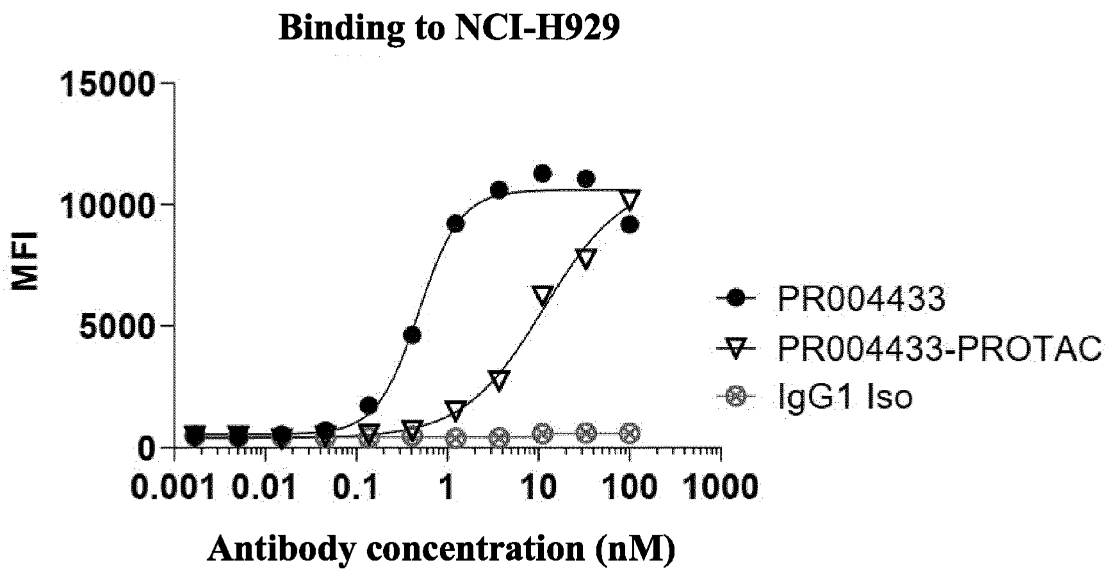


FIG. 66

# Structure of 2129/4433

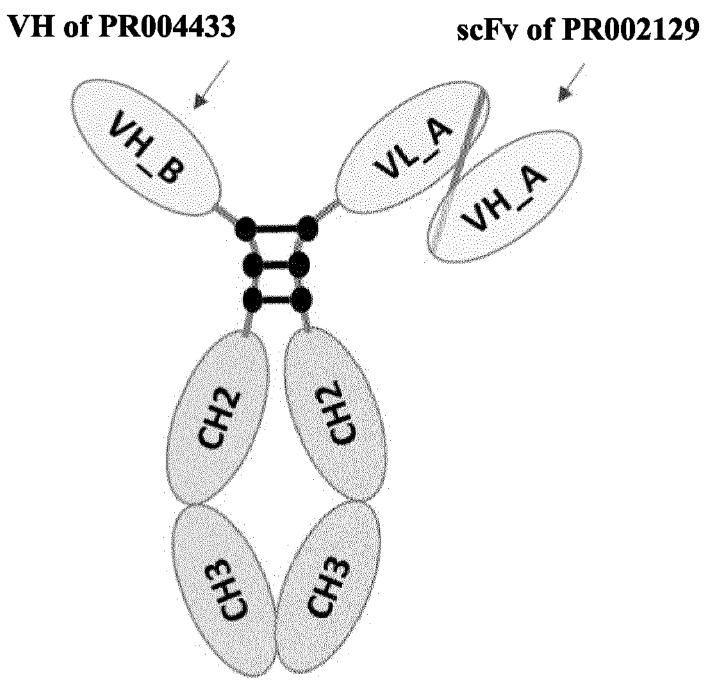


FIG. 67

(Lot.# CY20210214) before purification of 2129/4433-ADC, HIC-HPLC

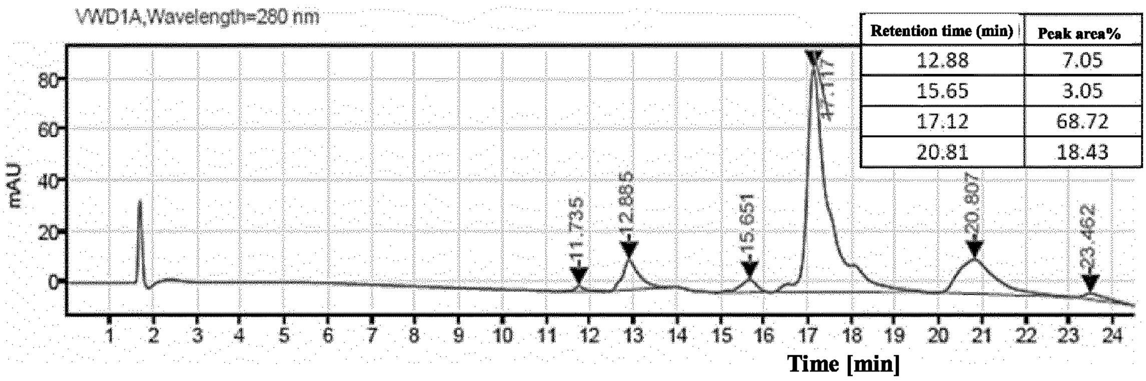


| Retention time (min) | Peak area% |
|---|---|
| 12.88 | 7.05 |
| 15.65 | 3.05 |
| 17.12 | 68.72 |
| 20.81 | 18.43 |

FIG. 68

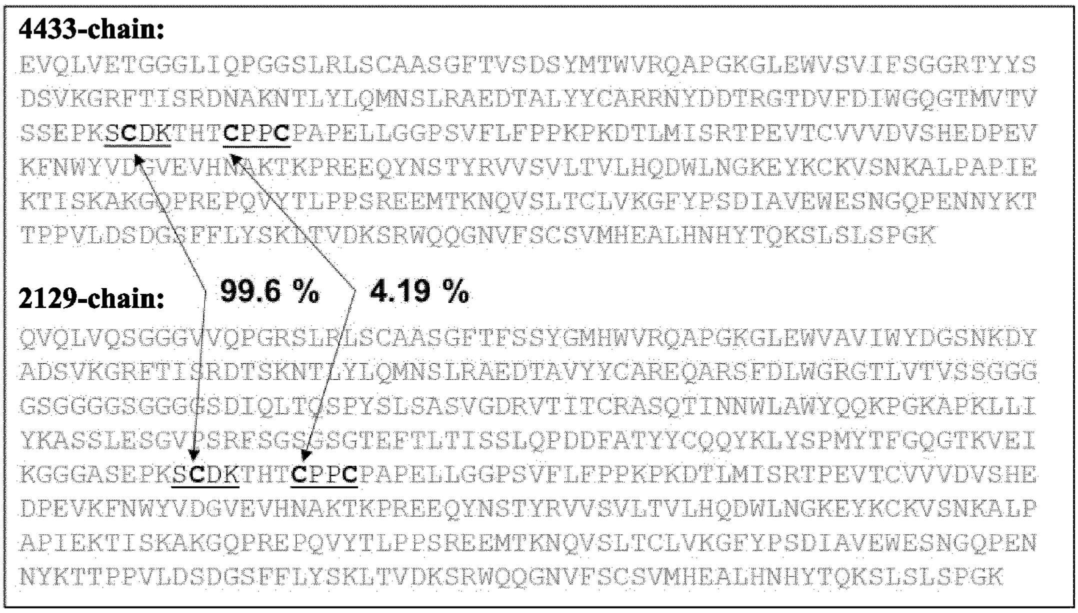


1. S**C**(+1315.78)DK
2. S**C**(+1315.78)DKTHTCPPCPAPELLGGPSVFLFPPK
3. THT**C**(+1315.78)PP**C**PAPELLGGPSVFLFPPK

| | Attribute Name | Value |
|---|---|---|
| 1 | MMAE@SCDK | 99.60 % |
| 2 | MMAE@CPPC | 4.19 % |

**FIG. 69**

**PR001046-ADC peptide fragment 'SC (+ 1315.78) DKTHTC (+ 57.02) PPC (+ 57.02) PAPELLGGPSVFLFPPKPK' secondary MS map**

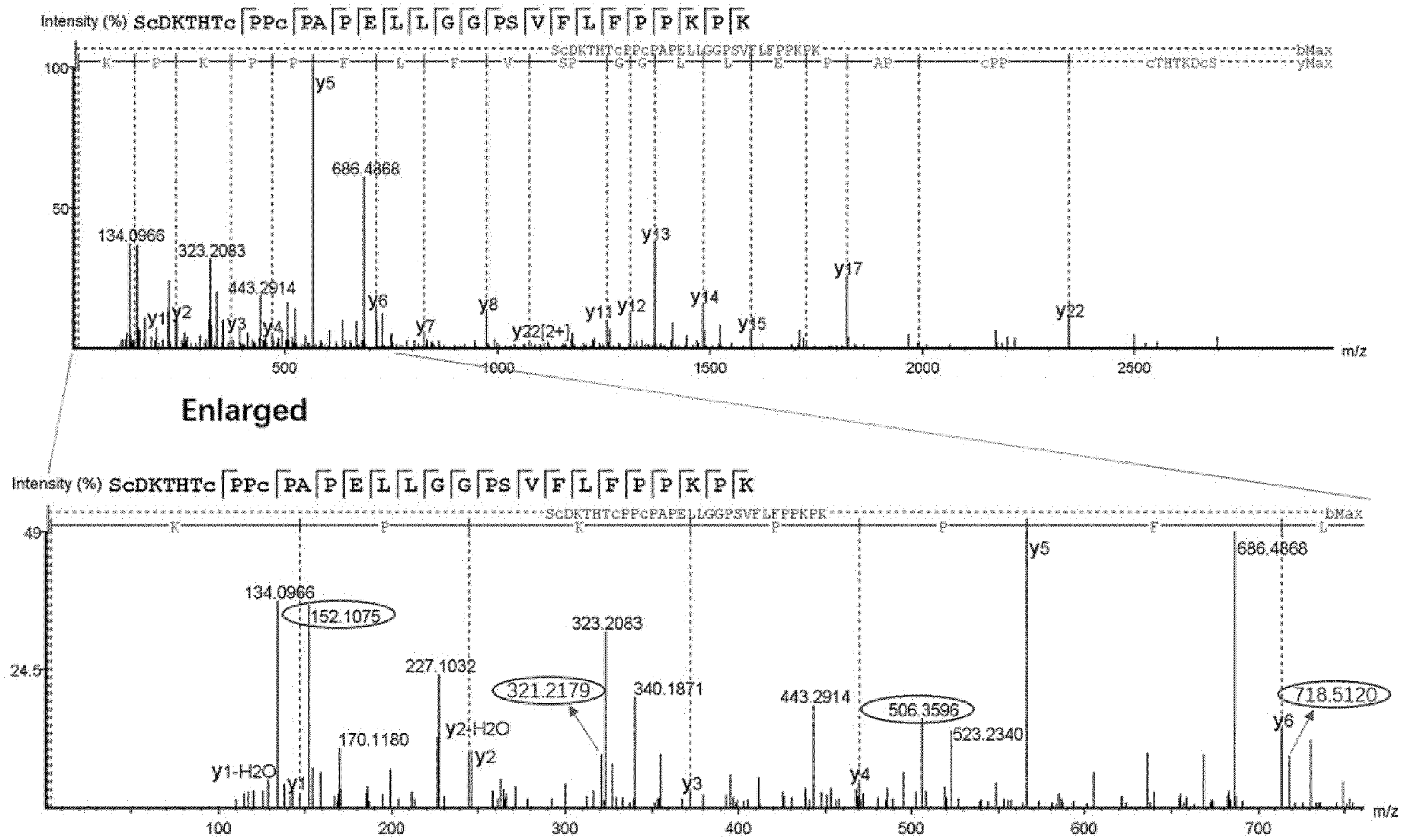


FIG. 70

PR006468-ADC peptide fragment 'VPITQNPC (+ 1315.78) PPLK' secondary MS map

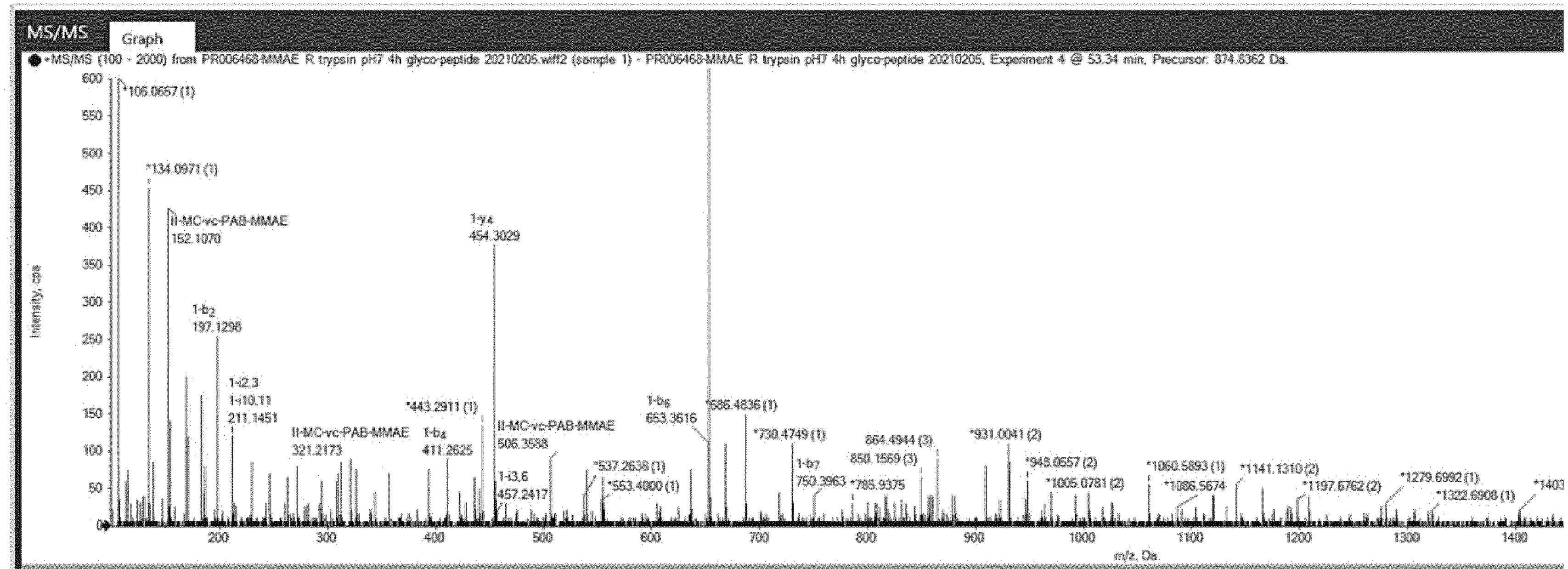


Show: Basic / Advanced (including internal fragments)

| Fragment | Modifications | Type | Neut... Loss | Theoretical Mono m/z | Observed Mono m/z | Error (Da) | Char... | Theoretical Mono Mass |
|---|---|---|---|---|---|---|---|---|
| PITQNPCPPL | MC-vc-PAB-MMAE@7 | i | | 1189.1656 | 1189.1620 | -0.0037 | 2 | 2376.3167 |
| PITQNPCPP | MC-vc-PAB-MMAE@7 | i | | 1132.6236 | 1132.6206 | -0.0030 | 2 | 2263.2326 |
| TQNPCPP | MC-vc-PAB-MMAE@5 | i | | 1027.5552 | 1027.5875 | 0.0323 | 2 | 2053.0958 |
| NPCPPL | MC-vc-PAB-MMAE@3 | i | | 969.5441 | 969.5653 | 0.0212 | 2 | 1937.0736 |
| PCP | MC-vc-PAB-MMAE@2 | i | | 1613.9012 | 1613.8776 | -0.0235 | 1 | 1612.8939 |
| PCP | MC-vc-PAB-MMAE@2 | i | | 807.4542 | 807.4384 | -0.0158 | 2 | 1612.8939 |
| CPP | MC-vc-PAB-MMAE@1 | i | | 1613.9012 | 1613.8776 | -0.0235 | 1 | 1612.8939 |
| CPP | MC-vc-PAB-MMAE@1 | i | | 807.4542 | 807.4384 | -0.0158 | 2 | 1612.8939 |
| VPITQNP | | b | | 750.4145 | 750.3963 | -0.0182 | 1 | 749.4072 |

FIG. 71

## 2129/4433-ADC peptide fragment 'SC (+ 1315.78) DK' secondary MS map

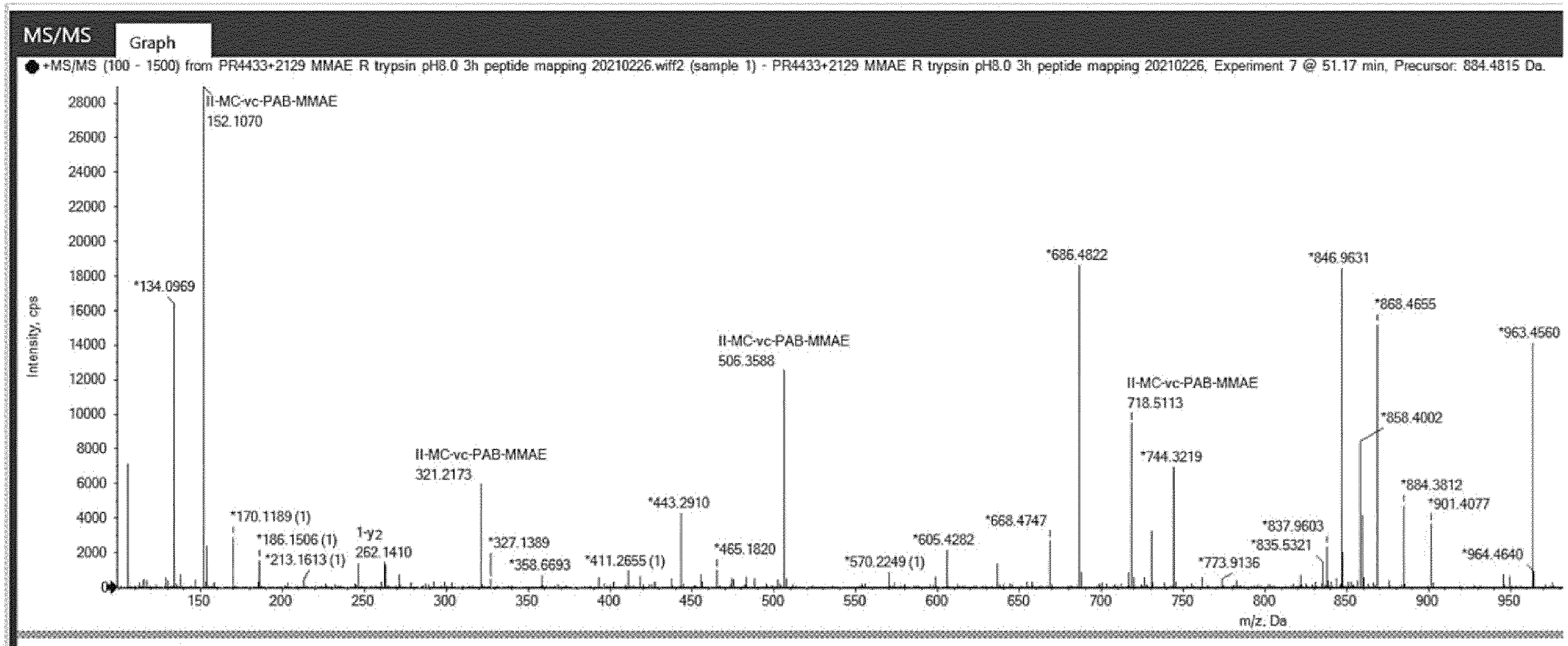


Show: Basic Advanced (including internal fragments)

| Fragment | Modifications | Type | Neut... Loss | Theoretical Mono m/z | Observed Mono m/z | Error (Da) | Char... | Theoretical Mono Mass |
|---|---|---|---|---|---|---|---|---|
| K | | y | | 147.1128 | 147.1144 | 0.0016 | 1 | 146.1055 |
| DK | | y | | 262.1397 | 262.1410 | 0.0012 | 1 | 261.1325 |
| SCDK | MC-vc-PAB-MMAE@2 | Precursor | | 884.4837 | 884.4807 | -0.0030 | 2 | 1766.9528 |

FIG. 72

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011423832
- WO 2010109165 A2
- WO 2017025038 A1
- US 10829771 B2
- CN 111234020 B


### Non-patent literature cited in the description


- **KABAT et al.** Sequences of Proteins of Immunological Interest. National Institutes of Health (U.S.), 1991
- *J Mol Biol,* 1997, vol. 273, 927-948
- **KUMARA et al.** *Bioorganic & Medicinal Chemistry Letters,* 2018, vol. 28, 3617-3621
- **W. R. PEARSON ; D. J. LIPMAN.** Improved Tools for Biological Sequence Comparison. *Proc. Natl. Acad. Sci. USA,* 1988, vol. 85, 2444-2448
- **D. J. LIPMAN ; W. R. PEARSON.** Rapid and Sensitive Protein Similarity Searches. *Science,* 1989, vol. 227, 1435-1441
- **S. ALTSCHUL ; W. GISH ; W. MILLER ; E. W. MYERS ; D. LIPMAN.** A Basic Local Alignment Search Tool. *Journal of Molecular Biology,* 1990, vol. 215, 403-410
- *CHEMICAL ABSTRACTS,* 646502-53-6
- **LIU, H. et al.** *mAbs,* 2012, vol. 4 (1), 17-23
- **KAUDER SE et al.** *PLoS ONE,* 2018, vol. 13 (8), e0201832
- **MANEIRO MA et al.** *ACS Chem Biol.,* 2020, vol. 15 (6), 1306-1312